# EUROPEAN PATENT APPLICATION

(11) **EP 4 501 923 A1**
(43) Date of publication of application: **05.02.2025**
(21) Application number: 23779620.6
(22) Date of filing: 15.03.2023
(51) Int. Cl.: C07D 401/14, A01N 43/58, A01P 7/04, A61K 31/501, A61P 33/14, C07D 403/04, C07D 405/14

(54) **PYRIDAZINONE COMPOUND OR SALT THEREOF AND PEST CONTROL AGENT CONTAINING SAME**

(30) Priority: 30.03.2022 JP 2022056258
(71) Applicant: Ishihara Sangyo Kaisha, Ltd., Osaka-shi, Osaka 550-0002 (JP)
(72) Inventor: KIRIYAMA, Kazuhisa, Osaka-shi, Osaka 550-0002 (JP); TAZAWA, Yuta, Osaka-shi, Osaka 550-0002 (JP); JUKUROGI, Tatsuya, Osaka-shi, Osaka 550-0002 (JP); KAWAKAMI, Shota, Osaka-shi, Osaka 550-0002 (JP); YOSHIMURA, Tadahiro, Osaka-shi, Osaka 550-0002 (JP); ISHIHARA, Katsuya, Osaka-shi, Osaka 550-0002 (JP); TORII, Ayaka, Osaka-shi, Osaka 550-0002 (JP); DOI, Ryusuke, Osaka-shi, Osaka 550-0002 (JP)
(74) Representative: Wächtershäuser & Hartz Patentanwaltspartnerschaft mbB
(86) International application number: PCT/JP2023/010103
(87) International publication number: WO 2023/189602

(57) **Abstract**

Many pest control agents have been used over a period of time, however, quite a few of them have various problems such that their effects are insufficient, and that pests have acquired resistance and their use is restricted. Accordingly, development of a novel pest control agent with few such defects has been desired.

A pyridazinone compound represented by the formula (I) or a salt thereof: formula (I): 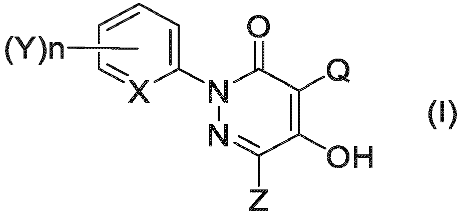 wherein the symbols are as defined in the specification, a pest control agent comprising the compound, and a method for controlling pests by applying the compound.

## Description

### TECHNICAL FIELD

The present invention relates to a novel pyridazinone compound or a salt thereof and a pest control agent containing it as an active ingredient.

### BACKGROUND ART

Patent Document 1 discloses 4-(heterocyclyl)pyridazinone compounds. The compounds have an alkyl, a cycloalkyl or the like at the 2-position of the pyridazinone ring, but do not have an aromatic ring at the 2-position as in compounds represented by the formula (I) described later. Thus, both the compounds are different in their chemical structures.

Patent Documents 2 and 3 disclose pyridazinone compounds. The compounds have an alkyl, a cycloalkyl, tetrahydropyranyl or the like at the 2-position of the pyridazinone ring, but do not have an aromatic ring at the 2-position as in compounds represented by the formula (I) described later. Thus, both the compounds are different in their chemical structures.

Patent Document 4 discloses pyridazinone compounds. The compounds have A-R³ (A includes NR⁴, and R⁴ is a C₁-C₆ alkyl or may form a 5-membered or 6-membered ring together with R³) at the 4-position of the pyridazinone ring, but do not have pyrazole or triazole at the 4-position as in compounds represented by the formula (I) described later. Thus, both the compounds are different in their chemical structures.

Patent Document 5 discloses antimicrobial agents containing a pyridazinone compound. Patent Document 6 discloses 4-position substituted pyridazinone compounds and c-Met inhibitors. Patent Document 7 discloses pyridazinone compounds and therapeutic drugs for proliferative diseases. Patent Document 8 discloses pyridazinone compounds and P2X7 receptor inhibitors.

However, Patent Document 5 to 8 do not specifically disclose compounds having an aromatic ring at the 2-position of the pyridazinone ring and having pyrazole or triazole at the 4-position of the pyridazinone ring, as compounds represented by the formula (I) described later. They do not disclose control of agricultural and horticultural pests and animal-parasitic organisms either.

### PRIOR ART DOCUMENTS

### PATENT DOCUMENTS

Patent Document 1: WO2011/045271
Patent Document 2: WO2009/086041
Patent Document 3: JP-A-2012-56903
Patent Document 4: JP-A-S63-185966
Patent Document 5: WO2015/143164
Patent Document 6: WO2008/103277
Patent Document 7: WO2008/080056
Patent Document 8: WO2010/126104

### DISCLOSURE OF INVENTION

### TECHNICAL PROBLEM

Many pest control agents have been used over a period of time, however, quite a few of them have various problems such that their effects are insufficient, and that pests have acquired resistance and their use is restricted. Accordingly, development of a novel pest control agent with few such defects has been desired. The object of the present invention is to provide a compound highly active against pests, to provide a pest control agent containing the compound, and a method for controlling pests by applying the compound.

### SOLUTION TO PROBLEM

To find more excellent pest control agents, the present inventors have conducted extensive studies on pyridazinone compounds and as a result, found that pyridazinone compounds represented by the following formula (I) and salts thereof, which are novel diarylpyridazinone compounds having an aromatic ring at the 2-position of the pyridazinone ring and having, at the 4-position of the pyridazinone ring, pyrazole or triazole bonded via its nitrogen atom, have controlling effects on pests, and have accomplished the present invention.

That is, the present invention relates to a pyridazinone compound represented by the formula (I) or a salt thereof: 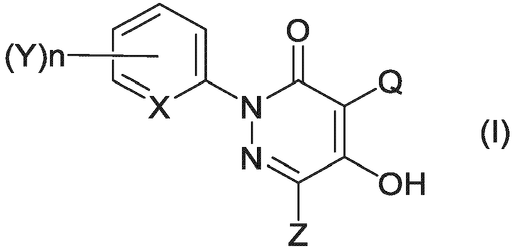 wherein Q is Q¹, Q², Q³ or Q⁴: 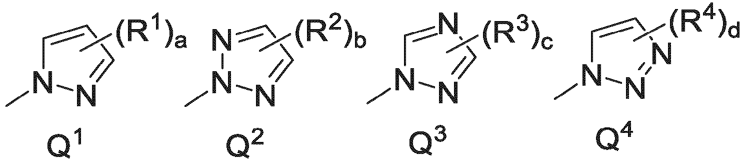
X is CH, C(R^{A}) or N;
Y and R^{A} are each a halogen, an alkyl, an alkenyl, an alkynyl, a cycloalkyl, a haloalkyl, an alkoxy, a haloalkoxy, an alkylthio, an alkylsulfinyl, an alkylsulfonyl, a haloalkylthio, a haloalkylsulfinyl, a haloalkylsulfonyl, hydroxy, cyano, nitro, -SFs, - NR^{B}R^{C}, -C(=O)R^{D} or -C(=O)NR^{E}R^{F};
Z is an alkyl, an alkenyl, an alkynyl, a cycloalkyl, an alkoxyalkyl, a cycloalkylalkyl or a haloalkyl;
R¹, R², R³ and R⁴ are each a halogen, an alkyl, an alkenyl, an alkynyl, a cycloalkyl, a haloalkyl, an alkoxy, a cycloalkoxy, a haloalkoxy, an alkylthio, an alkylsulfinyl, an alkylsulfonyl, a haloalkylthio, a haloalkylsulfinyl, a haloalkylsulfonyl, hydroxy, cyano, nitro, -NR^{G}R^{H}, -C(=O)R^{I}, -C(=O)NR^{J}R^{K}, -C(=NOR^{L})R^{M}, dimethoxymethyl, 1,3-dioxan-2-yl or 1,3-dioxolan-2-yl;
R^{B}, R^{C}, R^{E}, R^{F}, R^{G}, R^{H}, R^{J} and R^{K} are each a hydrogen atom, an alkyl or a haloalkyl;
R^{D} and R^{I} are each a hydrogen atom, hydroxy, an alkyl, a haloalkyl or an alkoxy;
R^{L} and R^{M} are each a hydrogen atom or an alkyl;
n is an integer of from 0 to 4;
a is an integer of from 0 to 3; and
b, c and d are each 0, 1 or 2.

The present invention further relates to a pest control agent comprising as an active ingredient the pyridazinone compound represented by the above formula (I) or a salt thereof, and a method for controlling pests, which comprises applying a pesticidally effective amount of the compound or a salt thereof to the pests or to a place where they grow.

### ADVANTAGEOUS EFFECTS OF INVENTION

The pyridazinone compound represented by the above formula (I) or a salt thereof has controlling effects on pests.

### DESCRIPTION OF EMBODIMENTS

In the above formula (I), the halogen atom or the halogen atom as a substituent may be an each atom of fluorine, chlorine, bromine or iodine. The number of the halogen atom as a substituent may be one or more, and in the case of two or more, the respective halogen atoms may be identical or different from each other. Further, the position of the halogen atom as a substituent may be any position.

In the above formula (I), the alkyl or the alkyl moiety may be a linear or branched (C₁-C₅) group having 1 to 5 carbon atoms such as methyl, ethyl, n-propyl, isopropyl, n-butyl, isobutyl, sec-butyl, tert-butyl, n-pentyl, isopentyl or neopentyl.

Particularly, in the above formula (I), the alkyl or the alkyl moiety in Z is preferably a linear or branched (C₁-C₄) group having 1 to 4 carbon atoms, more preferably methyl, ethyl, isopropyl or tert-butyl.

In the above formula (I), the alkoxy or the alkoxy moiety may be a linear or branched (C₁-C₄) group having 1 to 4 carbon atoms, such as methoxy, ethoxy, propoxy, isopropoxy, n-butoxy, isobutoxy, sec-butoxy or tert-butoxy.

In the above formula (I), the alkenyl may be a linear or branched (C₂-C₄) group having 2 to 4 carbon atoms, having at least one double bond at an optional position, such as vinyl, 1-propenyl, 2-propenyl, isopropenyl, 2-methyl-1-propenyl, 1-methyl-1-propenyl, 2-methyl-2-propenyl, 1-methyl-2-propenyl, 1-butenyl, 2-butenyl or 3-butenyl.

In the above formula (I), the alkynyl may be a linear or branched (C₂-C₄) group having 2 to 4 carbon atoms, having at least one triple bond at an optional position, such as ethynyl, 1-propynyl, 2-propynyl, 1-butynyl, 2-butynyl, 3-butynyl or 1-methyl-2-propynyl.

In the above formula (I), the cycloalkyl or the cycloalkyl moiety may be a (C₃-C₆) group having 3 to 6 carbon atoms, such as cyclopropyl, cyclobutyl, cyclopentyl or cyclohexyl.

Particularly in the above formula (I), the cycloalkyl or the cycloalkyl moiety in Z is preferably a (C₃-C₆) group having 3 to 6 carbon atoms, more preferably a (C₃-C₄) group having 3 to 4 carbon atoms, such as cyclopropyl or cyclobutyl.

In the above formula (I), the cycloalkoxy may be a (C₃-C₆) group having 3 to 6 carbon atoms, such as cyclopropyloxy, cyclobutyloxy, cyclopentyloxy or cyclohexyloxy.

In the above formula (I), when n is an integer of 2 or more, the respective substituents Y may be identical or different from each other. Likewise, when a is an integer of 2 or more, the respective substituents R' may be identical or different from each other. Likewise, when each of b, c and d is 2 , the respective substituents R², R³ and R⁴ may be identical or different from each other.

In the above formula (I), since Q is represented by Q¹ to Q⁴, the compound of the formula (I) is specifically represented by the following formulae (i) to (iv).

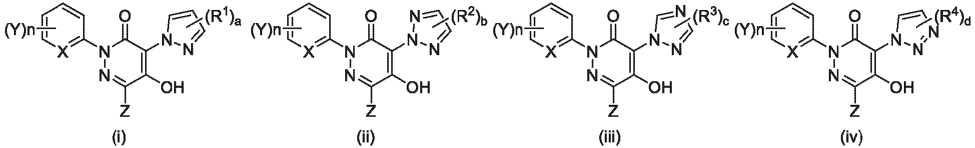

The compound of the formula (i) includes compounds of the following formulae (i-a) to (i-h) depending upon the number and the position of the substituent in Q¹.

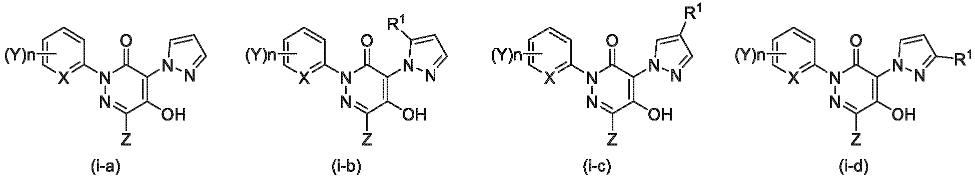

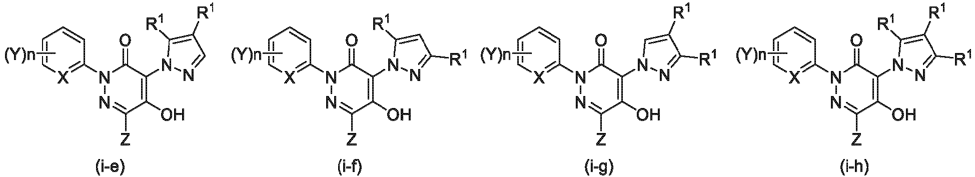

Likewise, the compound of the formula (ii) includes compounds of the following formulae (ii-a) to (ii-c) depending upon the number and the position of the substituent in Q².

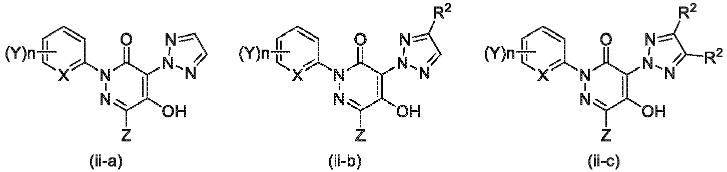

Likewise, the compound of the formula (iii) includes compounds of the following formulae (iii-a) to (iii-d) depending upon the number and the position of the substituent in Q³.

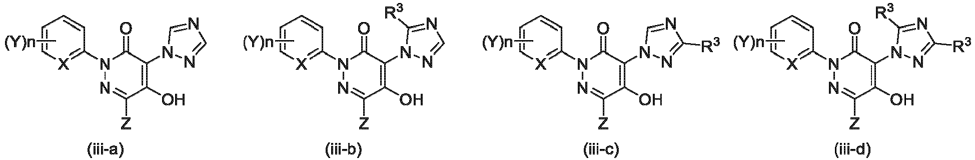

Likewise, the compound of the formula (iv) includes compounds of the following formulae (iv-a) to (iv-d) depending upon the number and the position of the substituent in Q⁴.

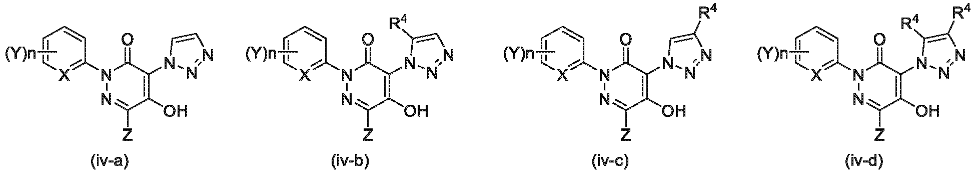

The symbols in the compounds of the formulae (i) to (iv), (i-a) to (i-h), (ii-a) to (ii-c), (iii-a) to (iii-d) and (iv-a) to (iv-d) are as defined for the formula (I). Further, description regarding the compound of the formula (I) in this specification includes descriptions regarding the compounds of the above formulae included in the formula (I).

As the compound represented by the formula (i), preferred are compounds represented by the formulae (i-a), (i-c), (i-d) and (i-g), more preferred are compounds represented by the formulae (i-c), (i-d) and (i-g). As the compound represented by the formulae (ii) to (iv), preferred are compounds represented by the formulae (ii-a), (ii-b), (ii-c), (iii-a), (iii-c), (iv-a) and (iv-c), more preferred are compounds represented by the formulae (ii-b), (ii-c), (iii-c) and (iv-c). In the formula (I), Q is preferably Q¹ or Q³, that is, the compounds represented by the formulae (i) and (iii) are preferred. Further, as the compounds represented by the formulae (i) and (iii), preferred are compounds represented by the formulae (i-a), (i-c), (i-d), (i-g), (iii-a) and (iii-c), more preferred are compounds represented by the formulae (i-c), (i-d), (i-g) and (iii-c).

The salts of the compound of the formula (I) include all kinds of salts so long as they are acceptable in this technical field, and, for example, alkali metal salts such as a sodium salt and a potassium salt; alkaline earth metal salts such as a magnesium salt and a calcium salt; quaternary alkylammonium salts such as a tetrabutylammonium salt; amine salts such as a dimethylamine salt and a triethylamine salt; inorganic acid salts such as a hydrochloride, a perchlorate, a sulfate and a nitrate; and organic acid salts such as an acetate and a methanesulfonate may be mentioned.

As the compound of the formula (I) or a salt thereof, isomers such as diastereoisomers and optical isomers may sometimes be present, and the present invention includes the respective isomers and isomer mixtures. In this specification, isomers are described as a mixture, unless otherwise specified. Various isomers other than the above-mentioned isomers are included in the present invention within a range of common knowledge in this technical field.

Further, depending upon the type of an isomer, it may have a chemical structure different from the above formula (I), but for those skilled in the art, it is sufficiently recognizable that such a chemical structure is in a relation of an isomer with the formula (I), and such an isomer is evidently within the scope of the present invention. For example, the compound of the formula (I) or a salt thereof includes the following tautomers.

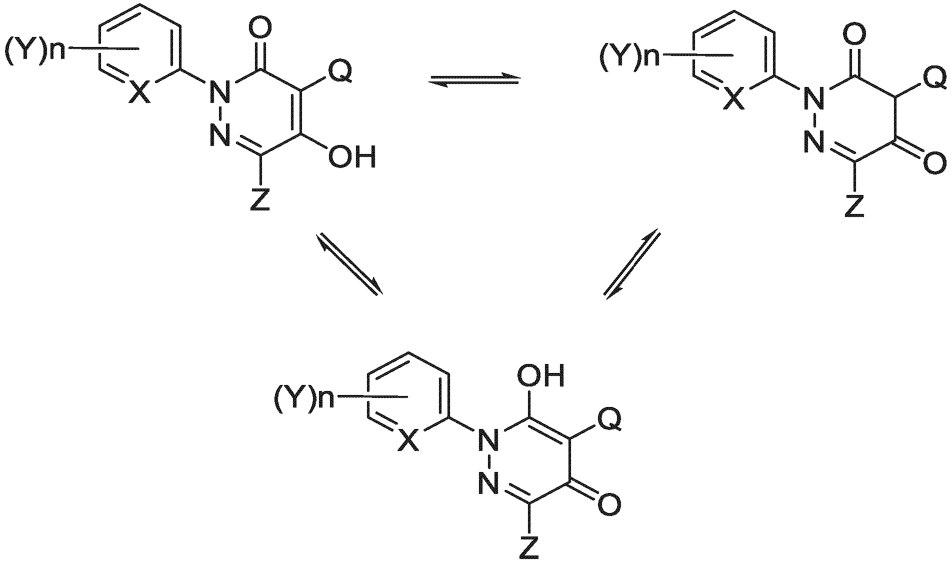

The compound of the formula (I) or a salt thereof (hereinafter sometimes referred to as the compound of the present invention) may be produced in accordance with the following Production Process or a conventional method for producing a salt, however, its production is not limited to such a method. For example, the compound (I) of the present invention may be produced by applying a substituent transformation (such as alkylation, haloalkylation, cross coupling reaction such as Suzuki coupling, Sandmeyer reaction, halogenation, oxidation or reduction) known in this technical field, on the substituent on the pyridazinone ring and the pyrazolyl group, the triazolyl group, the phenyl group and the pyridyl group. Further, as the case requires, optional functional groups in the staring material and in the intermediate may be protected by protecting groups known in this technical field. Such protecting groups can be removed at appropriate stages in the reaction scheme by a method known in this technical field. In the following Production Processes, geometrical isomers derived from a carbon-nitrogen double bond may sometimes be present in the chemical structural formulae, and the following description includes the respective geometrical isomers and a mixture of them in an optional ratio. For example, in the chemical structural formula, a wave line indicates both geometrical isomers derived from a carbon-nitrogen double bond are included. Further, the reaction may be carried out under an atmosphere of an inert gas such as nitrogen or argon as the case requires.

### Production Process [1]

A compound of the formula (I-a) which is the compound of the formula (I) wherein X is N may be produced by continuously carrying out step 1 of reacting a compound of the formula (II) with a compound of the formula (III) to obtain a compound of the formula (IV), and step 2 of reacting the compound of the formula (IV) in the presence of a base.

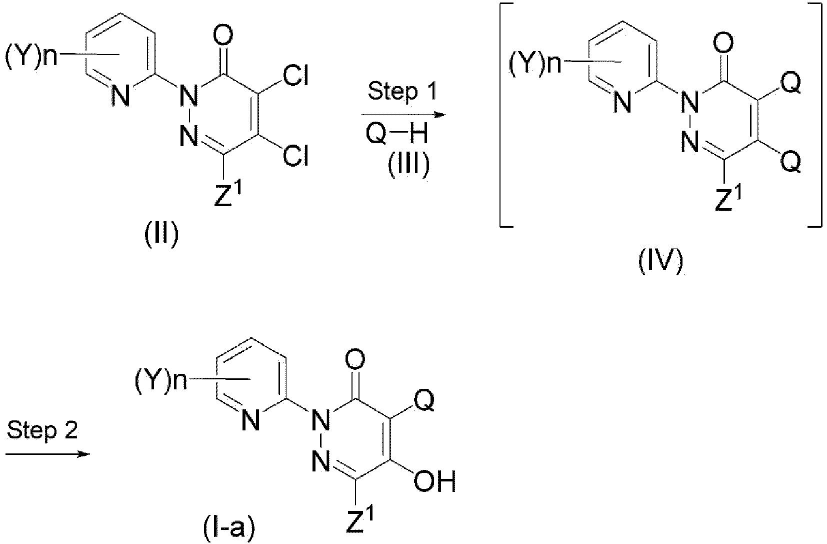

In the formulae, Z¹ is an alkyl, and the other symbols are as defined above.

In step 1 of Production Process [1], the compound of the formula (II) and the compound of the formula (III) are reacted in the presence of a base to produce the compound of the formula (IV). The compound of the formula (III) may be commercially available or may be produced in accordance with a known method.

The base may, for example, be an alkali metal hydride such as sodium hydride, an alkali metal carbonate such as sodium carbonate, potassium carbonate or cesium carbonate, or an alkali metal hydroxide such as lithium hydroxide, sodium hydroxide or potassium hydroxide. The base may be used in an amount of from 0.5 to 10 equivalents to 1 equivalent of the compound of the formula (II).

The reaction of step 1 in Production Process [1] may be carried out optionally in the presence of a solvent. The solvent may be any solvent and is not particularly limited so long as it is inert to the reaction, and one or more may properly be selected or mixed from ethers such as diethyl ether, butyl methyl ether, tetrahydrofuran, ethylene glycol dimethyl ether, 1,4-dioxane and dimethoxyethane; aliphatic halogenated hydrocarbons such as methylene chloride, dichloroethane and chloroform; aromatic hydrocarbons such as benzene, toluene, xylene and chlorobenzene; aprotic polar solvents such as N,N-dimethylformamide, N,N-dimethylacetamide, N-methyl-2-pyrrolidone, dimethyl sulfoxide and sulfolane; and aliphatic hydrocarbons such as pentane, hexane, heptane, octane and cyclohexane.

In step 1 of Production Process [1], the reaction temperature is about -20°C to 150°C. The reaction time is about 0.5 hour to 24 hours.

In step 2 of Production Process [1], the compound of the formula (IV) obtained in step 1 and a base are reacted to produce the compound of the formula (I-a).

As the base, the bases described for the above step 1 may be mentioned.

The reaction of step 2 in Production Process [1] may be carried out optionally in the presence of a solvent. The solvent may be any solvent and is not particularly limited so long as it is inert to the reaction, and one or more may properly be selected or mixed from ethers such as diethyl ether, butyl methyl ether, tetrahydrofuran, ethylene glycol dimethyl ether, 1,4-dioxane and dimethoxyethane; aliphatic halogenated hydrocarbons such as methylene chloride, dichloroethane and chloroform; aromatic hydrocarbons such as benzene, toluene, xylene and chlorobenzene; aprotic polar solvents such as N,N-dimethylformamide, N,N-dimethylacetamide, N-methyl-2-pyrrolidone, dimethyl sulfoxide and sulfolane; aliphatic hydrocarbons such as pentane, hexane, heptane, octane and cyclohexane; and water.

In step 2 of Production Process [1], the reaction temperature is about -20°C to 150°C. The reaction time is about 0.5 hour to 24 hours.

### Intermediate Production Process [1-1]

The compound of the formula (II) may be produced by reacting a compound of the formula (V) with a compound of the formula (VI) in the presence of a base. The compound of the formula (VI) may be commercially available or may be produced in accordance with a known method.

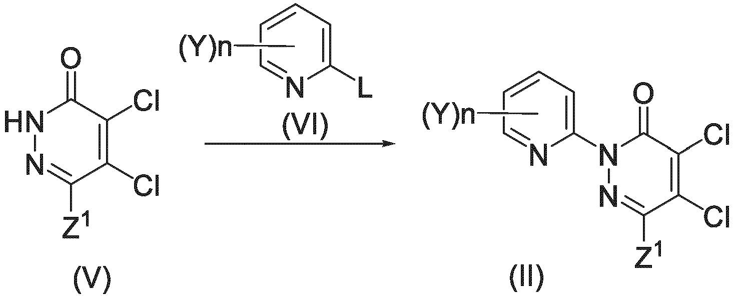

In the formulae, L is a halogen atom, and the other symbols are as defined above.

The base may, for example, be an alkali metal hydride such as sodium hydride; an alkali metal carbonate such as sodium carbonate, potassium carbonate or cesium carbonate; an alkali metal hydroxide such as lithium hydroxide, sodium hydroxide or potassium hydroxide; or a tertiary amine such as triethylamine, 4-methylmorpholine or diisopropylethylamine. The base may be used in an amount of from 0.5 to 10 equivalents to 1 equivalent of the compound of the formula (V).

The reaction of Intermediate Production Process [1-1] may be carried out optionally in the presence of a solvent. The solvent may be any solvent and is not particularly limited so long as it is inert to the reaction, and one or more may properly be selected or mixed from the solvents as described for step 1 of Production Process [1].

In Intermediate Production Process [1-1], the reaction temperature is about 0°C to 150°C. The reaction time is about 0.5 hour to 24 hours.

### Intermediate Production Process [1-2]

The compound of the formula (V) may be produced in accordance with a known literature (for example, Protective Groups in Organic Synthesis Fourth Edition, John Wiley & Sons, Inc). For example, the compound of the formula (V) may be produced by reacting a compound of the formula (VII) in the presence of an acid.

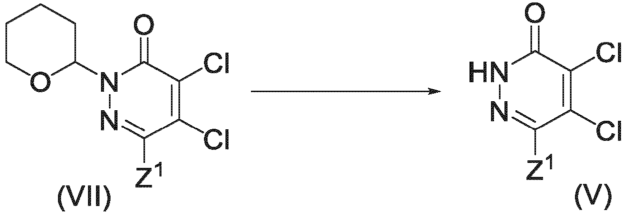

The symbols in the formulae are as defined above.

The acid may, for example, be an inorganic acid such as hydrochloric acid; or an organic acid such as acetic acid, formic acid or trifluoroacetic acid.

The reaction in Intermediate Production Process [1-2] may be carried out optionally in the presence of a solvent. The solvent may be any solvent and is not particularly limited so long as it is inert to the reaction, and one or more may properly be selected or mixed from ethers such as diethyl ether, butyl methyl ether, tetrahydrofuran, ethylene glycol dimethyl ether, 1,4-dioxane and dimethoxyethane; aliphatic halogenated hydrocarbons such as methylene chloride, dichloroethane and chloroform; aromatic hydrocarbons such as benzene, toluene, xylene and chlorobenzene; aprotic polar solvents such as N,N-dimethylformamide, N,N-dimethylacetamide, N-methyl-2-pyrrolidone, dimethyl sulfoxide and sulfolane; nitriles such as acetonitrile and propionitrile; esters such as ethyl acetate and ethyl propionate; aliphatic hydrocarbons such as pentane, hexane, heptane, octane and cyclohexane; and ketones such as acetone and ethyl methyl ketone.

In Intermediate Production Process [1-2], the reaction temperature is about 0°C to 150°C. The reaction time is about 0.5 hour to 24 hours.

### Intermediate Production Process [1-3]

The compound of the formula (VII) may be produced by reacting a compound of the formula (VIII) with a compound of the formula (IX) in the presence of a metal catalyst and a base. The compound of the formula (IX) may be commercially available or may be produced in accordance with a known method.

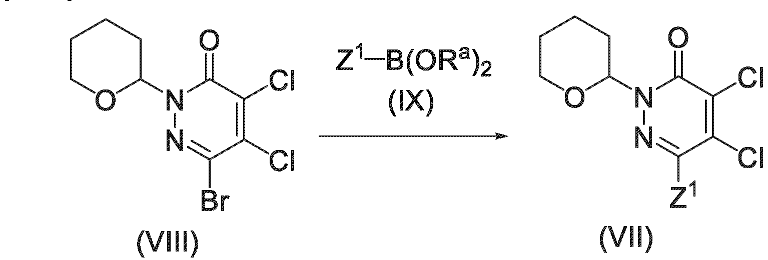

In the formulae, the two R^{a} are each a hydrogen atom or a (C₁-C₆)alkyl and the respective (C₁-C₆)alkyl may be identical or different from each other, or the two R^{a} together may form -CH₂CH₂- or -C(CH₃)₂C(CH₃)₂-, and the other symbols are as defined above.

The metal catalyst may be a conventional catalyst used for a cross coupling reaction. It may, for example, be a palladium catalyst such as palladium on carbon, palladium chloride, palladium acetate, tetrakis(triphenylphosphine)palladium, bis(triphenylphosphine)palladium dichloride, or [1,1'-bis(diphenylphosphino)ferrocene]palladium dichloride dichloromethane adduct; or a copper catalyst such as metal copper, copper(I) acetate, copper(II) acetate, copper(I) oxide, copper(II) oxide or copper iodide.

As the base, one or more may properly be selected and mixed from tertiary amines such as triethylamine and diisopropylethylamine, an alkali metal carbonates such as sodium carbonate, potassium carbonate and cesium carbonate; alkaline earth metal carbonates such as calcium carbonate and barium carbonate; alkali metal hydroxides such as lithium hydroxide, sodium hydroxide and potassium hydroxide; alkaline earth metal hydroxides such as calcium hydroxide and barium hydroxide; alkali metal phosphates such as sodium phosphate and potassium phosphate; and alkaline earth metal phosphates such as calcium phosphate. The base may be used in an amount of from 0.5 to 10 equivalents to 1 equivalent of the compound of the formula (VIII).

The reaction in Intermediate Production Process [1-3] may be carried out optionally in the presence of a solvent. The solvent may be any solvent and is not particularly limited so long as it is inert to the reaction, and one or more may properly be selected or mixed from ethers such as diethyl ether, butyl methyl ether, tetrahydrofuran, ethylene glycol dimethyl ether, 1,4-dioxane and dimethoxyethane; aliphatic halogenated hydrocarbons such as methylene chloride, dichloroethane and chloroform; aromatic hydrocarbons such as benzene, toluene, xylene and chlorobenzene; aprotic polar solvents such as N,N-dimethylformamide, N,N-dimethylacetamide, N-methyl-2-pyrrolidone, dimethyl sulfoxide and sulfolane; nitriles such as acetonitrile and propionitrile; esters such as ethyl acetate and ethyl propionate; aliphatic hydrocarbons such as pentane, hexane, heptane, octane and cyclohexane; ketones such as acetone and ethyl methyl ketone; alcohols such as methanol, ethanol and isopropyl alcohol; and water.

In Intermediate Production Process [1-3], the reaction temperature is about 20°C to 150°C. The reaction time is about 0.5 hour to 24 hours.

### Intermediate Production Process [1-4]

The compound of the formula (VIII) may be produced in accordance with a known literature (for example, Protective Groups in Organic Synthesis Fourth Edition, John Wiley & Sons, Inc). For example, the compound of the formula (VIII) may be produced by reacting a compound of the formula (X) with 3,4-dihydro-2H-pyran in the presence of an acid. The compound of the formula (X) may be commercially available or may be produced in accordance with a known method.

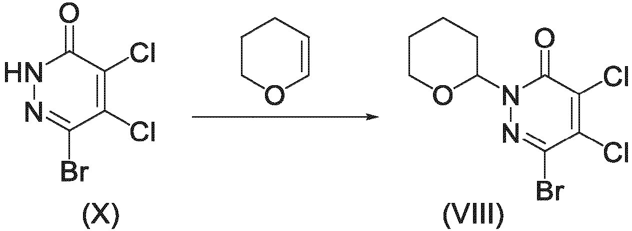

The acid may be an organic acid such as methanesulfonic acid, trifluoroacetic acid, p-toluenesulfonic acid or pyridinium p-toluenesulfonate.

The reaction in Intermediate Production Process [1-4] may be carried out optionally in the presence of a solvent. The solvent may be any solvent and is not particularly limited so long as it is inert to the reaction, and one or more may properly be selected or mixed from ethers such as diethyl ether, butyl methyl ether, tetrahydrofuran, ethylene glycol dimethyl ether, 1,4-dioxane and dimethoxyethane; aliphatic halogenated hydrocarbons such as methylene chloride, dichloroethane and chloroform; aromatic hydrocarbons such as benzene, toluene, xylene and chlorobenzene; aprotic polar solvents such as N,N-dimethylformamide, N,N-dimethylacetamide, N-methyl-2-pyrrolidone, dimethyl sulfoxide and sulfolane; nitriles such as acetonitrile and propionitrile; esters such as ethyl acetate and ethyl propionate; and aliphatic hydrocarbons such as pentane, hexane, heptane, octane and cyclohexane.

In Intermediate Production Process [1-4], the reaction temperature is about 10°C to 150°C. The reaction time is about 0.5 hour to 24 hours.

### Production Process [2]

The compound of the formula (I) may be produced by reacting a compound of the formula (XI) in the presence of a base.

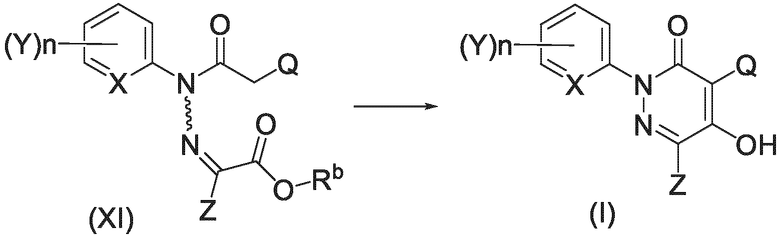

In the formulae, R^{b} is a (C₁₋₆)alkyl, and the other symbols are as defined above.

The base may, for example, be an alkali metal carbonate such as sodium carbonate, potassium carbonate or cesium carbonate; an alkaline earth metal carbonate such as calcium carbonate or barium carbonate; an alkali metal hydroxide such as lithium hydroxide, sodium hydroxide or potassium hydroxide; an alkaline earth metal hydroxide such as calcium hydroxide or barium hydroxide; an alkali metal hydride such as sodium hydride; an alkyllithium such as n-butyllithium or tert-butyllithium; a metal amide such as lithium diisopropylamide, lithium hexamethyldisilazide or potassium hexamethyldisilazide; a metal alkoxide such as sodium methoxide or potassium tert-butoxide; a tertiary amine such as triethylamine, 4-methylmorpholine or diisopropylethylamine; 1,8-diazabicyclo[5.4.0]-7-undecene, 1,4-diazabicyclo[2.2.2]octane, pyridine, 4-(dimethylamino)pyridine or 2,6-lutidine. The base may be used in an amount of from 0.5 to 10 equivalents to 1 equivalent of the compound of the formula (XI).

The reaction in Production Process [2] may be carried out optionally in the presence of a solvent. The solvent may be any solvent and is not particularly limited so long as it is inert to the reaction, and one or more may properly be selected or mixed from the solvents described for Intermediate Production Process [1-2].

In Production Process [2], the reaction temperature is about -20°C to 150°C. The reaction time is about 0.5 hour to 24 hours.

### Intermediate Production Process [2-1]

The compound of the formula (XI) may be produced by reacting a compound of the formula (XII) with a compound of the formula (XIII) optionally in the presence of an acid. The compound of the formula (XIII) may be commercially available or may be produced in accordance with a known method, for example, may be produced in accordance with a known literature (for example, US patent application US2004/087569 A1 (e.g. [0092] at page 6, [0093] at page 7).

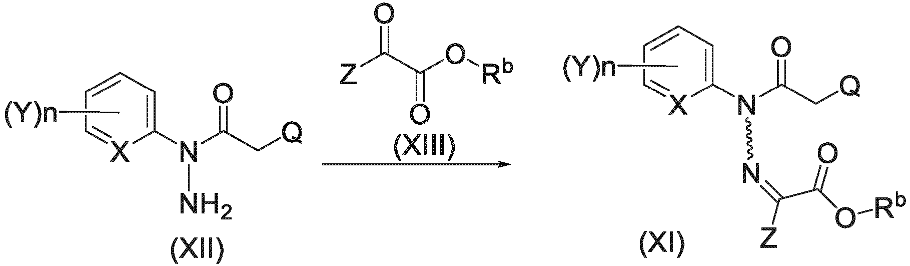

The symbols in the formulae are as defined above.

The acid may, for example, be an inorganic acid such as hydrochloric acid or sulfuric acid; or an organic acid such as acetic acid, formic acid, methanesulfonic acid, trifluoroacetic acid or p-toluenesulfonic acid.

The reaction in Intermediate Production Process [2-1] may be carried out optionally in the presence of a solvent. The solvent may be any solvent and is not particularly limited so long as it is inert to the reaction, and one or more may properly be selected or mixed from ethers such as diethyl ether, butyl methyl ether, tetrahydrofuran, ethylene glycol dimethyl ether, 1,4-dioxane and dimethoxyethane; aliphatic halogenated hydrocarbons such as methylene chloride, dichloroethane and chloroform; aromatic hydrocarbons such as benzene, toluene, xylene and chlorobenzene; aprotic polar solvents such as N,N-dimethylformamide, N,N-dimethylacetamide, N-methyl-2-pyrrolidone, dimethyl sulfoxide and sulfolane; nitriles such as acetonitrile and propionitrile; esters such as ethyl acetate and ethyl propionate; aliphatic hydrocarbons such as pentane, hexane, heptane, octane and cyclohexane; and alcohols such as methanol, ethanol and isopropyl alcohol.

In Intermediate Production Process [2-1], the reaction temperature is about -20°C to 150°C. The reaction time is about 0.5 hour to 24 hours.

### Intermediate Production Process [2-2]

The compound of the formula (XII) may be produced in accordance with a known literature (for example, Protective Groups in Organic Synthesis Fourth Edition, John Wiley & Sons, Inc). For example, the compound of the formula (XII) may be produced by reacting a compound of the formula (XIV) in the presence of an acid.

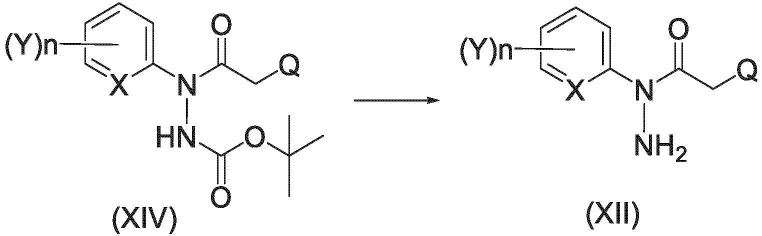

The symbols in the formulae are as defined above.

The acid may be an inorganic acid such as hydrochloric acid; or an organic acid such as trifluoroacetic acid.

The reaction in Intermediate Production Process [2-2] may be carried out optionally in the presence of a solvent. The solvent may be any solvent and is not particularly limited so long as it is inert to the reaction, and one or more may properly be selected or mixed from ethers such as diethyl ether, butyl methyl ether, tetrahydrofuran, ethylene glycol dimethyl ether, 1,4-dioxane and dimethoxyethane; aliphatic halogenated hydrocarbons such as methylene chloride, dichloroethane and chloroform; aromatic hydrocarbons such as benzene, toluene, xylene and chlorobenzene; esters such as ethyl acetate and ethyl propionate; and aliphatic hydrocarbons such as pentane, hexane, heptane, octane and cyclohexane.

In Intermediate Production Process [2-2], the reaction temperature is about 0°C to 150°C. The reaction time is about 0.5 hour to 24 hours.

### Intermediate Production Process [2-3]

The compound of the formula (XIV) may be produced by reacting a compound of the formula (XV) with a compound of the formula (XVI) in the presence of a base. The compound of the formula (XVI) may be commercially available or may be produced by the method in Intermediate Production Process [2-6].

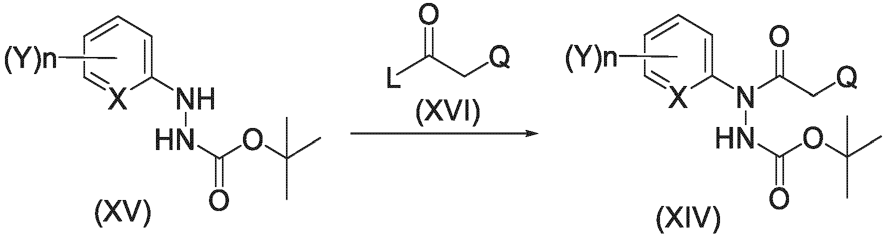

The symbols in the formulae are as defined above.

As the base, the bases described for Production Process [2] may be mentioned. The base may be used in an amount of from 0.5 to 10 equivalents to 1 equivalent of the compound of the formula (XV).

The reaction in Intermediate Production Process [2-3] may be carried out optionally in the presence of a solvent. The solvent may be any solvent and is not particularly limited so long as it is inert to the reaction, and one or more may properly be selected or mixed from the solvents described for Intermediate Production Process [1-2].

In Intermediate Production Process [2-3], the reaction temperature is about -20°C to 150°C. The reaction time is about 0.5 hour to 24 hours.

### Intermediate Production Process [2-4]

The compound of the formula (XV) may be produced in accordance with a known literature (for example, Protective Groups in Organic Synthesis Fourth Edition, John Wiley & Sons, Inc). For example, the compound of the formula (XV) may be produced by reacting a compound of the formula (XVII) with a protecting reagent such as di-tert-butyl dicarbonate ((Boc)₂O) optionally in the presence of a base.

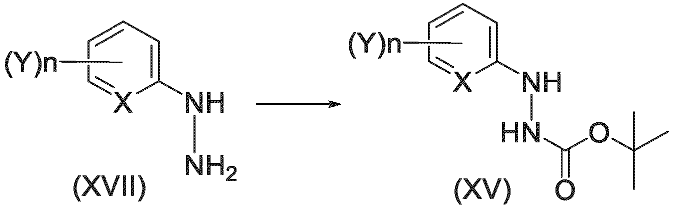

The symbols in the formulae are as defined above.

The reaction in Intermediate Production Process [2-4] may be carried out optionally in the presence of a base. The base may, for example, be an alkali metal carbonate such as sodium carbonate, potassium carbonate or cesium carbonate; an alkaline earth metal carbonate such as calcium carbonate or barium carbonate; an alkali metal hydride such as sodium hydride; a tertiary amine such as triethylamine, 4-methylmorpholine or diisopropylethylamine; 1,8-diazabicyclo[5.4.0]-7-undecene or 4-(dimethylamino)pyridine.

The reaction in Intermediate Production Process [2-4] may be carried out optionally in the presence of a solvent. The solvent may be any solvent and is not particularly limited so long as it is inert to the reaction, and one or more may properly be selected or mixed from ethers such as diethyl ether, butyl methyl ether, tetrahydrofuran, ethylene glycol dimethyl ether, 1,4-dioxane and dimethoxyethane; aliphatic halogenated hydrocarbons such as methylene chloride, dichloroethane and chloroform; aprotic polar solvents such as N,N-dimethylformamide, N,N-dimethylacetamide, N-methyl-2-pyrrolidone, dimethyl sulfoxide and sulfolane; esters such as ethyl acetate and ethyl propionate; and water.

In Intermediate Production Process [2-4], the reaction temperature is about 0°C to 150°C. The reaction time is about 0.5 hour to 24 hours.

### Intermediate Production Process [2-5]

The compound of the formula (XVII) may be commercially available, or may be produced by reacting a compound of the formula (XVIII) with a nitrite such as sodium nitrite in an aqueous solution containing an acid to prepare a corresponding diazonium salt of the formula (XIX), and reacting the prepared diazonium salt with a reducing agent in an aqueous solution containing an acid. The compound of the formula (XVIII) may be commercially available or may be produced in accordance with a known method, and for example, may be produced in accordance with a known literature (for example, WO2021/097057 (page 348, Step 1 to Step 3 of Intermediate A-7)).

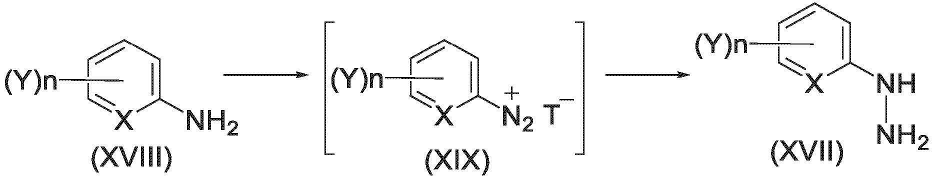

In the formulae, T⁻ is a chloride ion, a sulfate ion, an acetate ion or a trifluoracetate anion, and the other symbols are as defined above.

As the acid, one or more may properly be selected or mixed from inorganic acids such as hydrochloric acid and sulfuric acid; and organic acids such as acetic acid and trifluoroacetic acid.

The reducing agent may, for example, be tin(II) chloride.

In Intermediate Production Process [2-5], the reaction temperature is about -20°C to 100°C. The reaction time is about 0.5 hour to 24 hours.

### Intermediate Production Process [2-6]

The compound of the formula (XVI) may be produced by reacting a compound of the formula (XX) with a halogenating agent. The compound of the formula (XX) may be commercially available or may be produced by the method of Intermediate Production Process [2-7] or Intermediate Production Process [2-9].

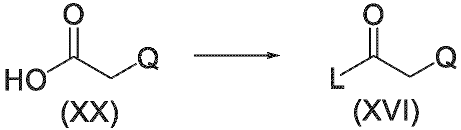

The symbols in the formulae are as defined above.

The halogenating agent is not particularly limited and may, for example, be thionyl chloride, oxalyl chloride, phosphoryl chloride, sulfuryl chloride, phosphorus trichloride or phosphorus pentachloride.

The reaction in Intermediate Production Process [2-6] may be carried out optionally in the presence of a solvent. The solvent may be any solvent and is not particularly limited so long as it is inert to the reaction, and one or more may properly be selected or mixed from the solvents described for Intermediate Production Process [1-2].

In Intermediate Production Process [2-6], the reaction temperature is about 0°C to 150°C. The reaction time is about 0.5 hour to 24 hours.

### Intermediate Production Process [2-7]

The compound of the formula (XX) may be produced by hydrolyzing a compound of the formula (XXI) in the presence of a base.

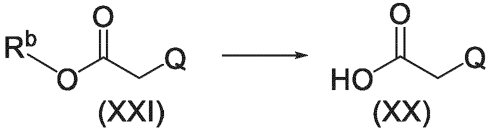

The symbols in the formulae are as defined above.

The base may, for example, be an alkali metal carbonate such as sodium carbonate, potassium carbonate or cesium carbonate; an alkaline earth metal carbonate such as calcium carbonate or barium carbonate; an alkali metal hydroxide such as lithium hydroxide, sodium hydroxide or potassium hydroxide; or an alkaline earth metal hydroxide such as calcium hydroxide or barium hydroxide. The base may be used in an amount of from 0.5 to 10 equivalents to 1 equivalent of the compound of the formula (XXI).

The reaction in Intermediate Production Process [2-7] may be carried out optionally in the presence of a solvent. The solvent may be any solvent and is not particularly limited so long as it is inert to the reaction, and one or more may properly be selected or mixed from ethers such as diethyl ether, butyl methyl ether, tetrahydrofuran, ethylene glycol dimethyl ether, 1,4-dioxane and dimethoxyethane; aprotic polar solvents such as N,N-dimethylformamide, N,N-dimethylacetamide, N-methyl-2-pyrrolidone, dimethyl sulfoxide and sulfolane; aliphatic hydrocarbons such as pentane, hexane, heptane, octane and cyclohexane; alcohols such as methanol, ethanol and isopropyl alcohol; and water.

In Intermediate Production Process [2-7], the reaction temperature is about 0°C to 150°C. The reaction time is about 0.5 hour to 24 hours.

### Intermediate Production Process [2-8]

The compound of the formula (XXI) may be commercially available, or may be produced by reacting a compound of the formula (XXII) with the compound of the formula (III) in the presence of a base. The compound of the formula (XXII) is commercially available.

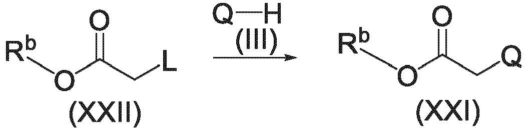

The symbols in the formulae are as defined above.

The base may, for example, be an alkali metal carbonate such as sodium carbonate, potassium carbonate or cesium carbonate; an alkaline earth metal carbonate such as calcium carbonate or barium carbonate; an alkali metal hydroxide such as lithium hydroxide, sodium hydroxide or potassium hydroxide; an alkaline earth metal hydroxide such as calcium hydroxide or barium hydroxide; an alkali metal hydride such as sodium hydride; a metal amide such as lithium diisopropylamide, lithium hexamethyldisilazide or potassium hexamethyldisilazide; a metal alkoxide such as sodium methoxide or potassium tert-butoxide; a tertiary amine such as triethylamine, 4-methylmorpholine or diisopropylethylamine; 1,8-diazabicyclo[5.4.0]-7-undecene, 1,4-diazabicyclo[2.2.2]octane, pyridine, 4-(dimethylamino)pyridine or 2,6-lutidine. The base may be used in an amount of from 0.5 to 10 equivalents to 1 equivalent of the compound of the formula (XXII).

The reaction in Intermediate Production Process [2-8] may be carried out optionally in the presence of a solvent. The solvent may be any solvent and is not particularly limited so long as it is inert to the reaction, and one or more may properly be selected or mixed from ethers such as diethyl ether, butyl methyl ether, tetrahydrofuran, ethylene glycol dimethyl ether, 1,4-dioxane and dimethoxyethane; aprotic polar solvents such as N,N-dimethylformamide, N,N-dimethylacetamide, N-methyl-2-pyrrolidone, dimethyl sulfoxide and sulfolane; nitriles such as acetonitrile and propionitrile; and esters such as ethyl acetate and ethyl propionate.

In Intermediate Production Process [2-8], the reaction temperature is about 0°C to 150°C. The reaction time is about 0.5 hour to 24 hours.

### Intermediate Production Process [2-9]

The compound of the formula (XX) may be produced by reacting a compound of the formula (XXIII) with the compound of the formula (III) in the presence of a base. The compound of the formula (XXIII) is commercially available.

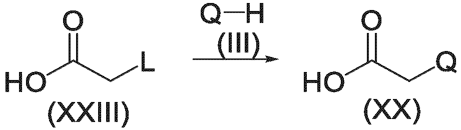

The symbols in the formulae are as defined above.

The base may, for example, be an alkali metal carbonate such as sodium carbonate, potassium carbonate or cesium carbonate; an alkaline earth metal carbonate such as calcium carbonate or barium carbonate; an alkali metal hydroxide such as lithium hydroxide, sodium hydroxide or potassium hydroxide; an alkaline earth metal hydroxide such as calcium hydroxide or barium hydroxide; an alkali metal hydride such as sodium hydride; a metal amide such as lithium diisopropylamide, lithium hexamethyldisilazide or potassium hexamethyldisilazide; or a metal alkoxide such as sodium methoxide or potassium tert-butoxide. The base may be used in an amount of from 0.5 to 10 equivalents to 1 equivalent of the compound of the formula (XXIII).

The reaction in Intermediate Production Process [2-9] may be carried out optionally in the presence of a solvent. The solvent may be any solvent and is not particularly limited so long as it is inert to the reaction, and one or more may properly be selected or mixed from ethers such as diethyl ether, butyl methyl ether, tetrahydrofuran, ethylene glycol dimethyl ether, 1,4-dioxane and dimethoxyethane; aprotic polar solvents such as N,N-dimethylformamide, N,N-dimethylacetamide, N-methyl-2-pyrrolidone, dimethyl sulfoxide and sulfolane; and water.

In Intermediate Production Process [2-9], the reaction temperature is about 20°C to 150°C. The reaction time is about 0.5 hour to 24 hours.

### Intermediate Production Process [2-10]

The compound of the formula (XIV) may be produced by reacting a compound of the formula (XXIV) with the compound of the formula (III) in the presence of a base.

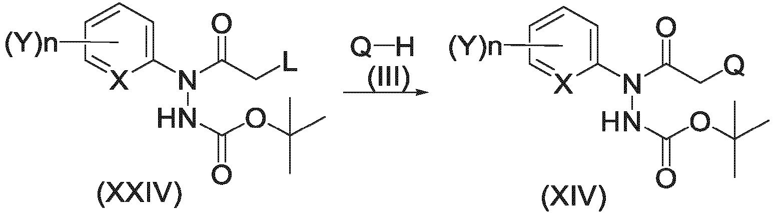

The symbols in the formulae are as defined above.

As the base, the bases described for Intermediate Production Process [2-8] may be mentioned. The base may be used in an amount of from 0.5 to 10 equivalents to 1 equivalent of the compound of the formula (XXIV).

The reaction in Intermediate Production Process [2-10] may be carried out optionally in the presence of a solvent. The solvent may be any solvent and is not particularly limited so long as it is inert to the reaction, and one or more may properly be selected or mixed from the solvents described for Intermediate Production Process [2-8].

In Intermediate Production Process [2-10], the reaction temperature is about 0°C to 150°C. The reaction time is about 0.5 hour to 24 hours.

### Intermediate Production Process [2-11]

The compound of the formula (XXIV) may be produced by reacting the compound of the formula (XV) with a compound of the formula (XXV) in the presence of a base. The compound of the formula (XXV) is commercially available.

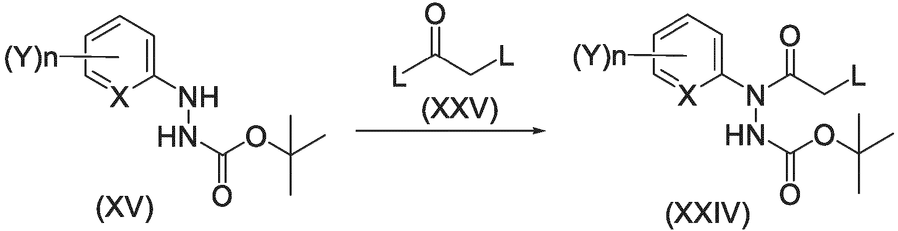

The symbols in the formulae are as defined above.

As the base, the bases described for Production Process [2] may be mentioned. The base may be used in an amount of from 0.5 to 10 equivalents to 1 equivalent of the compound of the formula (XV).

The reaction in Intermediate Production Process [2-11] may be carried out optionally in the presence of a solvent. The solvent may be any solvent and is not particularly limited so long as it is inert to the reaction, and one or more may properly be selected or mixed from the solvents described for Intermediate Production Process [1-2].

In Intermediate Production Process [2-11], the reaction temperature is about -20°C to 150°C. The reaction time is about 0.5 hour to 24 hours.

### Production Process [3]

The compound of the formula (I) may be produced by reacting a compound of the formula (XXVI) with the compound of the formula (XVI).

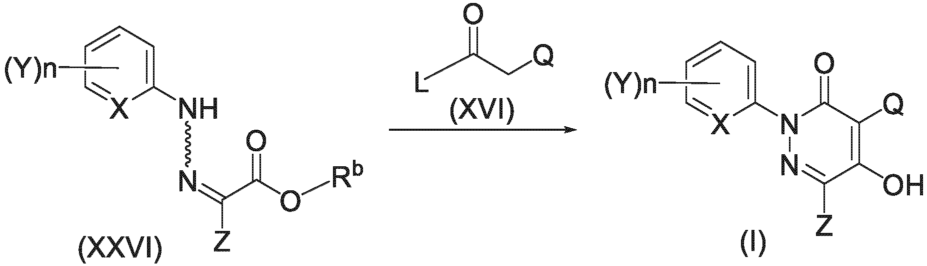

The symbols in the formulae are as defined above.

The reaction in Production Process [3] may be carried out optionally in the presence of a base. As the base, the bases described for Production Process [2] may be mentioned. The base may be used in an amount of from 0.5 to 10 equivalents to 1 equivalent of the compound of the formula (XXVI).

The reaction in Production Process [3] may be carried out optionally in the presence of a solvent. The solvent may be any solvent and is not particularly limited so long as it is inert to the reaction, and one or more may properly be selected or mixed from ethers such as diethyl ether, butyl methyl ether, tetrahydrofuran, ethylene glycol dimethyl ether, 1,4-dioxane and dimethoxyethane; aliphatic halogenated hydrocarbons such as methylene chloride, dichloroethane and chloroform; aromatic hydrocarbons such as benzene, toluene, xylene and chlorobenzene; aprotic polar solvents such as N,N-dimethylformamide, N,N-dimethylacetamide, N-methyl-2-pyrrolidone, dimethyl sulfoxide and sulfolane; nitriles such as acetonitrile and propionitrile; esters such as ethyl acetate and ethyl propionate; aliphatic hydrocarbons such as pentane, hexane, heptane, octane and cyclohexane; ketones such as acetone and ethyl methyl ketone; and nitroalkanes such as nitromethane and nitroethane.

In Production Process [3], the reaction temperature is about -20°C to 150°C. The reaction time is about 0.5 hour to 24 hours.

### Intermediate Production Process [3-1]

The compound of the formula (XXVI) may be produced by reacting the compound of the formula (XVII) with the compound of the formula (XIII) optionally in the presence of an acid.

The reaction of Intermediate Production Process [3-1] may be carried out in the same manner as Intermediate Production Process [2-1].

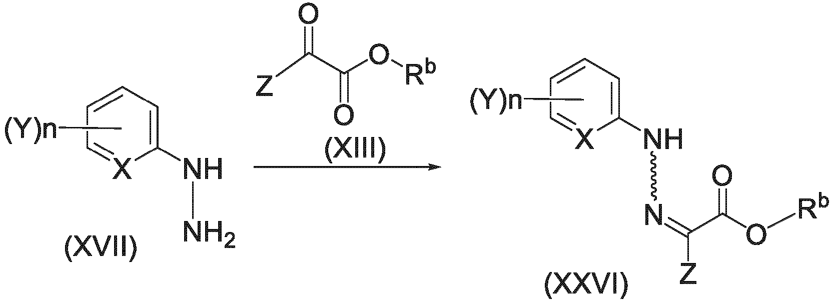

The symbols in the formulae are as defined above.

The acid may, for example, be an inorganic acid such as hydrochloric acid or sulfuric acid; or an organic acid such as acetic acid, formic acid, methanesulfonic acid, trifluoroacetic acid or p-toluenesulfonic acid.

The compound of the formula (XXVI) may be produced in accordance with a known literature (for example, Bioorganic and Medicinal Chemistry Letters, 2014,24,1944-1947).

### Production Process [4]

A compound of the formula (I-b) which is the compound of the formula (I) wherein Z is an alkoxyalkyl may be produced by reacting a compound of the formula (XXVII) with a compound of the formula (XXVIII) in the presence of a base. The compound of the formula (XXVIII) may be commercially available or may be produced in accordance with a known method.

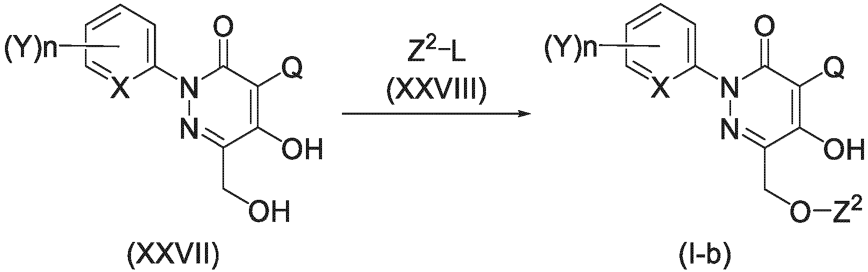

In the formulae, Z² is an alkyl, and the other symbols are as defined above.

As the base, the bases described for Intermediate Production Process [2-8] may be mentioned. The base may be used in an amount of from 0.5 to 10 equivalents to 1 equivalent of the compound of the formula (XXVII).

The reaction in Production Process [4] may be carried out optionally in the presence of a solvent. The solvent may be any solvent and is not particularly limited so long as it is inert to the reaction, and one or more may properly be selected or mixed from the solvents as described for step 1 of Production Process [1].

In Production Process [4], the reaction temperature is about 0°C to 150°C. The reaction time is about 0.5 hour to 24 hours.

In the reaction of Production Process [4], optionally the following base and/or water may be added to a reactor during the reaction. The stirring time after the addition of the base is about 0.5 hour to 12 hours.

As the base to be added, the bases described for Intermediate Production Process [2-7] may be mentioned. The base may be used in an amount of from 0.5 to 10 equivalents to 1 equivalent of the compound of the formula (XXVII). Or, in some cases, it may be used in excess over the compound of the formula (XXVII).

### Intermediate Production Process [4-1]

The compound of the formula (XXVII) may be produced by reacting a compound of the formula (XXIX) with a reducing agent. The compound of the formula (XXIX) may be produced in accordance with Production Process [2] and Intermediate Production Process [2-1], or Production Process [3] and Intermediate Production Process [3-1], except that in the compound of the formula (I), Z is -C(=O)OR^{b}.

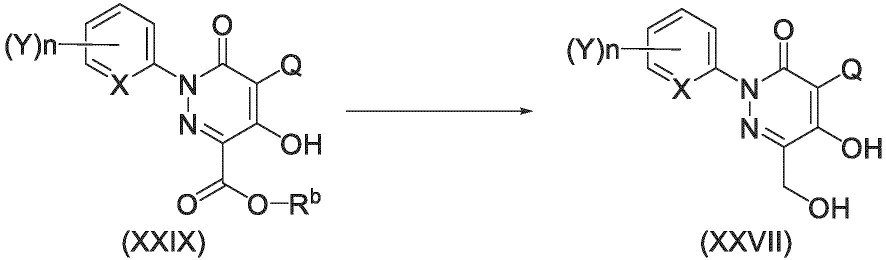

The symbols in the formulae are as defined above.

The reducing agent is not particularly limited and may, for example, be lithium aluminum hydride, diisobutyl aluminum hydride, sodium bis(2-methoxyethoxy)aluminum hydride, sodium borohydride, lithium borohydride or a borane-tetrahydrofuran complex.

The reaction in Intermediate Production Process [4-1] may be carried out optionally in the presence of a solvent. The solvent may be any solvent and is not particularly limited so long as it is inert to the reaction, and one or more may properly be selected or mixed from halogenated hydrocarbons such as methylene chloride, chloroform, dichloroethane, trichloroethane and carbon tetrachloride; aromatic hydrocarbons such as benzene, toluene, xylene and chlorobenzene; hydrocarbons such as n-hexane and n-heptane; ethers such as diethyl ether, tert-butyl methyl ether, 1,4-dioxane, tetrahydrofuran and 1,2-dimethoxyethane; and alcohols such as methanol, ethanol and isopropyl alcohol.

In Intermediate Production Process [4-1], the reaction temperature is about -20°C to 150°C. The reaction time is about 0.5 hour to 24 hours.

### Production Process [5]

A compound of the formula (I-d) which is the compound of the formula (I) wherein Q is Q^{b}, may be produced by reacting a compound of the formula (I-c) with a compound of the formula (XXX). The compound of the formula (XXX) may be commercially available or may be produced in accordance with a known method.

The reaction of Production Process [5] may be carried out in the same manner as Intermediate Production Process [1-3].

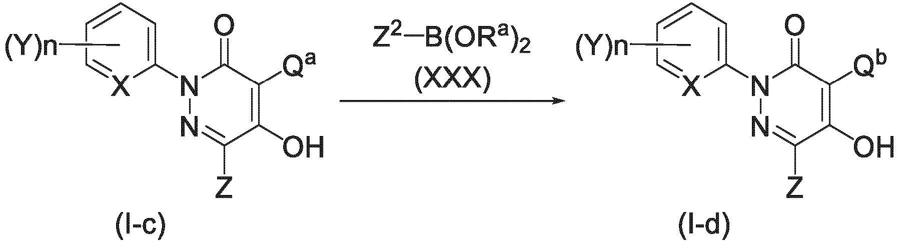

In the formulae, Q^{a} is Q¹, Q², Q³ or Q⁴ having at least one chlorine, bromine or iodine as a substituent on the ring, Q^{b} is Q¹, Q², Q³ or Q⁴ having at least one Z² as a substituent on the ring, and the other symbols are as defined above.

### Production Process [6]

The compound of the formula (I-d) may be produced also by carrying out step 1 of reacting the compound of the formula (I-c) with a compound of the formula (XXXI) to obtain a compound of the formula (I-e), and subsequent step 2. The compound of the formula (XXXI) may be commercially available or may be produced in accordance with a known method.

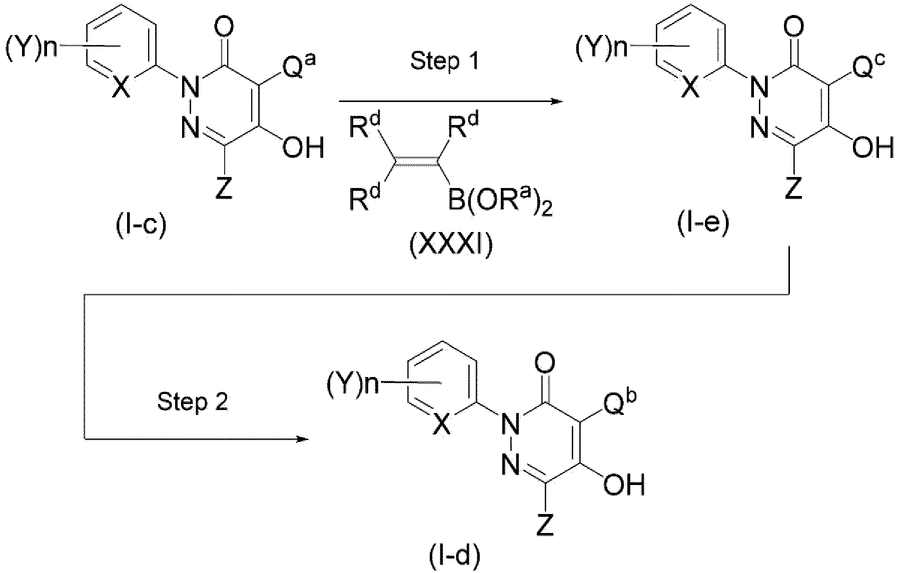

In the formulae, R^{d} are each a hydrogen atom or an alkyl, Q^{c} is Q¹, Q², Q³ or Q⁴ having at least one alkenyl as a substituent on the ring, and the other symbols are as defined above.

The reaction of step 1 of Production Process [6] may be carried out in the same manner as Intermediate Production Process [1-3].

In step 2 of Production Process [6], under a hydrogen atmosphere, the compound of the formula (I-e) obtained in step 1 is reacted in the presence of a metal catalyst to produce the compound of the formula (I-d).

The metal catalyst may be a conventional catalyst used for a reduction reaction. It may, for example, be a palladium catalyst such as palladium on carbon or palladium hydroxide; a nickel catalyst such as Raney nickel; or a platinum catalyst such as platinum on carbon or platinum oxide.

The reaction in step 2 of Production Process [6] may be carried out optionally in the presence of a solvent. The solvent may be any solvent and is not particularly limited so long as it is inert to the reaction, and one or more may properly be selected or mixed from ethers such as diethyl ether, butyl methyl ether, tetrahydrofuran, ethylene glycol dimethyl ether, 1,4-dioxane and dimethoxyethane; aliphatic halogenated hydrocarbons such as methylene chloride, dichloroethane and chloroform; aromatic hydrocarbons such as benzene, toluene, xylene and chlorobenzene; aprotic polar solvents such as N,N-dimethylformamide, N,N-dimethylacetamide, N-methyl-2-pyrrolidone, dimethyl sulfoxide and sulfolane; aliphatic hydrocarbons such as pentane, hexane, heptane, octane and cyclohexane; ketones such as acetone and ethyl methyl ketone; alcohols such as methanol, ethanol and isopropyl alcohol; and water.

In step 2 of Production Process [6], the reaction temperature is about 20°C to 100°C. The reaction time is about 0.5 hour to 24 hours.

### Production Process [7]

A compound of the formula (I-f) which is the compound of the formula (I) having a formyl group as Y, may be produced by reacting a compound of the formula (XXXII) with an oxidizing agent.

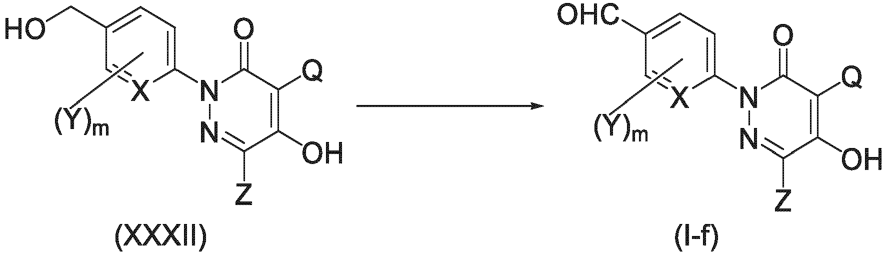

In the formulae, m is an integer of from 0 to 3, and the other symbols are as defined above.

The oxidizing agent may, for example, be dimethyl sulfoxide-activating agent, 2,2,6,6-tetramethylpiperidine 1-oxyl, 1,1,1-triacetoxy-1,1-dihydro-1,2-benziodoxol-3-(1H)-one, manganese dioxide, pyridinium chlorochromate or pyridinium dichromate.

In the case of oxidation by dimethyl sulfoxide-activating agent, the activating agent may, for example, be an acid anhydride such as acetic anhydride or trifluoroacetic anhydride; an acid chloride such as thionyl chloride or oxalyl chloride; chlorine or N-chlorosuccinimide.

2,2,6,6-tetramethylpiperidine 1-oxyl may be used in a catalytic amount, and in such a case, as a reactivating agent, sodium hypochlorite, iodobenzene diacetate or the like may be used.

The reaction in Production Process [7] may be carried out optionally in the presence of a solvent. The solvent may be any solvent and is not particularly limited so long as it is inert to the reaction, and one or more may properly be selected or mixed from ethers such as diethyl ether, butyl methyl ether, tetrahydrofuran, ethylene glycol dimethyl ether, 1,4-dioxane and dimethoxyethane; aliphatic halogenated hydrocarbons such as methylene chloride, dichloroethane and chloroform; aromatic hydrocarbons such as benzene, toluene, xylene and chlorobenzene; aliphatic hydrocarbons such as pentane, hexane, heptane, octane and cyclohexane; ketones such as acetone and ethyl methyl ketone; and alcohols such as methanol, ethanol and isopropyl alcohol.

In Production Process [7], the reaction temperature is about -78°C to 150°C. The reaction time is about 0.5 hour to 24 hours.

### Intermediate Production Process [7-1]

The compound of the formula (XXXII) may be produced by reacting a compound of the formula (I-g) with a reducing agent. The compound of the formula (I-g) may be produced by the method of Production Process [2] or Production Process [3].

The reaction of Intermediate Production Process [7-1] may be carried out in the same manner as Intermediate Production Process [4-1].

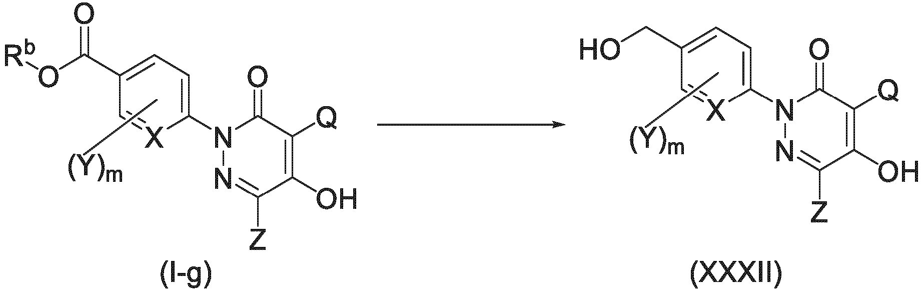

The symbols in the formulae are as defined above.

The reducing agent is not particularly limited and may, for example, be lithium aluminum hydride, diisobutyl aluminum hydride, sodium bis(2-methoxyethoxy)aluminum hydride, sodium borohydride, lithium borohydride or a borane-tetrahydrofuran complex.

### Production Process [8]

A compound of the formula (I-h) which is the compound of the formula (I) having a cyano group as Y, may be produced by reacting a compound of the formula (XXXIII) with acetic anhydride.

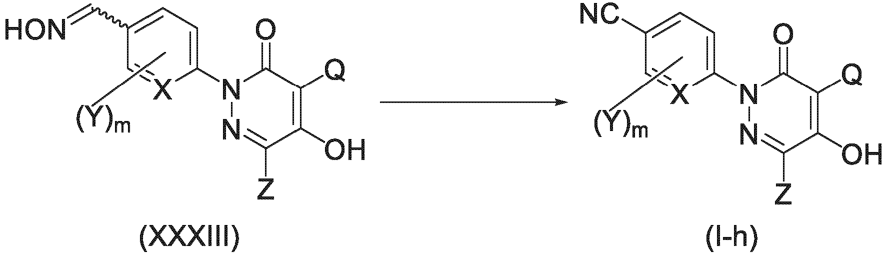

The symbols in the formulae are as defined above.

The reaction in Production Process [8] may be carried out optionally in the presence of a solvent. The solvent may be any solvent and is not particularly limited so long as it is inert to the reaction, and one or more may properly be selected or mixed from aromatic hydrocarbons such as benzene, toluene, xylene and chlorobenzene; and aprotic polar solvents such as N,N-dimethylformamide, N,N-dimethylacetamide, N-methyl-2-pyrrolidone, dimethyl sulfoxide and sulfolane.

In Production Process [8], the reaction temperature is about 50°C to 200°C. The reaction time is about 0.5 hour to 24 hours.

In the reaction of Production Process [8], optionally the following base and/or solvent may be added to a reactor during the reaction. Further, the mixture obtained after post-treatment of the reaction and purification, may be mixed with the following base or solvent. The stirring time after the addition of the base or solvent is about 0.5 hour to 12 hours.

As the base to be added, the bases described for Intermediate Production Process [2-7] may be mentioned. The base may be used in an amount of from 0.5 to 10 equivalents to 1 equivalent of the compound of the formula (XXXIII). Or, in some cases, it may be used in excess over the compound of the formula (XXXIII).

The solvent is not particularly limited, and one or more may properly be selected or mixed from ethers such as diethyl ether, butyl methyl ether, tetrahydrofuran, ethylene glycol dimethyl ether, 1,4-dioxane and dimethoxyethane; aliphatic hydrocarbons such as pentane, hexane, heptane, octane and cyclohexane; alcohols such as methanol, ethanol and isopropyl alcohol; and water.

### Intermediate Production Process [8-1]

The compound of the formula (XXXIII) may be produced by reacting a compound of the formula (XXXIV) with hydroxylamine in the presence of a base. The hydroxylamine may be used as a hydroxylamine solution or a hydroxylamine salt.

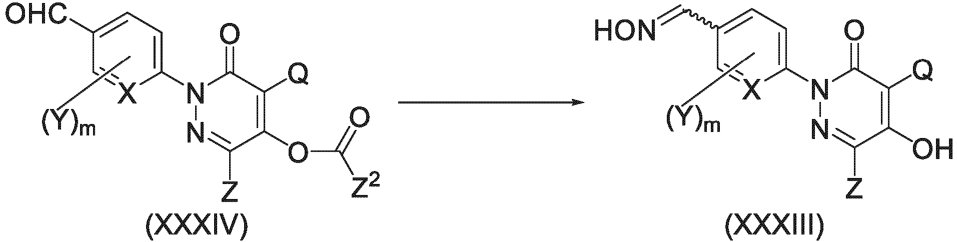

The symbols in the formulae are as defined above.

The hydroxylamine solution may, for example, be an aqueous hydroxylamine solution or a hydroxylamine-ethanol mixed solution. The hydroxylamine salt may, for example, be hydroxylamine hydrochloride or hydroxylamine sulfate.

As the base, one or more may properly be selected and mixed from tertiary amines such as triethylamine and diisopropylethylamine; alkali metal carbonates such as sodium carbonate, potassium carbonate and cesium carbonate; alkaline earth metal carbonates such as calcium carbonate and barium carbonate; alkali metal hydroxides such as lithium hydroxide, sodium hydroxide and potassium hydroxide; alkaline earth metal hydroxides such as calcium hydroxide and barium hydroxide; and acetates such as sodium acetate and potassium acetate. The base may be used in an amount of from 0.5 to 10 equivalents to 1 equivalent of the compound of the formula (XXXIV).

The reaction in Intermediate Production Process [8-1] may be carried out optionally in the presence of a solvent. The solvent may be any solvent and is not particularly limited so long as it is inert to the reaction, and one or more may properly be selected or mixed from ethers such as diethyl ether, butyl methyl ether, tetrahydrofuran, ethylene glycol dimethyl ether, 1,4-dioxane and dimethoxyethane; aliphatic halogenated hydrocarbons such as methylene chloride, dichloroethane and chloroform; aprotic polar solvents such as N,N-dimethylformamide, N,N-dimethylacetamide, N-methyl-2-pyrrolidone, dimethyl sulfoxide and sulfolane; alcohols such as methanol, ethanol and isopropyl alcohol; and water.

In Intermediate Production Process [8-1], the reaction temperature is about -20°C to 150°C. The reaction time is about 0.5 hour to 24 hours.

In the reaction of Intermediate Production Process [8-1], optionally the following base and/or water may be added to a reactor during the reaction. The stirring time after the addition of the base is about 5 minutes to 12 hours.

As the base to be added, the bases described for Intermediate Production Process [2-7] may, for example, be mentioned. The base may be used in an amount of from 0.5 to 10 equivalents to 1 equivalent of the compound of the formula (XXXIV). Or, in some cases, it may be used in excess over the compound of the formula (XXXIV).

### Intermediate Production Process [8-2]

The compound of the formula (XXXIV) may be produced by reacting the compound of the formula (I-f) with a compound of the formula (XXXV) or a compound of the formula (XXXVI), in the presence of a base. The compound of the formula (XXXV) and the compound of the formula (XXXVI) may be commercially available or may be produced in accordance with a known method.

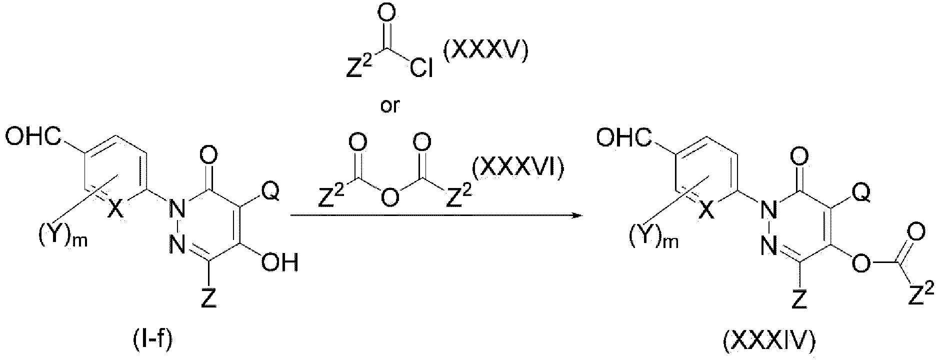

The symbols in the formulae are as defined above.

As the base, the bases described for Intermediate Production Process [2-8] may be mentioned. The base may be used in an amount of from 0.5 to 10 equivalents to 1 equivalent of the compound of the formula (I-f).

The reaction in Intermediate Production Process [8-2] may be carried out optionally in the presence of a solvent. The solvent may be any solvent and is not particularly limited so long as it is inert to the reaction, and one or more may properly be selected or mixed from the solvents as described for step 1 of Production Process [1].

In Intermediate Production Process [8-2], the reaction temperature is about -20°C to 150°C. The reaction time is about 0.5 hour to 24 hours.

### Production Process [9]

A compound of the formula (I-i) which is the compound of the formula (I) having an alkylsulfinyl, an alkylsulfonyl, a haloalkylsulfinyl or a haloalkylsulfonyl as Y, may be produced by reacting a compound of the formula (I-j) with an oxidizing agent. The compound of the formula (I-j) may be produced by the method of Production Process [1] and Intermediate Production Processes [1-1] to [1-4], Production Process [2] and Intermediate Production Processes [2-1] to [2-11], or Production Process [3] and Intermediate Production Process [3-1].

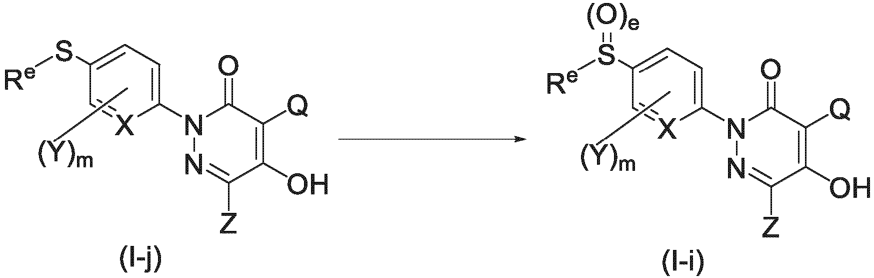

In the formulae, R^{e} is an alkyl or a haloalkyl, e is 1 or 2, and the other symbols are as defined above.

The oxidizing agent may, for example, be a peroxide such as hydrogen peroxide, acetic peroxide, benzoic peroxide or m-chloroperbenzoic acid. In a case where hydrogen peroxide is used as the oxidizing agent, optionally sodium tungstate or the like may be added.

The reaction of Production Process [9] may be carried out optionally in the presence of a solvent. The solvent may be any solvent and is not particularly limited so long as it is inert to the reaction, and one or more may properly be selected or mixed from ethers such as diethyl ether, butyl methyl ether, tetrahydrofuran, ethylene glycol dimethyl ether, 1,4-dioxane and dimethoxyethane; aliphatic halogenated hydrocarbons such as methylene chloride, dichloroethane and chloroform; aromatic hydrocarbons such as benzene, toluene, xylene and chlorobenzene; aprotic polar solvents such as N,N-dimethylformamide, N,N-dimethylacetamide, N-methyl-2-pyrrolidone, dimethyl sulfoxide and sulfolane; nitriles such as acetonitrile and propionitrile; esters such as ethyl acetate and ethyl propionate; aliphatic hydrocarbons such as pentane, hexane, heptane, octane and cyclohexane; and water.

In Production Process [9], the reaction temperature is about -20°C to 100°C. The reaction time is about 0.5 hour to 24 hours.

### Production Process [10]

The compound of the formula (I-i) may be produced also by reacting a compound of the formula (XXXVII) in the presence of a base.

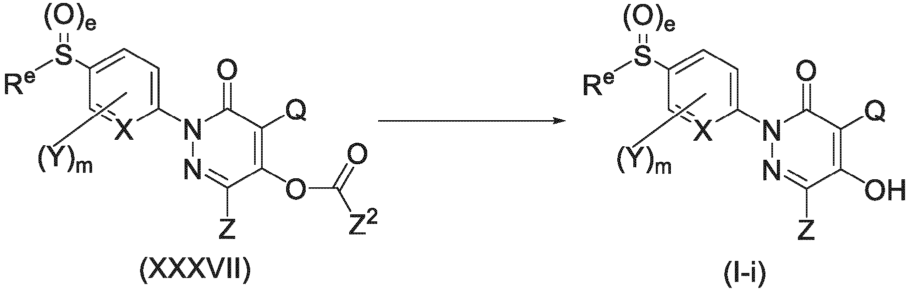

The symbols in the formulae are as defined above.

As the base, the bases described for Intermediate Production Process [2-7] may be mentioned. The base may be used in an amount of from 0.5 to 10 equivalents to 1 equivalent of the compound of the formula (XXXVII).

The reaction in Production Process [10] may be carried out optionally in the presence of a solvent. The solvent may be any solvent and is not particularly limited so long as it is inert to the reaction, and one or more may properly be selected or mixed from ethers such as diethyl ether, butyl methyl ether, tetrahydrofuran, ethylene glycol dimethyl ether, 1,4-dioxane and dimethoxyethane; aliphatic halogenated hydrocarbons such as methylene chloride, dichloroethane and chloroform; aromatic hydrocarbons such as benzene, toluene, xylene and chlorobenzene; aprotic polar solvents such as N,N-dimethylformamide, N,N-dimethylacetamide, N-methyl-2-pyrrolidone, dimethyl sulfoxide and sulfolane; aliphatic hydrocarbons such as pentane, hexane, heptane, octane and cyclohexane; alcohols such as methanol, ethanol and isopropyl alcohol; and water.

In Production Process [10], the reaction temperature is about 0°C to 150°C. The reaction time is about 0.5 hour to 24 hours.

### Intermediate Production Process [10-1]

The compound of the formula (XXXVII) may be produced by reacting a compound of the formula (XXXVIII) with an oxidizing agent.

The reaction of Intermediate Production Process [10-1] may be carried out in the same manner as Production Process [9].

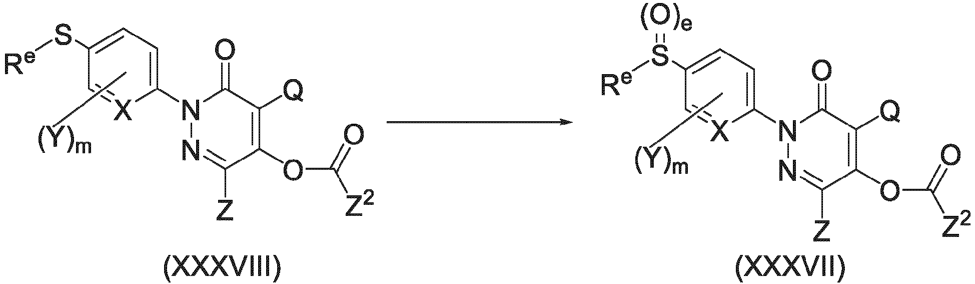

The symbols in the formulae are as defined above.

The oxidizing agent may, for example, be a peroxide such as hydrogen peroxide, acetic peroxide, benzoic peroxide or m-chloroperbenzoic acid. In a case where hydrogen peroxide is used as the oxidizing agent, optionally sodium tungstate or the like may be added.

### Intermediate Production Process [10-2]

The compound of the formula (XXXVIII) may be produced by reacting the compound of the formula (I-j) with the compound of the formula (XXXV) or the compound of the formula (XXXVI) in the presence of a base.

The reaction in Intermediate Production Process [10-2] may be carried out in the same manner as Intermediate Production Process [8-2].

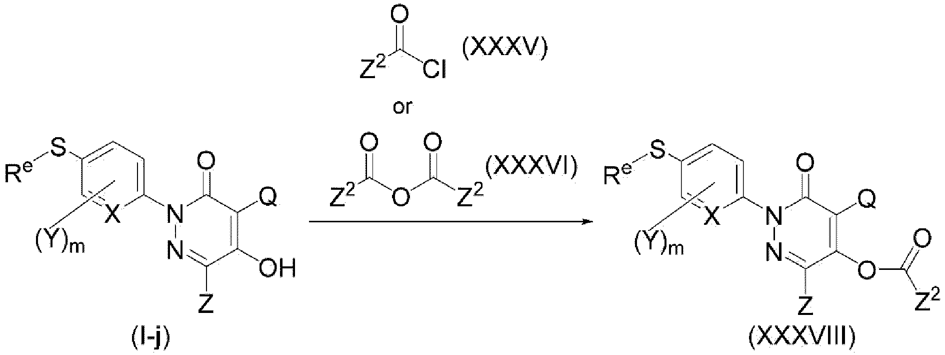

The symbols in the formulae are as defined above.

The base may, for example, be an alkali metal carbonate such as sodium carbonate, potassium carbonate or cesium carbonate; an alkaline earth metal carbonate such as calcium carbonate or barium carbonate; an alkali metal hydroxide such as lithium hydroxide, sodium hydroxide or potassium hydroxide; an alkaline earth metal hydroxide such as calcium hydroxide or barium hydroxide; an alkali metal hydride such as sodium hydride; a metal amide such as lithium diisopropylamide, lithium hexamethyldisilazide or potassium hexamethyldisilazide; a metal alkoxide such as sodium methoxide or potassium tert-butoxide; a tertiary amine such as triethylamine, 4-methylmorpholine or diisopropylethylamine; 1,8-diazabicyclo[5.4.0]-7-undecene, 1,4-diazabicyclo[2.2.2]octane, pyridine, 4-(dimethylamino)pyridine or 2,6-lutidine. The base may be used in an amount of from 0.5 to 10 equivalents to 1 equivalent of the compound of the formula (I-j).

Now, preferred embodiments of the pest control agent containing the compound of the present invention will be described below.

The pest control agent containing the compound of the present invention is useful as a controlling agent against insects, mites, nematodes or soil insect pests problematic in agricultural and horticultural fields, that is as an agricultural and horticultural insecticide, miticide, nematicide or soil pesticide. It is also useful as a controlling agent against animal-parasitic pests, that is as an animal-parasitic pesticide.

The pests problematic in agricultural and horticultural fields may, for example, be agricultural pests such as aphids (such as green peach aphid and cotton aphid), planthoppers (such as brown planthopper), leafhoppers, scales, bugs, whiteflies (such as sweetpotato whitefly), thrips, grasshoppers, anthomyiid flies, scarabs, ants, diamondback moth, cabbage armyworm, cotton leafworm, codling moth, bollworm, tobacco budworm, gypsy moth, rice leafroller, smaller tea tortrix, Colorado potato beetle, cucurbit leaf beetle, boll weevil, black cutworm and cutworm; gastropods such as slugs and snails; sanitary insect pests such as tropical rat mite, cockroaches, housefly and common house mosquito; stored grain insect pests such as angoumois grain moth, adzuki bean weevil, red flour beetle and mealworms; clothes and household goods insect pests such as case-making clothes moth, black carpet beetle and termites.

The mites problematic in agricultural and horticultural fields may, for example, be plant parasitic mites such as two-spotted spider mite, carmine spider mite, kanzawa spider mite, citrus red mite, European red mite, broad mite, pink citrus rust mite and bulb mite; and domestic mites such as mould mite, Dermatophagoides farinae and

### Chelacaropsis moorei.

The nematodes problematic in agricultural and horticultural fields may, for example, be plant parasitic nematodes such as root-knot nematodes, cyst nematodes, root-lesion nematodes, white tip nematode, strawberry bud nematode and pine wood nematode.

The soil pests problematic in agricultural and horticultural fields may, for example, be isopods such as pill bugs and sow bugs.

The pest control agent containing the compound of the present invention is effective for controlling, among the above, for example, insect pests of the order Hemiptera, insect pests of the order Thysanoptera, insect pests of the order Coleoptera, insect pests of the order Acari, insect pests of the order Lepidoptera, and nematodes, and is particularly effective for controlling insect pests of the order Hemiptera and insect pests of the order Acari.

As specific examples of such insect pests and nematodes, the following may be mentioned.

Insect pests of the order Hemiptera, such as Aphididae such as pea aphid (Acyrthosiphon pisum), oriental grass root aphid (Tetraneura nigriabdominalis), sugarcane woolly aphid (Ceratovacuna lanigera), foxglove aphid (Aulacorthum solani), cabbage aphid (Brevicoryne brassicae), soybean aphid (Aphis glycines), potato aphid (Macrosiphum euphorbiae), corn leaf aphid (Rhopalosiphum maidis), turnip aphid (Lipaphis erysimi), sugarcane aphid (Melanaphis sacchari), black bean aphid (Aphis fabae), brown citrus aphid (Toxoptera citricida), bird cherry-oat aphid (Rhopalosiphum padi), leaf curl plum aphid (Brachycaudus helichrysi), green peach aphid (Myzus persicae), mealy plum aphid (Hyalopterus pruni), green citrus aphid (Aphis spiraecola), woolly apple aphid (Eriosoma lanigerum), currant-lettuce aphid (Nasonovia ribisnigri), cotton aphid (Aphis gossypii), apple aphid (Aphis pomi), English grain aphid (Sitobion avenae) and rosy apple aphid (Dysaphis plantaginea); Phylloxeridae such as grape phylloxera (Daktulosphaira vitifoliae), pecan leaf phylloxera (Phylloxera notabilis), pecan phylloxera (Phylloxera devastatrix), southern pecan leaf phylloxera (Phylloxera russelae); Adelgidae such as hemlock woolly aphid (Adelges tsugae), Aphrastasia pectinatae, balsam woolly aphid (Adelges piceae); Aleyrodidae such as greenhouse whitefly (Trialeurodes vaporariorum), sweetpotato whitefly (Bemisia tabaci), tea spiny whitefly (Aleurocanthus camelliae), Pealius euryae, citrus whitefly (Dialeurodes citri) and orange spiny whitefly (Aleurocanthus spiniferus); Delphacidae such as cereal leafhopper (Javesella pellucida), sugarcane planthopper (Perkinsiella saccharicida), white backed planthopper (Sogatella furcifera), corn planthopper (Peregrinus maidis), brown planthopper (Nilaparvata lugens), small brown planthopper (Laodelphax striatellus) and rice delphacid (Tagosodes orizicolus); Cicadellidae such as zigzagstripped leafhopper (Recilia dorsalis), rice leafhopper (Nephotettix nigropictus), potato leafhopper (Empoasca fabae), white rice leafhopper (Cofana spectra), green leafhopper (Nephotettix virescens), tea green leafhopper (Empoasca onukii), green rice leafhopper (Nephotettix cincticeps), cotton jassid (Amrasca biguttula) and corn leafhopper (Dalbulus maidis); Aphrophoridae such as meadow spittlebug (Philaenus spumarius); Cercopidae such as Mahanarva fimbriolata; Pentatomidae such as eastern green stink bug (Nezara antennata), stink bug (Scotinophara lurida), lewis spined bug (Eysarcoris lewisi), brown marmorated stink bug (Halyomorpha halys), white-spotted stink bug (Eysarcoris ventralis), brown-winged green bug (Plautia stali), white-spotted spined bug (Eysarcoris aeneus), southern green stink bug (Nezara viridula), Eysarcoris annamita, black paddy bug (Scotinophara coarctata), brown stink bug (Euschistus heros), greenbelly stink bug (Dichelops melacanthus), redbanded stink bug (Piezodorus quildinii) and rice stink bug (Oebalus pugnax); Cydnidae such as subterraneous stink bug (Scaptocoris castanea); Alydidae such as corbett rice bug (Leptocorisa chinensis), paddy earhead bug (Leptocorisa acuta) and bean bug (Riptortus clavatus); Coreidae such as leaf-footed plant bug (Leptoglossus australis) and rice stink bug (Cletus punctiger); Lygaeidae such as chinch bug (Blissus leucopterus), oriental chinch bug (Cavelerius saccharivorus) and seed bug (Togo hemipterus); Miridae such as sorghum plant bug (Stenotus rubrovittatus), rice leaf bug (Trigonotylus caelestialium), tarnished plant bug (Lygus lineolaris), European tarnished plant bug (Lygus rugulipennis) and white leaf bug (Stenodema calcarata); Diaspididae such as California red scale (Aonidiella aurantii), white prunicola scale (Pseudaulacaspis prunicola), white peach scale (Pseudaulacaspis pentagona), cyanophyllum scale (Abgrallaspis cyanophylli), San Jose scale (Diaspidiotus perniciosus) and arrowhead snow scale (Unaspis vanonensis); Coccidae such as red wax scale (Ceroplastes rubens); Margarodidae such as cottony cushion scale (Icerva purchasi) and yellow cottony cushion scale (Icerva sevchellarum); Pseudococcidae such as citrophilus mealybug (Pseudococcus calceolariae), cotton mealybug (Phenacoccus solenopsis), comstock mealybug (Pseudococcus comstocki), longtailed mealybug (Pseudococcus longispinus), solanum mealybug (Phenacoccus solani), Japanese mealybug (Planococcus kraunhiae), citrus mealybug (Planococcus citri) and rice mealybug (Brevennia rehi); Psyllidae such as potato/tomato psyllid (Bactericera cockerelli), Chinese pear psyllid (Cacopsylla chinensis), pear sucker (Cacopsylla pyrisuga), Asian citrus psyllid (Diaphorina citri), African citrus psyllid (Trioza erytreae) and pear psylla (Cacopsylla pyricola); Tingidae such as chrysanthemum lace bug (Corythucha marmorata), azalea lace bug (Stephanitis pyrioides), Japanese pear lace bug (Stephanitis nashi) and sycamore lace bug (Corythucha ciliata); Cimicidae such as bed bug (Cimex lectularius) and tropical bed bug (Cimex hemipterus); Cicadidae such as Quesada gigas; and Reduviidae such as kissing bug (Triatoma rubrofasciata), blood sucking bug (Triatoma infestans), Rhodnius prolixus and Triatoma dimidiate.

Insect pests of the order Thysanoptera, such as Thripidae such as rice thrips (Stenchaetothrips biformis), chilli thrips (Scirtothrips dorsalis), onion thrips (Thrips tabaci), flower thrips (Frankliniella intonsa), western flower thrips (Frankliniella occidentalis), melon thrips (Thrips palmi), poinsettia thrips (Echinothrips americanus) and avocado thrips (Scirtothrips perseae); and Phlaeothripidae such as rice aculeated thrips (Haplothrips aculeatus).

Insect pests of the order Coleoptera, such as Chrysomelidae such as rice hispa (Dicladispa armigera), rice leaf beetle (Oulema oryzae), cucurbit leaf beetle (Aulacophora femoralis), striped flea beetle (Phyllotreta striolata), cereal leaf beetle (Oulema melanopus), Colorado potato beetle (Leptinotarsa decemlineata), sweetpotato flea beetle (Chaetocnema confinis), spotted cucumber beetle (Diabrotica undecimpunctata Mannerheim), tobacco flea beetle (Epitrix hirtipennis), Laccoptera quadrimaculata, banded cucumber beetle (Diabrotica balteata), bean leaf beetle (Cerotoma trifurcata), cabbage-stem flea beetle (Psylliodes chrysocephala), corn flea beetle (Chaetocnema pulicaria), crucifer flea beetle (Phyllotreta cruciferae), cucurbit beetle (Diabrotica speciosa), grape colaspis (Colaspis brunnea), hop flea beetle (Psylliodes punctulata), northern corn rootworm (Diabrotica barberi), potato flea beetle (Epitrix cucumeris), southern corn leaf beetle (Myochrous denticollis), western black flea beetle (Phyllotreta pusilla), western corn rootworm (Diabrotica virgifera virgifera) and Mexican corn rootworm (Diabrotica virgifera zeae); Carabidae such as slender seedcorn beetle (Clivina impressifrons) and Leconte's seedcorn beetle (Stenolophus lecontei); Scarabaeidae such as green chafer (Anomala albopilosa), scarab beetle (Holotrichia kiotonensis), large black chafer (Holotrichia parallela), carrot beetle (Tomarus gibbosus), cupreous chafer (Anomala cuprea), yellowish elongate chafer (Heptophylla picea), soybean beetle (Anomala rufocuprea), Japanese beetle (Popillia japonica), European chafer (Rhizotrogus majalis), June beetle (Phyllophaga crinita), Phyllophaga anxia, Phyllophaga crassissima and Diloboderus abderus; Anthriibidae such as coffee bean beetle (Araecerus coffeae); Aponidae such as sweetpotato weevil (Cylas formicarius); Bruchidae such as azuki bean weevil (Callosobruchus chinensis) and Mexican bean weevil (Zabrotes subfasciatus); Scolytidae such as pine shoot beetle (Tomicus piniperda) and coffee berry borer (Hypothenemus hampei); Curculionidae such as alfalfa weevil (Hypera postica), rice plant weevil (Echinocnemus squameus), rice water weevil (Lissorhoptrus orvzophilus), West Indian sweetpotato weevil (Euscepes postfasciatus), granary weevil (Sitophilus granarius), maize weevil (Sitophilus zeamais), rice weevil (Sitophilus oryzae), rusty gourd-shaped weevil (Scepticus griseus), hunting billbug (Sphenophorus venatus), Asiatic palm weevil (Rhabdoscelus lineaticollis), brown gourd-shaped weevil (Scepticus uniformis), boll weevil (Anthonomus grandis), cotton root borer (Eutinobothrus brasiliensis), southern corn billbug (Sphenophorus callosus), sugarcane weevil (Sphenophorus levis), Aracanthus mourei and soybean stalk weevil (Sternechus subsignatus); Tenebrionidae such as lesser mealworm (Alphitobius diaperinus), red flour beetle (Tribolium castaneum) and confused flour beetle (Tribolium confusum); Coccinellidae such as 28-spotted potato ladybird (Epilachna vigintioctomaculata) and 28-spotted ladybird (Epilachna vigintioctopunctata); Bostrychidae such as lesser grain borer (Rhizopertha dominica) and powder post beetle (Lyctus brunneus); Ptinidae; Cerambycidae such as red necked longhorn beetle (Aromia bungii), white-spotted longicorn beetle (Anoplophora malasiaca) and Migdolus fryanus; Elateridae such as sugarcane wireworm (Melanotus okinawensis), Melanotus legatus, barley wireworm (Agriotes fuscicollis), Aeolus spp., Anchastus spp., Conoderus spp., Ctenicera spp. and Limonius spp.; Staphylinidae such as skirt and blouse beetle (Paederus fuscipes); Dermestidae such as hide beetle (Dermestes maculatus), khapra beetle (Trogoderma granarium) and varied carpet beetle (Anthrenus verbasci); Anobiidae such as drugstore beetle (Stegobium paniceum) and cigarette beetle (Lasioderma serricorne); Laemophloeidae such as flat grain beetle (Cryptolestes ferrugineus); Silvanidae such as sawtoothed grain beetle (Oryzaephilus surinamensis); and Nitidulidae such as pollen beetle (Brassicogethes aeneus).

Insect pests of the order Acari, such as Tetranychidae such as Oligonychus spp., Kanzawa spider mite (Tetranychus kanzawai), two-spotted spider mite (Tetranychus urticae), citrus red mite (Panonychus citri), red spider mite (Tetranychus evansi) and European red mite (Panonychus ulmi); Eriophyidae such as persimmon bud mite (Aceria diospyri), Shevtchenkella sp., purple tea mite (Calacarus carinatus), tea rust mite (Acaphylla theavagrans), tomato russet mite (Aculops lycopersici), Japanese pear rust mite (Eriophyes chibaensis), Japanese citrus rust mite (Aculops pelekassi), Phyllocoptruta citri, apple rust mite (Aculus schlechtendali) and Aceria tosichella; Tarsonemidae such as broad mite (Polyphagotarsonemus latus); Tenuipalpidae such as red and black flat mite (Brevipalpus phoenicis); Tuckerellidae; Acaridae such as mould mite (Tyrophagus putrescentiae) and grassland mite (Tyrophagus similis); and Pyroglyphidae such as American house dust mite (Dermatophagoides farinae) and European house dust mite (Dermatophagoides pteronyssinus).

Insect pests of the order Lepidoptera, such as Crambidae such as adzuki bean borer (Ostrinia scapulalis), Asian corn borer (Ostrinia furnacalis), yellow stem borer (Scirpophaga incertulas), Marasmia exiqua, rice leaf roller (Cnaphalocrocis medinalis), bluegrass webworm (Pediasia teterrellus), striped rice stemborer (Chilo suppressalis), cabbage webworm (Hellula undalis), grape leafroller (Herpetogramma luctuosale), cotton leaf roller (Haritalodes derogata), dark-headed stem borer (Chilo polychrysus), rice caseworm (Nymphula depunctalis), rice white stemborer (Scirpophaga innotata), sugarcane borer (Diatraea saccharalis), eggplant fruit and shoot borer (Leucinodes orbonalis) and European corn worm (Ostrinia nubilalis); Pyralidae such as Mediterranean flour moth (Ephestia kuehniella), dried currant moth (Cadra cautella), Indian meal moth (Plodia interpunctella), persimmon bark borer (Euzophera batangensis) and lesser cornstalk borer (Elasmopalpus lignosellus); Noctuidae such as African armyworm (Spodoptera exempta), rice armyworm (Mythimna separata), rice looper (Plusia festucae), pink rice stem borer (Sesamia inferens), beet armyworm (Spodoptera exigua), lawn armyworm (Spodoptera Mauritia), beet semi-looper (Autographa nigrisigna), black cutworm (Agrotis ipsilon), fall armyworm (Spodoptera frugiperda), tobacco budworm (Heliothis virescens), tabacco cutworm (Spodoptera litura), rice green caterpillar (Naranga aenescens), cabbage moth (Mamestra brassicae), southern armyworm (Spodoptera eridania), soybean looper (Chrysodeixis includens), Spodoptera cosmioides, Trichoplusia spp., corn earworm (Helicoverpa zea), cotton bollworm (Helicoverpa armigera), cabbage looper (Trichoplusia ni), turnip moth (Agrotis segetum), lawn cutworm (Spodoptera depravata), oriental tobacco budworm (Helicoverpa assulta), sweet potato leaf worm (Aedia leucomelas), African pink stem borer (Sesamia calamistis), cotton leafworm (Alabama argillacea), hop vine borer moth (Hydraecia immanis), soybean looper (Pseudoplusia includens) and velvetbean caterpillar (Anticarsia gemmatalis); Pieridae such as large white (Pieris brassicae) and small white (Pieris rapae); Tortricidae such as Matsumuraeses azukivora, apple leafroller (Archips xylosteanus), grey borer of sugarcane (Tetramoera schistaceana), codling moth (Cydia pomonella), Grapholita dimorpha, smaller tea tortrix (Adoxophyes honmai), oriental tea tortrix moth (Homona magnanima), oriental fruit moth (Grapholita molesta), soybean pod borer (Leguminivora glycinivorella), apple tortrix (Archips fuscocupreanus), summer fruit tortrix (Adoxophyes orana fasciata), citrus fruit borer (Citripestis sagittiferella), European grapevine moth (Lobesia botrana) and Epinotia aporema; Gracillariidae such as apple leafminer (Phyllonorycter ringoniella), tea leaf roller (Caloptilia theivora) and citrus leafminer (Phyllocnistis citrella); Carposinidae such as peach fruit moth (Carposina sasakii); Lyonetiidae such as Lyonetia prunifoliella, peach leafminer (Lyonetia clerkella) and coffee leafminer (Leucoptera coffeella); Lymantriidae such as gypsy moth (Lymantria dispar) and tea tussock mot (Euproctis pseudoconspersa); Plutellidae such as diamondback moth (Plutella xylostella); Gelechiidae such as Helcystogramma triannulella, potato tuber moth (Phthorimaea operculella), tomato leafminer (Tuta absoluta), peach twig borer (Anarsia lineatella) and pink bollworm (Pectinophora gossypiella); Arctiidae such as fall webworm (Hyphantria cunea); Castniidae such as giant sugarcane borer (Telchin licus); Cossidae such as carpenter moth (Cossus insularis); Geometridae such as plum cankerworm (Cystidia couaggaria) and mugwort looper (Ascotis selenaria); Limacodidae such as Parasa consocia, Monema flavescens and blue-striped nettle grub (Parasa lepida); Stathmopodidae such as persimmon fruit moth (Stathmopoda masinissa); Sphingidae such as greater death's head hawkmoth (Acherontia lachesis); Sesiidae such as Nokona feralis, cherry tree borer (Synanthedon hector) and Synanthedon tenuis; Hesperiidae such as rice skipper (Parnara guttata); Tineidae such as clothes moth (Tinea translucens) and common clothes moth (Tineola bisselliella); and Papilionidae such as Asian swallowtail (Papilio Xuthus) and old world swallowtail (Papilio machaon hippocrates).

Nematodes, such as Aphelenchoididae such as rice white tip nematode (Aphelenchoides besseyi); Pratylenchidae such as cobb root-lesion nematode (Pratylenchus penetrans), coffee root lesion nematode (Pratylenchus coffeae), California meadow nematode (Pratylenchus neglectus), Pratylenchus brachyrus and Radopholus similis; Heteroderidae such as northern root-knot nematode (Meloidogyne hapla), southern rook-knot nematode (Meloidogyne incognita), Javanese root-knot nematode (Meloidogyne iavanica), potato cyst nematode (Globodera rostochiensis), white potato cyst nematode (Globodera pallida), soybean cyst nematode (Heterodera glycines) and guava root-knot nematode (Meloidogyne enterolobii); Hoplolaimidae such as Rotylenchulus reniformis; Anguinidae such as strawberry bud nematode (Nothotylenchus acris) and stem and bulb nematode (Ditylenchus dipsaci); Tylenchulidae such as citrus root nematode (Tylenchulus semipenetrans); Longidoridae such as dagger nematode (Xiphinema index); Trichodoridae; and Parasitaphelenchidae such as pine wood nematode (Bursaphelenchus xylophilus).

The agricultural and horticultural insecticide, miticide, nematicide or soil pesticide, containing the compound of the present invention, is especially effective in controlling plant-parasitic mites, agricultural pests, plant-parasitic nematodes, or the like, more highly effective in controlling plant-parasitic mites and agricultural pests, and is exceedingly useful as insecticides or miticides.

Furthermore, the agricultural and horticultural insecticide, miticide, nematicide or soil pesticide, containing the compound of the present invention, is effective also in controlling various pests that have acquired resistance to existing chemicals such as organophosphorus pesticides, carbamate pesticides, synthetic pyrethroid pesticides, neonicotinoid pesticides, or the like.

Further, since the compounds of the present invention have excellent systemic property, not only soil insect pests, mites, nematodes, gastropods, isopods, and the like, but also pests living in or on the stalks and leaves can be controlled by treating the soil with the agricultural and horticultural insecticide, miticide, nematicide or soil pesticide, containing the compound of the present invention.

Other preferred embodiments of the insecticide, the miticide, the nematocide and the soil pesticide, containing the compound of the present invention, may be agricultural and horticultural insecticides, miticides, nematicides and soil pesticides for comprehensively controlling the above-described agricultural pests, gastropods, plant-parasitic mites, plant-parasitic nematodes, soil insect pests, and the like.

The pest control agent containing the compound of the present invention may be used for agriculturally and horticulturally useful plants, such as fruit vegetables such as tomato, cherry tomato, sweet pepper, eggplant, cucumber, zucchini, watermelon, melon, pumpkin, okra, hot pepper, Oriental pickling melon, wax gourd, bitter melon and Oriental melon; leaf vegetables such as lettuce, leaf lettuce, spinach, cabbage, onion, garlic, asparagus, broccoli, cauliflower, Chinese cabbage, Malabar spinach, leek, Potherb mustard, Perilla, Komatsuna and celery; root vegetables such as potato, sweet potato, Japanese radish, carrot, burdock, Lotus root, Eddoe, Konjac, Japanese yam, ginger, turnip, allium chinense, Myoga and Cirsium dipsacolepis; beans such as soybean, adzuki bean, pea, kidney bean, peanut, broad bean and Edamame; cereal crops such as rice, wheat, oat, rye and corn, fruit trees; fruits and nuts such as apple, citrus fruits, pear, grape, strawberry, walnut, almond, banana, fig, pineapple, peach, nectarine, cherry, apricot, coconut, avocado, persimmon, plum, pistachio, kiwifruit and loquat; lawn such as Zoysia tenuifolia and bentgrass; flowers and ornamental plants such as daisy, lily, rose, carnation, dahlia, gerbera, saintpaulia, tulip, daffodil, petunia, snowflake, peony, cyclamen, rose balsam and Eustoma; flowering trees such as azalea; industrial crops such as cotton, rapeseed, beet, tea, tobacco, coffee, olive, sugar cane, hop, sesame, parsley, thymus, rosemary, basil and lavender; foliage plants such as orchidaceae, cactus and fern; and Pasture plants such as clover, alfalfa, orchard grass and Johnson grass.

Such plants include plants having, imparted by classical breeding methods, resistance to a herbicide (for example, a HPPD inhibitor such as isoxaflutole; an ALS inhibitor such as imazethapyr or thifensulfuron-methyl; an EPSP synthase inhibitor such as glyphosate; a glutamine synthase inhibitor such as glufosinate; an acetyl CoA carboxylase inhibitor such as sethoxydim; bromoxynil; dicamba; or 2,4-D). The plants further include transgenic plants generated by gene modification or gene editing. Examples of the transgenic plants include herbicide-resistant transgenic plants, noxious insect-resistant transgenic plants, transgenic plants relating to plant components, and phytopathogen-resistant transgenic plants.

The pest control agent containing the compound of the present invention may be obtained by mixing the compound with additives and applied in the form of various formulations such as dusts, granules, water dispersible granules, wettable powders, water-based suspensions, oil-based suspensions, water soluble granules, water soluble powders, emulsifiable concentrates, liquid, pastes, aerosols and ultra low-volume formulations. It may be formed into any usual formulation used in this field, so long as the object of the present invention is thereby met. The additives to be used for formulation include, for example, a solid carrier such as diatomaceous earth, slaked lime, calcium carbonate, talc, white carbon, kaoline, bentonite, kaolinite, sericite, clay, sodium carbonate, sodium bicarbonate, salt cake, zeolite or starch; a liquid carrier such as water, toluene, xylene, solvent naphtha, dioxane, acetone, isophorone, methyl isobutyl ketone, chlorobenzene, cyclohexane, dimethyl sulfoxide, N,N-dimethylformamide, dimethylacetamide, N-methyl-2-pyrrolidone or an alcohol; an anionic surfactant such as a salt of fatty acid, a benzoate, an alkylsulfosuccinate, a dialkylsulfosuccinate, a polycarboxylate, a salt of alkylsulfuric acid ester, an alkyl sulfate, an alkylaryl sulfate, an alkyl diglycol ether sulfate, a salt of alcohol sulfuric acid ester, an alkyl sulfonate, an alkylaryl sulfonate, an aryl sulfonate, a lignin sulfonate, an alkyldiphenyl ether disulfonate, a polystyrene sulfonate, a salt of alkylphosphoric acid ester, an alkylaryl phosphate, a styrylaryl phosphate, a salt of polyoxyethylene alkyl ether sulfuric acid ester, a polyoxyethylene alkylaryl ether sulfate, a salt of polyoxyethylene alkylaryl ether sulfuric acid ester, a polyoxyethylene alkyl ether phosphate, a salt of polyoxyethylene alkylaryl phosphoric acid ester or a salt of naphthalene sulfonate condensed with formaldehyde; a nonionic surfactant such as a sorbitan fatty acid ester, a glycerin fatty acid ester, a fatty acid polyglyceride, a fatty acid alcohol polyglycol ether, acetylene glycol, acetylene alcohol, an oxyalkylene block polymer, a polyoxyethylene alkyl ether, a polyoxyethylene alkylaryl ether, a polyoxyethylene styrylaryl ether, a polyoxyethylene glycol alkyl ether, a polyethylene glycol, a polyoxyethylene fatty acid ester, a polyoxyethylene sorbitan fatty acid ester, a polyoxyethylene glycerin fatty acid ester, a polyoxyethylene hydrogenated castor oil or a polyoxypropylene fatty acid ester; or a vegetable oil or mineral oil such as olive oil, kapok oil, castor oil, palm oil, camellia oil, coconut oil, sesame oil, corn oil, rice bran oil, peanut oil, cottonseed oil, soybean oil, rapeseed oil, linseed oil, tung oil or liquid paraffins; or silicone oil. These additive components may suitably be selected for use alone or in combination as a mixture of two or more of them, so long as the object of the present invention is met. Further, in addition to the above, various additives known in this technical field, such as a filler, a thickener, an anti-settling agent, an anti-freezing agent, a dispersion stabilizer, a safener and an anti-mold agent, may be used. The mixing ratio (weight ratio) of the compound of the present invention to such various additives is usually from 0.001:99.999 to 95:5, preferably from 0.005:99.995 to 90:10. In the actual application of such a formulation, it may be used as it is, or may be diluted to a predetermined concentration with a diluent such as water, and may be mixed with various spreaders (e.g. surfactants, vegetable oils or mineral oils), as the case requires.

Application of the pest control agent containing the compound of the present invention cannot generally be defined, as it varies depending upon the weather conditions, the type of the formulation, the application season, the application site, the type or degree of outbreak of pests, etc. However, it is carried out at an active ingredient concentration of generally from 0.1 to 50,000 ppm, preferably from 1 to 30,000 ppm, and the dose per unit area is such that the amount of the compound of the present invention is from 0.01 to 50,000 g, preferably from 1 to 30,000 g, per hectare. The present invention includes method for controlling pests, mites, nematodes or soil insect pests, particularly for controlling plant-parasitic mites, agricultural pests or plant-parasitic nematodes, by such application methods.

Various formulations of the pest control agent containing the compound of the present invention or dilutions thereof can be usually applied by a conventional application method. That is, the formulations or dilutions can be applied by spray application (spraying, misting, atomizing, granule scattering, application to water surface, or the like), soil application (mixing, irrigation, or the like), surface application (coating, dust coating, covering, or the like), use of an impregnated toxic bait, or the like. It is also possible to use a method in which the active ingredient is supplied as mixed with a feed to a farm animal to inhibit pests, particularly, insect pests, from breeding and growing on the excreta. Furthermore, the active ingredient may also be applied by a so-called ultra low volume application method. In this method, the active ingredient can be contained at a concentration of 100%.

For example, in a case where the pest control agent containing the compound of the present invention is applied to a plant, by applying an effective amount of the pest control agent directly to pests such as insect pests, mites, nematodes or soil insects pests and/or to soil or a plant body, the pests can be controlled. The plant body may be above-ground parts (e.g. stems, leaves, trunks), below-ground parts (e.g. roots) or seeds of a plant to which the pest control agent is to be applied. The seeds mean diaspores (so-called seeds) formed by sexual reproduction, plant organs (e.g. tubers, scaly bulbs, corms, rhizomes and seed tubers), and scions of fruit trees, flowering trees, flowers and ornamental plants (for example, branches of grape which is a fruit tree).

In such a case, the pest control agent containing the compound of the present invention may be applied, for example, by soil treatment, foliar treatment, irrigation treatment, seed treatment, trunk injection or nursery box application. In the case of soil treatment, foliar treatment or irrigation treatment, the application amount is such that the amount of the compound of the present invention is from 0.01 g to 50,000 g, preferably from 1 g to 30,000 g, per hectare. In the case of seed treatment, the application amount is such that the amount of the compound of the present invention is from 0.01 g to 200 g, preferably from 0.05 g to 100 g per 1 kg of seeds. In the case of nursery box application, the application amount is such that the amount of the compound of the present invention is from 0.001 g to 3,000 g, preferably from 0.01 g to 1,000 g per nursery box. In the case of trunk injection, the application amount is such that the amount of the compound of the present invention is from 0.001 g to 5,000 g, preferably from 0.01 g to 3,000 g per tree.

The soil treatment may, for example, be a method wherein the compound of the present invention is formulated into a liquid formulation (liquid, water-based suspension, oil-based suspension, emulsifiable concentrate or the like) or a solid formulation (granules, dusts, wettable powder, water soluble powder, water dispersible granules, water soluble granules or the like), and the formulation as it is or after diluted with water is applied over the entire surface of the soil and mixed, before planting of the plant body or before sowing; the formulation is spread into holes made to plant the plant body (planting holes); or the formulation is spread into furrows made with a certain width for sowing or planting of the plant body.

The foliar treatment may, for example, be a method wherein the compound of the present invention is formulated into a liquid formulation (liquid, water-based suspension, oil-based suspension, emulsifiable concentrate or the like) or a solid formulation

(wettable powder, water soluble powder, water dispersible granules, water soluble granules or the like), and the formulation is sprayed after diluted with water to the entire plant body.

The irrigation treatment may, for example, be a method wherein the compound of the present invention is formulated into a liquid formulation (liquid, water-based suspension, oil-based suspension or the like) or a solid formulation (wettable powder, water soluble powder, water dispersible granules, water soluble granules or the like), and the formulation as it is or after diluted with water is applied by irrigation to a seedling container, to the plant foot during cultivation, or to a vicinity thereof.

The seed treatment may, for example, be a method wherein the compound of the present invention is formulated into a liquid formulation (water-based suspension, oil-based suspension, emulsifiable concentrate, liquid or the like) or a solid formulation (dusts, wettable powder, water soluble powder, water dispersible granules, water soluble granules or the like), and the formulation as it is or after diluted with water is mixed with seeds to attach the compound of the present invention to the surface of the seeds; the formulation is mixed with seeds together with a coating material and attached to the surface of the seeds; the formulation is sprayed and attached to the surface of seeds; or seeds are dipped in the formulation and infiltrated with the chemical.

The nursery box application may, for example, be a method wherein the compound of the present invention is formulated into a solid formulation (granules, dusts or the like) or a liquid formulation (water-based suspension, oil-based suspension, liquid or the like), and the formulation as it is or after diluted with water is applied to a nursery box before sowing, after sowing, before and after sowing, at the time of cultivation in the nursery box, or before planting into paddies; or a method wherein when seeds are sown into a nursery box, the compound of the present invention is mixed with culture soil, for example, the compound of the present invention is formulated into a solid formulation (granules, dusts, water dispersible granules or the like), and the formulation is mixed with the entire seedbed soil, cover soil or culture soil.

The trunk injection may, for example, be a method wherein the compound of the present invention is formulated into a liquid formulation (liquid or the like), and is injected as it is into a plant body through a hole made on the trunk.

The agricultural and horticultural insecticide, miticide, nematicide or soil pesticide, containing the compound of the present invention, may be mixed with or used in combination with other agricultural chemicals, fertilizers, safeners, or the like, whereby more excellent effects or activities may sometimes be obtained.

Mixing with or use in combination with other components means that the compound of the present invention and other components are used simultaneously, separately or with an interval.

Examples of other agricultural chemicals include herbicides, insecticides, miticides, nematicides, soil pesticides, fungicides, antivirus agents, attractants, antibiotics, plant hormones and plant growth regulators. Particularly, with an insecticidal composition, a miticidal composition, a nematicidal composition or a soil pesticidal composition in which the compound of the present invention is mixed with or used in combination with one or more active ingredient compounds for other pesticides, preferable improvements in application range, application time for pesticide treatment, control activity, or the like may be achieved. The compound of the present invention and the active ingredient compounds for other agricultural chemicals may be separately formulated and thereafter mixed together just before application, or may be formulated together. Such insecticidal compositions, miticidal compositions, nematicidal compositions and soil pesticidal compositions are included in the present invention.

The mixing ratio of the compound of the present invention to the active ingredient compounds of other insecticide and/or nematicide can not generally be defined, as it varies depending upon the weather conditions, the type of the formulation, the crop plant to be treated, the application time, the application site, the type or degree of outbreak of pests or nematodes, etc. However, it is usually within a range of from 1:300 to 300:1, preferably from 1:100 to 100:1, by weight ratio. Further, the dose for the application is such that the total amount of the active ingredient compounds is from 0.1 to 70,000 g, preferably from 5 to 50,000 g, per hectare. The present invention includes a method for controlling pests or nematodes by application of such a mixed insecticidal or nematicidal composition.

The active ingredient compounds of an insecticide, a miticide, a nematicide or a soil insect pesticide, that is a pesticide, in the above other agricultural chemicals may properly be selected, for example, from the following group of compounds (by common names or test codes of Japan Plant Protection Association). In a case where these compounds have their salts, alkyl esters, various structural isomers such as optical isomers, etc., all of them are included, even if no specific disclosure thereof is made.

Organic phosphate compounds such as profenofos, dichlorvos, fenamiphos, fenitrothion, EPN ((RS)-(O-ethyl-O-4-nitrophenyl phenylphosphonothioate)), diazinon, chlorpyrifos, chlorpyrifos-methyl, acephate, prothiofos, fosthiazate, cadusafos, disulfoton, isoxathion, isofenphos, ethion, etrimfos, quinalphos, dimethylvinphos, dimethoate, sulprofos, thiometon, vamidothion, pyraclofos, pyridaphenthion, pirimiphosmethyl, propaphos, phosalone, formothion, malathion, tetrachlorvinphos, chlorfenvinphos, cyanophos, trichlorfon, methidathion, phenthoate, oxydeprofos (another name: ESP), azinphos-methyl, fenthion, heptenophos, parathion, phosphocarb, demeton-S-methyl, monocrotophos, methamidophos, imicyafos, parathion-methyl, terbufos, phosphamidon, phosmet and phorate;
carbamate compounds such as carbaryl, propoxur, aldicarb, carbofuran, thiodicarb, methomyl, oxamyl, ethiofencarb, pirimicarb, fenobucarb, carbosulfan, benfuracarb, bendiocarb, furathiocarb, isoprocarb, metolcarb, xylylcarb, XMC (3,5-xylyl methylcarbamate) and fenothiocarb;
nereistoxin derivatives such as cartap, thiocyclam, thiocyclam oxalate, thiocyclam hydrochloride, bensultap, thiosultap, monosultap (another name: thiosultapmonosodium), bisultap (another name: thiosultap-disodium) and polythialan;
organic chlorine compounds such as dicofol, tetradifon, endosulfan, dienochlor, dieldrin and methoxychlor;
organic tin compounds such as fenbutatin oxide, cyhexatin and azocyclotin;
pyrethroid compounds such as fenvalerate, permethrin, cypermethrin, alphacypermethrin, zeta-cypermethrin, theta-cypermethrin, beta-cypermethrin, deltamethrin, cyhalothrin, gamma-cyhalothrin, lambda-cyhalothrin, tefluthrin, kappa-tefluthrin, ethofenprox, flufenprox, cyfluthrin, beta-cyfluthrin, fenpropathrin, flucythrinate, fluvalinate, cycloprothrin, pyrethrins, esfenvalerate, tetramethrin, resmethrin, protrifenbute, bifenthrin, kappa-bifenthrin, acrinathrin, allethrin, tau-fluvalinate, tralomethrin, profluthrin, metofluthrin, epsilon-metofluthrin, heptafluthrin, phenothrin, flumethrin, momfluorothrin, epsilon-momfluorothrin, silafluofen and chloroprallethrin;
benzoylurea compounds such as diflubenzuron, chlorfluazuron, teflubenzuron, flufenoxuron, lufenuron, novaluron, triflumuron, hexaflumuron, bistrifluron, noviflumuron, fluazuron and flufenoxuron;
juvenile hormone-like compounds such as methoprene, pyriproxyfen, fenoxycarb and diofenolan;
pyridazinone compounds such as pyridaben;
pyrazole compounds such as fenpyroximate, cyenopyrafen and cyetpyrafen;
phenylpyrazole compounds such as acetoprole, ethiprole, fipronil, flufiprole, pyrafluprole, pyriprole, nicofluprole and vaniliprole;
pyrazole carboxamide compounds such as pyflubumide, tebufenpyrad, tolfenpyrad and dimpropyridaz;
pyridyl pyrazole compounds such as chlorantraniliprole, cyantraniliprole, cyclaniliprole, tetrachlorantraniliprole, tetraniliprole, tyclopyrazoflor, fluchlordiniliprole and tiorantraniliprole;
neonicotinoid compounds such as imidacloprid, nitenpyram, acetamiprid, thiacloprid, thiamethoxam, clothianidin, dinotefuran and nithiazine;
hydrazine compounds such as tebufenozide, methoxyfenozide, chromafenozide and halofenozide;
pyridine compounds such as pyridalyl and flonicamid;
tetronic acid compounds such as spirodiclofen, spiromesifen and spirobudifen;
tetramic acid compounds such as spirotetramat, spiropidion and spidoxamat;
strobilurin compounds such as fluacrypyrim, pyriminostrobin and flupyroxystrobin;
pyrimidinamine compounds such as flufenerim and pyrimidifen;
organosulfur compounds such as malathion;
triazine compounds such as cyromazine;
hydrazone compounds such as hydramethylnon;
phthalamide compounds such as flubendiamide and cyhalodiamide;
meta-diamide compounds such as broflanilide, cyproflanilide and modoflaner;
thiourea compounds such as diafenthiuron and chloromethiuron;
formamidine compounds such as amitraz, chlordimeform and chloromebuform;
pyridine azomethine compounds such as pymetrozine and pyrifluquinazone;
isoxazoline compounds such as afoxolaner, fluralaner, fluxametamide, sarolaner, isocycloseram, umifoxolaner, tigolaner and mivorilaner:
   fluoroalkenyl compounds such as fluensulfone and trifluenfuronate;
   mesoionic compounds such as dicloromezotiaz, triflumezopyrim and fenmezoditiaz;
   pyropen compounds such as afidopyropen; and
   other compounds such as buprofezin, hexythiazox, triazamate, chlorfenapyr, indoxacarb, acequinocyl, etoxazole, 1,3-dichloropropene, benclothiaz, bifenazate, propargite, clofentezine, metaflumizone, cyflumetofen, fenazaquin, amidoflumet, sulfluramid, hydramethylnon, metaldehyde, sulfoxaflor, verbutin, fluhexafon, tioxazafen, flometoquin, flupyradifurone, fluazaindolizine, acynonapyr, benzpyrimoxan, flupyrimin, oxazosulfyl, cyclobutrifluram, flupentiofenox and indazapyroxamet.

Further, the compound of the present invention may be mixed with or used in combination with the following compounds.

Microbial pesticides such as insecticidal crystal proteins produced by Bacillus thuringiensis aizawai, Bacillus thuringiensis kurstaki, Bacillus thuringiensis israelensis, Bacillus thuringiensis japonensis, Bacillus thuringiensis tenebrionis, Bacillus thuringiensis, Paecilomyces lilacinus, Bacillus methylotrophicus, Bacillus subtilis, Bacillus amyloliquefaciens or Bacillus licheniformis, insect viruses, entomopathogenic fungi, and nematophagous fungi;
antibiotics or semisynthetic antibiotics such as abamectin, emamectin benzoate, ivermectin, milbemectin, milbemycin oxime, lepimectin, spinosad and spinetoram;
natural products such as azadirachtin, rotenone and ryanodine;
repellents such as deet;
physical preservatives such as a paraffin oil and a mineral oil; and
RNAi drugs such as ledprona.

The active ingredient compounds of a fungicide in the above-mentioned other agricultural chemicals may properly be selected, for example, from the following group of compounds (by common names or test codes of Japan Plant Protection Association). In a case where these compounds have their salts, alkyl esters, various structural isomers such as optical isomers, etc., all of them are included, even if no specific disclosure thereof is made.
Anilinopyrimidine compounds such as mepanipyrim, pyrimethanil and cyprodinil;
triazolopyrimidine compounds such as ametoctradin;
triazolobenzothiazole compounds such as tricyclazole;
pyridinamine compounds such as fluazinam;
azole compounds such as triadimefon, bitertanol, triflumizole, etaconazole, propiconazole, penconazole, flusilazole, myclobutanil, cyproconazole, tebuconazole, hexaconazole, furconazole-cis, prochloraz, metconazole, epoxiconazole, tetraconazole, oxpoconazole fumarate, prothioconazole, triadimenol, flutriafol, difenoconazole, fluquinconazole, fenbuconazole, bromuconazole, diniconazole, simeconazole, pefurazoate, ipconazole, imibenconazole, azaconazole, triticonazole, imazalil, ipfentrifluconazole, mefentrifluconazole and fluoxytioconazole;
quinoxaline compounds such as chinomethionat;
dithiocarbamate compounds such as maneb, zineb, mancozeb, polycarbamate, metiram, propineb and thiram;
organic chlorine compounds such as phthalide, chlorothalonil and quintozene;
benzimidazole compounds such as benomyl, thiophanate-methyl, carbendazim, thiabendazole and fuberiazole;
cyanoacetamide compounds such as cymoxanil;
phenylamide compounds such as metalaxyl, metalaxyl-M (another name: mefenoxam), oxadixyl, ofurace, benalaxyl, benalaxyl-M (another name: kiralaxyl, chiralaxyl), furalaxyl and valifenalate;
anilide compounds such as cyprofuram, carboxin, oxycarboxin, thifluzamide, boscalid, fenhexamid, isotianil, tiadinil and pyraziflumid;
sulfamide (sulfenic acid) compounds such as dichlofluanid and tolylfluanid;
copper compounds such as cupric hydroxide, oxine copper, anhydrous copper sulfate, copper nonylphenol sulfonate, copper 8-hydroxyquinoline and dodecylbenzenesulfonic acid bisethylenediamine copper(II) salt (another name: DBEDC);
organophosphorus compounds such as fosetyl-Al, tolclofos-Methyl, edifenphos and iprobenfos;
phthalimide compounds such as captan, captafol and folpet;
dicarboxyimide compounds such as procymidone, iprodione and vinclozolin;
benzanilide compounds such as flutolanil, mepronil, benodanil and flufenoxadiazam;
amide compounds such as carpropamid, diclocymet, silthiopham and fenoxanil;
pyrazolecarboxamide compounds such as benzovindiflupyr, bixafen, fluindapyr, fluxapyroxad, furametpyr, isopyrazam, penflufen, penthiopyrad, pydiflumetofen, sedaxane, isoflucypram, inpyrfluxam, pyrapropoyne and flubeneteram;
benzamide compounds such as fluopicolide, fluopyram, zoxamide and fluopimomide;
furanilide compounds such as fenfuram;
thiophenamide compounds such as isofetamid;
piperazine compounds such as triforine;
pyridine compounds such as pyrifenox, pyrisoxazole and aminopyrifen;
pyrimidine compounds such as fenarimol, ferimzone, nuarimol and flumethylsulforim;
piperidine compounds such as fenpropidin;
morpholine compounds such as fenpropimorph and tridemorph;
organic tin compounds such as fentin hydroxide and fentin acetate;
urea compounds such as pencycuron;
carboxylic acid amide compounds such as dimethomorph, flumorph, pyrimorph, iprovalicarb, benthiavalicarb-isopropyl and mandipropamid;
phenylcarbamate compounds such as diethofencarb;
cyanopyrrole compounds such as fludioxonil and fenpiclonil;
strobilurin compounds such as azoxystrobin, kresoxim-methyl, metominostrobin, trifloxystrobin, picoxystrobin, orysastrobin, dimoxystrobin, pyraclostrobin, fluoxastrobin, pyraoxystrobin, pyrametostrobin, coumoxystrobin, enoxastrobin, fenaminstrobin, flufenoxystrobin, triclopyricarb and mandestrobin;
oxazole compounds such as famoxadone, oxathiapiprolin and fluoxapiprolin;
thiazolecarboxamide compounds such as ethaboxam;
imidazolinone compounds such fenamidone;
benzenesulfonamide compounds such as flusulfamide;
oxime ether compounds such as cyflufenamid;
anthraquinone compounds such as dithianon;
crotonic acid compounds such as meptyldinocap;
antibiotics such as validamycin, kasugamycin, streptomycin and polyoxins;
guanidine compounds such as iminoctadine, dodine and guazatine;
aliphatic nitrogen-containing compounds such as butylamine and seboctylamine;
quinoline compounds such as tebufloquin, quinoxyfen, quinofumelin and ipflufenoquin;
thiazolidine compounds such as flutianil;
carbamate compounds such as propamocarb hydrochloride, pyribencarb and tolprocarb;
tetrazole compounds such as picarbutrazox and metyltetraprole;
sulfonamide compounds such as amisulbrom and cyazofamid;
allyl phenyl ketone compounds such as metrafenone and pyriofenone;
benzothiazole compounds such as probenazole and dichlobentiazox;
phenylpyrazole compounds such as fenpyrazamine;
dithiolane compounds such as isoprothiolane;
picolinamide compounds such as fenpicoxamid, florylpicoxamid and metarylpicoxamid;
pyridazine compounds such as pyridachlometyl;
sulfur compounds such as sulfur and lime sulfur;
hydrazide compounds such as chloroinconazide;
other compounds such as pyroquilon, diclomezine, chloropicrin, dazomet, metamsodium, proquinazid, spiroxamine and dipymetitrone;
microbial fungicides such as Bacillus amyloliqefaciens strain QST713, Bacillus amyloliqefaciens strain FZB24, Bacillus amyloliqefaciens strain MBI600, Bacillus amyloliqefaciens strain D747, Pseudomonas fluorescens, Bacillus subtilis and Trichoderma atroviride SKT-1; and
plant extracts such as tea tree oil.

Preferred embodiments of the present invention will be described below. However, it should be understood that the present invention is by no means restricted thereto.
(1) A pyridazinone compound represented by the formula (I) or a salt thereof.
(2) The pyridazinone compound or a salt thereof according to (1), wherein
   Y and R^{A} are each a halogen, a (C₁-C₄)alkyl, a (C₂-C₄)alkenyl, a (C₂-C₄)alkynyl, a (C₃-C₆)cycloalkyl, a (C₁-C₄)haloalkyl, a (C₁-C₄)alkoxy, a (C₁-C₄)haloalkoxy, a (C₁-C₄)alkylthio, a (C₁-C₄)alkylsulfinyl, a (C₁-C₄)alkylsulfonyl, a (C₁-C₄)haloalkylthio, a (C₁-C₄)haloalkylsulfinyl, a (C₁-C₄)haloalkylsulfonyl, hydroxy, cyano, nitro, -SFs, -NR^{B}R^{C}, - C(=O)R^{D} or -C(=O)NR^{E}R^{F};
   Z is a (C₁-C₅)alkyl, a (C₂-C₄)alkenyl, a (C₂-C₄)alkynyl, a (C₃-C₆)cycloalkyl, a (C₁-C₄)alkoxy(C₁-C₄)alkyl, a (C₃-C₆)cycloalkyl(C₁-C₄)alkyl or a (C₁-C₄)haloalkyl;
   R¹, R², R³ and R⁴ are each a halogen, a (C₁-C₄)alkyl, a (C₂-C₄)alkenyl, a (C₂-C₄)alkynyl, a (C₃-C₆)cycloalkyl, a (C₁-C₄)haloalkyl, a (C₁-C₄)alkoxy, a (C₃-C₆)cycloalkoxy, a (C₁-C₄)haloalkoxy, a (C₁-C₄)alkylthio, a (C₁-C₄)alkylsulfinyl, a (C₁-C₄)alkylsulfonyl, a (C₁-C₄)haloalkylthio, a (C₁-C₄)haloalkylsulfinyl, a (C₁-C₄)haloalkylsulfonyl, hydroxy, cyano, nitro, -NR^{G}R^{H}, -C(=O)R^{I}, -C(=O)NR^{J}R^{k,} - C(=NOR^{L})R^{M}, dimethoxymethyl, 1,3-dioxan-2-yl or 1,3-dioxolan-2-yl;
   R^{B}, R^{C}, R^{E}, R^{F}, R^{G}, R^{H}, R^{J} and R^{K} are each a hydrogen atom, a (C₁-C₄)alkyl or a (C₁-C₄)haloalkyl;
   R^{D} and R^{I} are each a hydrogen atom, hydroxy, a (C₁-C₄)alkyl, a (C₁-C₄)haloalkyl or a (C₁-C₄)alkoxy; and
   R^{L} and R^{M} are each a hydrogen atom or a (C₁-C₄)alkyl.
(3) The pyridazinone compound or a salt thereof according to (1), wherein
   Y and R^{A} are each a halogen, a (C₁-C₄)alkyl, a (C₂-C₄)alkenyl, a (C₂-C₄)alkynyl, a (C₃-C₆)cycloalkyl, a (C₁-C₄)haloalkyl, a (C₁-C₄)alkoxy, a (C₁-C₄)haloalkoxy, a (C₁-C₄)alkylsulfonyl, a (C₁-C₄)haloalkylthio, a (C₁-C₄)haloalkylsulfinyl, a (C₁-C₄)haloalkylsulfonyl, cyano, nitro or -C(=O)R^{D};
   Z is a (C₁-C₅)alkyl, a (C₃-C₆)cycloalkyl, a (C₁-C₄)alkoxy(C₁-C₄)alkyl or a (C₁-C₄)haloalkyl;
   R¹, R², R³ and R⁴ are each a halogen, a (C₁-C₄)alkyl, a (C₂-C₄)alkynyl, a (C₃-C₆)cycloalkyl, a (C₁-C₄)haloalkyl, a (C₁-C₄)alkoxy, a (C₁-C₄)haloalkoxy, a (C₁-C₄)alkylthio, a (C₁-C₄)alkylsulfinyl, a (C₁-C₄)alkylsulfonyl, cyano, nitro, -NR^{G}R^{H}, - C(=O)R^{I}, -C(=NOR^{L})R^{M}, dimethoxymethyl, 1,3-dioxan-2-yl or 1,3-dioxolan-2-yl;
   R^{G} and R^{H} are each a hydrogen atom or a (C₁-C₄)alkyl;
   R^{D} and R^{I} are each a hydrogen atom, a (C₁-C₄)alkyl or a (C₁-C₄)alkoxy; and
   R^{L} and R^{M} are each a hydrogen atom or a (C₁-C₄)alkyl.
(4) The pyridazinone compound or a salt thereof according to (1), wherein
   Y and R^{A} are each a halogen, a (C₁-C₄)alkyl, a (C₂-C₄)alkenyl, a (C₂-C₄)alkynyl, a (C₃-C₆)cycloalkyl, a (C₁-C₄)haloalkyl, a (C₁-C₄)alkoxy, a (C₁-C₄)haloalkoxy, a (C₁-C₄)alkylsulfonyl, cyano, nitro or -C(=O)R^{D};
   Z is a (C₁-C₄)alkyl, a (C₃-C₆)cycloalkyl, a (C₁-C₄)alkoxy(C₁-C₄)alkyl or a (C₁-C₄)haloalkyl;
   R¹, R², R³ and R⁴ are each a halogen, a (C₁-C₄)alkyl, a (C₂-C₄)alkynyl, a (C₃-C₆)cycloalkyl, a (C₁-C₄)haloalkyl, a (C₁-C₄)alkoxy, a (C₁-C₄)haloalkoxy, a (C₁-C₄)alkylthio, a (C₁-C₄)alkylsulfinyl, a (C₁-C₄)alkylsulfonyl, a (C₁-C₄)haloalkylthio, a (C₁-C₄)haloalkylsulfinyl, a (C₁-C₄)haloalkylsulfonyl, cyano, nitro, -C(=O)R^{I}, -C(=NOR^{L})R^{M}, dimethoxymethyl, 1,3-dioxan-2-yl or 1,3-dioxolan-2-yl;
   R^{D} is a (C₁-C₄)alkyl;
   R^{I} is a hydrogen atom; and
   R^{L} and R^{M} are each a hydrogen atom or a (C₁-C₄)alkyl.
(5) The pyridazinone compound or a salt thereof according to (1), wherein
   Y and R^{A} are each a halogen, a (C₁-C₄)alkyl, a (C₁-C₄)alkenyl, a (C₁-C₄)alkynyl, a (C₃-C₆)cycloalkyl, a (C₁-C₄)haloalkyl, a (C₁-C₄)alkoxy, a (C₁-C₄)haloalkoxy, a (C₁-C₄)alkylsulfonyl, a (C₁-C₄)haloalkylthio, a (C₁-C₄)haloalkylsulfinyl, a (C₁-C₄)haloalkylsulfonyl, cyano or nitro;
   Z is a (C₁-C₄)alkyl, a (C₃-C₆)cycloalkyl, a (C₁-C₄)alkoxy(C₁-C₄)alkyl or a (C₁-C₄)haloalkyl; and
   R¹, R², R³ and R⁴ are each a halogen, a (C₁-C₄)alkyl, a (C₃-C₆)cycloalkyl, a (C₁-C₄)haloalkyl, a (C₁-C₄)alkoxy, a (C₁-C₄)haloalkoxy, a (C₁-C₄)alkylthio, a (C₁-C₄)alkylsulfinyl or nitro.
(6) The pyridazinone compound or a salt thereof according to (1), wherein
   Y and R^{A} are each a halogen, a (C₁-C₄)alkyl, a (C₁-C₄)alkenyl, a (C₁-C₄)alkynyl, a (C₃-C₆)cycloalkyl, a (C₁-C₄)haloalkyl, a (C₁-C₄)alkoxy, a (C₁-C₄)haloalkoxy, a (C₁-C₄)alkylsulfonyl, a (C₁-C₄)haloalkylthio, a (C₁-C₄)haloalkylsulfinyl, a (C₁-C₄)haloalkylsulfonyl, cyano or nitro;
   Z is a (C₁-C₄)alkyl, a (C₃-C₆)cycloalkyl, a (C₁-C₄)alkoxy(C₁-C₄)alkyl or a (C₁-C₄)haloalkyl; and
   R¹, R², R³ and R⁴ are each a halogen, a (C₁-C₄)alkyl, a (C₃-C₆)cycloalkyl, a (C₁-C₄)haloalkyl, a (C₁-C₄)alkoxy, a (C₁-C₄)haloalkoxy or nitro.
(7) The pyridazinone compound or a salt thereof according to (1) or (2), which has Y bonded at the p-position of the pyridazinone ring, and wherein Y is a halogen, a (C₁-C₄)alkyl, a (C₂-C₄)alkenyl, a (C₂-C₄)alkynyl, a (C₁-C₄)haloalkyl, a (C₁-C₄)alkoxy, a (C₁-C₄)haloalkoxy, a (C₁-C₄)alkylthio, a (C₁-C₄)alkylsulfinyl, a (C₁-C₄)alkylsulfonyl, a (C₁-C₄)haloalkylthio, a (C₁-C₄)haloalkylsulfinyl, a (C₁-C₄)haloalkylsulfonyl, cyano, nitro, -SFs or -C(=O)R^{D}.
(8) The pyridazinone compound or a salt thereof according to (1) or (2), which has Y bonded at the p-position of the pyridazinone ring, and wherein Y is a halogen, a (C₂-C₄)alkynyl, a (C₁-C₄)haloalkyl, a (C₁-C₄)haloalkoxy, a (C₁-C₄)alkylsulfinyl, a (C₁-C₄)alkylsulfonyl, a (C₁-C₄)haloalkylthio, a (C₁-C₄)haloalkylsulfinyl, a (C₁-C₄)haloalkylsulfonyl or cyano.
(9) The pyridazinone compound or a salt thereof according to any one of (1) to (8), wherein Q is Q¹ or Q³.
(10) The pyridazinone compound or a salt thereof according to any one of (1) to (9), wherein X is CH or C(R^{A}).
(11) The pyridazinone compound or a salt thereof according to any one of (1) to (9), wherein X is C(R^{A}).
(12) The pyridazinone compound or a salt thereof according to (11), wherein R^{A} is a halogen, a (C₁-C₄)alkyl, a (C₂-C₄)alkenyl, a (C₂-C₄)alkynyl, a (C₃-C₆)cycloalkyl, a (C₁-C₄)haloalkyl, a (C₁-C₄)alkoxy, a (C₁-C₄)haloalkoxy, cyano, nitro or -C(=O)R^{D}.
(13) The pyridazinone compound or a salt thereof according to (11), wherein R^{A} is a halogen, a (C₁-C₄)alkyl, a (C₂-C₄)alkenyl, a (C₂-C₄)alkynyl, a (C₁-C₄)haloalkyl or a (C₁-C₄)alkoxy.
(14) The pyridazinone compound or a salt thereof according to any one of (1) to (9), wherein X is N.
(15) The pyridazinone compound or a salt thereof according to any one of (1) to (9), wherein X is CH.
(16) The pyridazinone compound or a salt thereof according to any one of (1) to (9), wherein X is C(R^{A}) or N, n is 1 or 2, and at least one Y substitutes at the p-position of the pyridazinone ring.
(17) The pyridazinone compound or a salt thereof according to any one of (1) to (4) and (7) to (16), wherein R¹, R², R³ and R⁴ are each a halogen, a (C₁-C₄)alkyl, a (C₃-C₆)cycloalkyl, a (C₁-C₄)haloalkyl, a (C₁-C₄)alkoxy, a (C₁-C₄)haloalkoxy, a (C₁-C₄)alkylthio, a (C₁-C₄)alkylsulfinyl, a (C₁-C₄)alkylsulfonyl, cyano, nitro, -C(=O)R^{I} or dimethoxymethyl.
(18) The pyridazinone compound or a salt thereof according to any one of (1) to (4) and (7) to (16), wherein R¹, R², R³ and R⁴ are each a halogen, a (C₁-C₄)alkyl, a (C₃-C₆)cycloalkyl, a (C₁-C₄)haloalkyl, a (C₁-C₄)alkoxy, a (C₁-C₄)haloalkoxy, nitro or dimethoxymethyl.
(19) The pyridazinone compound or a salt thereof according to any one of (1), (2) and (7) to (18), wherein Z is a (C₁-C₅)alkyl, a (C₃-C₄)cycloalkyl, a (C₁-C₄)alkoxy(C₁-C₄)alkyl or a (C₁-C₄)haloalkyl.
(20) The pyridazinone compound or a salt thereof according to any one of (1) to (19), wherein n is 1 or 2.
(21) The pyridazinone compound or a salt thereof according to any one of (1) to (19), wherein n is 2.
(22) The pyridazinone compound or a salt thereof according to any one of (1) to (21), wherein a, b, c and d are each 1 or 2.
(23) A pyridazinone compound represented by the formula (i) or a salt thereof.
(24) The pyridazinone compound or a salt thereof according to (23), which is represented by the formula (i-a), (i-c), (i-d) or (i-g).
(25) The pyridazinone compound or a salt thereof according to (23), which is represented by the formula (i-c), (i-d) or (i-g).
(26) The pyridazinone compound or a salt thereof according to (23), wherein a is 1 or 2.
(27) A pyridazinone compound represented by the formula (ii) or a salt thereof.
(28) The pyridazinone compound or a salt thereof according to (27), which is represented by the formula (ii-b) or (ii-c).
(29) The pyridazinone compound or a salt thereof according to (27), wherein b is 1 or 2.
(30) A pyridazinone compound represented by the formula (iii) or a salt thereof.
(31) The pyridazinone compound or a salt thereof according to (30), which is represented by the formula (iii-a) or (iii-c).
(32) The pyridazinone compound or a salt thereof according to (30), which is represented by the formula (iii-c).
(33) The pyridazinone compound or a salt thereof according to (30), wherein c is 1 or 2.
(34) A pyridazinone compound represented by the formula (iv) or a salt thereof.
(35) The pyridazinone compound or a salt thereof according to (34), which is represented by the formula (iv-a) or (iv-c).
(36) The pyridazinone compound or a salt thereof according to (34), which is represented by the formula (iv-c).
(37) The pyridazinone compound or a salt thereof according to (34), wherein d is 1 or 2.
(38) The pyridazinone compound or a salt thereof according to any one of (23) to (37), wherein
   Y and R^{A} are each a halogen, a (C₁-C₄)alkyl, a (C₂-C₄)alkenyl, a (C₂-C₄)alkynyl, a (C₃-C₆)cycloalkyl, a (C₁-C₄)haloalkyl, a (C₁-C₄)alkoxy, a (C₁-C₄)haloalkoxy, a (C₁-C₄)alkylthio, a (C₁-C₄)alkylsulfinyl, a (C₁-C₄)alkylsulfonyl, a (C₁-C₄)haloalkylthio, a (C₁-C₄)haloalkylsulfinyl, a (C₁-C₄)haloalkylsulfonyl, hydroxy, cyano, nitro, -SFs, -NR^{B}R^{C}, - C(=O)R^{D} or -C(=O)NR^{E}R^{F};
   Z is a (C₁-C₅)alkyl, a (C₂-C₄)alkenyl, a (C₂-C₄)alkynyl, a (C₃-C₆)cycloalkyl, a (C₁-C₄)alkoxy(C₁-C₄)alkyl, a (C₃-C₆)cycloalkyl(C₁-C₄)alkyl or a (C₁-C₄)haloalkyl,
   R¹, R², R³ and R⁴ are each a halogen, a (C₁-C₄)alkyl, a (C₂-C₄)alkenyl, a (C₂-C₄)alkynyl, a (C₃-C₆)cycloalkyl, a (C₁-C₄)haloalkyl, a (C₁-C₄)alkoxy, a (C₃-C₆)cycloalkoxy, a (C₁-C₄)haloalkoxy, a (C₁-C₄)alkylthio, a (C₁-C₄)alkylsulfinyl, a (C₁-C₄)alkylsulfonyl, a (C₁-C₄)haloalkylthio, a (C₁-C₄)haloalkylsulfinyl, a (C₁-C₄)haloalkylsulfonyl, hydroxy, cyano, nitro, -NR^{G}R^{H}, -C(=O)R^{I}, -C(=O)NR^{J}R^{k}, - C(=NOR^{L})R^{M}, dimethoxymethyl, 1,3-dioxan-2-yl or 1,3-dioxolan-2-yl;
   R^{B}, R^{C,} R^{E}, R^{F}, R^{G}, R^{H}, R^{J} and R^{K} are each a hydrogen atom, a (C₁-C₄)alkyl or a (C₁-C₄)haloalkyl;
   R^{D} and R^{I} are each a hydrogen atom, hydroxy, a (C₁-C₄)alkyl, a (C₁-C₄)haloalkyl or a (C₁-C₄)alkoxy; and
   R^{L} and R^{M} are each a hydrogen atom or a (C₁-C₄)alkyl.
(39) The pyridazinone compound or a salt thereof according to any one of (23) to (37), wherein
   Y and R^{A} are each a halogen, a (C₁-C₄)alkyl, a (C₂-C₄)alkenyl, a (C₂-C₄)alkynyl, a (C₃-C₆)cycloalkyl, a (C₁-C₄)haloalkyl, a (C₁-C₄)alkoxy, a (C₁-C₄)haloalkoxy, a (C₁-C₄)alkylsulfonyl, a (C₁-C₄)haloalkylthio, a (C₁-C₄)haloalkylsulfinyl, a (C₁-C₄)haloalkylsulfonyl, cyano, nitro or -C(=O)R^{D;}
   Z is a (C₁-C₅)alkyl, a (C₃-C₆)cycloalkyl, a (C₁-C₄)alkoxy(C₁-C₄)alkyl or a (C₁-C₄)haloalkyl;
   R¹, R², R³ and R⁴ are each a halogen, a (C₁-C₄)alkyl, a (C₂-C₄)alkynyl, a (C₃-C₆)cycloalkyl, a (C₁-C₄)haloalkyl, a (C₁-C₄)alkoxy, a (C₁-C₄)haloalkoxy, a (C₁-C₄)alkylthio, a (C₁-C₄)alkylsulfinyl, a (C₁-C₄)alkylsulfonyl, cyano, nitro, -NR^{G}R^{H}, - C(=O)R^{I}, -C(=NOR^{L})R^{M}, dimethoxymethyl, 1,3-dioxan-2-yl or 1,3-dioxolan-2-yl;
   R^{G} and R^{H} are each a hydrogen atom or a (C₁-C₄)alkyl;
   R^{D} and R^{I} are each a hydrogen atom, a (C₁-C₄)alkyl or a (C₁-C₄)alkoxy; and
   R^{L} and R^{M} are each a hydrogen atom or a (C₁-C₄)alkyl.
(40) The pyridazinone compound or a salt thereof according to any one of (23) to (37), wherein
   Y and R^{A} are each a halogen, a (C₁-C₄)alkyl, a (C₂-C₄)alkenyl, a (C₂-C₄)alkynyl, a (C₃-C₆)cycloalkyl, a (C₁-C₄)haloalkyl, a (C₁-C₄)alkoxy, a (C₁-C₄)haloalkoxy, a (C₁-C₄)alkylsulfonyl, cyano, nitro or -C(=O)R^{D};
   Z is a (C₁-C₄)alkyl, a (C₃-C₆)cycloalkyl, a (C₁-C₄)alkoxy(C₁-C₄)alkyl or a (C₁-C₄)haloalkyl;
   R¹, R², R³ and R⁴ are each a halogen, a (C₁-C₄)alkyl, a (C₂-C₄)alkynyl, a (C₃-C₆)cycloalkyl, a (C₁-C₄)haloalkyl, a (C₁-C₄)alkoxy, a (C₁-C₄)haloalkoxy, a (C₁-C₄)alkylthio, a (C₁-C₄)alkylsulfinyl, a (C₁-C₄)alkylsulfonyl, a (C₁-C₄)haloalkylthio, a (C₁-C₄)haloalkylsulfinyl, a (C₁-C₄)haloalkylsulfonyl, cyano, nitro, -C(=O)R^{I}, -C(=NOR^{L})R^{M}, dimethoxymethyl, 1,3-dioxan-2-yl or 1,3-dioxolan-2-yl;
   R^{D} is a (C₁-C₄)alkyl;
   R^{I} is a hydrogen atom; and
   R^{L} and R^{M} are each a hydrogen atom or a (C₁-C₄)alkyl.
(41) The pyridazinone compound or a salt thereof according to any one of (23) to (37), wherein
   Y and R^{A} are each a halogen, a (C₁-C₄)alkyl, a (C₁-C₄)alkenyl, a (C₁-C₄)alkynyl, a (C₃-C₆)cycloalkyl, a (C₁-C₄)haloalkyl, a (C₁-C₄)alkoxy, a (C₁-C₄)haloalkoxy, a (C₁-C₄)alkylsulfonyl, a (C₁-C₄)haloalkylthio, a (C₁-C₄)haloalkylsulfinyl, a (C₁-C₄)haloalkylsulfonyl, cyano or nitro;
   Z is a (C₁-C₄)alkyl, a (C₃-C₆)cycloalkyl, a (C₁-C₄)alkoxy(C₁-C₄)alkyl or a (C₁-C₄)haloalkyl;
   R¹, R², R³ and R⁴ are each a halogen, a (C₁-C₄)alkyl, a (C₃-C₆)cycloalkyl, a (C₁-C₄)haloalkyl, a (C₁-C₄)alkoxy, a (C₁-C₄)haloalkoxy, a (C₁-C₄)alkylthio, a (C₁-C₄)alkylsulfinyl or nitro.
(42) The pyridazinone compound or a salt thereof according to any one of (23) to (37), wherein
   Y and R^{A} are each a halogen, a (C₁-C₄)alkyl, a (C₁-C₄)alkenyl, a (C₁-C₄)alkynyl, a (C₃-C₆)cycloalkyl, a (C₁-C₄)haloalkyl, a (C₁-C₄)alkoxy, a (C₁-C₄)haloalkoxy, a (C₁-C₄)alkylsulfonyl, a (C₁-C₄)haloalkylthio, a (C₁-C₄)haloalkylsulfinyl, a (C₁-C₄)haloalkylsulfonyl, cyano or nitro;
   Z is a (C₁-C₄)alkyl, a (C₃-C₆)cycloalkyl, a (C₁-C₄)alkoxy(C₁-C₄)alkyl or a (C₁-C₄)haloalkyl; and
   R¹, R², R³ and R⁴ are each a halogen, a (C₁-C₄)alkyl, a (C₃-C₆)cycloalkyl, a (C₁-C₄)haloalkyl, a (C₁-C₄)alkoxy, a (C₁-C₄)haloalkoxy or nitro.
(43) The pyridazinone compound or a salt thereof according to any one of (23) to (38), which has Y bonded at the p-position of the pyridazinone ring, and wherein Y is a halogen, a (C₁-C₄)alkyl, a (C₂-C₄)alkenyl, a (C₂-C₄)alkynyl, a (C₁-C₄)haloalkyl, a (C₁-C₄)alkoxy, a (C₁-C₄)haloalkoxy, a (C₁-C₄)alkylthio, a (C₁-C₄)alkylsulfinyl, a (C₁-C₄)alkylsulfonyl, a (C₁-C₄)haloalkylthio, a (C₁-C₄)haloalkylsulfinyl, a (C₁-C₄)haloalkylsulfonyl, cyano, nitro, -SFs or -C(=O)R^{D}.
(44) The pyridazinone compound or a salt thereof according to any one of (23) to (38), which has Y bonded at the p-position of the pyridazinone ring, and wherein Y is a halogen, a (C₂-C₄)alkynyl, a (C₁-C₄)haloalkyl, a (C₁-C₄)haloalkoxy, a (C₁-C₄)alkylsulfinyl, a (C₁-C₄)alkylsulfonyl, a (C₁-C₄)haloalkylthio, a (C₁-C₄)haloalkylsulfinyl, a (C₁-C₄)haloalkylsulfonyl or cyano.
(45) The pyridazinone compound or a salt thereof according to any one of (23) to (44), wherein X is CH or C(R^{A}).
(46) The pyridazinone compound or a salt thereof according to any one of (23) to (44), wherein X is C(R^{A}).
(47) The pyridazinone compound or a salt thereof according to (46) wherein R^{A} is a halogen, a (C₁-C₄)alkyl, a (C₂-C₄)alkenyl, a (C₂-C₄)alkynyl, a (C₃-C₆)cycloalkyl, a (C₁-C₄)haloalkyl, a (C₁-C₄)alkoxy, a (C₁-C₄)haloalkoxy, cyano, nitro or -C(=O)R^{D}
(48) The pyridazinone compound or a salt thereof according to (46), wherein R^{A} is a halogen, a (C₁-C₄)alkyl, a (C₂-C₄)alkenyl, a (C₂-C₄)alkynyl, a (C₁-C₄)haloalkyl or a (C₁-C₄)alkoxy.
(49) The pyridazinone compound or a salt thereof according to any one of (23) to (44), wherein X is N.
(50) The pyridazinone compound or a salt thereof according to any one of (23) to (44), wherein X is CH.
(51) The pyridazinone compound or a salt thereof according to any one of (23) to (44), wherein X is C(R^{A}) or N, n is 1 or 2, and at least one Y substitutes at the p-position of the pyridazinone ring.
(52) The pyridazinone compound or a salt thereof according to any one of (23) to (40) and (43) to (51), wherein R¹, R², R³ and R⁴ are each a halogen, a (C₁-C₄)alkyl, a (C₃-C₆)cycloalkyl, a (C₁-C₄)haloalkyl, a (C₁-C₄)alkoxy, a (C₁-C₄)haloalkoxy, a (C₁-C₄)alkylthio, a (C₁-C₄)alkylsulfinyl, a (C₁-C₄)alkylsulfonyl, cyano, nitro, -C(=O)R^{I} or dimethoxymethyl.
(53) The pyridazinone compound or a salt thereof according to any one of (23) to (40) and (43) to (51), wherein R¹, R², R³ and R⁴ are each a halogen, a (C₁-C₄)alkyl, a (C₃-C₆)cycloalkyl, a (C₁-C₄)haloalkyl, a (C₁-C₄)alkoxy, a (C₁-C₄)haloalkoxy, nitro or dimethoxymethyl.
(54) The pyridazinone compound or a salt thereof according to any one of (23) to (38) and (43) to (53), wherein Z is a (C₁-C₅)alkyl, a (C₃-C₄)cycloalkyl, a (C₁-C₄)alkoxy(C₁-C₄)alkyl or a (C₁-C₄)haloalkyl.
(55) The pyridazinone compound or a salt thereof according to any one of (23) to (54), wherein n is 1 or 2.
(56) The pyridazinone compound or a salt thereof according to any one of (23) to (54), wherein n is 2.
(57) A pest control agent comprising the compound or a salt thereof as defined in any one of (1) to (56) as an active ingredient.
(58) An insecticide, a miticide, a nematocide or a soil pesticide comprising the compound or a salt thereof as defined in any one of (1) to (56) as an active ingredient.
(59) An insecticide comprising the compound or a salt thereof as defined in any one of (1) to (56) as an active ingredient.
(60) A method for controlling pests, which comprises applying an effective amount of the compound or a salt thereof as defined in any one of (1) to (56).

### EXAMPLES

Now, examples of the present invention will be described, however, the present invention is by no means restricted thereto. Synthesis Examples of the compounds of the present invention will be described.

### Synthesis Example 1

### Synthesis of 2-[3-chloro-5-(trifluoromethyl)pyridin-2-yl]-6-ethyl-5-hydroxy-4-[3-(trifluoromethyl)-1H-pyrazol-1-yl]pyridazin-3(2H)-one (the after-described Compound No. 1-57)

(1) A mixture of 6-bromo-4,5-dichloropyridazin-3(2H)-one (19.0 g, 77.9 mmol), 3,4-dihydro-2H-pyrane (13.1 g, 156 mmol), p-toluenesulfonic acid monohydrate (1.48 g, 7.79 mmol) and tetrahydrofuran (130 mL) was stirred at 80°C for 16 hours. After the reaction, the mixture was cooled to room temperature, and a saturated aqueous sodium hydrogen carbonate solution was added. The resulting mixture was extracted with ethyl acetate, and the organic extract was dried over sodium sulfate, filtrated and concentrated to obtain a crude product. The crude product was purified by silica gel column chromatography (eluent: ethyl acetate/n-heptane) to afford 19.1 g of 6-bromo-4,5-dichloro-2-(tetrahydro-2H-pyran-2-yl)pyridazin-3(2H)-one (yield: 75%). Its ¹H-NMR spectrum data are as follows.
   ¹H-NMR (CDCl₃, 500 MHz): 5.95 (1H, dd), 4.07-4.15 (1H, m), 3.66-3.75 (1H, m), 1.97-2.25 (2H, m), 1.63-1.80 (4H, m)
(2) A mixture of 6-bromo-4,5-dichloro-2-(tetrahydro-2H-pyran-2-yl)pyridazin-3(2H)-one (2.0 g, 6.1 mmol), ethylboronic acid (0.54g, 7.3 mmol), [1,1'-bis(diphenylphosphino)ferrocene]palladium(II) dichloride dichloromethane adduct (0.50 g, 0.61 mmol), cesium carbonate (2.3 g, 7.0 mmol) and 1,4-dioxane (18 mL) was stirred at reflux for 16 hours under a nitrogen atmosphere. After the reaction, the mixture was cooled with ice bath, and water was added. The resulting mixture was extracted with ethyl acetate, and the organic extract was dried over sodium sulfate, filtrated and concentrated to obtain a crude product. The crude product was purified by silica gel column chromatography (eluent: ethyl acetate/n-heptane) to afford 1.1 g of 4,5-dichloro-6-ethyl-2-(tetrahydro-2H-pyran-2-yl)pyridazin-3(2H)-one (yield: 62%). Its ¹H-NMR spectrum data are as follows.
   ¹H-NMR (CDCl₃, 500 MHz): 6.00 (1H, dd), 4.05-4.20 (1H, m), 3.70-3.83 (1H, m), 2.77-2.87 (2H, m), 2.17-2.34 (1H, m), 1.95-2.13 (2H, m), 1.60-1.80 (4H, m), 1.28 (3H, t)
(3) To a solution of 4,5-dichloro-6-ethyl-2-(tetrahydro-2H-pyran-2-yl)pyridazin-3(2H)-one (3.9 g, 14 mmol) in ethanol (100 mL), concentrated hydrochloric acid (10 mL) was added at room temperature. Then, the mixture was stirred at 80°C for 2 hours. After the reaction, the mixture was cooled to room temperature, and the obtained mixture was concentrated. Ethanol was added to the residue, followed by azeotropic dehydration under reduced pressure to afford 2.3 g of 4,5-dichloro-6-ethylpyridazin-3(2H)-one (yield: 85%). Its ¹H-NMR spectrum data are as follows.
   ¹H-NMR (CDCl₃, 500 MHz): 10.47 (1H, brs), 2.80 (2H, q), 1.26 (3H, t)
(4) To a solution of 4,5-dichloro-6-ethylpyridazin-3(2H)-one (2.2 g, 11 mmol) in N,N-dimethylformamide (20 mL), sodium hydride (dispersed in about 60% liquid paraffin) (0.53 g, 13 mmol) in small portions was added at 0°C. The mixture was stirred at 0°C for 30 minutes and 3-chloro-2-fluoro-5-(trifluoromethyl)pyridine (2.7 g, 13 mmol) was added. Then, the mixture was stirred at room temperature for 4 hours. After the reaction, water was added. The mixture was extracted with ethyl acetate, and the organic extract was dried over anhydrous sodium sulfate, filtrated and concentrated to obtain a crude product. The crude product was purified by silica gel column chromatography (eluent: ethyl acetate/n-heptane) to afford 2.9 g of 4,5-dichloro-2-[3-chloro-5-(trifluoromethyl)pyridin-2-yl]-6-ethylpyridazin-3(2H)-one (yield: 70%). Its ¹H-NMR spectrum data are as follows.
   ¹H-NMR (CDCl₃, 500 MHz): 8.81 (1H, d), 8.18 (1H, d), 2.87 (2H, q), 1.27 (3H, t)
(5) To a solution of 3-(trifluoromethyl)pyrazole (1.5 g, 11 mmol) in N,N-dimethylformamide (21 mL), sodium hydride (dispersed in about 60% liquid paraffin) (0.45 g, 11 mmol) in small portions was added at 0°C. The mixture was stirred at 0°C for 30 minutes, and 4,5-dichloro-2-[3-chloro-5-(trifluoromethyl)pyridin-2-yl]-6-ethylpyridazin-3(2H)-one (2.0 g, 5.4 mmol) was added. Then, the mixture was stirred at 60°C for 2 hours. After cooling down to room temperature, a 1N aqueous sodium hydroxide solution (7 mL, 7 mmol) was added to the obtained reaction mixture, followed by stirring at 60°C for 1 hour. After cooling down to room temperature, the solution was acidified with 1N hydrochloric acid. The mixture was extracted with ethyl acetate, and the organic extract was dried over sodium sulfate, filtrated and concentrated to obtain a crude product. The crude product was purified by silica gel column chromatography (eluent: ethyl acetate/n-heptane) to afford 1.4 g of a desired product (yield: 57%).

### Synthesis Example 2

### Synthesis of 2-[2,6-dimethyl-4-(trifluoromethyl)phenyl]-6-ethyl-5-hydroxy-4-(3-nitro-1H-1,2,4-triazol-1-yl)pyridazin-3(2H)-one (the after-described Compound No. 6-65)

(1) A mixture of 3-nitro-1H-1,2,4-triazole (1.0 g, 8.77 mmol), methyl bromoacetate (1.88 g, 12.3 mmol), potassium carbonate (2.18g, 15.78 mmol) and acetonitrile (15 mL) was stirred at 80°C for 4 hours. After cooling down to room temperature, ethyl acetate was added and the mixture was filtrated. To the obtained filtrate, 1N hydrochloric acid was added, and the obtained mixture was extracted with ethyl acetate. The organic extract was washed with brine, dried over anhydrous sodium sulfate, filtrated, and concentrated to afford 1.95 g of crude methyl 2-(3-nitro-1H-1,2,4-triazol-1-yl) acetate. Its ¹H-NMR spectrum data are as follows.
   ¹H-NMR (CDCl₃, 500 MHz):8.21 (1H, s), 5.10 (2H, s), 3.86 (3H, s)
(2) To a solution of methyl 2-(3-nitro-1H-1,2,4-triazol-1-yl) acetate (1.31 g, 7.04 mmol) in tetrahydrofuran (10 mL), a solution of sodium hydroxide (0.60 g, 15 mmol) in water (5 mL) was added, followed by stirring at room temperature for 3 hours. After the reaction, heptane was added, and the obtained mixture was extracted with water twice. The collected aqueous layer was acidified with concentrated hydrochloric acid, and the obtained mixture was extracted with ethyl acetate. The organic extract was washed with brine, dried over anhydrous sodium sulfate, filtrated and concentrated to afford 1.32 g of crude 2-(3-nitro-1H-1,2,4-triazol-1-yl)acetic acid. Its ¹H-NMR spectrum data are as follows.
   ¹H-NMR (DMSO-d₆, 500 MHz):8.86 (1H, s), 5.30 (2H, s)
(3) To a solution of 2,6-dimethyl-4-(trifluoromethyl)phenylaniline (2.49 g, 13.16 mmol) in concentrated hydrochloric acid (12 mL), a solution of sodium nitrite (1.10 g, 15.94 mmol) in water (12 mL) was added at 0°C and the reaction mixture was stirred at this temperature for 1 hour to prepare an aqueous diazonium salt solution. Separately, tin(II) chloride dihydrate (8.91 g, 15.5 mmol) was dissolved in concentrated hydrochloric acid (28 mL), then the aqueous diazonium salt solution prepared as above was dropwise added at room temperature. After the dropping, the mixture was stirred at room temperature for 16 hours. After the reaction, heptane was added, the obtained mixture was filtrated, and ethanol was added to the resulting solid, followed by azeotropic dehydration under reduced pressure to afford 2.84 g of crude [2,6-dimethyl-4-(trifluoromethyl)phenyl]hydrazine hydrochloride (yield: 90%). Its ¹H-NMR spectrum data are as follows.
   ¹H-NMR (DMSO-d₆, 500 MHz): 9.77 (3H, br), 7.46 (2H, s), 6.96 (1H, br), 2.43 (6H, s)
(4) [2,6-dimethyl-4-(trifluoromethyl)phenyl]hydrazine hydrochloride (2.00 g, 8.31 mmol) and triethylamine (4.63 mL, 33.2 mmol) were dissolved in dichloromethane (21 mL), and (Boc)₂O (3.63 g, 33.2 mmol) was added at 0°C. Then, the mixture was stirred at room temperature for 12 hours. After the reaction, 2,2,2-trifluoroethanol (0.10 g, 0.83 mmol) and 4-dimethylaminopyridine (0.10 g, 0.83 mmol) were added, and the mixture was stirred at room temperature for 1 hour. Then, the mixture was concentrated to obtain a residue. To the obtained residue, ethyl acetate and 1N hydrochloric acid were added, and the mixture was extracted with ethyl acetate. The organic extract was washed with brine, dried over anhydrous sodium sulfate, filtrated and concentrated to obtain a crude product. The crude product was purified by silica gel column chromatography (eluent: ethyl acetate/n-heptane) to afford 1.76 g of tert-butyl 2-[2,6-dimethyl-4-(trifluoromethyl)phenyl]hydrazine-1-formate (yield: 70%). Its ¹H-NMR spectrum data are as follows.
   ¹H-NMR (CDCl₃, 500 MHz):7.22 (2H, s), 6.34 (1H, br), 5.83 (1H, d), 2.40 (6H, s), 1.40 (9H, brs)
(5) 2-(3-nitro-1H-1,2,4-triazol-1-yl)acetic acid (0.23 g, 0.76 mmol) and N,N-dimethylformamide (10 mg, 0.14 mmol) were dissolved in dichloromethane (4 mL), and oxalyl chloride (0.50 mL, 5.71 mmol) was added. The mixture was stirred at room temperature for 1 hour. After the reaction, the mixture was concentrated under reduced pressure to obtain an acid chloride. Separately, to a solution of tert-butyl 2-[2,6-dimethyl-4-(trifluoromethyl)phenyl]hydrazine-1-formate (0.20 g, 0.66 mmol) in dichloromethane (2 mL), potassium carbonate (0.18 g, 1.31 mmol) was added at 0°C. The mixture was stirred at 0°C for 10 minutes, and then a solution of the acid chloride prepared as above in dichloromethane (2 mL) was dropwise added. After the dropping, the mixture was stirred at 0°C for 10 minutes and then warmed, and stirred at room temperature for 21 hours. After the reaction, ethyl acetate and water were added, and the mixture was extracted with ethyl acetate. The organic extract was washed with brine, dried over anhydrous sodium sulfate, filtrated and concentrated to obtain a crude product. The crude product was purified by silica gel column chromatography (eluent: ethyl acetate/n-heptane) to afford 0.13 g of tert-butyl 2-[2,6-dimethyl-4-(trifluoromethyl)phenyl]-2-[2-(3-nitro-1H-1,2,4-triazol-1-yl)acetyl]hydrazine-1-formate (yield: 38%). Its ¹H-NMR spectrum data are as follows.
   ¹H-NMR (CDCl₃, 500 MHz):8.39 (1H, s), 8.38 (0.6H, s), 7.48 (1.2H, s), 7.39 (2H, s), 6.79 (1H, s), 6.78 (0.6H, s), 5.10-5.80 (2H, m), 4.75 (1.2H, s), 2.53 (3.6H, s), 2.39 (6H, br), 1.49 (9H, s), 1.47 (5.4H, s)
(6) To a solution of tert-butyl 2-[2,6-dimethyl-4-(trifluoromethyl)phenyl]-2-[2-(3-nitro-1H-1,2,4-triazol-1-yl)acetyl]hydrazine-1-formate (0.13 g, 0.29 mmol) in dichloromethane (4 mL), a solution of trifluoroacetic acid (1.00 mL, 13 mmol) in dichloromethane (1 mL) was added, followed by stirring at room temperature for 1 hour. After the reaction, a saturated aqueous sodium hydrogen carbonate solution was added, and the mixture was extracted with ethyl acetate. The organic extract was washed with brine, dried over anhydrous sodium sulfate and filtrated, and the filtrate was concentrated. To the obtained residue, p-toluenesulfonic acid monohydrate (10 mg, 0.05 mmol), methanol (5 mL) and methyl 2-oxobutyrate (50 mg, 0.43 mmol) were added in this order, followed by stirring at 70°C for 30 minutes. After the reaction, the mixture was cooled to room temperature and concentrated to obtain a crude product. The crude product was purified by silica gel column chromatography (eluent: ethyl acetate/n-heptane) to afford 95 mg of methyl 2-{2-[2,6-dimethyl-4-(trifluoromethyl)phenyl]-2-[2-(3-nitro-1H-1 ,2,4-triazol-1-yl)acetyl]hydrazinylidene} butyrate (yield: 73%). Its ¹H-NMR spectrum data are as follows.
   ¹H-NMR (CDCl₃, 500 MHz):8.38 (0.6H, s), 8.36 (1H, s), 7.42 (1.2H, s), 7.34 (2H, s), 5.78 (1.2H, s), 5.77 (2H, s), 3.88 (1.8H, s), 3.09 (3H, s), 2.51 (2H, q), 2.20 (3.6H, s), 2.17 (6H, s), 1.91 (1.2H, q), 1.20 (3H, t), 0.67 (1.8H, t)
(7) To a solution of methyl 2-{2-[2,6-dimethyl-4-(trifluoromethyl)phenyl]-2-[2-(3-nitro-1H-1,2,4-triazol-1-yl)acetyl]hydrazinylidene} butyrate (94 mg, 0.21 mmol) in acetonitrile (4 mL), 1,8-diazabicyclo[5.4.0]-7-undecene (0.10 mL, 0.66 mmol) was added, followed by stirring at 70°C for 30 minutes. After the reaction, the mixture was cooled to room temperature, ethyl acetate and water were added, the solution was acidified with 2N hydrochloric acid, and the mixture was extracted with ethyl acetate. The organic extract was washed with brine, dried over anhydrous sodium sulfate, filtrated and concentrated to obtain a crude product. The crude product was purified by silica gel column chromatography (eluent: ethyl acetate/n-heptane) to afford 24 mg of a desired product (yield: 27%).

### Synthesis Example 3

### Synthesis of 2-[2,6-dichloro-4-(trifluoromethyl)phenyl]-6-ethyl-5-hydroxy-4-[3-(trifluoromethyl)-1H-pyrazol-1-yl]pyridazin-3(2H)-one (the after-described Compound No. 2-97)

(1) To a solution of sodium hydroxide (2.12 g, 52.9 mmol) in water (30 mL), 3-(trifluoromethyl)pyrazole (3.00 g, 22.1 mmol) and bromoacetic acid (3.22 g, 23.2 mmol) were added in this order. Then, the mixture was stirred at 100°C for 15 hours. After the reaction, the solution was cooled to 0°C and acidified with 2N hydrochloric acid, and n-heptane was further added. The precipitated solid was recovered by filtration to afford 3.41 g of crude 2-[3-(trifluoromethyl) -1H-pyrazol-1-yl]acetic acid (yield: 80%). Its ¹H-NMR spectrum data are as follows.
   ¹H-NMR (CDCl₃, 500 MHz):7.55 (1H, d), 6.62 (1H, d), 5.04 (2H, s)
(2) A mixture of [2,6-dichloro-4-(trifluoromethyl)phenyl]hydrazine (500 mg, 2.04 mmol), methyl 2-oxobutyrate (284 mg, 2.45 mmol) and methanol (10 mL) was stirred at 65°C for 15 hours. The mixture was then diluted with ethyl acetate, and water was added. The mixture was extracted with ethyl acetate, and the organic extract was washed with brine, dried over anhydrous sodium sulfate, filtrated and concentrated to obtain a crude product. The crude product was purified by silica gel column chromatography (eluent: ethyl acetate/n-heptane) to afford 526 mg of methyl 2-{2-[2,6-dichloro-4-(trifluoromethyl)phenyl]hydrazinylidene} butyrate (yield: 75%). Its ¹H-NMR spectrum data are as follows.
   ¹H-NMR (CDCl₃, 500 MHz):12.28 (1H, s), 7.54 (2H, s), 3.86 (3H, s), 2.57 (2H, q), 1.16 (3H, t)
(3) A mixture of 2-[3-(trifluoromethyl)-1H-pyrazol-1-yl]butyric acid (357 mg, 1.84 mmol), N,N-dimethylformamide (10 mg, 0.14 mmol) and dichloromethane (10 mL) was stirred under cooling with ice bath for several minutes, and then oxalyl chloride (0.17 mL, 1.99 mmol) was slowly dropwise added. After the dropping, the mixture was stirred at room temperature for 3 hours and then concentrated under reduced pressure to obtain an acid chloride. Separately, a mixture of methyl 2-{2-[2,6-dichloro-4-(trifluoromethyl)phenyl]hydrazinylidene} butyrate (526 mg, 1.53 mmol) and tetrahydrofuran (15 mL) was stirred under cooling with ice bath for several minutes under a nitrogen atmosphere, and a solution of lithium hexamethyldisilazide in tetrahydrofuran(2.95 mL, 1.3 molar, 3.83 mmol) was added, followed by stirring for 45 minutes, and then the acid chloride prepared as above was added. Then, the mixture was stirred at room temperature for 16 hours. The solution was acidified with 2N hydrochloric acid, water was added, and the resulting mixture was extracted with ethyl acetate. The organic extract was washed with brine, dried over anhydrous sodium sulfate, filtrated and concentrated to obtain a crude product. The crude product was purified by silica gel column chromatography (eluent: ethyl acetate/n-heptane) to afford 82 mg of a desired product (yield: 11%).

### Synthesis Example 4

### Synthesis of 2-[2,6-dimethyl-4-(trifluoromethyl)phenyl]-6-ethyl-5-hydroxy-4-(3-cyano-1H-1,2,4-triazol-1-yl)pyridazin-3(2H)-one (the after-described Compound No. 6-60)

(1) To a solution of tert-butyl 2-[2,6-dimethyl-4-(trifluoromethyl)phenyl]hydrazine-1-formate (0.50 g, 1.64 mmol) in dichloromethane (11 mL), potassium carbonate (0.45 g, 3.29 mmol) was added at room temperature. The mixture was stirred at room temperature for 10 minutes, and bromoacetyl bromide (0.17 mL, 1.97 mmol) was dropwise added at 0°C. After the dropping, the mixture was stirred at 0°C for 30 minutes and warmed, and stirred at room temperature for 21 hours. After the reaction, ethyl acetate and water were added, and the obtained mixture was extracted with ethyl acetate. The organic extract was washed with brine, dried over anhydrous sodium sulfate, filtrated and concentrated to obtain a crude product. The crude product was purified by silica gel column chromatography (eluent: ethyl acetate/n-heptane) to afford 0.34 g of tert-butyl 2-(2-bromoacetyl)-2-[2,6-dimethyl-4-(trifluoromethyl)phenyl]hydrazine-1-formate (yield: 49%). Its ¹H-NMR spectrum data are as follows.
   ¹H-NMR (CDCl₃, 300 MHz):7.42 (1.4H, s), 7.35 (2H, s), 6.97 (1H, s), 6.77 (0.7H, s), 4.05 (2H, s), 3.64 (1.4H, s), 2.49 (4.2H, s), 2.42 (6H, s), 1.48 (6.3H, s), 1.47 (9H, s)
(2) To a solution of 3-cyano-1H-1,2,4-triazole (50 mg, 0.57 mmol) in N,N-dimethylformamide (3 mL), potassium carbonate (0.16 g, 1.14 mmol) was added at room temperature. The mixture was stirred at room temperature for 30 minutes, and then a solution of tert-butyl 2-(2-bromoacetyl)-2-[2,6-dimethyl-4-(trifluoromethyl)phenyl]hydrazine-1-formate (0.22 g, 0.52 mmol) in N,N-dimethylformamide (3 mL) was dropwise added. After the dropping, the mixture was stirred at room temperature for 17 hours. After the reaction, ethyl acetate and water were added, and the obtained mixture was extracted with ethyl acetate. The organic extract was washed with brine, dried over anhydrous sodium sulfate, filtrated and concentrated to obtain a crude product. The crude product was purified by silica gel column chromatography (eluent: ethyl acetate/n-heptane) to afford 0.17 g of tert-butyl 2-[2,6-dimethyl-4-(trifluoromethyl)phenyl]-2-[2-(3-cyano-1H-1,2,4-triazol-1-yl)acetyl]hydrazine-1-formate (yield: 75%). Its ¹H-NMR spectrum data are as follows.
   ¹H-NMR (CDCl₃, 300 MHz):8.39 (1H, s), 8.35 (0.9H, s), 7.49 (1.8H, s), 7.41 (2H, s), 6.83 (1H, s), 6.77 (0.9H, s), 5.48 (2H, brs), 4.71 (1.8H, s), 2.53 (5.4H, s), 2.39 (6H, brs), 1.49 (9H, s), 1.48 (8.1H, s)
(3) By the method of Synthesis Example 2, (6) and (7), from tert-butyl 2-[2,6-dimethyl-4-(trifluoromethyl)phenyl]-2-[2-(3-cyano-1H-1 ,2,4-triazol-1-yl)acetyl]hydrazine-1-formate, desired product 2-[2,6-dimethyl-4-(trifluoromethyl)phenyl]-6-ethyl-5-hydroxy-4-(3-cyano-1H-1,2,4-triazol-1-yl)pyridazin-3(2H)-one was prepared.

### Synthesis Example 5

### Synthesis of 4-(3-chloro-1H-pyrazol-1-yl)-2-[2,6-dimethyl-4-(trifluoromethyl)phenyl]-5-hydroxy-6-(methoxymethyl)pyridazin-3(2H)-one (the after-described Compound No. 2-444)

(1) A mixture of ethyl 5-(3-chloro-1H-pyrazol-1-yl)-1-[2,6-dimethyl-4-(trifluoromethyl)phenyl]-4-hydroxy-6-oxo-1,6-dihydropyridazin-3-carboxylate (0.40 g, 0.88 mmol) prepared by the method of Synthesis Example 2, lithium aluminum hydride (0.10 g, 2.63 mmol) and tetrahydrofuran (18 mL) was stirred at 0°C for 3 hours. After the reaction, ethyl acetate and water were added, and the mixture was extracted with an aqueous sodium hydroxide solution. The aqueous layer was acidified with 2N hydrochloric acid, and the mixture was extracted with ethyl acetate. The organic extract was washed with brine, dried over anhydrous sodium sulfate, filtrated and concentrated to afford 0.35 g of crude 4-(3-chloro-1H-pyrazol-1-yl)-2-[2,6-dimethyl-4-(trifluoromethyl)phenyl]-5-hydroxy-6-(hydroxymethyl)pyridazin-3(2H)-one. Its ¹H-NMR spectrum data are as follows.
   ¹H-NMR (CDCl₃, 300 MHz):13.06 (1H, brs), 9.23 (1H, d), 7.49 (2H, s), 6.48 (1H, d), 4.85 (2H, s), 2.18 (6H, s)
(2) To a solution of 4-(3-chloro-1H-pyrazol-1-yl)-2-[2,6-dimethyl-4-(trifluoromethyl)phenyl]-5-hydroxy-6-(hydroxymethyl)pyridazin-3(2H)-one (0.27g, 0.66 mmol) in N,N-dimethylformamide (4 mL), sodium hydride (dispersed in about 60% liquid paraffin) (0.10g, 2.62 mmol) and methyl iodide (0.37 g, 2.62 mmol) were added at 0°C. Then, the mixture was stirred at room temperature for 15 hours. To the obtained reaction mixture, a 2N aqueous sodium hydroxide solution was added, followed by stirring at room temperature for 1 hour. The mixture was diluted with ethyl acetate, and 0.5N hydrochloric acid was added. The obtained mixture was extracted with ethyl acetate. The organic extract was washed with brine, dried over anhydrous sodium sulfate, filtrated and concentrated to obtain a crude product. The crude product was purified by silica gel column chromatography (eluent: ethyl acetate/n-heptane) to afford 87 mg of a desired product (yield: 31%).

### Synthesis Example 6

### Synthesis of 2-[2,6-dimethyl-4-(trifluoromethyl)phenyl]-5-hydroxy-4-(3-methyl-1H-pyrazol-1-yl)-6-(trifluoromethyl)pyridazin-3(2H)-one (the after-described Compound No. 2-516)

(1) 4-(3-bromo-1H-pyrazol-1-yl)-2-[2,6-dimethyl-4-(trifluoromethyl)phenyl]-5-hydroxy-6-(trifluoromethyl)pyridazin-3(2H)-one (81mg, 0.16 mmol) prepared by the method of Synthesis Example 2, trimethylboroxine (0.11 mL, 0.81 mmol), [1,1'-bis(diphenylphosphino)ferrocene]palladium(II) dichloride dichloromethane adduct (13 mg, 0.020 mmol) and potassium carbonate (68 mg, 0.49 mmol) were dissolved in water (2 mL) and 1,4-dioxane (4 mL). The mixture was stirred at 140°C for 6 hours in a microwave generator. After the reaction, the mixture was cooled to room temperature. Water and 1N hydrochloric acid were added, and then ethyl acetate was added, followed by cerite filtration. The filtrate mixture was extracted with ethyl acetate. The organic extract was washed with brine, dried over anhydrous sodium sulfate, filtrated and concentrated to obtain a crude product. The crude product was purified by silica gel column chromatography (eluent: ethyl acetate/n-heptane) to afford 46 mg of a desired product (yield: 65%).

### Synthesis Example 7

### Synthesis of 2-[2,6-dimethyl-4-(trifluoromethyl)phenyl]-4-(3-ethyl-1H-1 ,2,4-triazol-1-yl)-5-hydroxy-6-isopropylpyridazin-3(2H)-one (the after-described Compound No. 6-134)

(1) 4-(3-bromo-1H-1,2,4-triazol-1-yl)-2-[2,6-dimethyl-4-(trifluoromethyl)phenyl]-5-hydroxy-6-isopropylpyridazin-3(2H)-one (50 mg, 0.11 mmol) prepared by the method of Synthesis Example 2, 4,4,5,5-tetramethyl-2-vinyl-1,3,2-dioxaborolane (50 mg, 0.32 mmol), [1,1'-bis(diphenylphosphino)ferrocene]palladium(II) dichloride dichloromethane adduct (8.7mg, 0.011 mmol) and potassium carbonate (40 mg, 0.32 mmol) were dissolved in water (0.5 mL) and 1,4-dioxane (1 mL). The mixture was stirred at 140°C for 6 hours by a microwave generator. After the reaction, the mixture was cooled to room temperature. Water and 1N hydrochloric acid were added, and then ethyl acetate was added, followed by celite filtration. The filtrate mixture was extracted with ethyl acetate. The organic extract was washed with brine, dried over anhydrous sodium sulfate, filtrated and concentrated to obtain a crude product. The crude product was purified by silica gel column chromatography (eluent: ethyl acetate/n-heptane) to afford 30 mg of 2-[2,6-dimethyl-4-(trifluoromethyl)phenyl]-5-hydroxy-6-isopropyl-4-(3-vinyl-1H-1,2,4-triazol-1-yl)pyridazin-3(2H)-one (yield: 68%). Its ¹H-NMR spectrum data are as follows.
   ¹H-NMR (CDCl₃, 500 MHz):12.86 (1H, brs), 9.77 (1H, s), 7.46 (2H, s), 6.78 (1H, dd), 6.45 (1H, d), 5.74 (1H, d), 3.42 (1H, sep), 2.15 (6H, s), 1.28 (6H, d)
(2) To a solution of 2-[2,6-dimethyl-4-(trifluoromethyl)phenyl]-5-hydroxy-6-isopropyl-4-(3-vinyl-1H-1,2,4-triazol-1-yl)pyridazin-3(2H)-one (30 mg, 0.070 mmol) in an ethanol (1.4 mL), 5% palladium on carbon (wetted with water) (7mg) was added, followed by stirring at room temperature for 12 hours. The mixture was then diluted with ethyl acetate, followed by celite filtration. The filtrate was concentrated to obtain a crude product. The crude product was purified by silica gel column chromatography (eluent: ethanol/ethyl acetate) to afford 20 mg of a desired product (yield: 66%).

### Synthesis Example 8

### Synthesis of 4-[5-(3-chloro-1H-1,2,4-triazol-1-yl)-3-ethyl-4-hydroxy-6-oxopyridazin-1(6H)-yl]-3,5-dimethylbenzonitrile (the after-described Compound No. 6-136)

(1) A mixture of methyl 4-[5-(3-chloro-1H-1,2,4-triazol-1-yl)-3-ethyl-4-hydroxy-6-oxopyridazin-1(6H)-yl]-3,5-dimethylbenzoate (91 mg, 0.23 mmol) prepared by the method of Synthesis Example 2, lithium aluminum hydride (21 mg, 0.56 mmol) and tetrahydrofuran (4 mL) was stirred at -10°C for 3 hours. The reaction mixture was put in a funnel packed with celite and silica gel, and ethyl acetate and 2N hydrochloric acid were further added to carry out filtration. The filtrate was diluted with ethyl acetate, and water was added. The mixture was extracted with ethyl acetate. The organic extract was washed with brine, dried over anhydrous sodium sulfate, filtrated and concentrated to afford 88 mg of crude 4-(3-chloro-1H-1,2,4-triazol-1-yl)-6-ethyl-5-hydroxy-2-[4-(hydroxymethyl)-2,6-dimethylphenyl]pyridazin-3(2H)-one. Its ¹H-NMR spectrum data are as follows.
   ¹H-NMR (CDCl₃, 300 MHz):11.86 (1H, brs), 9.81 (1H, s), 7.21 (2H, s), 4.68 (2H, s), 2.87 (2H, q), 2.10 (6H, s), 1.29 (3H, t)
(2) To a solution of 4-(3-chloro-1H-1,2,4-triazol-1-yl)-6-ethyl-5-hydroxy-2-[4-(hydroxymethyl)-2,6-dimethylphenyl]pyridazin-3(2H)-one (85 mg, 0.23 mmol) in dichloromethane (5 mL), activated manganese dioxide (0.59 g, 6.79 mmol) was added, followed by stirring at room temperature for 22 hours. Celite filtration was carried out, and the filtrate was concentrated to afford 79 mg of crude 4-[5-(3-chloro-1H-1,2,4-triazol-1-yl)-3-ethyl-4-hydroxy-6-oxopyridazin-1(6H)-yl]-3,5-dimethylbenzaldehyde. Its ¹H-NMR spectrum data are as follows.
   ¹H-NMR (CDCl₃, 300 MHz):11.93 (1H, brs), 10.04 (1H, s), 9.78 (1H, s), 7.74 (2H, s), 2.88 (2H, q), 2.20 (6H, s), 1.30 (3H, t)
(3) 4-[5-(3-chloro-1H-1,2,4-triazol-1-yl)-3-ethyl-4-hydroxy-6-oxopyridazin-1(6H)-yl]-3,5-dimethylbenzaldehyde (79 mg, 0.21 mmol) and diisopropylethylamine (54 mg, 0.42 mmol) were dissolved in tetrahydrofuran (4 mL), and isobutyryl chloride (36 mg, 0.34 mmol) was added. Then, the mixture was stirred at room temperature for 16 hours. The mixture was diluted with ethyl acetate, and water was added. The mixture was extracted with ethyl acetate. The organic extract was washed with brine, dried over anhydrous sodium sulfate, filtrated and concentrated to obtain a crude product. The crude product was purified by silica gel column chromatography (eluent: ethyl acetate/n-heptane) to afford 75 mg of 4-[5-(3-chloro-1H-1,2,4-triazol-1-yl)-3-ethyl-4-isobutyryloxy-6-oxopyridazin-1(6H)-yl]-3,5-dimethylbenzaldehyde (yield: 80%). Its ¹H-NMR spectrum data are as follows.
   ¹H-NMR (CDCl₃, 300 MHz):10.05 (1H, s), 9.20 (1H, s), 7.75 (2H, s), 2.99 (1H, sep), 2.76 (2H, q), 2.22 (6H, s), 1.40 (6H, d), 1.27 (3H, t)
(4) 4-[5-(3-chloro-1H-1,2,4-triazol-1-yl)-3-ethyl-4-isobutyryloxy-6-oxopyridazin-1(6H)-yl]-3,5-dimethylbenzaldehyde (75 mg, 0.17 mmol) and sodium acetate (14 mg, 0.17 mmol) were dissolved in tetrahydrofuran (1 mL) and ethanol (3 mL), and hydroxylamine (50% aqueous solution) (56 mg, 0.85 mmol) was added. Then, the mixture was stirred at room temperature for 20 hours. To the obtained reaction mixture, a 2N aqueous sodium hydroxide solution (2 mL) was added, followed by stirring at room temperature for 15 minutes, and ethyl acetate and heptane were added. The mixture was extracted with water, and the aqueous layer was acidified with 2N hydrochloric acid, and the mixture was extracted with ethyl acetate. The organic extract was washed with brine, dried over anhydrous sodium sulfate, filtrated, and concentrated to afford 69 mg of crude (*E*)-4-[5-(3-chloro-1H-1,2,4-triazol-1-yl)-3-ethyl-4-hydroxy-6-oxopyridazin-1(6H)-yl]-3,5-dimethylbenzaldoxime. Its ¹H-NMR spectrum data are as follows.
   ¹H-NMR (CDCl₃, 300 MHz):11.90 (1H, brs), 9.81 (1H, s), 8.13 (1H, s), 7.43 (2H, s), 2.88 (2H, q), 2.14 (6H, s), 1.30 (3H, t)
(5) A mixture of (*E*)-4-[5-(3-chloro-1H-1,2,4-triazol-1-yl)-3-ethyl-4-hydroxy-6-oxopyridazin-1(6H)-yl]-3,5-dimethylbenzaldoxime (69mg, 0.18 mmol) and acetic anhydride( 5 mL) was stirred at 140°C for 16 hours. After cooling down to room temperature, the mixture was diluted with ethyl acetate and water. The mixture was extracted with ethyl acetate. The organic layer was washed with brine, dried over anhydrous sodium sulfate, filtrated and concentrated to obtain a crude product. The crude product was purified by silica gel column chromatography (eluent: ethyl acetate/n-heptane) to afford a mixture containing a desired product. To a solution of the mixture in tetrahydrofuran (2 mL), a 2N aqueous sodium hydroxide solution (2 mL) was added, and the mixture was stirred at room temperature for 2 hours. The mixture was acidified with 1N hydrochloric acid, and the mixture was extracted with ethyl acetate. The organic extract was washed with brine, dried over anhydrous sodium sulfate, filtrated and concentrated to afford 22 mg of a desired product (yield: 33%).

### Synthesis Example 9

### Synthesis of 4-(3-chloro-1H-pyrazol-1-yl)-2-{2,6-dimethyl-4-[(trifluoromethyl)sulfonyl]phenyl}-6-ethyl-5-hydroxypyridazin-3(2H)-one (the after-described Compound No. 2-191)

(1) 4-(3-chloro-1H-pyrazol-1-yl)-2-{2,6-dimethyl-4-[(trifluoromethyl)thio]phenyl}-6-ethyl-5-hydroxypyridazin-3(2H)-one (70 mg, 0.16 mmol) prepared by the method of Synthesis Example 2 and diisopropylethylamine (31 mg, 0.24 mmol) were dissolved in tetrahydrofuran (4 mL), and isobutyryl chloride (20 mg, 0.19 mmol) was added. Then, the mixture was stirred at room temperature for 3 hours. The mixture was diluted with ethyl acetate, and water was added. The mixture was extracted with ethyl acetate. The organic extract was washed with brine, dried over anhydrous sodium sulfate, filtrated and concentrated to obtain a crude product. The crude product was purified by silica gel column chromatography (eluent: ethyl acetate/n-heptane) to afford 80 mg of 4-(3-chloro-1H-pyrazol-1-yl)-2-{2,6-dimethyl-4-[(trifluoromethyl)thio]phenyl}-6-ethyl-5-(isobutyryloxy)pyridazin-3(2H)-one (yield: 99%). Its ¹H-NMR spectrum data are as follows.
   ¹H-NMR (CDCl₃, 300 MHz):8.43 (1H, d), 7.49 (2H, s), 6.35 (1H, d), 2.94 (1H, sep), 2.71 (2H, q), 2.13 (6H, s), 1.35 (6H, d), 1.24 (3H, t)
(2) A mixture of 4-(3-chloro-1H-pyrazol-1-yl)-2-{2,6-dimethyl-4-[(trifluoromethyl)thio]phenyl}-6-ethyl-5-(isobutyryloxy)pyridazin-3(2H)-one (35 mg, 0.070 mmol), 72% m-chloroperbenzoic acid (with water) (33 mg, 0.14 mmol) and dichloroethane (1 mL) was stirred at 50°C for 16 hours. After cooling down to room temperature, the mixture was diluted with ethyl acetate, and a 0.1N aqueous sodium hydroxide solution was added. The mixture was extracted with ethyl acetate. The organic extract was washed with brine, dried over anhydrous sodium sulfate, filtrated and concentrated to obtain a crude product. The crude product was purified by silica gel column chromatography (eluent: ethyl acetate/n-heptane) to afford 38 mg of 4-(3-chloro-1H-pyrazol-1-yl)-2-{2,6-dimethyl-4-[(trifluoromethyl)sulfonyl]phenyl}-6-ethyl-5-(isobutyryloxy)pyridazin-3(2H)-one (yield: 102%). Its ¹H-NMR spectrum data are as follows.
   ¹H-NMR (CDCl₃, 300 MHz):8.41 (1H, d), 7.87 (2H, s), 6.37 (1H, d), 2.96 (1H, sep), 2.72 (2H, q), 2.24 (6H, s), 1.36 (6H, d), 1.24 (3H, t)
(3) To a solution of 4-(3-chloro-1H-pyrazol-1-yl)-2-{2,6-dimethyl-4-[(trifluoromethyl)sulfonyl]phenyl}-6-ethyl-5-(isobutyryloxy)pyridazin-3(2H)-one (38 mg, 0.070 mmol) in tetrahydrofuran (2 mL), a 2N aqueous sodium hydroxide solution (2 mL) was added, followed by stirring at room temperature for 1 hour. The mixture was acidified with 2N hydrochloric acid, and the mixture was extracted with ethyl acetate. The organic extract was washed with brine, dried over anhydrous sodium sulfate, filtrated and concentrated to obtain a crude product. The crude product was purified by silica gel column chromatography (eluent: ethyl acetate/n-heptane) to afford 29 mg of a desired product (yield: 88%).

### Synthesis Example 10

### Preparation of tetrabutylammonium salt of 2-[2,6-dimethyl-4-(trifluoromethyl)phenyl]-6-ethyl-4-(4-fluoro-1H-pyrazol-1-yl)-5-hydroxypyridazin-3(2H)-one (the after-described Compound No. 2-555)

(1) To a solution of 2-[2,6-dimethyl-4-(trifluoromethyl)phenyl]-6-ethyl-4-(4-fluoro-1H-pyrazol-1-yl)-5-hydroxypyridazin-3(2H)-one (50 mg, 0.13 mmol) in tetrahydrofuran (4 mL), tetrabutylammonium hydroxide (10% methanol solution ) (0.67 g, 0.26 mmol) was added, followed by stirring at room temperature for 2 hours. The mixture was concentrated, tert-butyl methyl ether was added, and the mixture was filtrated. The solid on the filter was further washed with ethyl acetate, and the solid was collected by filtration to afford 28 mg of a desired product (yield: 35%).

Now, representative examples of the compound of the formula (I) are shown in Tables 1 to 8. These compounds may be synthesized by the above Synthesis Example or the above Production Process. Further, as physical properties of the typical examples of the compound of the formula (I), ¹H-NMR spectrum data are shown in Table 9. In ¹H-NMR spectrum data in Table 9, s is singlet, brs is broad singlet, d is doublet, t is triplet, q is quartet, quin is quintet, sep is septet, m is multiplet and br is broad.

In each Table, No. represents Compound No. Abbreviations used in Tables 1 to 8 represent the following substituents. Me is a methyl group, Et is an ethyl group, n-Pr is a n-propyl group, i-Pr is an isopropyl group, nBu is a n-butyl group, sBu is a sec-butyl group, tBu is a tert-butyl group, cPr is a cyclopropyl group, cBu is a cyclobutyl group, cPen is a cyclopentyl group, cHex is a cyclohexyl group, DXAN is a 1,3-dioxan-2-yl group, and DXOL is a 1,3-dioxolan-2-yl group. In Tables, for example, "OMe" is a methoxy group, "SMe" is a methylthio group, and "OcPr" is a cyclopropyloxy group.

Further, in Table 1, for example, the compound indicated with "3-CF₃" in the column (R¹)a, is a compound wherein the pyrazole ring is substituted by CF₃ at the substitution position of "3" in the chemical formula in the Table, that is a compound of "a=1" in which the pyrazole ring is substituted by one R¹ at the 3-position of the pyrazole ring. Further, in Table 1, the compound indicated with "a=0" in the column (R¹)a, is a compound not substituted by R¹, and a compound indicated with "n=0" in the column (Y)n, is a compound not substituted by Y. The same applies to the other description in Tables 1 to 8.

Further, in Table 2, compounds indicated with " 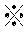 " on the Compound Nos are salts of the compound of the formula (I), and the salts are described on the margin of Table 2.

**Table 1**

| 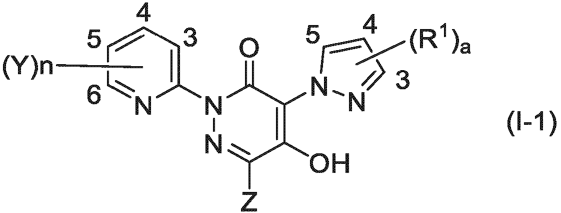 | | | |
|---|---|---|---|
| No. | (R¹)ₐ | (Y₎ₙ | Z |
| 1-1 | 3-CF₃ | 3-Br, 5-CF₃ | Et |
| 1-2 | 3-CF₃ | 3-F, 5-CF₃ | Et |
| 1-3 | 3-CF₃ | 3-Me, 5-CF₃ | Et |
| 1-4 | 3-CF₃ | 3-I, 5-CF₃ | Et |
| 1-5 | 3-CF₃ | 5-CF₃ | Et |
| 1-6 | 3-CF₃ | 3-NO₂, 5-CF₃ | Et |
| 1-7 | 3-CF₃ | 3-CH=CH₂, 5-CF₃ | Et |
| 1-8 | 3-CF₃ | 3-Et, 5-CF₃ | Et |
| 1-9 | 3-CF₃ | 3-Et, 5-CF₃ | Me |
| 1-10 | 3-CF₃ | 3-CN, 5-CF₃ | Et |
| 1-11 | 3-CF₃ | 3-COMe, 5-CF₃ | Et |
| 1-12 | 3-CF₃ | 3-iPr, 5-CF₃ | Et |
| 1-13 | 3-CF₃ | 3-OMe, 5-CF₃ | Et |
| 1-14 | 3-CF₃ | 3-CF₃, 5-CF₃ | Et |
| 1-15 | 3-CF₃ | 3-OCF₃, 5-CF₃ | Et |
| 1-16 | 3-CF₃ | 3-SMe, 5-CF₃ | Et |
| 1-17 | 3-CF₃ | 3-SOMe, 5-CF₃ | Et |
| 1-18 | 3-CF₃ | 3-SO₂Me, 5-CF₃ | Et |
| 1-19 | 3-CF₃ | 3-NH₂, 5-CF₃ | Et |
| 1-20 | 3-CF₃ | 3-NHMe, 5-CF₃ | Et |
| 1-21 | 3-CF₃ | 3-NMe₂, 5-CF₃ | Et |
| 1-22 | 3-CF₃ | 3-NHCH₂CF₃, 5-CF₃ | Et |
| 1-23 | 3-CF₃ | 3-N(CH₂CF₃)₂, 5-CF₃ | Et |
| 1-24 | 3-CF₃ | 3-OH, 5-CF₃ | Et |
| 1-25 | 3-CF₃ | 3-Cl | Et |
| 1-26 | 3-CF₃ | 3-Cl, 5-OCF₃ | Et |
| 1-27 | 3-CF₃ | 3-Cl, 5-NO₂ | Et |
| 1-28 | 3-CF₃ | 3-Cl, 5-cPr | Et |
| 1-29 | 3-CF₃ | 3-Cl, 5-SCF₃ | Et |
| 1-30 | 3-CF₃ | 3-Cl, 5-SOCF₃ | Et |
| 1-31 | 3-CF₃ | 3-Cl, 5-SO₂CF₃ | Et |
| 1-32 | 3-CF₃ | 3-Cl, 5-CN | Et |
| 1-33 | 3-CF₃ | 3-Cl, 5-SF₅ | Et |
| 1-34 | 3-CF₃ | 3-Cl, 5-COMe | Et |
| 1-35 | 3-CF₃ | 3-Cl, 5-COCF₃ | Et |
| 1-36 | 3-CF₃ | 3-Cl, 5-CO₂Me | Et |
| 1-37 | 3-CF₃ | 3-Cl, 5-CONH₂ | Et |
| 1-38 | 3-CF₃ | 3-Cl, 5-CONHMe | Et |
| 1-39 | 3-CF₃ | 3-Cl, 5-CONMe₂ | Et |
| 1-40 | 3-CF₃ | 3-Cl, 5-CONHCH₂CF₃ | Et |
| 1-41 | 3-CF₃ | 3-Cl, 5-CON(CH₂CF₃)₂ | Et |
| 1-42 | 3-CF₃ | 5-Br | Et |
| 1-43 | 3-CF₃ | 3-Br | Et |
| 1-44 | 3-CF₃ | 5-Me | Et |
| 1-45 | 3-CF₃ | 3-CF₃ | Et |
| 1-46 | 3-CF₃ | 3-Me | Et |
| 1-47 | 3-CF₃ | 3-C≡CH, 5-CF₃ | Et |
| 1-48 | 3-CF₃ | 3-C(CH₃)=CH₂, 5-CF₃ | Et |
| 1-49 | 3-CF₃ | 3-cPr, 5-CF₃ | Et |
| 1-50 | 3-CF₃ | n=0 | Et |
| 1-51 | 3-CF₃ | 3-Cl, 6-Cl | Et |
| 1-52 | 3-CF₃ | 3-Cl, 6-CF₃ | Et |
| 1-53 | 3-CF₃ | 4-CF₃ | Et |
| 1-54 | 3-CF₃ | 6-CF₃ | Et |
| 1-55 | 3-CF₃ | 6-Br | Et |
| 1-56 | 3-CF₃ | 6-Me | Et |
| 1-57 | 3-CF₃ | 3-Cl, 5-CF₃ | Et |
| 1-58 | 3-C CH | 3-Cl, 5-CF₃ | Et |
| 1-59 | 3-CH=CH₂ | 3-Cl, 5-CF₃ | Et |
| 1-60 | 3-Cl | 3-Cl, 5-CF₃ | Et |
| 1-61 | 3-OMe | 3-Cl, 5-CF₃ | Et |
| 1-62 | 3-OH | 3-Cl, 5-CF₃ | Et |
| 1-63 | a=0 | 3-Cl, 5-CF₃ | Et |
| 1-64 | 3-I | 3-Cl, 5-CF₃ | Et |
| 1-65 | 3-Br | 3-Cl, 5-CF₃ | Et |
| 1-66 | 3-C≡CMe | 3-Cl, 5-CF₃ | Et |
| 1-67 | 3-Me | 3-Cl, 5-CF₃ | Et |
| 1-68 | 3-Et | 3-Cl, 5-CF₃ | Et |
| 1-69 | 3-F | 3-Cl, 5-CF₃ | Et |
| 1-70 | 3-CN | 3-Cl, 5-CF₃ | Et |
| 1-71 | 3-SOMe | 3-Cl, 5-CF₃ | Et |
| 1-72 | 3-SOCF₃ | 3-Cl, 5-CF₃ | Et |
| 1-73 | 3-CH(OMe)₂ | 3-Cl, 5-CF₃ | Et |
| 1-74 | 3-SMe | 3-Cl, 5-CF₃ | Et |
| 1-75 | 3-SCF₃ | 3-Cl, 5-CF₃ | Et |
| 1-76 | 3-OCH₂CF₃ | 3-Cl, 5-CF₃ | Et |
| 1-77 | 3-NO₂ | 3-Cl, 5-CF₃ | Et |
| 1-78 | 3-CH=N-OMe | 3-Cl, 5-CF₃ | Et |
| 1-79 | 3-C(Me)=N-OMe | 3-Cl, 5-CF₃ | Et |
| 1-80 | 3-CH=N-OH | 3-Cl, 5-CF₃ | Et |
| 1-81 | 3-C(Me)=N-OH | 3-Cl, 5-CF₃ | Et |
| 1-82 | 3-NMe₂ | 3-Cl, 5-CF₃ | Et |
| 1-83 | 3-NHMe | 3-Cl, 5-CF₃ | Et |
| 1-84 | 3-NH₂ | 3-Cl, 5-CF₃ | Et |
| 1-85 | 3-N(CH₂CF₃)₂ | 3-Cl, 5-CF₃ | Et |
| 1-86 | 3-NHCH₂CF₃ | 3-Cl, 5-CF₃ | Et |
| 1-87 | 3-CHO | 3-Cl, 5-CF₃ | Et |
| 1-88 | 3-COMe | 3-Cl, 5-CF₃ | Et |
| 1-89 | 3-COCF₃ | 3-Cl, 5-CF₃ | Et |
| 1-90 | 3-CO₂Me | 3-Cl, 5-CF₃ | Et |
| 1-91 | 3-CONH₂ | 3-Cl, 5-CF₃ | Et |
| 1-92 | 3-CONHMe | 3-Cl, 5-CF₃ | Et |
| 1-93 | 3-CONMe₂ | 3-Cl, 5-CF₃ | Et |
| 1-94 | 3-CONHCH₂CF₃ | 3-Cl, 5-CF₃ | Et |
| 1-95 | 3-CON(CH₂CF₃)₂ | 3-Cl, 5-CF₃ | Et |
| 1-96 | 3-iPr | 3-Cl, 5-CF₃ | Et |
| 1-97 | 3-SO₂Me | 3-Cl, 5-CF₃ | Et |
| 1-98 | 3-SO₂CF₃ | 3-Cl, 5-CF₃ | Et |
| 1-99 | 3-tBu | 3-Cl, 5-CF₃ | Et |
| 1-100 | 3-CF₂H | 3-Cl, 5-CF₃ | Et |
| 1-101 | 3-cPr | 3-Cl, 5-CF₃ | Et |
| 1-102 | 3-OcPr | 3-Cl, 5-CF₃ | Et |
| 1-103 | 3-DXOL | 3-Cl, 5-CF₃ | Et |
| 1-104 | 3-DXAN | 3-Cl, 5-CF₃ | Et |
| 1-105 | 4-F | 3-Cl, 5-CF₃ | Et |
| 1-106 | 4-Cl | 3-Cl, 5-CF₃ | Et |
| 1-107 | 4-CF₃ | 3-Cl, 5-CF₃ | Et |
| 1-108 | 4-CN | 3-Cl, 5-CF₃ | Et |
| 1-109 | 4-NO₂ | 3-Cl, 5-CF₃ | Et |
| 1-110 | 4-Br | 3-Cl, 5-CF₃ | Et |
| 1-111 | 4-OMe | 3-Cl, 5-CF₃ | Et |
| 1-112 | 4-OCF₃ | 3-Cl, 5-CF₃ | Et |
| 1-113 | 4-Me | 3-Cl, 5-CF₃ | Et |
| 1-114 | 4-cPr | 3-Cl, 5-CF₃ | Et |
| 1-115 | 4-I | 3-Cl, 5-CF₃ | Et |
| 1-116 | 4-SMe | 3-Cl, 5-CF₃ | Et |
| 1-117 | 4-SCF₃ | 3-Cl, 5-CF₃ | Et |
| 1-118 | 4-SO₂Me | 3-Cl, 5-CF₃ | Et |
| 1-119 | 4-SO₂CF₃ | 3-Cl, 5-CF₃ | Et |
| 1-120 | 4-NH₂ | 3-Cl, 5-CF₃ | Et |
| 1-121 | 4-NHMe | 3-Cl, 5-CF₃ | Et |
| 1-122 | 4-NMe₂ | 3-Cl, 5-CF₃ | Et |
| 1-123 | 4-COMe | 3-Cl, 5-CF₃ | Et |
| 1-124 | 4-COCF₃ | 3-Cl, 5-CF₃ | Et |
| 1-125 | 5-Cl | 3-Cl, 5-CF₃ | Et |
| 1-126 | 5-Me | 3-Cl, 5-CF₃ | Et |
| 1-127 | 5-CF₃ | 3-Cl, 5-CF₃ | Et |
| 1-128 | 3-Br, 4-Br | 3-Cl, 5-CF₃ | Et |
| 1-129 | 3-Cl, 4-Cl | 3-Cl, 5-CF₃ | Et |
| 1-130 | 3-Me, 4-Me | 3-Cl, 5-CF₃ | Et |
| 1-131 | 3-CF₃, 4-NO₂ | 3-Cl, 5-CF₃ | Et |
| 1-132 | 3-I, 4-NO₂ | 3-Cl, 5-CF₃ | Et |
| 1-133 | 3-Br, 4-NO₂ | 3-Cl, 5-CF₃ | Et |
| 1-134 | 3-Br, 4-NO₂ | 3-Cl, 5-CF₃ | Me |
| 1-135 | 3-Cl, 4-NO₂ | 3-Cl, 5-CF₃ | Et |
| 1-136 | 3-CF₃, 4-Cl | 3-Cl, 5-CF₃ | Et |
| 1-137 | 3-CF₃, 4-Cl | 3-F, 5-CF₃ | Et |
| 1-138 | 3-CF₃, 4-Br | 3-Cl, 5-CF₃ | Et |
| 1-139 | 3-OMe, 4-NO₂ | 3-Cl, 5-CF₃ | Et |
| 1-140 | 3-Cl, 4-CF₃ | 3-Cl, 5-CF₃ | Et |
| 1-141 | 3-Cl, 4-CF₃ | 3-Cl, 5-CF₃ | Me |
| 1-142 | 3-Cl, 4-CF₃ | 3-Cl, 5-CF₃ | iPr |
| 1-143 | 3-Cl, 4-CF₃ | 3-F, 5-CF₃ | Et |
| 1-144 | 3-Cl, 4-CF₃ | 3-Br, 5-CF₃ | Et |
| 1-145 | 3-Cl, 4-CF₃ | 3-Et, 5-CF₃ | Et |
| 1-146 | 3-Br, 4-CF₃ | 3-Cl, 5-CF₃ | Et |
| 1-147 | 3-NO₂, 4-CF₃ | 3-Cl, 5-CF₃ | Et |
| 1-148 | 3-1, 4-Cl | 3-Cl, 5-CF₃ | Et |
| 1-149 | 3-Br, 4-Cl | 3-Cl, 5-CF₃ | Et |
| 1-150 | 3-NO₂, 4-Cl | 3-Cl, 5-CF₃ | Et |
| 1-151 | 3-Cl, 4-Br | 3-Cl, 5-CF₃ | Et |
| 1-152 | 3-Cl, 4-Br | 3-Cl, 5-CF₃ | Me |
| 1-153 | 3-Cl, 4-Br | 3-F, 5-CF₃ | Et |
| 1-154 | 3-Cl, 4-Br | 3-Br, 5-CF₃ | Et |
| 1-155 | 3-Cl, 4-I | 3-Cl, 5-CF₃ | Et |
| 1-156 | 3-Me, 4-Br | 3-Cl, 5-CF₃ | Et |
| 1-157 | 3-Me, 4-Cl | 3-Cl, 5-CF₃ | Et |
| 1-158 | 3-Me, 4-Cl | 3-Cl, 5-CF₃ | Me |
| 1-159 | 3-Me, 4-Cl | 3-F, 5-CF₃ | Et |
| 1-160 | 3-OMe, 4-Cl | 3-Cl, 5-CF₃ | Et |
| 1-161 | 3-Me, 4-I | 3-Cl, 5-CF₃ | Et |
| 1-162 | 3-OMe, 4-I | 3-Cl, 5-CF₃ | Et |
| 1-163 | 3-OMe, 4-Br | 3-Cl, 5-CF₃ | Et |
| 1-164 | 3-Br, 4-F | 3-Cl, 5-CF₃ | Et |
| 1-165 | 3-Br, 4-Me | 3-Cl, 5-CF₃ | Et |
| 1-166 | 3-NO₂, 4-Me | 3-Cl, 5-CF₃ | Et |
| 1-167 | 3-Cl, 4-Me | 3-Cl, 5-CF₃ | Et |
| 1-168 | 3-Cl, 4-SMe | 3-Cl, 5-CF₃ | Et |
| 1-169 | 3-Cl, 4-SO₂Me | 3-Cl, 5-CF₃ | Et |
| 1-170 | 3-iPr, 4-Cl | 3-Cl, 5-CF₃ | Et |
| 1-171 | 3-Et, 4-Cl | 3-Cl, 5-CF₃ | Et |
| 1-172 | 3-iPr, 4-Br | 3-Cl, 5-CF₃ | Et |
| 1-173 | 3-iPr, 4-I | 3-Cl, 5-CF₃ | Et |
| 1-174 | 3-Cl, 4-NH₂ | 3-Cl, 5-CF₃ | Et |
| 1-175 | 3-cPr, 4-Cl | 3-Cl, 5-CF₃ | Et |
| 1-176 | 3-Et, 4-Br | 3-Cl, 5-CF₃ | Et |
| 1-177 | 3-Et, 4-I | 3-Cl, 5-CF₃ | Et |
| 1-178 | 3-cPr, 4-Br | 3-Cl, 5-CF₃ | Et |
| 1-179 | 3-cPr, 4-I | 3-Cl, 5-CF₃ | Et |
| 1-180 | 3-CH(OMe)₂, 4-I | 3-Cl, 5-CF₃ | Et |
| 1-181 | 3-CHO, 4-I | 3-Cl, 5-CF₃ | Et |
| 1-182 | 3-CH=N-OH, 4-I | 3-Cl, 5-CF₃ | Et |
| 1-183 | 3-CN, 4-I | 3-Cl, 5-CF₃ | Et |
| 1-184 | 3-CH(OMe)₂, 4-Br | 3-Cl, 5-CF₃ | Et |
| 1-185 | 3-CH=N-OMe, 4-I | 3-Cl, 5-CF₃ | Et |
| 1-186 | 3-CH=N-OEt, 4-I | 3-Cl, 5-CF₃ | Et |
| 1-187 | 3-CHO, 4-Br | 3-Cl, 5-CF₃ | Et |
| 1-188 | 3-CH=N-OH, 4-Br | 3-Cl, 5-CF₃ | Et |
| 1-189 | 3-CN, 4-Br | 3-Cl, 5-CF₃ | Et |
| 1-190 | 3-F, 4-Br | 3-Cl, 5-CF₃ | Et |
| 1-191 | 3-CH(OMe)₂, 4-Cl | 3-Cl, 5-CF₃ | Et |
| 1-192 | 3-CF₃, 4-I | 3-Cl, 5-CF₃ | Et |
| 1-193 | 3-CF₃, 4-I | 3-Cl, 5-CF₃ | Me |
| 1-194 | 3-CF₃, 4-I | 3-F, 5-CF₃ | Et |
| 1-195 | 3-Me, 4-F | 3-Cl, 5-CF₃ | Et |
| 1-196 | 3-Me, 4-OMe | 3-Cl, 5-CF₃ | Et |
| 1-197 | 3-DXAN, 4-Br | 3-Cl, 5-CF₃ | Et |
| 1-198 | 3-DXAN, 4-Cl | 3-Cl, 5-CF₃ | Et |
| 1-199 | 3-Me, 5-Me | 3-Cl, 5-CF₃ | Et |
| 1-200 | 3-Cl, 5-Cl | 3-Cl, 5-CF₃ | Et |
| 1-201 | 3-CF₃, 5-Me | 3-Cl, 5-CF₃ | Et |
| 1-202 | 4-Cl, 5-Me | 3-Cl, 5-CF₃ | Et |
| 1-203 | 4-Cl, 5-Cl | 3-Cl, 5-CF₃ | Et |
| 1-204 | 3-I | 3-F, 5-CF₃ | Et |
| 1-205 | 3-Br | 3-F, 5-CF₃ | Et |
| 1-206 | 3-Cl | 3-F, 5-CF₃ | Et |
| 1-207 | 3-I | 3-Br, 5-CF₃ | Et |
| 1-208 | 3-Br | 3-Br, 5-CF₃ | Et |
| 1-209 | 3-Cl | 3-Br, 5-CF₃ | Et |
| 1-210 | 3-Cl | 3-Et, 5-CF₃ | Et |
| 1-211 | 3-Cl | 3-Br, 5-CF₃ | Me |
| 1-212 | 3-F | 3-Br, 5-CF₃ | Et |
| 1-213 | 3-I | 3-Me, 5-CF₃ | Et |
| 1-214 | 4-F | 3-F, 5-CF₃ | Et |
| 1-215 | 4-NO₂ | 3-F, 5-CF₃ | Et |
| 1-216 | 4-Cl | 3-F, 5-CF₃ | Et |
| 1-217 | 4-F | 3-Br, 5-CF₃ | Et |
| 1-218 | 4-Cl | 3-Br, 5-CF₃ | Et |
| 1-219 | 4-Cl | 3-Et, 5-CF₃ | Et |
| 1-220 | 4-F | 3-Me, 5-CF₃ | Et |
| 1-221 | 4-Cl | 3-Me, 5-CF₃ | Et |
| 1-222 | 4-F | 3-CH=CH₂, 5-CF₃ | Et |
| 1-223 | 4-F | 3-Et, 5-CF₃ | Et |
| 1-224 | 3-I, 4-NO₂ | 3-Br, 5-CF₃ | Et |
| 1-225 | 3-I, 4-NO₂ | 3-F, 5-CF₃ | Et |
| 1-226 | 3-Br, 4-NO₂ | 3-Br, 5-CF₃ | Et |
| 1-227 | 3-Cl, 4-NO₂ | 3-Br, 5-CF₃ | Et |
| 1-228 | 3-Cl, 4-NO₂ | 3-Br, 5-CF₃ | Me |
| 1-229 | 3-Cl, 4-NO₂ | 3-Br, 5-CF₃ | iPr |
| 1-230 | 3-Cl, 4-NO₂ | 3-Et, 5-CF₃ | Et |
| 1-231 | 3-Cl, 4-NO₂ | 3-F, 5-CF₃ | Et |
| 1-232 | 3-Br, 4-NO₂ | 3-F, 5-CF₃ | Et |
| 1-233 | 3-Me, 4-NO₂ | 3-Cl, 5-CF₃ | Et |
| 1-234 | 3-Me, 4-NO₂ | 3-Cl, 5-CF₃ | Me |
| 1-235 | 3-Me, 4-NO₂ | 3-Br, 5-CF₃ | Et |
| 1-236 | 3-Me, 4-NO₂ | 3-F, 5-CF₃ | Et |
| 1-237 | 3-CF₃, 4-Br | 3-Br, 5-CF₃ | Et |
| 1-238 | 3-CF₃, 4-Br | 3-Br, 5-CF₃ | Me |
| 1-239 | 3-CF₃, 4-Cl | 3-Br, 5-CF₃ | Et |
| 1-240 | 3-OMe, 4-NO₂ | 3-F, 5-CF₃ | Et |
| 1-241 | 3-OMe, 4-NO₂ | 3-Br, 5-CF₃ | Et |
| 1-242 | 3-CF₃, 4-Cl | 3-Me, 5-CF₃ | Et |
| 1-243 | 3-Br, 4-F | 3-Me, 5-CF₃ | Et |
| 1-244 | 3-CF₃, 4-Cl | 3-CH=CH₂, 5-CF₃ | Et |
| 1-245 | 3-CF₃, 4-Cl | 3-Et, 5-CF₃ | Et |
| 1-246 | 3-CF₃, 4-Cl | 3-C(Me)=CH₂, 5-CF₃ | Et |
| 1-247 | 3-CF₃, 4-Cl | 3-iPr, 5-CF₃ | Et |
| 1-248 | 3-CF₃, 4-Cl | 3-C≡CH, 5-CF₃ | Et |
| 1-249 | 3-Cl, 4-Me | 3-Br, 5-CF₃ | Et |
| 1-250 | 3-Cl, 4-Me | 3-Me, 5-CF₃ | Et |
| 1-251 | 3-Me, 4-Cl | 3-Br, 5-CF₃ | Et |
| 1-252 | 3-Me, 4-Cl | 3-Me, 5-CF₃ | Et |
| 1-253 | 3-CF₃, 4-I | 3-Br, 5-CF₃ | Et |
| 1-254 | 3-CF₃, 4-Cl | 3-CF₃, 5-Cl | Et |
| 1-255 | 3-CF₃, 4-Cl | 3-CF₃, 5-Br | Et |
| 1-256 | 3-CF₃ | 3-Cl, 5-CF₃ | Me |
| 1-257 | 3-CF₃ | 3-Cl, 5-CF₃ | nPr |
| 1-258 | 3-CF₃ | 3-Cl, 5-CF₃ | iPr |
| 1-259 | 3-CF₃ | 3-Cl, 5-CF₃ | sBu |
| 1-260 | 3-CF₃ | 3-Cl, 5-CF₃ | tBu |
| 1-261 | 3-CF₃ | 3-Cl, 5-CF₃ | CH₂iPr |
| 1-262 | 3-CF₃ | 3-Cl, 5-CF₃ | cPr |
| 1-263 | 3-CF₃ | 3-Cl, 5-CF₃ | cPen |
| 1-264 | 3-CF₃ | 3-Cl, 5-CF₃ | cHex |
| 1-265 | 3-CF₃ | 3-Cl, 5-CF₃ | CH=CH₂ |
| 1-266 | 3-CF₃ | 3-Cl, 5-CF₃ | C≡CH |
| 1-267 | 3-CF₃ | 3-Cl, 5-CF₃ | CH₂OMe |
| 1-268 | 3-CF₃ | 3-Cl, 5-CF₃ | CH₂cPr |
| 1-269 | 3-CF₃ | 3-Cl, 5-CF₃ | CF₃ |
| 1-270 | 3-CF₃ | 3-Cl, 5-CF₃ | CF₂H |
| 1-271 | 3-CF₃ | 3-Cl, 5-CF₃ | CBr₂H |
| 1-272 | 3-CF₃ | 3-Cl, 5-CF₃ | CBrH₂ |
| 1-273 | 3-CF₃ | 3-Br, 5-CF₃ | iPr |
| 1-274 | 3-CF₃ | 3-Br, 5-CF₃ | cPr |
| 1-275 | 3-CF₃ | 3-F, 5-CF₃ | cPr |
| 1-276 | 3-CF₃ | 3-F, 5-CF₃ | iPr |
| 1-277 | 3-CF₃ | 3-Br, 5-CF₃ | Me |
| 1-278 | 3-CF₃ | 3-Me, 5-CF₃ | iPr |
| 1-279 | 3-CF₃ | 3-Et, 5-CF₃ | iPr |
| 1-280 | 3-CF₃ | 3-Me, 5-CF₃ | Me |
| 1-281 | 3-Cl | 3-Me, 5-CF₃ | iPr |
| 1-282 | 4-F | 3-Me, 5-CF₃ | iPr |
| 1-283 | 3-CF₃ | 3-Me, 5-CF₃ | cPr |
| 1-284 | 3-CF₃ | 3-F, 5-CF₃ | Me |
| 1-285 | 4-F | 3-Cl, 5-CF₃ | iPr |
| 1-286 | 3-CF₃, 4-Cl | 3-Cl, 5-CF₃ | iPr |
| 1-287 | 3-Cl, 4-NO₂ | 3-Cl, 5-CF₃ | iPr |
| 1-288 | 3-Cl, 4-NO₂ | 3-F, 5-CF₃ | iPr |
| 1-289 | 3-CF₃, 4-Cl | 3-Cl, 5-CF₃ | Me |
| 1-290 | 3-CF₃, 4-Cl | 3-F, 5-CF₃ | Me |
| 1-291 | 3-Cl, 4-NO₂ | 3-Cl, 5-CF₃ | Me |
| 1-292 | 3-CF₃, 4-Cl | 3-Br, 5-CF₃ | iPr |
| 1-293 | 3-CF₃, 4-Cl | 3-Br, 5-CF₃ | Me |
| 1-294 | 3-Br, 4-CF₃ | 3-Br, 5-CF₃ | Et |
| 1-295 | 3-Br, 4-NO₂ | 3-Et, 5-CF₃ | Et |
| 1-296 | 3-CF₃, 4-Me | 3-Cl, 5-CF₃ | Et |
| 1-297 | 3-CF₃, 4-Me | 3-Br, 5-CF₃ | Et |
| 1-298 | 3-CF₃, 4-Me | 3-Me, 5-CF₃ | Et |
| 1-299 | 3-Me, 4-CF₃ | 3-Me, 5-CF₃ | Et |
| 1-300 | 3-Br, 4-CF₃ | 3-Cl, 5-CF₃ | Me |
| 1-301 | 3-CF₃, 4-Cl | 3-Me, 5-CF₃ | iPr |

**Table 2**

| 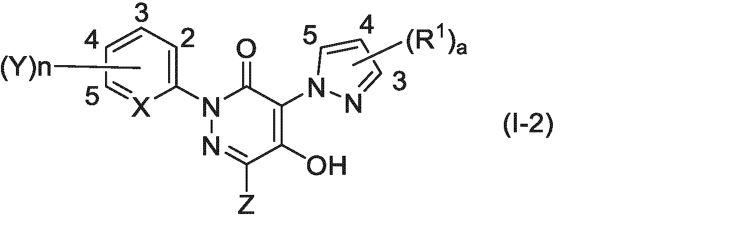 | | | | |
|---|---|---|---|---|
| No. | (R¹)ₐ | X | (Y)ₙ | Z |
| 2-1 | 3-CF₃ | C-H | n=0 | Et |
| 2-2 | 3-CF₃ | C-H | 2-Me | Et |
| 2-3 | 3-CF₃ | C-H | 2-CF₃ | Et |
| 2-4 | 3-CF₃ | C-H | 3-CF₃ | Et |
| 2-5 | 3-CF₃ | C-H | 3-Me | Et |
| 2-6 | 3-CF₃ | C-H | 4-CF₃ | Et |
| 2-7 | 3-CF₃ | C-H | 4-Me | Et |
| 2-8 | 3-CF₃ | C-H | 2-Cl, 3-Cl | Et |
| 2-9 | 3-CF₃ | C-H | 2-Me, 3-CF₃ | Et |
| 2-10 | 3-CF₃ | C-H | 2-Cl, 3-CF₃ | Et |
| 2-11 | 3-CF₃ | C-H | 2-CF₃, 3-Cl | Et |
| 2-12 | 3-CF₃ | C-H | 2-Cl, 4-CF₃ | Et |
| 2-13 | 3-CF₃ | C-H | 2-Cl, 4-Cl | Et |
| 2-14 | 3-CF₃ | C-H | 2-Br, 4-CF₃ | Et |
| 2-15 | 3-CF₃ | C-H | 2-Me, 4-CF₃ | Et |
| 2-16 | 3-CF₃ | C-H | 2-Me, 4-CF₃ | Me |
| 2-17 | 3-CF₃ | C-H | 2-Me, 4-CF₃ | iPr |
| 2-18 | 3-CF₃ | C-H | 2-Cl, 4-F | Et |
| 2-19 | 3-CF₃ | C-H | 2-Cl, 4-Br | Et |
| 2-20 | 3-CF₃ | C-H | 2-l, 4-CF₃ | Et |
| 2-21 | 3-CF₃ | C-H | 2-I, 4-CF₃ | Me |
| 2-22 | 3-CF₃ | C-H | 2-I, 4-CF₃ | iPr |
| 2-23 | 3-CF₃ | C-H | 2-I, 4-Cl | Et |
| 2-24 | 3-CF₃ | C-H | 2-Cl, 4-I | Et |
| 2-25 | 3-CF₃ | C-H | 2-F, 4-Cl | Et |
| 2-26 | 3-CF₃ | C-H | 2-CF₃, 4-CF₃ | Et |
| 2-27 | 3-CF₃ | C-H | 2-CF₃, 4-Cl | Et |
| 2-28 | 3-CF₃ | C-H | 2-CH=CH₂, 4-CF₃ | Et |
| 2-29 | 3-CF₃ | C-H | 2-Et, 4-CF₃ | Et |
| 2-30 | 3-CF₃ | C-H | 2-Cl, 4-OMe | Et |
| 2-31 | 3-CF₃ | C-H | 2-CN, 4-CF₃ | Et |
| 2-32 | 3-CF₃ | C-H | 2-Me, 4-OCF₃ | Et |
| 2-33 | 3-CF₃ | C-H | 2-Me, 4-OCF₃ | Me |
| 2-34 | 3-CF₃ | C-H | 2-Me, 4-OCF₃ | iPr |
| 2-35 | 3-CF₃ | C-H | 2-CF₃, 4-OMe | Et |
| 2-36 | 3-CF₃ | C-H | 2-Cl, 4-OCF₃ | Et |
| 2-37 | 3-CF₃ | C-H | 2-OMe, 4-CF₃ | Et |
| 2-38 | 3-CF₃ | C-H | 2-C(Me)=CH₂, 4-CF₃ | Et |
| 2-39 | 3-CF₃ | C-H | 2-cPr, 4-CF₃ | Et |
| 2-40 | 3-CF₃ | C-H | 2-iPr, 4-CF₃ | Et |
| 2-41 | 3-CF₃ | C-H | 2-F, 4-CF₃ | Et |
| 2-42 | 3-CF₃ | C-H | 2-C≡CH, 4-CF₃ | Et |
| 2-43 | 3-CF₃ | C-H | 2-Me, 4-OCF₂H | Et |
| 2-44 | 3-CF₃ | C-H | 2-Me, 4-SO₂Me | Et |
| 2-45 | 3-CF₃ | C-H | 2-Me, 4-CH₂CF₃ | Et |
| 2-46 | 3-CF₃ | C-H | 2-Cl, 5-Cl | Et |
| 2-47 | 3-CF₃ | C-H | 2-Me, 5-Me | Et |
| 2-48 | 3-CF₃ | C-H | 2-Cl, 5-CF₃ | Et |
| 2-49 | 3-CF₃ | C-H | 2-Br, 5-Br | Et |
| 2-50 | 3-CF₃ | C-H | 2-F, 5-F | Et |
| 2-51 | 3-CF₃ | C-H | 2-Cl, 5-Me | Et |
| 2-52 | 3-CF₃ | C-H | 2-Me, 5-Cl | Et |
| 2-53 | 3-CF₃ | C-H | 2-Cl, 5-OMe | Et |
| 2-54 | 3-CF₃ | C-H | 2-Br, 5-F | Et |
| 2-55 | 3-CF₃ | C-H | 2-Cl, 5-F | Et |
| 2-56 | 3-CF₃ | C-H | 2-Cl, 5-OCF₃ | Et |
| 2-57 | 3-CF₃ | C-H | 2-F, 5-Br | Et |
| 2-58 | 3-CF₃ | C-H | 2-F, 5-Cl | Et |
| 2-59 | 3-CF₃ | C-H | 2-Cl, 5-Br | Et |
| 2-60 | 3-CF₃ | C-H | 2-1, 5-Cl | Et |
| 2-61 | 3-CF₃ | C-H | 2-Br, 5-Cl | Et |
| 2-62 | 3-CF₃ | C-H | 2-F, 5-I | Et |
| 2-63 | 3-CF₃ | C-H | 2-Br, 5-I | Et |
| 2-64 | 3-CF₃ | C-H | 2-Cl, 5-I | Et |
| 2-65 | 3-CF₃ | C-Cl | 2-Cl | Et |
| 2-66 | 3-CF₃ | C-Me | 2-CF₃ | Et |
| 2-67 | 3-CF₃ | C-Cl | 2-CF₃ | Et |
| 2-68 | 3-CF₃ | C-H | 3-Cl, 4-Cl | Et |
| 2-69 | 3-CF₃ | C-H | 3-F, 4-CF₃ | Et |
| 2-70 | 3-CF₃ | C-H | 3-Cl. 4-CF₃ | Et |
| 2-71 | 3-CF₃ | C-H | 3-CF₃, 4-Cl | Et |
| 2-72 | 3-CF₃ | C-H | 3-Cl, 5-Cl | Et |
| 2-73 | 3-CF₃, 4-Cl | C-Cl | 2-Cl, 4-OCF₃ | Et |
| 2-74 | 3-CF₃ | C-H | 2-CF₃, 5-Cl | Et |
| 2-75 | 3-CF₃ | C-H | 2-Me, 3-F, 4-F | Et |
| 2-76 | 3-CF₃ | C-H | 2-Cl, 3-Cl, 5-Cl | Et |
| 2-77 | 3-CF₃ | C-Cl | 2-Cl, 3-Cl | Et |
| 2-78 | 3-CF₃ | C-H | 2-Cl, 4-Cl, 5-Cl | Et |
| 2-79 | 3-CF₃ | C-H | 2-F, 4-Br, 5-F | Et |
| 2-80 | 3-CF₃ | C-H | 2-F, 4-F, 5-F | Et |
| 2-81 | 3-CF₃ | C-H | 2-F, 4-Cl, 5-F | Et |
| 2-82 | 3-CF₃ | C-H | 2-1, 4-Br, 5-F | Et |
| 2-83 | 3-CF₃ | C-H | 2-1, 4-F, 5-F | Et |
| 2-84 | 3-CF₃ | C-H | 2-1, 4-CF₃, 5-Br | Et |
| 2-85 | 3-CF₃ | C-H | 2-Br, 4-F, 5-Br | Et |
| 2-86 | 3-CF₃ | C-H | 2-F, 4-Cl, 5-Cl | Et |
| 2-87 | 3-CF₃ | C-H | 2-Br, 4-F, 5-Cl | Et |
| 2-88 | 3-CF₃ | C-H | 2-Cl, 4-Cl, 5-F | Et |
| 2-89 | 3-CF₃ | C-H | 2-Br, 4-F, 5-F | Et |
| 2-90 | 3-CF₃ | C-H | 2-F, 4-Br, 5-Cl | Et |
| 2-91 | 3-CF₃ | C-H | 2-F, 4-F, 5-Br | Et |
| 2-92 | 3-CF₃ | C-H | 2-Cl, 4-F, 5-F | Et |
| 2-93 | 3-CF₃ | C-H | 2-F, 4-CF₃, 5-F | Et |
| 2-94 | 3-CF₃ | C-H | 2-Cl, 4-CF₃, 5-F | Et |
| 2-95 | 3-CF₃ | C-H | 2-Br, 4-CF₃, 5-Cl | Et |
| 2-96 | 3-CF₃ | C-H | 2-Me, 4-F, 5-Br | Et |
| 2-97 | 3-CF₃ | C-Cl | 2-Cl, 4-CF₃ | Et |
| 2-98 | 3-CF₃ | C-Cl | 2-Cl, 4-Cl | Et |
| 2-99 | 3-CF₃ | C-Br | 2-Br, 4-CF₃ | Et |
| 2-100 | 3-CF₃ | C-Me | 2-Me, 4-CF₃ | Et |
| 2-101 | 3-CF₃ | C-F | 2-F, 4-CF₃ | Et |
| 2-102 | 3-CF₃ | C-Cl | 2-Cl, 4-OCF₃ | Et |
| 2-103 | 3-CF₃ | C-I | 2-Cl, 4-CF₃ | Et |
| 2-104 | 3-CF₃ | C-F | 2-Cl, 4-CF₃ | Et |
| 2-105 | 3-CF₃ | C-Cl | 2-Br, 4-CF₃ | Et |
| 2-106 | 3-CF₃ | C-I | 2-Br, 4-CF₃ | Et |
| 2-107 | 3-CF₃ | C-Cl | 2-Me, 4-Cl | Et |
| 2-108 | 3-CF₃ | C-Cl | 2-Cl, 4-OMe | Et |
| 2-109 | 3-CF₃ | C-Br | 2-Br, 4-OCF₃ | Et |
| 2-110 | 3-CF₃ | C-Cl | 2-Cl, 4-iPr | Et |
| 2-111 | 3-CF₃ | C-Cl | 2-Me, 4-CF₃ | Et |
| 2-112 | 3-CF₃ | C-Br | 2-Me, 4-CF₃ | Et |
| 2-113 | 3-CF₃ | C-Cl | 2-Cl, 4-tBu | Et |
| 2-114 | 3-CF₃ | C-CH=CH₂ | 2-CH=CH₂, 4-CF₃ | Et |
| 2-115 | 3-CF₃ | C-Et | 2-Et, 4-CF₃ | Et |
| 2-116 | 3-CF₃ | C-Br | 2-F, 4-Cl | Et |
| 2-117 | 3-CF₃ | C-Br | 2-Br, 4-Br | Et |
| 2-118 | 3-CF₃ | C-Me | 2-Br, 4-F | Et |
| 2-119 | 3-CF₃ | C-F | 2-Br, 4-CF₃ | Et |
| 2-120 | 3-CF₃ | C-Cl | 2-Me, 4-OCF₃ | Et |
| 2-121 | 3-CF₃ | C-Br | 2-Me, 4-OCF₃ | Et |
| 2-122 | 3-CF₃ | C-Cl | 2-Cl, 4-F | Et |
| 2-123 | 3-CF₃ | C-Me | 2-Me, 4-OCF₃ | Et |
| 2-124 | 3-CF₃ | C-Me | 2-Me, 4-OCF₃ | Me |
| 2-125 | 3-CF₃ | C-Me | 2-Me, 4-OCF₃ | iPr |
| 2-126 | 3-CF₃ | C-F | 2-F, 4-F | Et |
| 2-127 | 3-CF₃ | C-F | 2-Me, 4-CF₃ | Et |
| 2-128 | 3-CF₃ | C-Me | 2-Me, 4-F | Et |
| 2-129 | 3-CF₃ | C-Cl | 2-cPr, 4-CF₃ | Et |
| 2-130 | 3-CF₃ | C-Cl | 2-CH=CH₂, 4-CF₃ | Et |
| 2-131 | 3-CF₃ | C-Cl | 2-Et, 4-CF₃ | Et |
| 2-132 | 3-CF₃ | C-Cl | 2-Et, 4-CF₃ | Me |
| 2-133 | 3-CF₃ | C-Cl | 2-Me, 4-Br | Et |
| 2-134 | 3-CF₃ | C-Me | 2-Me, 4-Br | Et |
| 2-135 | 3-CF₃ | C-Me | 2-Me, 4-Me | Et |
| 2-136 | 3-CF₃ | C-Cl | 2-Me, 4-CF(CF₃)₂ | Et |
| 2-137 | 3-CF₃ | C-Cl | 2-F, 3-F, 4-CF₃ | Et |
| 2-138 | 3-CF₃ | C-Cl | 2-Cl, 3-F, 4-CF₃, 5-F | Et |
| 2-139 | 3-CF₃ | C-F | 2-F, 3-F, 4-CF₃, 5-F | Et |
| 2-140 | 3-F | C-Me | 2-Me, 4-CF₃ | Et |
| 2-141 | 3-F | C-Me | 2-Me, 4-CF₃ | Me |
| 2-142 | 3-F | C-Me | 2-Me, 4-CF₃ | iPr |
| 2-143 | 3-Cl | C-Me | 2-Me, 4-CF₃ | Et |
| 2-144 | 3-Br | C-Me | 2-Me, 4-CF₃ | Et |
| 2-145 | 3-Br | C-Me | 2-Me, 4-CF₃ | Me |
| 2-146 | 3-Br | C-Me | 2-Me, 4-CF₃ | iPr |
| 2-147 | 3-Me | C-Me | 2-Me, 4-CF₃ | Et |
| 2-148 | 3-Me | C-Me | 2-Me, 4-CF₃ | Me |
| 2-149 | 3-Me | C-Me | 2-Me, 4-CF₃ | iPr |
| 2-150 | 3-cPr | C-Me | 2-Me, 4-CF₃ | Et |
| 2-151 | 3-OMe | C-Me | 2-Me, 4-CF₃ | Et |
| 2-152 | 3-OCF₃ | C-Me | 2-Me, 4-CF₃ | Et |
| 2-153 | 3-SOMe | C-Me | 2-Me, 4-CF₃ | Et |
| 2-154 | 3-SO₂Me | C-Me | 2-Me, 4-CF₃ | Et |
| 2-155 | 3-SCF₃ | C-Me | 2-Me, 4-CF₃ | Et |
| 2-156 | 3-SOCF₃ | C-Me | 2-Me, 4-CF₃ | Et |
| 2-157 | 3-SO₂CF₃ | C-Me | 2-Me, 4-CF₃ | Et |
| 2-158 | 3-CN | C-Me | 2-Me, 4-CF₃ | Et |
| 2-159 | 3-NO₂ | C-Me | 2-Me, 4-CF₃ | Et |
| 2-160 | 3-NMe₂ | C-Me | 2-Me, 4-CF₃ | Et |
| 2-161 | 3-NHMe | C-Me | 2-Me, 4-CF₃ | Et |
| 2-162 | 3-NH₂ | C-Me | 2-Me, 4-CF₃ | Et |
| 2-163 | 3-N(CH₂CF₃)₂ | C-Me | 2-Me, 4-CF₃ | Et |
| 2-164 | 3-NHCH₂CF₃ | C-Me | 2-Me, 4-CF₃ | Et |
| 2-165 | 3-COCF₃ | C-Me | 2-Me, 4-CF₃ | Et |
| 2-166 | 3-CO₂Me | C-Me | 2-Me, 4-CF₃ | Et |
| 2-167 | 3-I | C-Cl | 2-Cl, 4-CF₃ | Et |
| 2-168 | 3-Cl | C-Cl | 2-Cl, 4-CF₃ | Et |
| 2-169 | 3-F | C-Cl | 2-Cl, 4-CF₃ | Et |
| 2-170 | 3-F | C-Cl | 2-Cl, 4-CF₃ | Me |
| 2-171 | 3-Me | C-Cl | 2-Me, 4-OCF₃ | Et |
| 2-172 | 3-Me | C-Cl | 2-Me, 4-CF₃ | Et |
| 2-173 | 3-Cl | C-Cl | 2-Me, 4-CF₃ | Et |
| 2-174 | 3-Cl | C-Cl | 2-Me, 4-CF₃ | Me |
| 2-175 | 3-Cl | C-Cl | 2-Me, 4-OCF₃ | Et |
| 2-176 | 3-Cl | C-Cl | 2-Me, 4-OCF₃ | Me |
| 2-177 | 3-Br | C-Cl | 2-Me, 4-CF₃ | Et |
| 2-178 | 3-Br | C-Cl | 2-Me, 4-OCF₃ | Et |
| 2-179 | 3-F | C-Cl | 2-Me, 4-OCF₃ | Et |
| 2-180 | a=0 | C-Cl | 2-Cl, 4-CF₃ | Et |
| 2-181 | a=0 | C-H | 2-Me, 4-CF₃ | Et |
| 2-182 | a=0 | C-Cl | 2-Me, 4-CF₃ | Et |
| 2-183 | a=0 | C-Cl | 2-Me, 4-OCF₃ | Et |
| 2-184 | a=0 | C-Me | 2-Me, 4-CF₃ | Et |
| 2-185 | a=0 | C-Me | 2-Me, 4-OCF₃ | Et |
| 2-186 | 3-Cl | C-Me | 2-Me, 4-OCF₃ | Et |
| 2-187 | 3-Cl | C-Me | 2-Me, 4-OCF₃ | Me |
| 2-188 | 3-Cl | C-Me | 2-Me, 4-cPr | Et |
| 2-189 | 3-Cl | C-Me | 2-Me, 4-SCF₃ | Et |
| 2-190 | 3-Cl | C-Me | 2-Me, 4-SOCF₃ | Et |
| 2-191 | 3-Cl | C-Me | 2-Me, 4-SO₂CF₃ | Et |
| 2-192 | 3-I | C-Me | 2-Me, 4-CF₃ | Et |
| 2-193 | 3-I | C-Me | 2-Me, 4-CF₃ | Me |
| 2-194 | 3-I | C-Me | 2-Me, 4-CF₃ | iPr |
| 2-195 | 3-Br | C-Me | 2-Me, 4-OCF₃ | Et |
| 2-196 | 3-Br | C-Me | 2-Me, 4-OCF₃ | Me |
| 2-197 | 3-CF₃ | C-Me | 2-Me, 4-Cl | Et |
| 2-198 | 3-Cl | C-Me | 2-Me, 4-Cl | Et |
| 2-199 | 3-Cl | C-Me | 2-Me, 4-CN | Et |
| 2-200 | 3-Cl | C-Me | 2-Me, 4-NO₂ | Et |
| 2-201 | 3-Cl | C-Me | 2-Me, 4-CO₂Me | Et |
| 2-202 | 3-Cl | C-Me | 2-Me, 4-COMe | Et |
| 2-203 | 3-Cl | C-Me | 2-Me, 4-COCF₃ | Et |
| 2-204 | 3-Cl | C-Me | 2-Me, 4-SMe | Et |
| 2-205 | 3-Cl | C-Me | 2-Me, 4-SOMe | Et |
| 2-206 | 3-Cl | C-Me | 2-Me, 4-SO₂Me | Et |
| 2-207 | 3-Cl | C-Me | 2-Me, 4-SO₂Et | Et |
| 2-208 | 3-Cl | C-Me | 2-Me, 4-CH₂CF₃ | Et |
| 2-209 | 3-Cl | C-Me | 2-Me, 4-OCF₂H | Et |
| 2-210 | 3-Cl | C-Me | 2-Me, 4-CF₂CF₃ | Et |
| 2-211 | 3-Cl | C-Me | 2-Me, 4-CF₂H | Et |
| 2-212 | 3-Cl | C-Me | 2-Me, 4-CHO | Et |
| 2-213 | 3-Cl | C-Me | 2-Me, 4-CO₂H | Et |
| 2-214 | 3-Cl | C-Me | 2-Me, 4-OH | Et |
| 2-215 | 3-Cl | C-Me | 2-Me, 4-C≡CH | Et |
| 2-216 | 3-Cl | C-Me | 2-Me, 4-CH=CH₂ | Et |
| 2-217 | 3-S(nPr) | C-Me | 2-Me, 4-CF₃ | Et |
| 2-218 | 3-Cl | C-Me | 2-Me, 4-Br | Et |
| 2-219 | 3-Cl | C-Me | 2-Me, 4-Br | Me |
| 2-220 | 3-Cl | C-Me | 2-Me, 4-Br | iPr |
| 2-221 | 3-Cl | C-Me | 2-Me, 4-SF₅ | Et |
| 2-222 | 3-Cl | C-H | 2-Me, 4-CF₃ | Et |
| 2-223 | 3-Cl | C-H | 2-Me, 4-OCF₃ | Et |
| 2-224 | 3-Cl | C-CH=CH₂ | 2-Me, 4-CF₃ | Et |
| 2-225 | 3-Cl | C-Et | 2-Me, 4-CF₃ | Et |
| 2-226 | 3-Cl | C-C≡CH | 2-Me, 4-CF₃ | Et |
| 2-227 | 3-Cl | C-OMe | 2-Me, 4-CF₃ | Et |
| 2-228 | 3-Cl | C-OH | 2-Me, 4-CF₃ | Et |
| 2-229 | 3-Cl | C-NH₂ | 2-Me, 4-CF₃ | Et |
| 2-230 | 3-Cl | C-NHMe | 2-Me, 4-CF₃ | Et |
| 2-231 | 3-Cl | C-NMe₂ | 2-Me, 4-CF₃ | Et |
| 2-232 | 3-Cl | C-NHCH₂CF₃ | 2-Me, 4-CF₃ | Et |
| 2-233 | 3-Cl | C-N(CH₂CF₃)₂ | 2-Me, 4-CF₃ | Et |
| 2-234 | 4-F | C-Me | 2-Me, 4-CF₃ | Et |
| 2-235 | 4-Cl | C-Me | 2-Me, 4-CF₃ | Et |
| 2-236 | 4-Cl | C-Me | 2-Me, 4-CF₃ | Me |
| 2-237 | 4-Cl | C-Me | 2-Me, 4-CF₃ | iPr |
| 2-238 | 4-Br | C-Me | 2-Me, 4-CF₃ | Et |
| 2-239 | 4-Br | C-Me | 2-Me, 4-CF₃ | Me |
| 2-240 | 4-Br | C-Me | 2-Me, 4-CF₃ | iPr |
| 2-241 | 4-Me | C-Me | 2-Me, 4-CF₃ | Et |
| 2-242 | 4-Me | C-Me | 2-Me, 4-CF₃ | Me |
| 2-243 | 4-Me | C-Me | 2-Me, 4-CF₃ | iPr |
| 2-244 | 4-cPr | C-Me | 2-Me, 4-CF₃ | Et |
| 2-245 | 4-OMe | C-Me | 2-Me, 4-CF₃ | Et |
| 2-246 | 4-OCF₃ | C-Me | 2-Me, 4-CF₃ | Et |
| 2-247 | 4-SMe | C-Me | 2-Me, 4-CF₃ | Et |
| 2-248 | 4-SOMe | C-Me | 2-Me, 4-CF₃ | Et |
| 2-249 | 4-SO₂Me | C-Me | 2-Me, 4-CF₃ | Et |
| 2-250 | 4-SCF₃ | C-Me | 2-Me, 4-CF₃ | Et |
| 2-251 | 4-SOCFs | C-Me | 2-Me, 4-CF₃ | Et |
| 2-252 | 4-SO₂CF₃ | C-Me | 2-Me, 4-CF₃ | Et |
| 2-253 | 4-CN | C-Me | 2-Me, 4-CF₃ | Et |
| 2-254 | 4-NO₂ | C-Me | 2-Me, 4-CF₃ | Et |
| 2-255 | 4-NMe₂ | C-Me | 2-Me, 4-CF₃ | Et |
| 2-256 | 4-NHMe | C-Me | 2-Me, 4-CF₃ | Et |
| 2-257 | 4-NH₂ | C-Me | 2-Me, 4-CF₃ | Et |
| 2-258 | 4-N(CH₂CF₃)₂ | C-Me | 2-Me, 4-CF₃ | Et |
| 2-259 | 4-NHCH₂CF₃ | C-Me | 2-Me, 4-CF₃ | Et |
| 2-260 | 4-COCF₃ | C-Me | 2-Me, 4-CF₃ | Et |
| 2-261 | 4-CO₂Me | C-Me | 2-Me, 4-CF₃ | Et |
| 2-262 | 4-Cl | C-H | 2-Br, 4-CF₃ | Et |
| 2-263 | 4-F | C-Cl | 2-Cl, 4-CF₃ | Et |
| 2-264 | 4-F | C-Cl | 2-Cl, 4-CF₃ | Me |
| 2-265 | 4-F | C-F | 2-Cl, 4-CF₃ | Et |
| 2-266 | 4-F | C-Br | 2-Br, 4-CF₃ | Et |
| 2-267 | 4-F | C-Me | 2-Me, 4-SMe | Et |
| 2-268 | 4-F | C-Me | 2-Me, 4-SOMe | Et |
| 2-269 | 4-F | C-Me | 2-Me, 4-SO₂Me | Et |
| 2-270 | 4-F | C-H | 2-Br, 4-CF₃ | Et |
| 2-271 | 4-Cl | C-H | 2-Me, 4-CF₃ | Et |
| 2-272 | 4-Cl | C-H | 2-Me, 4-CF₃ | Me |
| 2-273 | 4-Cl | C-H | 2-Me, 4-CF₃ | iPr |
| 2-274 | 4-F | C-H | 2-Me, 4-CF₃ | Et |
| 2-275 | 4-F | C-H | 2-Me, 4-CF₃ | Me |
| 2-276 | 4-F | C-H | 2-Me, 4-CF₃ | iPr |
| 2-277 | 4-F | C-CH=CH₂ | 2-Me, 4-CF₃ | Et |
| 2-278 | 4-F | C-Et | 2-Me, 4-CF₃ | Et |
| 2-279 | 4-F | C-Cl | 2-Me, 4-OCF₃ | Et |
| 2-280 | 4-F | C-Cl | 2-Me, 4-OCF₃ | Me |
| 2-281 | 4-F | C-Br | 2-Me, 4-OCF₃ | Et |
| 2-282 | 4-F | C-Br | 2-Me, 4-OCF₃ | Me |
| 2-283 | 4-Cl | C-Br | 2-Me, 4-OCF₃ | Et |
| 2-284 | 4-F | C-Cl | 2-Me, 4-CF₃ | Et |
| 2-285 | 4-F | C-F | 2-Br, 4-CF₃ | Et |
| 2-286 | 4-Cl | C-F | 2-Br, 4-CF₃ | Et |
| 2-287 | 4-Cl | C-Br | 2-Me, 4-CF₃ | Et |
| 2-288 | 4-F | C-H | 2-Me, 4-OCF₃ | Et |
| 2-289 | 4-F | C-H | 2-Me, 4-OCF₃ | Me |
| 2-290 | 4-F | C-H | 2-Me, 4-OCF₃ | iPr |
| 2-291 | 4-F | C-Br | 2-Me, 4-CF₃ | Et |
| 2-292 | 4-Cl | C-Cl | 2-Me, 4-CF₃ | Et |
| 2-293 | 4-F | C-Cl | 2-Cl, 4-F | Et |
| 2-294 | 4-F | C-F | 2-F, 4-F | Et |
| 2-295 | 4-Cl | C-Cl | 2-Cl, 4-CF₃ | Et |
| 2-296 | 4-F | C-F | 2-Me, 4-CF₃ | Et |
| 2-297 | 4-Br | C-Cl | 2-Cl, 4-CF₃ | Et |
| 2-298 | 4-I | C-Cl | 2-Cl, 4-CF₃ | Et |
| 2-299 | 4-Cl | C-Br | 2-Br, 4-CF₃ | Et |
| 2-300 | 4-Cl | C-Cl | 2-Me, 4-OCF₃ | Et |
| 2-301 | 4-Cl | C-F | 2-Me, 4-CF₃ | Et |
| 2-302 | 4-Me | C-Cl | 2-Me, 4-CF₃ | Et |
| 2-303 | 4-Me | C-Cl | 2-Me, 4-OCF₃ | Et |
| 2-304 | 4-F | C-Me | 2-Me, 4-F | Et |
| 2-305 | 4-F | C-Me | 2-Me, 4-SCF₃ | Et |
| 2-306 | 4-F | C-Me | 2-Me, 4-SOCF₃ | Et |
| 2-307 | 4-F | C-Me | 2-Me, 4-SO₂CF₃ | Et |
| 2-308 | 4-F | C-Me | 2-Me, 4-NO₂ | Et |
| 2-309 | 4-Br | C-Cl | 2-Me, 4-CF₃ | Et |
| 2-310 | 4-Br | C-Cl | 2-Me, 4-OCF₃ | Et |
| 2-311 | 4-F | C-Cl | 2-Br, 4-CF₃ | Et |
| 2-312 | 4-F | C-Cl | 2-Br, 4-CF₃ | Me |
| 2-313 | 4-CF₃ | C-Cl | 2-Me, 4-CF₃ | Et |
| 2-314 | 4-CF₃ | C-Cl | 2-Me, 4-OCF₃ | Et |
| 2-315 | 3-Cl | C-H | 2-1, 4-CF₃ | Et |
| 2-316 | 4-F | C-H | 2-1, 4-CF₃ | Et |
| 2-317 | 4-F | C-Cl | 2-CH=CH₂, 4-CF₃ | Et |
| 2-318 | 4-F | C-Cl | 2-Et, 4-CF₃ | Et |
| 2-319 | 4-F | C-Me | 2-Me, 4-OCF₃ | Et |
| 2-320 | 4-F | C-Me | 2-Me, 4-OCF₃ | Me |
| 2-321 | 4-F | C-Me | 2-Me, 4-SF₅ | Et |
| 2-322 | 4-F | C-Me | 2-Me, 4-CO₂Me | Et |
| 2-323 | 4-F | C-F | 2-F, 4-CF₃ | Et |
| 2-324 | 4-I | C-Me | 2-Me, 4-CF₃ | Et |
| 2-325 | 4-Br | C-Me | 2-Me, 4-OCF₃ | Et |
| 2-326 | 4-Cl | C-Me | 2-Me, 4-OCF₃ | Et |
| 2-327 | 4-Cl | C-Me | 2-Me, 4-OCF₃ | Me |
| 2-328 | 4-F | C-Me | 2-Me, 4-Cl | Et |
| 2-329 | 4-F | C-Me | 2-Me, 4-Br | Et |
| 2-330 | 4-F | C-Me | 2-Me, 4-Br | Me |
| 2-331 | 4-F | C-Me | 2-Me, 4-Br | iPr |
| 2-332 | 4-F | C-C≡CH | 2-Me, 4-CF₃ | Et |
| 2-333 | 4-F | C-OMe | 2-Me, 4-CF₃ | Et |
| 2-334 | 4-F | C-OH | 2-Me, 4-CF₃ | Et |
| 2-335 | 3-CF₃, 4-Br | C-Cl | 2-Cl, 4-CF₃ | Et |
| 2-336 | 3-Me, 4-NO₂ | C-Cl | 2-Cl, 4-CF₃ | Et |
| 2-337 | 3-Me, 4-NO₂ | C-Me | 2-Me, 4-CF₃ | Et |
| 2-338 | 3-CF₃, 4-Cl | C-Cl | 2-Cl, 4-CF₃ | Et |
| 2-339 | 3-CF₃, 4-Cl | C-H | 2-1, 4-CF₃ | Et |
| 2-340 | 3-Cl, 4-NO₂ | C-Cl | 2-Cl, 4-CF₃ | Et |
| 2-341 | 3-Br, 4-NO₂ | C-Me | 2-Me, 4-CF₃ | Et |
| 2-342 | 3-CF₃, 4-Cl | C-H | 2-Me, 4-CF₃ | Et |
| 2-343 | 3-CF₃, 4-Cl | C-H | 2-Me, 4-CF₃ | Me |
| 2-344 | 3-CF₃, 4-Cl | C-H | 2-Me, 4-CF₃ | iPr |
| 2-345 | 3-CF₃, 4-Cl | C-H | 2-Br, 4-CF₃ | Et |
| 2-346 | 3-CF₃ | C-H | 2-Me, 4-OCF₂CClBrH | Et |
| 2-347 | 3-CF₃, 4-Cl | C-H | 2-Br, 4-F, 5-Cl | Et |
| 2-348 | 3-CF₃, 4-Cl | C-H | 2-F, 4-Br, 5-Cl | Et |
| 2-349 | 3-CF₃, 4-Cl | C-H | 2-Cl, 5-Cl | Et |
| 2-350 | 3-CF₃, 4-Cl | C-H | 2-Cl, 5-Cl | Me |
| 2-351 | 3-CF₃, 4-Cl | C-H | 2-Cl, 5-Cl | iPr |
| 2-352 | 3-CF₃ | C-H | 3-CF₃, 5-Cl | Et |
| 2-353 | 3-CF₃, 4-Cl | C-Br | 2-F, 4-Cl | Et |
| 2-354 | 3-CF₃, 4-Cl | C-Br | 2-F, 4-Cl | Me |
| 2-355 | 3-CF₃, 4-Cl | C-Br | 2-F, 4-Cl | iPr |
| 2-356 | 3-CF₃, 4-Cl | C-H | 2-Me, 4-F, 5-Br | Et |
| 2-357 | 3-CF₃, 4-Cl | C-H | 2-Me, 4-Cl, 5-Br | Et |
| 2-358 | 3-CF₃, 4-Cl | C-Br | 2-Br, 4-Br | Et |
| 2-359 | 3-CF₃, 4-Cl | C-Br | 2-Br, 4-F | Et |
| 2-360 | 3-CF₃, 4-Cl | C-H | 2-Me, 3-F, 4-F | Et |
| 2-361 | 3-CF₃, 4-Cl | C-H | 2-Me, 4-Br, 5-Cl | Et |
| 2-362 | 3-CF₃, 4-Cl | C-H | 2-Me, 3-Br, 5-F | Et |
| 2-363 | 3-CF₃, 4-Cl | C-H | 2-Me, 4-Br, 5-F | Et |
| 2-364 | 3-CF₃, 4-Cl | C-F | 2-F, 4-Br | Et |
| 2-365 | 3-CF₃, 4-Cl | C-H | 2-Me, 4-F, 5-F | Et |
| 2-366 | 3-CF₃, 4-Cl | C-H | 2-Br, 4-Cl | Et |
| 2-367 | 3-CF₃, 4-Cl | C-Br | 2-Me, 4-Br | Et |
| 2-368 | 3-CF₃, 4-Cl | C-Br | 2-Br, 4-Cl | Et |
| 2-369 | 3-CF₃, 4-Cl | C-Me | 2-Br, 4-F | Et |
| 2-370 | 3-CF₃, 4-Cl | C-Me | 2-Cl, 4-Br | Et |
| 2-371 | 3-CF₃, 4-Cl | C-H | 2-Me, 3-F, 4-Br | Et |
| 2-372 | 3-CF₃, 4-Cl | C-H | 2-Me, 3-Cl, 4-Br | Et |
| 2-373 | 3-CF₃, 4-Cl | C-Me | 2-Me, 4-CF₃ | Et |
| 2-374 | 3-CF₃, 4-Cl | C-Me | 2-Me, 4-CF₃ | Me |
| 2-375 | 3-CF₃, 4-Cl | C-Me | 2-Me, 4-CF₃ | iPr |
| 2-376 | 3-CF₃, 4-I | C-Me | 2-Me, 4-CF₃ | Et |
| 2-377 | 3-CF₃, 4-Cl | C-Cl | 2-Me, 4-CF₃ | Et |
| 2-378 | 3-CF₃, 4-Cl | C-Br | 2-Me, 4-CF₃ | Et |
| 2-379 | 3-CF₃, 4-Cl | C-H | 2-C(Me)=CH₂, 4-CF₃ | Et |
| 2-380 | 3-CF₃, 4-Cl | C-H | 2-cPr, 4-CF₃ | Et |
| 2-381 | 3-CF₃, 4-Cl | C-H | 2-iPr, 4-CF₃ | Et |
| 2-382 | 3-CF₃, 4-Cl | C-H | 2-OMe, 4-CF₃ | Et |
| 2-383 | 3-CF₃, 4-Cl | C-H | 2-C≡CH, 4-CFs | Et |
| 2-384 | 3-CF₃, 4-Cl | C-Cl | 2-Cl, 4-F | Et |
| 2-385 | 3-CF₃, 4-Cl | C-F | 2-Me, 4-CF₃ | Et |
| 2-386 | 3-CF₃, 4-Cl | C-H | 2-Cl, 4-OCF₃ | Et |
| 2-387 | 3-CF₃, 4-Cl | C-H | 2-Me, 4-OCF₃ | Et |
| 2-388 | 3-CF₃, 4-Cl | C-Br | 2-Me, 4-OCF₃ | Et |
| 2-389 | 3-CF₃, 4-Cl | C-F | 2-F, 4-F | Et |
| 2-390 | 3-Me, 4-Cl | C-Cl | 2-Cl, 4-CF₃ | Et |
| 2-391 | 3-Me, 4-Cl | C-Cl | 2-Cl, 4-CF₃ | Me |
| 2-392 | 3-Me, 4-Cl | C-Cl | 2-Cl, 4-CF₃ | iPr |
| 2-393 | 3-Cl, 4-Me | C-Cl | 2-Cl, 4-CF₃ | Et |
| 2-394 | 3-Cl, 4-Br | C-Cl | 2-Cl, 4-CF₃ | Et |
| 2-395 | 3-Cl, 4-Cl | C-Cl | 2-Cl, 4-CF₃ | Et |
| 2-396 | 3-Cl, 4-I | C-Cl | 2-Cl, 4-CF₃ | Et |
| 2-397 | 3-Cl, 4-I | C-Me | 2-Me, 4-CF₃ | Et |
| 2-398 | 3-CF₃, 4-Cl | C-Cl | 2-Me, 4-OCF₃ | Et |
| 2-399 | 3-Cl, 4-CF₃ | C-Cl | 2-Me, 4-CF₃ | Et |
| 2-400 | 3-Cl, 4-CF₃ | C-Cl | 2-Me, 4-CF₃ | Me |
| 2-401 | 3-Cl, 4-CF₃ | C-Me | 2-Me, 4-CF₃ | Et |
| 2-402 | 3-Br, 4-CF₃ | C-Me | 2-Me, 4-CF₃ | Et |
| 2-403 | 3-Cl, 4-CF₃ | C-Cl | 2-Me, 4-OCF₃ | Et |
| 2-404 | 3-CF₃, 4-Cl | C-Cl | 2-Br, 4-CF₃ | Et |
| 2-405 | 3-CF₃, 4-Cl | C-Cl | 2-Br, 4-CF₃ | Me |
| 2-406 | 3-CF₃, 4-Cl | C-Cl | 2-cPr, 4-CF₃ | Et |
| 2-407 | 3-CF₃, 4-Cl | C-Cl | 2-CH=CH₂, 4-CF₃ | Et |
| 2-408 | 3-CF₃, 4-Cl | C-Cl | 2-Et, 4-CF₃ | Et |
| 2-409 | 3-Me, 4-Cl | C-Me | 2-Me, 4-CF₃ | Et |
| 2-410 | 3-Me, 4-Cl | C-Me | 2-Me, 4-CF₃ | Me |
| 2-411 | 3-Me, 4-Cl | C-Me | 2-Me, 4-CF₃ | iPr |
| 2-412 | 3-Cl, 4-Br | C-Me | 2-Me, 4-CF₃ | Et |
| 2-413 | 3-Cl, 4-Br | C-Me | 2-Me, 4-OCF₃ | Et |
| 2-414 | 3-Me, 4-Cl | C-Me | 2-Me, 4-OCF₃ | Et |
| 2-415 | 3-CF₃, 4-Cl | C-Me | 2-Me, 4-OCF₃ | Et |
| 2-416 | 3-CF₃, 5-Me | C-Me | 2-Me, 4-CF₃ | Et |
| 2-417 | 3-Me, 5-CF₃ | C-Me | 2-Me, 4-CF₃ | Et |
| 2-418 | 3-CF₃, 5-Cl | C-Me | 2-Me, 4-CF₃ | Et |
| 2-419 | 3-Cl, 5-CF₃ | C-Me | 2-Me, 4-CF₃ | Et |
| 2-420 | 3-Cl, 5-Cl | C-Me | 2-Me, 4-CF₃ | Et |
| 2-421 | 3-Br, 5-Br | C-Me | 2-Me, 4-CF₃ | Et |
| 2-422 | 3-Br, 4-Br, 5-Br | C-Me | 2-Me, 4-CF₃ | Et |
| 2-423 | 3-CF₃ | C-Cl | 2-Cl, 4-CF₃ | cPr |
| 2-424 | 3-CF₃ | C-Cl | 2-Cl, 4-CF₃ | iPr |
| 2-425 | 3-CF₃ | C-F | 2-Cl, 4-CF₃ | iPr |
| 2-426 | 3-CF₃ | C-Cl | 2-Cl, 4-CF₃ | Me |
| 2-427 | 3-CF₃ | C-F | 2-Cl, 4-CF₃ | Me |
| 2-428 | 3-CF₃ | C-Cl | 2-Me, 4-OCF₃ | iPr |
| 2-429 | 3-CF₃ | C-Cl | 2-Me, 4-OCF₃ | Me |
| 2-430 | 3-CF₃ | C-F | 2-Br, 4-CF₃ | Me |
| 2-431 | 3-CF₃ | C-Br | 2-Me, 4-CF₃ | iPr |
| 2-432 | 3-CF₃ | C-Cl | 2-Me, 4-CF₃ | iPr |
| 2-433 | 3-CF₃ | C-Cl | 2-Me, 4-CF₃ | Me |
| 2-434 | 3-CF₃ | C-Br | 2-Me, 4-CF₃ | Me |
| 2-435 | 3-CF₃ | C-F | 2-Me, 4-OCF₃ | Me |
| 2-436 | 3-CF₃ | C-Me | 2-Me, 4-CF₃ | Me |
| 2-437 | 4-F | C-Me | 2-Me, 4-CF₃ | Me |
| 2-438 | 3-CF₃ | C-Me | 2-Me, 4-CF₃ | iPr |
| 2-439 | 4-F | C-Me | 2-Me, 4-CF₃ | iPr |
| 2-440 | 3-CF₃ | C-Me | 2-Me, 4-CF₃ | cPr |
| 2-441 | 4-F | C-Me | 2-Me, 4-CF₃ | cPr |
| 2-442 | 3-Cl | C-Me | 2-Me, 4-CF₃ | CH=CH₂ |
| 2-443 | 3-Cl | C-Me | 2-Me, 4-CF₃ | C≡CH |
| 2-444 | 3-Cl | C-Me | 2-Me, 4-CF₃ | CH₂OMe |
| 2-445 | 3-Cl | C-Me | 2-Me, 4-CF₃ | CH₂cPr |
| 2-446 | 3-Cl | C-Me | 2-Me, 4-CF₃ | CF₃ |
| 2-447 | 3-Cl | C-Me | 2-Me, 4-CF₃ | Me |
| 2-448 | 3-Cl | C-Me | 2-Me, 4-CF₃ | iPr |
| 2-449 | 3-Cl | C-Me | 2-Me, 4-CF₃ | nPr |
| 2-450 | 3-Cl | C-Me | 2-Me, 4-CF₃ | nBu |
| 2-451 | 3-Cl | C-Me | 2-Me, 4-CF₃ | CH₂(CH₂)₃CH₃ |
| 2-452 | 3-Cl | C-Me | 2-Me, 4-CF₃ | cPr |
| 2-453 | 4-F | C-Cl | 2-Me, 4-CF₃ | iPr |
| 2-454 | 4-F | C-Cl | 2-Me, 4-OCF₃ | iPr |
| 2-455 | 3-CF₃, 4-Cl | C-Cl | 2-Me, 4-CF₃ | iPr |
| 2-456 | 4-CI | C-Cl | 2-Me, 4-CF₃ | iPr |
| 2-457 | 4-F | C-Br | 2-Br, 4-CF₃ | iPr |
| 2-458 | 4-F | C-F | 2-Br, 4-CF₃ | Me |
| 2-459 | 4-F | C-Br | 2-Me, 4-CF₃ | iPr |
| 2-460 | 4-F | C-Cl | 2-Me, 4-CF₃ | Me |
| 2-461 | 4-F | C-Br | 2-Me, 4-CF₃ | Me |
| 2-462 | 3-CF₃, 4-Cl | C-F | 2-Br, 4-CF₃ | Me |
| 2-463 | 4-F | C-Br | 2-Br, 4-CF₃ | Me |
| 2-464 | 4-F | C-F | 2-Me, 4-CF₃ | Me |
| 2-465 | 3-CF₃, 4-Cl | C-Cl | 2-Me, 4-CF₃ | Me |
| 2-466 | 3-CF₃, 4-Cl | C-Br | 2-Me, 4-CF₃ | Me |
| 2-467 | 3-CF₃, 4-Cl | C-Br | 2-Me, 4-CF₃ | iPr |
| 2-468 | 4-Cl | C-Br | 2-Me, 4-CF₃ | iPr |
| 2-469 | 4-Cl | C-Cl | 2-Me, 4-CF₃ | Me |
| 2-470 | 4-Cl | C-Br | 2-F, 4-OCF₃ | Me |
| 2-471 | 4-Cl | C-Br | 2-F, 4-CF₃ | Me |
| 2-472 | 3-CF₃ | C-F | 2-Br, 4-OCF₃ | Me |
| 2-473 | 3-CF₃, 4-Cl | C-F | 2-Br, 4-OCF₃ | Me |
| 2-474 | 4-F | C-F | 2-Br, 4-OCF₃ | Me |
| 2-475 | 4-Cl | C-Br | 2-Me, 4-CF₃ | Me |
| 2-476 | 4-Cl | C-Cl | 2-Me, 4-OCF₃ | Me |
| 2-477 | 4-Cl | C-Cl | 2-Me, 4-OCF₃ | iPr |
| 2-478 | 3-CF₃ | C-F | 2-Me, 4-CF₃ | Me |
| 2-479 | 3-CF₃, 4-Cl | C-F | 2-Me, 4-CF₃ | Me |
| 2-480 | 3-CF₃, 4-Cl | C-Cl | 2-Me, 4-OCF₃ | Me |
| 2-481 | 3-CF₃, 4-Cl | C-Cl | 2-Me, 4-OCF₃ | iPr |
| 2-482 | 4-CI | C-F | 2-Me, 4-OCF₃ | Me |
| 2-483 | 4-Cl | C-F | 2-Me, 4-CF₃ | Me |
| 2-484 | 3-CF₃, 4-Cl | C-F | 2-Me, 4-OCF₃ | Me |
| 2-485 | 4-F | C-F | 2-Me, 4-OCF₃ | Me |
| 2-486 | 3-Cl | C-Cl | 2-Cl, 4-CF₃ | Me |
| 2-487 | 3-Cl | C-Me | 2-Me, 4-SO₂Me | Me |
| 2-488 | 3-Cl | C-Me | 2-Me, 4-SO₂Et | Me |
| 2-489 | 3-Cl | C-Me | 2-Me, 4-CH₂CF₃ | Me |
| 2-490 | 3-Cl | C-Me | 2-Me, 4-OCF₂H | Me |
| 2-491 | 3-Cl | C-Me | 2-Me, 4-CF₂CF₃ | Me |
| 2-492 | 3-Cl | C-Me | 2-Me, 4-CF₂H | Me |
| 2-493 | 3-Cl | C-Me | 2-Me, 4-CF₃ | tBu |
| 2-494 | 3-Br, 4-NO₂ | C-Me | 2-Me, 4-CF₃ | Et |
| 2-495 | 3-Cl, 4-NO₂ | C-Me | 2-Me, 4-CF₃ | Et |
| 2-496 | 3-CF₃, 4-Br | C-H | 2-Me, 4-CF₃ | Et |
| 2-497 | 3-Br, 4-CF₃ | C-H | 2-Me, 4-CF₃ | Et |
| 2-498 | 3-Cl | C-Cl | 2-Cl, 4-SO₂Me | Et |
| 2-499 | 3-Cl | C-Br | 2-Br, 4-SO₂Me | Et |
| 2-500 | 3-Cl | C-CH=CH₂ | 2-Me, 4-CF₃ | Me |
| 2-501 | 3-Cl | C-CH=CH₂ | 2-Me, 4-CF₃ | iPr |
| 2-502 | 4-F | C-CH=CH₂ | 2-Me, 4-CF₃ | Me |
| 2-503 | 4-F | C-CH=CH₂ | 2-Me, 4-CF₃ | iPr |
| 2-504 | 3-Cl | C-Et | 2-Me, 4-CF₃ | Me |
| 2-505 | 3-Cl | C-Et | 2-Me, 4-CF₃ | iPr |
| 2-506 | 4-F | C-Et | 2-Me, 4-CF₃ | Me |
| 2-507 | 4-F | C-Et | 2-Me, 4-CF₃ | iPr |
| 2-508 | 3-SMe | C-Me | 2-Me, 4-CF₃ | Et |
| 2-509 | 4-Cl | C-Me | 2-Me, 4-CF₃ | CF₃ |
| 2-510 | 3-Cl, 4-Cl | C-H | 4-SO₂Me | iPr |
| 2-511 | 4-F | C-Me | 2-Me, 4-CF₃ | CF₃ |
| 2-512 | 3-OCH₂CF₃ | C-Me | 2-Me, 4-CF₃ | Et |
| 2-513 | 3-Me | C-Cl | 2-Cl, 4-CF₃ | iPr |
| 2-514 | 3-Me | C-Cl | 2-Cl, 4-CF₃ | cPr |
| 2-515 | 3-Br | C-Me | 2-Me, 4-CF₃ | CF₃ |
| 2-516 | 3-Me | C-Me | 2-Me, 4-CF₃ | CF₃ |
| 2-517 | 3-CF₃ | C-Me | 2-Me, 4-CF₃ | CF₃ |
| 2-518 | 4-Me | C-Cl | 2-Cl, 4-CF₃ | iPr |
| 2-519 | 4-Me | C-Cl | 2-Cl, 4-CF₃ | cPr |
| 2-520 | 4-Me | C-Me | 2-Me, 4-CF₃ | cPr |
| 2-521 | 4-Me | C-Me | 2-Me, 4-CF₃ | tBu |
| 2-522 | 3-cPr, 4-Cl | C-Me | 2-Me, 4-CF₃ | Et |
| 2-523 | 3-Cl | C-Me | 2-Me, 4-CF₃ | cBu |
| 2-524 | 3-Cl | C-F | 2-F, 4-CF₃ | Et |
| 2-525 | 3-Me | C-Me | 2-Me, 4-CF₃ | tBu |
| 2-526 | 3-Me | C-Me | 2-Me, 4-CF₃ | cPr |
| 2-527 | 3-Cl | C-Cl | 2-Cl, 4-SCF₃ | Et |
| 2-528 | 3-Cl | C-Br | 2-Br, 4-SCF₃ | Et |
| 2-529 | 3-CF₃, 4-Cl | C-Me | 2-Me, 4-CF₃ | CF₃ |
| 2-530 | 3-Me, 4-Cl | C-Me | 2-Me, 4-CF₃ | CF₃ |
| 2-531 | 3-Cl, 4-Me | C-Me | 2-Me, 4-CF₃ | CF₃ |
| 2-532 | 3-Et, 4-Cl | C-Me | 2-Me, 4-CF₃ | Et |
| 2-533 | 3-Cl, 4-Me | C-Me | 2-Me, 4-CF₃ | Et |
| 2-534 | 3-CF₃, 4-Me | C-Me | 2-Me, 4-CF₃ | Et |
| 2-535 | 3-CF₃ | C-Me | 2-Me, 4-CF₃ | tBu |
| 2-536 | 4-F | C-Me | 2-Me, 4-CF₃ | tBu |
| 2-537 | 4-Cl | C-Me | 2-Me, 4-CF₃ | cPr |
| 2-538 | 4-Cl | C-Me | 2-Me, 4-CF₃ | tBu |
| 2-539 | 3-Cl | C-Cl | 2-Cl, 4-CF₃ | cBu |
| 2-540 | 4-Br | C-Me | 2-Me, 4-CF₃ | cPr |
| 2-541 | 4-Br | C-Me | 2-Me, 4-CF₃ | tBu |
| 2-542 | 3-Cl | C-Br | 2-Br, 4-SO₂CF₃ | Et |
| 2-543 | 3-CF₂H | C-Me | 2-Me, 4-CF₃ | Et |
| 2-544 | 3-CF₃, 4-Me | C-Me | 2-Me, 4-CF₃ | cPr |
| 2-545 | 4-CF₃ | C-Me | 2-Me, 4-CF₃ | Me |
| 2-546 | 4-CF₃ | C-Me | 2-Me, 4-CF₃ | Et |
| 2-547 | 4-CF₃ | C-Me | 2-Me, 4-CF₃ | iPr |
| 2-548 | 4-CF₃ | C-Me | 2-Me, 4-CF₃ | cPr |
| 2-549 | 4-CF₃ | C-Me | 2-Me, 4-CF₃ | tBu |
| 2-550 | 3-CF₃, 4-Cl | C-Me | 2-Me, 4-CF₃ | cPr |
| 2-551 | 3-Cl | C-H | 2-Cl, 4-SO₂Me | Et |
| 2-552 | 3-CF₃, 4-Me | C-Me | 2-Me, 4-CF₃ | tBu |
| 2-553 | 4-Cl | C-H | 2-Cl, 4-SO₂Me | Et |
| 2-554 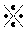 | 4-F | C-Me | 2-Me, 4-CF₃ | Et |
| 2-555 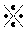 | 4-F | C-Me | 2-Me, 4-CF₃ | Et |

| | | | | |
|---|---|---|---|---|
| 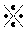 Compound No. 2-554 is sodium salt of compound No. 2-234, and Compound No. 2-555 is tetrabutylammonium salt of Compound No. 2-234. | | | | |

**Table 3**

| 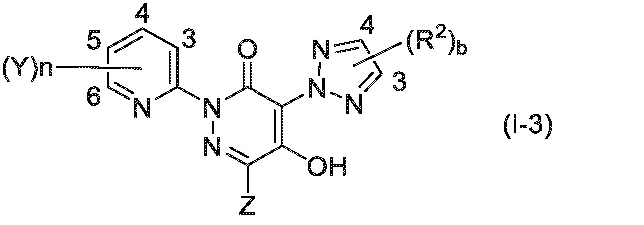 | | | |
|---|---|---|---|
| No. | (R²)_{b} | (Y)n | Z |
| 3-1 | b=0 | 3-Cl, 4-CF₃ | Et |
| 3-2 | 3-Br | 3-Cl, 4-CF₃ | Et |
| 3-3 | 3-Me | 3-Cl, 4-CF₃ | Et |
| 3-4 | 3-Br, 4-Br | 3-Cl, 4-CF₃ | Et |

**Table 4**

| 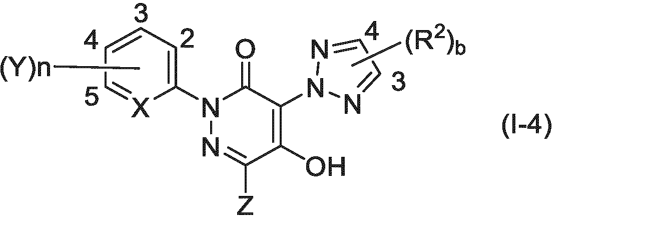 | | | | |
|---|---|---|---|---|
| No. | (R²)_{b} | X | (Y)n | Z |
| 4-1 | b=0 | C-Me | 2-Me, 4-CF₃ | Et |
| 4-2 | 3-Br | C-Me | 2-Me, 4-CF₃ | Et |
| 4-3 | 3-Me | C-Me | 2-Me, 4-CF₃ | Et |
| 4-4 | 3-Br, 4-Br | C-Me | 2-Me, 4-CF₃ | Et |
| 4-5 | 3-Br | C-Me | 2-Me, 4-CF₃ | iPr |
| 4-6 | 3-Cl | C-Me | 2-Me, 4-CF₃ | Et |

**Table 5**

| 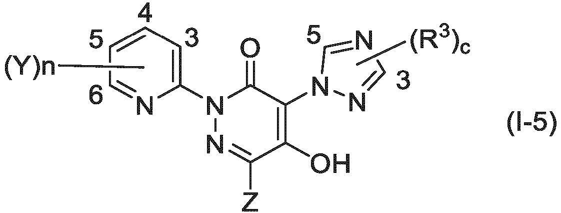 | | | |
|---|---|---|---|
| No. | (R³)_{c} | (Y)n | Z |
| 5-1 | c=0 | 3-Cl, 5-CF₃ | Et |
| 5-2 | 3-Cl | 3-Cl, 5-CF₃ | Et |
| 5-3 | 3-Cl | 3-F, 5-CF₃ | Et |
| 5-4 | 3-Cl | 3-Br, 5-CF₃ | Et |
| 5-5 | 3-Cl | 3-Et, 5-CF₃ | Et |
| 5-6 | 3-Cl | 3-Cl, 5-OCF₃ | Et |
| 5-7 | 3-Cl | 3-Cl, 5-Br | Et |
| 5-8 | 3-Cl | 3-Cl, 5-cPr | Et |
| 5-9 | 3-Cl | 3-Cl, 5-SMe | Et |
| 5-10 | 3-Cl | 3-Cl, 5-SOMe | Et |
| 5-11 | 3-Cl | 3-Cl 5-SO₂Me | Et |
| 5-12 | 3-Cl | 3-Cl, 5-SCF₃ | Et |
| 5-13 | 3-Cl | 3-Cl, 5-SOCF₃ | Et |
| 5-14 | 3-Cl | 3-Cl, 5-SO₂CF₃ | Et |
| 5-15 | 3-Cl | 3-Cl, 5-CN | Et |
| 5-16 | 3-Cl | 3-Cl, 5-NO₂ | Et |
| 5-17 | 3-Cl | 3-Cl, 5-SF₅ | Et |
| 5-18 | 3-Cl | 3-Cl, 5-COMe | Et |
| 5-19 | 3-Cl | 3-Cl, 5-COCF₃ | Et |
| 5-20 | 3-F | 3-F, 5-CF₃ | Et |
| 5-21 | 3-F | 3-Cl, 5-CF₃ | Et |
| 5-22 | 3-F | 3-Br, 5-CF₃ | Et |
| 5-23 | 3-Br | 3-Cl, 5-CF₃ | Et |
| 5-24 | 3-Br | 3-Br, 5-CF₃ | Et |
| 5-25 | 3-Me | 3-Cl, 5-CF₃ | Et |
| 5-26 | 3-Me | 3-Br, 5-CF₃ | Et |
| 5-27 | 3-CF₃ | 3-Cl, 5-CF₃ | Et |
| 5-28 | 3-CF₃ | 3-Br, 5-CF₃ | Et |
| 5-29 | 3-cPr | 3-Cl, 5-CF₃ | Et |
| 5-30 | 3-OMe | 3-Cl, 5-CF₃ | Et |
| 5-31 | 3-OMe | 3-Br, 5-CF₃ | Et |
| 5-32 | 3-OCF₃ | 3-Cl, 5-CF₃ | Et |
| 5-33 | 3-SCF₃ | 3-Cl, 5-CF₃ | Et |
| 5-34 | 3-SOCF₃ | 3-Cl, 5-CF₃ | Et |
| 5-35 | 3-SO₂CF₃ | 3-Cl, 5-CF₃ | Et |
| 5-36 | 3-CN | 3-Cl, 5-CF₃ | Et |
| 5-37 | 3-NO₂ | 3-Cl, 5-CF₃ | Et |
| 5-38 | 3-COMe | 3-Cl, 5-CF₃ | Et |
| 5-39 | 3-COCF₃ | 3-Cl, 5-CF₃ | Et |
| 5-40 | 3-Cl | 3-Cl, 5-CF₃ | Me |
| 5-41 | 3-Cl | 3-Br, 5-CF₃ | Me |
| 5-42 | 3-Cl | 3-Cl, 5-CF₃ | iPr |
| 5-43 | 3-Cl | 3-Br, 5-CF₃ | iPr |
| 5-44 | 3-Cl | 3-Cl, 5-CF₃ | cPr |
| 5-45 | 3-Cl | 3-Cl, 5-CF₃ | CH=CH₂ |
| 5-46 | 3-Cl | 3-Cl, 5-CF₃ | C≡CH |
| 5-47 | 3-Cl | 3-Cl, 5-CF₃ | CH₂OMe |
| 5-48 | 3-Cl | 3-Cl, 5-CF₃ | CH₂cPr |
| 5-49 | 3-Cl | 3-Cl, 5-CF₃ | CF₃ |
| 5-50 | 3-Cl | 3-Et, 5-CF₃ | Et |
| 5-51 | 3-CF₃ | 3-Me, 5-CF₃ | Et |

**Table 6**

| 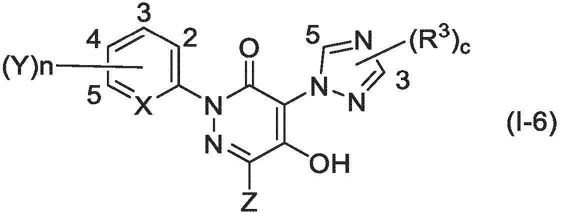 | | | | |
|---|---|---|---|---|
| No. | (R³)_{c} | X | (Y)n | Z |
| 6-1 | 3-CF₃ | C-Cl | 2-Cl, 4-CF₃ | Et |
| 6-2 | 3-CF₃ | C-Br | 2-Br, 4-CF₃ | Et |
| 6-3 | 3-CF₃ | C-Cl | 2-Me, 4-CF₃ | Et |
| 6-4 | 3-CF₃ | C-Br | 2-Me, 4-CF₃ | Et |
| 6-5 | 3-CF₃ | C-Me | 2-Me, 4-CF₃ | Et |
| 6-6 | 3-CF₃ | C-H | 2-Me, 4-CF₃ | Et |
| 6-7 | 3-CF₃ | C-Me | 2-Me, 4-OCF₃ | Et |
| 6-8 | 3-F | C-Cl | 2-Cl, 4-CF₃ | Et |
| 6-9 | 3-Cl | C-Cl | 2-Cl, 4-CF₃ | Me |
| 6-10 | 3-Cl | C-Cl | 2-Cl, 4-CF₃ | Et |
| 6-11 | 3-Cl | C-Cl | 2-Cl, 4-CF₃ | iPr |
| 6-12 | 3-Br | C-Cl | 2-Cl, 4-CF₃ | Et |
| 6-13 | 3-Cl | C-Br | 2-Br, 4-CF₃ | Et |
| 6-14 | 3-Br | C-Br | 2-Br, 4-CF₃ | Et |
| 6-15 | 3-Cl | C-Cl | 2-Me, 4-CF₃ | Et |
| 6-16 | 3-Cl | C-Br | 2-Me, 4-CF₃ | Et |
| 6-17 | 3-Cl | C-H | 2-1, 4-CF₃ | Et |
| 6-18 | 3-Cl | C-Me | 2-Me, 4-CF₃ | Et |
| 6-19 | 3-Cl | C-H | 2-Me, 4-CF₃ | Et |
| 6-20 | 3-Cl | C-H | 2-Me, 4-OCF₃ | Et |
| 6-21 | 3-Cl | C-Me | 2-Me, 4-OCF₃ | Et |
| 6-22 | 3-Cl | C-Me | 2-Me, 4-Cl | Et |
| 6-23 | 3-Cl | C-Me | 2-Me, 4-Br | Et |
| 6-24 | 3-Cl | C-Me | 2-Me, 4-cPr | Et |
| 6-25 | 3-Cl | C-Me | 2-Me, 4-CO₂Me | Et |
| 6-26 | 3-Cl | C-Me | 2-Me, 4-SMe | Et |
| 6-27 | 3-Cl | C-Me | 2-Me, 4-SOMe | Et |
| 6-28 | 3-Cl | C-Me | 2-Me, 4-SO₂Me | Et |
| 6-29 | 3-Cl | C-Me | 2-Me, 4-SO₂Et | Et |
| 6-30 | 3-Cl | C-Me | 2-Me, 4-CH₂CF₃ | Et |
| 6-31 | 3-Cl | C-Me | 2-Me, 4-OCF₂H | Et |
| 6-32 | 3-Cl | C-Me | 2-Me, 4-CF₂CF₃ | Et |
| 6-33 | 3-Cl | C-Me | 2-Me, 4-CF₂H | Et |
| 6-34 | 3-Cl | C-Me | 2-Me, 4-CHO | Et |
| 6-35 | 3-Cl | C-Me | 2-Me, 4-CO₂H | Et |
| 6-36 | 3-Cl | C-Me | 2-Me, 4-OH | Et |
| 6-37 | 3-Cl | C-Me | 2-Me, 4-C≡CH | Et |
| 6-38 | 3-Cl | C-Me | 2-Me, 4-CH=CH₂ | Et |
| 6-39 | 3-Cl | C-OMe | 2-Me, 4-CF₃ | Et |
| 6-40 | 3-Cl | C-CH=CH₂ | 2-Me, 4-CF₃ | Et |
| 6-41 | 3-Cl | C-Et | 2-Me, 4-CF₃ | Et |
| 6-42 | 3-CF₃ | C-Me | 2-Me, 4-Br | Et |
| 6-43 | 3-F | C-Me | 2-Me, 4-Br | Et |
| 6-44 | 3-Br | C-Me | 2-Me, 4-Br | Et |
| 6-45 | c=0 | C-Me | 2-Me, 4-CF₃ | Et |
| 6-46 | c=0 | C-Cl | 2-Cl, 4-CF₃ | Me |
| 6-47 | c=0 | C-Cl | 2-Cl, 4-CF₃ | Et |
| 6-48 | c=0 | C-Br | 2-Br, 4-CF₃ | Et |
| 6-49 | 3-F | C-Me | 2-Me, 4-CF₃ | Et |
| 6-50 | 3-F | C-H | 2-Me, 4-CF₃ | Et |
| 6-51 | 3-Br | C-Me | 2-Me, 4-CF₃ | Et |
| 6-52 | 3-Me | C-Me | 2-Me, 4-CF₃ | Et |
| 6-53 | 3-cPr | C-Me | 2-Me, 4-CF₃ | Et |
| 6-54 | 3-OMe | C-Me | 2-Me, 4-CF₃ | Et |
| 6-55 | 3-OCH₂CF₃ | C-Me | 2-Me, 4-CF₃ | Et |
| 6-56 | 3-OCF₃ | C-Me | 2-Me, 4-CF₃ | Et |
| 6-57 | 3-SCF₃ | C-Me | 2-Me, 4-CF₃ | Et |
| 6-58 | 3-SOCF₃ | C-Me | 2-Me, 4-CF₃ | Et |
| 6-59 | 3-SO₂CF₃ | C-Me | 2-Me, 4-CF₃ | Et |
| 6-60 | 3-CN | C-Me | 2-Me, 4-CF₃ | Et |
| 6-61 | 3-NO₂ | C-Me | 2-Me, 4-CF₃ | Et |
| 6-62 | 3-COMe | C-Me | 2-Me, 4-CF₃ | Et |
| 6-63 | 3-COCF₃ | C-Me | 2-Me, 4-CF₃ | Et |
| 6-64 | 3-Br | C-Me | 2-Me, 4-OCF₃ | Et |
| 6-65 | 3-NO₂ | C-Me | 2-Me, 4-CF₃ | Et |
| 6-66 | c=0 | C-Me | 2-Me, 4-OCF₃ | Et |
| 6-67 | 3-Br | C-Me | 2-Me, 4-Cl | Et |
| 6-68 | 5-Cl | C-Me | 2-Me, 4-CF₃ | Et |
| 6-69 | 5-Me | C-Me | 2-Me, 4-CF₃ | Et |
| 6-70 | 5-CF₃ | C-Me | 2-Me, 4-CF₃ | Et |
| 6-71 | 3-Br, 5-Br | C-Me | 2-Me, 4-CF₃ | Et |
| 6-72 | 3-Me, 5-Me | C-Me | 2-Me, 4-CF₃ | Et |
| 6-73 | 3-CF₃ | C-Me | 2-Me, 4-CF₃ | Me |
| 6-74 | 3-F | C-Me | 2-Me, 4-CF₃ | Me |
| 6-75 | 3-Cl | C-Me | 2-Me, 4-CF₃ | Me |
| 6-76 | 3-Br | C-Me | 2-Me, 4-CF₃ | Me |
| 6-77 | 3-Cl | C-Me | 2-Me, 4-OCF₃ | Me |
| 6-78 | 3-CF₃ | C-Me | 2-Me, 4-CF₃ | iPr |
| 6-79 | 3-F | C-Me | 2-Me, 4-CF₃ | iPr |
| 6-80 | 3-Cl | C-Me | 2-Me, 4-CF₃ | iPr |
| 6-81 | 3-CF₃ | C-Me | 2-Me, 4-CF₃ | cPr |
| 6-82 | 3-F | C-Me | 2-Me, 4-CF₃ | cPr |
| 6-83 | 3-Cl | C-Me | 2-Me, 4-CF₃ | cPr |
| 6-84 | 3-Cl | C-Me | 2-Me, 4-CF₃ | nPr |
| 6-85 | 3-Cl | C-Me | 2-Me, 4-CF₃ | nBu |
| 6-86 | 3-Cl | C-Me | 2-Me, 4-CF₃ | CH₂(CH₂)₃CH₃ |
| 6-87 | 3-Cl | C-Me | 2-Me, 4-CF₃ | CH=CH₂ |
| 6-88 | 3-Cl | C-Me | 2-Me, 4-CF₃ | C≡CH |
| 6-89 | 3-Cl | C-Me | 2-Me, 4-CF₃ | CH₂OMe |
| 6-90 | 3-Cl | C-Me | 2-Me, 4-CF₃ | CH₂cPr |
| 6-91 | 3-Cl | C-Me | 2-Me, 4-CF₃ | CF₃ |
| 6-92 | 3-Cl | C-Me | 2-Me, 4-SO₂Me | Me |
| 6-93 | 3-Cl | C-Me | 2-Me, 4-SO₂Et | Me |
| 6-94 | 3-Cl | C-Me | 2-Me, 4-CH₂CF₃ | Me |
| 6-95 | 3-Cl | C-Me | 2-Me, 4-OCF₂H | Me |
| 6-96 | 3-Cl | C-Me | 2-Me, 4-CF₂CF₃ | Me |
| 6-97 | 3-Cl | C-Me | 2-Me, 4-CF₂H | Me |
| 6-98 | 3-Cl | C-Me | 2-Me, 4-SCF₃ | Et |
| 6-99 | 3-Cl | C-Me | 2-Me, 4-SCF₃ | Me |
| 6-100 | 3-Cl | C-Me | 2-Me, 4-SO₂CF₃ | Et |
| 6-101 | 3-Cl | C-Me | 2-Me, 4-SO₂CF₃ | Me |
| 6-102 | 3-Cl | C-Me | 2-Me, 4-CF₃ | tBu |
| 6-103 | 3-F | C-Me | 2-Me, 4-OCF₃ | Et |
| 6-104 | 3-Cl | C-Me | 2-Me, 4-CF₃ | cBu |
| 6-105 | 3-CF₂H | C-Me | 2-Me, 4-CF₃ | Et |
| 6-106 | 3-Cl | C-CH=CH₂ | 2-Me, 4-CF₃ | Me |
| 6-107 | 3-Cl | C-CH=CH₂ | 2-Me, 4-CF₃ | iPr |
| 6-108 | 3-Cl | C-Et | 2-Me, 4-CF₃ | Me |
| 6-109 | 3-Cl | C-Et | 2-Me, 4-CF₃ | iPr |
| 6-110 | 3-CO₂Me | C-Me | 2-Me, 4-CF₃ | iPr |
| 6-111 | 3-Cl | C-H | 2-Me, 4-CF₃ | Me |
| 6-112 | 3-CF₂H | C-Me | 2-Me, 4-CF₃ | iPr |
| 6-113 | 3-CF₂H | C-Me | 2-Me, 4-CF₃ | tBu |
| 6-114 | 3-CF₂H | C-Me | 2-Me, 4-CF₃ | cPr |
| 6-115 | 3-F | C-Me | 2-Me, 4-CF₃ | tBu |
| 6-116 | 3-Cl | C-H | 2-Me, 4-CF₃ | iPr |
| 6-117 | 3-Cl | C-H | 2-1, 4-CF₃ | Et |
| 6-118 | 3-OCH₂CF₃ | C-Me | 2-Me, 4-CF₃ | iPr |
| 6-119 | 3-Br | C-Me | 2-Me, 4-CF₃ | iPr |
| 6-120 | 3-Me | C-Me | 2-Me, 4-CF₃ | iPr |
| 6-121 | 3-Cl | C-Cl | 2-Cl, 4-CF₃ | cPr |
| 6-122 | 3-Me | C-Cl | 2-Cl, 4-CF₃ | Et |
| 6-123 | 3-Me | C-Cl | 2-Cl, 4-CF₃ | cPr |
| 6-124 | 3-Cl | C-F | 2-F, 4-CF₃ | Et |
| 6-125 | 3-Cl | C-Br | 2-Br, 4-CF₃ | iPr |
| 6-126 | 3-Cl | C-Br | 2-Br, 4-CF₃ | cPr |
| 6-127 | 3-CF₃ | C-Me | 2-Me, 4-CF₃ | CF₃ |
| 6-128 | 3-Br | C-Me | 2-Me, 4-CF₃ | cPr |
| 6-129 | 3-Br | C-Me | 2-Me, 4-CF₃ | tBu |
| 6-130 | 3-Cl | C-Br | 2-Br, 4-SCF₃ | Et |
| 6-131 | 3-Me | C-Me | 2-Me, 4-CF₃ | cPr |
| 6-132 | 3-Me | C-Me | 2-Me, 4-CF₃ | tBu |
| 6-133 | 3-CF₃ | C-Me | 2-Me, 4-CF₃ | tBu |
| 6-134 | 3-Et | C-Me | 2-Me, 4-CF₃ | iPr |
| 6-135 | 3-iPr | C-Me | 2-Me, 4-CF₃ | iPr |
| 6-136 | 3-Cl | C-Me | 2-Me, 4-CN | Et |
| 6-137 | 3-Cl | C-Br | 2-Br, 4-SOCF₃ | Et |
| 6-138 | 3-Cl | C-Br | 2-Br, 4-SO₂CF₃ | Et |
| 6-139 | 3-Cl | C-Me | 2-Me, 4-SOCF₃ | Et |
| 6-140 | 3-cPr | C-Me | 2-Me, 4-CF₃ | iPr |
| 6-141 | 3-OMe | C-Me | 2-Me, 4-CF₃ | iPr |
| 6-142 | 3-Me | C-H | 2-Cl, 4-SO₂Me | Et |
| 6-143 | 3-Cl | C-H | 2-Cl, 4-SO₂Me | Et |

**Table 7**

| 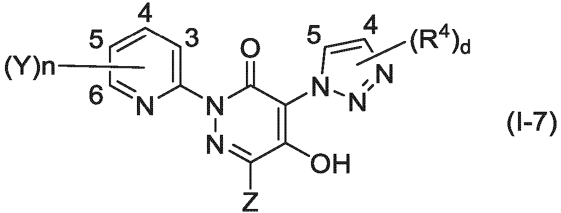 | | | |
|---|---|---|---|
| No. | (R⁴)_{d} | (Y)n | Z |
| 7-1 | d=0 | 3-Cl, 5-CF₃ | Et |
| 7-2 | 4-Br | 3-Cl, 5-CF₃ | Et |
| 7-3 | 5-Br | 3-Cl, 5-CF₃ | Et |
| 7-4 | 4-Br, 5-Br | 3-Cl, 5-CF₃ | Et |
| 7-5 | 4-Me | 3-Cl, 5-CF₃ | Et |
| 7-6 | 5-Me | 3-Cl, 5-CF₃ | Et |

**Table 8**

| 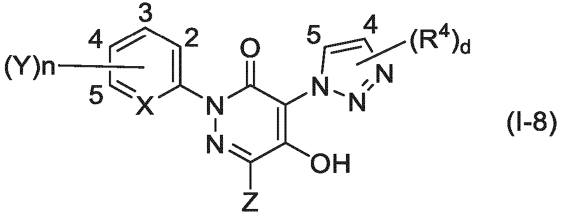 | | | | |
|---|---|---|---|---|
| No. | (R⁴)_{d} | X | (Y)ₙ | Z |
| 8-1 | d=0 | C-Me | 2-Me, 4-CF₃ | Et |
| 8-2 | 4-Br | C-Me | 2-Me, 4-CF₃ | Et |
| 8-3 | 5-Br | C-Me | 2-Me, 4-CF₃ | Et |
| 8-4 | 4-Me | C-Me | 2-Me, 4-CF₃ | Et |
| 8-5 | 5-Me | C-Me | 2-Me, 4-CF₃ | Et |
| 8-6 | 4-Br, 5-Br | C-Me | 2-Me, 4-CF₃ | Et |
| 8-7 | 4-Br, 5-Br | C-Me | 2-Me, 4-CF₃ | iPr |
| 8-8 | 4-Br, 5-Br | C-Me | 2-Me, 4-CF₃ | nPr |
| 8-9 | 4-Br, 5-Br | C-Me | 2-Me, 4-CF₃ | cPen |
| 8-10 | 4-Br, 5-Br | C-Me | 2-Me, 4-CF₃ | cPr |
| 8-11 | 4-Br, 5-Br | C-Me | 2-Me, 4-CF₃ | tBu |
| 8-12 | 4-Br | C-Me | 2-Me, 4-CF₃ | iPr |

**Table 9**

| No. | ¹H-NMR (300 MHz or 500 MHz/solvent) δ ppm |
|---|---|
| 1-1 | (500 MHz/CDCl₃) 12.96 (1H, s), 9.32 (1H, d), 8.87 (1H, d), 8.35 (1H, d), 6.77 (1H, d), 2.89 (2H, q), 1.31 (3H, t) |
| 1-2 | (500 MHz/CDCl₃) 12.97 (1H, s), 9.31 (1H, d), 8.76 (1H, s), 7.90 (1H, dd), 6.78 (1H, d), 2.88 (2H, q), 1.30 (3H, t) |
| 1-3 | (500 MHz/CDCl₃) 12.91 (1H, s), 9.34 (1H, d), 8.76 (1H, s), 7.98 (1H, s), 6.77 (1H, d), 2.88 (2H, q), 2.31 (3H, s), 1.30 (3H, t) |
| 1-4 | (500 MHz/CDCl₃) 12.98 (1H, s), 9.34 (1H, d), 8.90 (1H, s), 8.55 (1H, d), 6.79 (1H, d), 2.89 (2H, br), 1.32 (3H, t) |
| 1-5 | (500 MHz/CDCl₃) 12.97 (1H, s), 9.36 (1H, d), 8.94 (1H, s), 8.13 (1H, d), 7.78 (1H, d), 6.78 (1H, d), 2.89 (2H, q), 1.31 (3H, t) |
| 1-6 | (500 MHz/CDCl₃) 13.10 (1H, brs), 9.23 (1H, d), 9.15 (1H, d), 8.73 (1H, d), 6.78 (1H, d), 2.90 (2H, q), 1.32 (3H, t) |
| 1-7 | (300 MHz/CDCl₃) 12.98 (1H, s), 9.34-9.39 (1H, m), 8.84 (1H, d), 8.31 (1H, d), 6.78-6.81 (1H, m), 6.52 (1H, q), 5.95 (1H, d), 5.57 (1H, d), 2.89 (2H, q), 1.32 (3H, t) |
| 1-8 | (300 MHz/CDCl₃) 12.95 (1H, s), 9.35-9.41 (1H, m), 8.80 (1H, d), 8.05 (1H, d), 6.78-6.85 (1H, m), 2.88 (2H, q), 2.64 (2H, q), 1.31 (3H, t), 1.27 (3H, t) |
| 1-10 | (500 MHz/CDCl₃) 12.62 (1H, s), 9.23 (1H, d), 9.11 (1H, d), 8.90 (1H, d), 6.82 (1H, d), 3.08 (2H, q), 1.40 (3H, t) |
| 1-11 | (500 MHz/CDCl₃) 13.01 (1H, s), 9.28 (1H, d), 9.02 (1H, d), 8.35 (1H, d), 6.77 (1H, d), 2.89 (2H, q), 2.53 (3H, s), 1.30 (3H, t) |
| 1-12 | (500 MHz/CDCl₃) 12.78 (1H, brs), 9.34 (1H, d), 8.76 (1H, d), 8.06 (1H, d), 6.76 (1H, d), 2.88-2.83 (3H, m), 1.29-1.22 (9H, m) |
| 1-13 | (500 MHz/CDCl₃) 12.87 (1H, s), 9.33 (1H, s), 8.57 (1H, s), 7.59 (1H, s), 6.76 (1H, d), 3.93 (3H, s), 2.86 (2H, q), 1.30 (3H, t) |
| 1-25 | (500 MHz/CDCl₃) 12.92 (1H, s), 9.32 (1H, s), 8.87 (1H, d), 8.23 (1H, d), 7.65 (1H, t), 6.75 (1H, d), 2.86 (2H, q), 1.28 (3H, t) |
| 1-27 | (500 MHz/CDCl₃) 13.01 (1H, s), 9.38 (1H, d), 9.30 (1H, s), 8.74 (1H, d), 6.79 (1H, d), 2.88 (2H, q), 1.30 (3H, t) |
| 1-42 | (500 MHz/CDCl₃) 12.90 (1H, s), 9.36 (1H, d), 8.72 (1H, d), 8.02 (1H, t), 7.51 (1H, d), 6.76 (1H, d), 2.87 (2H, q), 1.30 (3H, t) |
| 1-43 | (500 MHz/CDCl₃) 12.89 (1H, s), 9.36 (1H, d), 8.62 (1H, t), 8.12 (1H, t), 7.37 (1H, d), 6.76 (1H, d), 2.87-2.85 (2H, m), 1.30 (3H, t) |
| 1-44 | (500 MHz/CDCl₃) 12.83 (1H, s), 9.39 (1H, d), 8.48 (1H, d), 7.70 (1H, dd), 7.44 (1H, d), 6.74 (1H, d), 2.86 (2H, q), 2.42 (3H, s), 1.29 (3H, t) |
| 1-45 | (500 MHz/CDCl₃) 12.88 (1H, s), 9.34 (1H, d), 8.57 (1H, t), 7.94 (1H, d), 7.43 (1H, d), 6.74 (1H, d), 2.85 (2H, q), 1.30 (3H, t) |
| 1-46 | (500 MHz/CDCl₃) 12.83 (1H, s), 9.38 (1H, d), 8.50 (1H, dd), 7.74 (1H, d), 7.37 (1H, q), 6.74 (1H, d), 2.86 (2H, q), 2.21 (3H, s), 1.29 (3H, t) |
| 1-47 | (500 MHz/CDCl₃) 12.93 (1H, s), 9.33 (1H, d), 8.88 (1H, d), 8.25 (1H, d), 6.77(1H, d), 3.33 (1H, s), 2.87 (2H, q), 1.29 (3H, t) |
| 1-48 | (500 MHz/CDCl₃) 12.86 (1H, s), 9.32 (1H, d), 8.82 (1H, d), 8.00 (1H, d), 6.75 (1H, d), 5.15 (1H, s), 4.96 (1H, s), 2.84 (2H, q), 2.00 (3H, s), 1.26 (3H, t) |
| 1-49 | (500 MHz/CDCl₃) 12.89 (1H, s), 9.36 (1H, d), 8.72 (1H, d), 7.72 (1H, d), 6.76 (1H, d), 2.87 (2H, q), 1.84-1.79 (1H, m), 1.29 (3H, t), 0.95 (2H, d), 0.74 (2H, brs) |
| 1-51 | (500 MHz/CDCl₃) 12.91 (1H, s), 9.31 (1H, d), 7.90 (1H, d), 7.48 (1H, d), 6.76 (1H, d), 2.87 (2H, q), 1.30 (3H, t) |
| 1-52 | (500 MHz/CDCl₃) 12.92 (1H, s), 9.30 (1H, d), 8.13 (1H, d), 7.82 (1H, d), 6.77 (1H, d), 2.88 (2H, q), 1.30 (3H, t) |
| 1-53 | (500 MHz/CDCl₃) 9.36 (1H, d), 8.86 (1H, d), 7.86 (1H, s), 7.63 (1H, d), 6.78 (1H, d), 2.88 (2H, q), 1.32 (3H, t) |
| 1-54 | (500 MHz/CDCl₃) 12.91 (1H, s), 9.33 (1H, d), 8.08 (1H, t), 7.79 (2H, dd), 6.77 (1H, d), 2.87 (2H, q), 1.30 (3H, t) |
| 1-55 | (500 MHz/CDCl₃) 12.88 (1H, s), 9.34 (1H, d), 7.77 (1H, t), 7.61 (1H, d), 7.53 (1H, d), 6.76 (1H, d), 2.88 (2H, q), 1.30 (3H, t) |
| 1-56 | (500 MHz/CDCl₃) 12.82 (1H, s), 9.38 (1H, d), 7.79 (1H, t), 7.31-7.25 (2H, m), 6.74 (1H, d), 2.86 (2H, q), 2.63 (3H, s), 1.29 (3H, t) |
| 1-57 | (300 MHz/CDCl₃) 12.98 (1H, s), 9.33 (1H, dd), 8.84 (1H, dd), 6.20 (1H, dd), 6.78 (1H, dd), 2.88 (2H, q), 1.31 (3H, t) |
| 1-58 | (500 MHz/CDCl₃) 13.59 (1H, brs), 9.19 (1H, d), 8.83 (1H, d), 8.18 (1H, d), 6.63 (1H, d), 3.27 (1H, s), 2.85 (2H, q), 1.29 (3H, t) |
| 1-60 | (500 MHz/CDCl₃) 13.00 (1H, s), 9.15 (1H, d), 8.82 (1H, s), 8.18 (1H, d), 6.42 (1H, d), 2.85 (2H, q), 1.28 (3H, t) |
| 1-61 | (300 MHz/CDCl₃) 13.47 (1H, s), 8.99 (1H, d), 8.83 (1H, dd), 8.18 (1H, dd), 5.92 (1H, d), 4.01 (3H, s), 2.83 (2H, q), 1.29 (3H, t) |
| 1-63 | (500 MHz/CDCl₃) 14.24 (1H, brs), 9.21 (1H, d), 8.83 (1H, d), 8.18 (1H, d), 7.77 (1H, d), 6.51 (1H, dd), 2.85 (2H, q), 1.29 (3H, t) |
| 1-64 | (500 MHz/CDCl₃) 13.26 (1H, brs), 9.03 (1H, d), 8.83 (1H, s), 8.18 (1H, d), 6.65 (1H, d), 2.85 (2H, q), 1.29 (3H, t) |
| 1-65 | (500 MHz/CDCl₃) 13.06 (1H, s), 9.11 (1H, d), 8.83 (1H, s), 8.18 (1H, s), 6.52 (1H, d), 2.85 (2H, q), 1.29 (3H, t) |
| 1-66 | (500 MHz/CDCl₃) 13.84 (1H, brs), 9.13 (1H, d), 8.82 (1H, s), 8.17 (1H, d), 6.50 (1H, d), 2.85 (2H, q), 2.10 (3H, s), 1.28 (3H, t) |
| 1-67 | (500 MHz/CDCl₃) 14.46 (1H, brs), 9.06 (1H, d), 8.82 (1H, d), 8.17 (1H, d), 6.26 (1H, d), 2.84 (2H, q), 2.40 (3H, s), 1.29 (3H, t) |
| 1-68 | (500 MHz/CDCl₃) 14.55 (1H, brs), 9.07 (1H, d), 8.83 (1H, s), 8.17 (1H, s), 6.29 (1H, d), 2.84 (2H, q), 2.77 (2H, q), 1.32 (3H, t), 1.29 (3H, t) |
| 1-69 | (500 MHz/CDCl₃) 12.59 (1H, brs), 9.06 (1H, dd), 8.82 (1H, s), 8.18 (1H, d), 6.07 (1H, dd), 2.84 (2H, q), 1.28 (3H, t) |
| 1-70 | (500 MHz/CDCl₃) 12.67 (1H, brs), 9.38 (1H, d), 8.83 (1H, s), 8.19 (1H, d), 6.92 (1H, d), 2.87 (2H, q), 1.30 (3H, t) |
| 1-71 | (500 MHz/CDCl₃) 12.90 (1H, brs), 9.34 (1H, d), 8.83 (1H, s), 8.19 (1H, d), 6.95 (1H, d), 2.99 (3H, s), 2.85 (2H, q), 1.28 (3H, t) |
| 1-73 | (500 MHz/CDCl₃) 13.98 (1H, brs), 9.15 (1H, d), 8.83 (1H, d), 8.17 (1H, d), 6.55 (1H, d), 5.57 (1H, s), 3.40 (6H, s), 2.85 (2H, q), 1.29 (3H, t) |
| 1-74 | (500 MHz/CDCl₃) 9.13 (1H, d), 8.82 (1H, s), 8.17 (1H, d), 6.38 (1H, d), 2.83 (2H, q), 2.60 (3H, s), 1.28 (3H, t) |
| 1-76 | (500 MHz/CDCl₃) 12.73 (1H, s), 9.03 (1H, d), 8.82 (1H, s), 8.17 (1H, d), 6.02 (1H, d), 4.61 (2H, q), 2.82 (2H, q), 1.28 (3H, t) |
| 1-77 | (500 MHz/CDCl₃) 12.93 (1H, brs), 10.02 (1H, s), 8.84 (1H, s), 8.36 (1H, s), 8.21 (1H, d), 2.88 (2H, q), 1.30 (3H, t) |
| 1-78 | (500 MHz/CDCl₃) 13.64 (1H, brs), 9.18 (1H, d), 8.83 (1H, d), 8.18 (1H, d), 8.14 (1H, s), 6.81 (1H, d), 4.01 (3H, s), 2.85 (2H, q), 1.29 (3H, t) |
| 1-80 | (500 MHz/CDCl₃) 13.32 (1H, brs), 10.10 (1H, s), 9.31 (1H, d), 8.83 (1H, d), 8.19 (1H, d), 7.02 (1H, d), 2.88 (2H, q), 1.30 (3H, t) |
| 1-87 | (500 MHz/CDCl₃) 13.32 (1H, brs), 10.10 (1H, s), 9.31 (1H, d), 8.83 (1H, s), 8.19 (1H, d), 7.02 (1H, d), 2.88 (2H, q), 1.30 (3H, t) |
| 1-96 | (500 MHz/CDCl₃) 14.61 (1H, brs), 9.06 (1H, d), 8.82 (1H, s), 8.17 (1H, d), 6.30 (1H, d), 3.08 (1H, sep), 2.84 (2H, q), 1.34 (6H, d), 1.29 (3H, t) |
| 1-99 | (500 MHz/CDCl₃) 9.05 (1H, s), 8.83 (1H, d), 8.16 (1H, d), 6.34 (1H, d), 2.84 (2H, q), 1.38 (9H, s), 1.29 (3H, t) |
| 1-100 | (300 MHz/CDCl₃) 13.16 (1H, brs), 9.21 (1H, d), 8.75-8.81 (1H, m), 8.10-8.15 (1H, m), 6.74 (1H, t), 6.65 (1H, d), 2.79 (2H, q), 1.23 (3H, t) |
| 1-101 | (500 MHz/CDCl₃) 14.24 (1H, brs), 9.03 (1H, d), 8.82 (1H, d), 8.16 (1H, d), 6.15 (1H, d), 2.83 (2H, q), 1.99-2.06 (1H, m), 1.28 (3H, t), 1.03-1.08 (2H, m), 0.82-0.87 (2H, m) |
| 1-103 | (500 MHz/CDCl₃) 13.81 (1H, brs), 9.18 (1H, d), 8.86 (1H, dd), 8.20 (1H, dd), 6.59 (1H, d), 6.07 (1H, s), 4.05-4.24 (4H, m), 2.88 (2H, q), 1.31 (3H, t) |
| 1-104 | (500 MHz/CDCl₃) 13.92 (1H, brs), 9.16 (1H, d), 8.85 (1H, dd), 8.35 (1H, dd), 6.62 (1H, d), 5.74 (1H, s), 4.24-4.28 (2H, m), 3.96-4.10 (2H, m), 2.87 (2H, q), 2.18-2.40 (1H, m), 1.45-1.60 (1H, m), 1.31 (3H, t) |
| 1-105 | (500 MHz/CDCl₃) 13.63 (1H, brs), 9.13 (1H, d), 8.83 (1H, s), 8.18 (1H, d), 7.65 (1H, d), 2.84 (2H, q), 1.28 (3H, t) |
| 1-106 | (500 MHz/CDCl₃) 13.50 (1H, brs), 9.22 (1H, s), 8.83 (1H, d), 8.18 (1H, d), 7.71 (1H, s), 2.85 (2H, q), 1.28 (3H,t) |
| 1-107 | (500 MHz/CDCl₃) 13.34 (1H, brs), 9.61 (1H, s), 8.84 (1H, s), 8.19 (1H, d), 7.98 (1H, s), 2.87 (2H, q), 1.29 (3H, t) |
| 1-109 | (500 MHz/CDCl₃) 12.53 (1H, brs), 9.34 (1H, d), 8.84 (1H, s), 8.20 (1H, d), 7.12 (1H, d), 2.89 (2H, q), 1.30 (3H, t) |
| 1-110 | (500 MHz/CDCl₃) 13.50 (1H, brs), 9.26 (1H, s), 8.83 (1H, s), 8.18 (1H, d), 7.74 (1H, s), 2.85 (2H, q), 1.28 (3H, t) |
| 1-111 | (500 MHz/CDCl₃) 13.94 (1H, brs), 8.88 (1H, s), 8.23 (1H, s), 8.18 (1H, d), 7.50 (1H, s), 3.79 (3H, s), 2.84 (2H, q), 1.28 (3H, t) |
| 1-113 | (500 MHz/CDCl₃) 8.96 (1H, s), 8.83 (1H, d), 8.17 (1H, d), 7.56 (1H, s),2.84 (2H, q), 2.16 (3H, s), 1.28 (3H, t) |
| 1-115 | (500 MHz/CDCl₃) 13.46 (1H, brs), 9.30 (1H, s), 8.83 (1H, s), 8.18 (1H, s), 7.78 (1H, s), 2.85 (2H, q), 1.28 (3H, t) |
| 1-116 | (500 MHz/CDCl₃) 13.78 (1H, brs), 9.17 (1H, s), 8.83 (1H, s), 8.18 (1H, d), 7.72 (1H, s), 2.85 (2H, q), 2.39 (3H, s), 1.28 (3H,t) |
| 1-118 | (500 MHz/CDCl₃) 13.07 (1H, brs), 9.78 (1H, s), 8.83 (1H, s), 8.20 (1H, d), 8.14 (1H, s), 3.15 (3H, s), 2.87 (2H, q), 1.29 (3H, t) |
| 1-128 | (500 MHz/CDCl₃) 12.60 (1H, s), 9.21 (1H, s), 8.83 (1H, d), 8.18 (1H, d), 2.85 (2H, q), 1.28 (3H, t) |
| 1-129 | (500 MHz/CDCl₃) 12.53 (1H, s), 9.23 (1H, s), 8.83 (1H, s), 8.18 (1H, d), 2.84 (2H, q), 1.28 (3H, t) |
| 1-130 | (500 MHz/CDCl₃) 8.85 (1H, s), 8.82 (1H, s), 8.16 (1H, d), 2.83 (2H, q),2.30 (3H, s), 2.06 (3H, s), 1.28 (3H, t) |
| 1-131 | (500 MHz/CDCl₃) 12.03 (1H, brs), 10.14 (1H, s), 8.86 (1H, d), 8.23 (1H, d), 2.90 (2H, q), 1.31 (3H, t) |
| 1-132 | (500 MHz/CDCl₃) 12.44 (1H, s), 9.94 (1H, s), 8.83 (1H, d), 8.20 (1H, d), 2.87 (2H, q), 1.30 (3H, t) |
| 1-133 | (500 MHz/CDCl₃) 12.24 (1H, s), 10.00 (1H, s), 8.84 (1H, s), 8.20 (1H, d), 2.87 (2H, q), 1.30 (3H, t) |
| 1-135 | (500 MHz/CDCl₃) 12.15 (1H, s), 10.03 (1H, s), 8.84 (1H, s), 8.21 (1H, d), 2.87 (2H, q), 1.30 (3H, t) |
| 1-136 | (500 MHz/CDCl₃) 12.50 (1H, s), 9.35 (1H, s), 8.84 (1H, s), 8.20 (1H, d), 2.87 (2H, q), 1.30 (3H, t) |
| 1-138 | (500 MHz/CDCl₃) 12.50 (1H, s), 9.38 (1H, s), 8.84 (1H, s), 8.20 (1H, d), 2.87 (2H, q), 1.30 (3H, t) |
| 1-139 | (500 MHz/CDCl₃) 12.50 (1H, s), 9.87 (1H, s), 8.84 (1H, s), 8.20 (1H, d), 4.19 (3H, s), 2.84 (2H, q), 1.29 (3H, t) |
| 1-140 | (500 MHz/CDCl₃) 12.38 (1H, s), 9.60 (1H, s), 8.83 (1H, s), 8.19 (1H, d), 2.86 (2H, q), 1.29 (3H, t) |
| 1-144 | (300 MHz/CDCl₃) 12.19 (1H, s), 9.35-9.42 (1H, m), 8.66-8.72 (1H, m), 8.14-8.20 (1H, m), 2.68 (2H, q), 1.11 (3H, t) |
| 1-146 | (500 MHz/CDCl₃) 12.38 (1H, s), 9.56 (1H, s), 8.83 (1H, s), 8.19 (1H, d), 2.86 (2H, q), 1.29 (3H, t) |
| 1-147 | (500 MHz/CDCl₃) 12.18 (1H, brs), 9.43 (1H, s), 8.84 (1H, s), 8.21 (1H, d), 2.88 (2H, q), 1.30 (3H, t) |
| 1-148 | (500 MHz/CDCl₃) 12.80 (1H, s), 9.13 (1H, s), 8.83 (1H, d), 8.18 (1H, d), 2.85 (2H, q), 1.28 (3H, t) |
| 1-149 | (500 MHz/CDCl₃) 12.60 (1H, s), 9.19 (1H, s), 8.83 (1H, s), 8.18 (1H, d), 2.85 (2H, q), 1.28 (3H, t) |
| 1-150 | (500 MHz/CDCl₃) 12.18 (1H, s), 9.40 (1H, s), 8.84 (1H, d), 8.20 (1H, d), 2.88 (2H, q), 1.30 (3H, t) |
| 1-151 | (500 MHz/CDCl₃) 12.53 (1H, s), 9.24 (1H, s), 8.83 (1H, d), 8.18 (1H, d), 2.84 (2H, q), 1.28 (3H, t) |
| 1-152 | (300 MHz/CDCl₃) 12.50 (1H, brs), 9.25 (1H, s), 8.79-8.89 (1H, m), 8.17-8.19 (1H, m), 2.46 (3H, s) |
| 1-155 | (500 MHz/CDCl₃) 12.55 (1H, s), 9.24 (1H, s), 8.83 (1H, s), 8.18 (1H, d), 2.85 (2H, q), 1.28 (3H, t) |
| 1-156 | (500 MHz/CDCl₃) 13.70 (1H, brs), 9.16 (1H, s), 8.82 (1H, d), 8.17 (1H, d), 2.84 (2H, q), 2.37 (3H, s), 1.28 (3H, t) |
| 1-157 | (500 MHz/CDCl₃) 13.72 (1H, brs), 9.12 (1H, s), 8.82 (1H, s), 8.17 (1H, s), 2.83 (2H, q), 2.36 (3H, s), 1.28 (3H, t) |
| 1-158 | (300 MHz/CDCl₃) 13.65 (1H, brs), 9.11-9.18 (1H, m), 8.80-8.87 (1H, m), 8.15-8.21 (1H, m), 2.45 (3H, s), 2.37 (3H, s) |
| 1-160 | (500 MHz/CDCl₃) 12.84 (1H, s), 9.08 (1H, s), 8.82 (1H, s), 8.17 (1H, d), 4.07 (3H, s), 2.81 (2H, q), 1.28 (3H, t) |
| 1-161 | (500 MHz/CDCl₃) 13.76 (1H, brs), 9.18 (1H, s), 8.82 (1H, s), 8.17 (1H, s), 2.84 (2H, q), 2.38 (3H, s), 1.28 (3H, t) |
| 1-162 | (500 MHz/CDCl₃) 12.89 (1H, s), 9.08 (1H, s), 8.82 (1H, d), 8.17 (1H, d), 4.06 (3H, s), 2.81 (2H, q), 1.28 (3H, t) |
| 1-163 | (500 MHz/CDCl₃) 12.85 (1H, s), 9.08 (1H, s), 8.82 (1H, s), 8.17 (1H, d), 4.07 (3H, s), 2.81 (2H, q), 1.28 (3H, t) |
| 1-164 | (500 MHz/CDCl₃) 12.68 (1H, s), 9.10 (1H, d), 8.82 (1H, s), 8.18 (1H, d), 2.84 (2H, q), 1.28 (3H, t) |
| 1-165 | (500 MHz/CDCl₃) 13.11 (1H, brs), 8.92 (1H, s), 8.82 (1H, s), 8.17 (1H, d), 2.84 (2H, q), 2.09 (3H, s), 1.28 (3H, t) |
| 1-166 | (500 MHz/CDCl₃) 12.68 (1H, s), 9.13 (1H, s), 8.84 (1H, d), 8.20 (1H, d), 2.88 (2H, q), 2.43 (3H, s), 1.30 (3H, t) |
| 1-167 | (500 MHz/CDCl₃) 13.04 (1H, brs), 8.95 (1H, s), 8.82 (1H, d), 8.17 (1H, d), 2.84 (2H, q), 2.11 (3H, s), 1.28 (3H, t) |
| 1-168 | (500 MHz/CDCl₃) 12.73 (1H, s), 9.16 (1H, s), 8.83 (1H, d), 8.18 (1H, d), 2.84 (2H, q), 2.37 (3H, s), 1.28 (3H, t) |
| 1-169 | (500 MHz/CDCl₃) 12.12 (1H, brs), 9.72 (1H, s), 8.83 (1H, s), 8.20 (1H, d), 3.20 (3H, s), 2.86 (2H, q), 1.29 (3H, t) |
| 1-170 | (500 MHz/CDCl₃) 13.90 (1H, brs), 9.11 (1H, s), 8.83 (1H, s), 8.17 (1H, d), 3.18 (1H, sep), 2.83 (2H, q), 1.36 (6H, d), 1.29 (3H, t) |
| 1-171 | (500 MHz/CDCl₃) 13.84 (1H, brs), 9.12 (1H, s), 8.82 (1H, s), 8.17 (1H, d), 2.83 (2H, q), 2.76 (2H, q), 1.33 (3H, t), 1.28 (3H, t) |
| 1-172 | (500 MHz/CDCl₃) 13.90 (1H, brs), 9.15 (1H, s), 8.82 (1H ,s), 8.17 (1H, d), 3.16 (1H, sep),2.84 (2H, q), 1.36 (6H, d), 1.29 (3H, t) |
| 1-174 | (500 MHz/CDCl₃) 12.80 (1H, brs), 8.82 (1H, s), 8.79 (1H, s), 8.16 (1H, d), 3.46 (2H, brs), 2.82 (2H, q), 1.27 (3H, t) |
| 1-175 | (500 MHz/CDCl₃) 13.62 (1H, brs), 9.09 (1H, s), 8.82 (1H, s), 8.17 (1H, d), 2.81 (2H, q), 2.01-2.08 (1H, m), 1.27 (3H, t), 1.07-1.13 (2H, m), 0.96-1.02 (2H, m) |
| 1-176 | (500 MHz/CDCl₃) 13.83 (1H, brs), 9.15 (1H, s), 8.83 (1H, d), 8.17 (1H, d), 2.84 (2H, q), 2.75 (2H, q), 1.33 (3H, t), 1.29 (3H, t) |
| 1-177 | (500 MHz/CDCl₃) 13.87 (1H, brs), 9.18 (1H, s), 8.83 (1H, d), 8.18 (1H, d), 2.84 (2H, q), 2.73 (2H, q), 1.33 (3H, t), 1.29 (3H, t) |
| 1-178 | (500 MHz/CDCl₃) 13.61 (1H, brs), 9.13 (1H, s), 8.82 (1H, s), 8.17 (1H ,d), 2.81 (2H, q), 2.00-2.07 (1H, m), 1.27 (3H, t), 1.06-1.13 (2H, m), 0.95-1.01 (2H, m) |
| 1-179 | (500 MHz/CDCl₃) 13.62 (1H, brs), 9.16 (1H, s), 8.82 (1H, s), 8.17 (1H, d), 2.82 (2H, q), 1.94-2.02 (1H, m), 1.27 (3H, t), 1.06-1.12 (2H, m), 0.91-0.98 (2H, m) |
| 1-180 | (500 MHz/CDCl₃) 13.56 (1H, brs), 9.26 (1H, s), 8.83 (1H, s), 8.18 (1H, d), 5.52 (1H, s), 3.43 (6H, s), 2.84 (2H, q), 1.28 (3H, t) |
| 1-181 | (500 MHz/CDCl₃) 12.95 (1H, brs), 10.05 (1H, s), 9.44 (1H, s), 8.84 (1H, s), 8.20 (1H, d), 2.88 (2H, q), 1.30 (3H, t) |
| 1-182 | (500 MHz/CDCl₃) 13.30 (1H, brs), 9.34 (1H, s), 8.84 (1H, s), 8.22 (1H, s), 8.19 (1H, d), 7.99 (1H, brs), 2.85 (2H, q), 1.29 (3H, t) |
| 1-183 | (500 MHz/CDCl₃) 12.24 (1H, s), 9.45 (1H, s), 8.83 (1H, d), 8.20 (1H, d), 2.87 (2H, q), 1.29 (3H, t) |
| 1-184 | (500 MHz/CDCl₃) 13.52 (1H, brs), 9.22 (1H, s), 8.83 (1H, s), 8.18 (1H, s), 5.58 (1H, s), 3.44 (6H, s), 2.84 (2H, q), 1.28 (3H, t) |
| 1-185 | (500 MHz/CDCl₃) 13.39 (1H, brs), 9.31 (1H, s), 8.83 (1H, s), 8.18 (1H, d), 8.11 (1H, s), 4.06 (3H, s), 2.85 (2H, q), 1.29 (3H, t) |
| 1-186 | (500 MHz/CDCl₃) 13.42 (1H, brs), 9.31 (1H, s), 8.83 (1H, s), 8.18 (1H, d), 8.13 (1H, s), 4.31 (2H, q), 2.85 (2H, q), 1.37 (3H, t), 1.29 (3H,t) |
| 1-187 | (500 MHz/CDCl₃) 12.94 (1H, s), 10.09 (1H, s), 9.40 (1H, s), 8.84 (1H, s), 8.20 (1H, s), 2.88 (2H, q), 1.30 (3H, t) |
| 1-188 | (500 MHz/CDCl₃) 13.28 (1H, brs), 9.30 (1H, s), 8.83 (1H, s), 8.23 (1H, s), 8.19 (1H, d), 7.92 (1H, brs), 2.85 (2H, q), 1.29 (3H, t) |
| 1-189 | (500 MHz/CDCl₃) 12.21 (1H, s), 9.43 (1H, s), 8.83 (1H, s), 8.20 (1H, s), 2.87 (2H, q), 1.29 (3H, t) |
| 1-190 | (500 MHz/CDCl₃) 12.09 (1H, s), 9.15 (1H, d), 8.83 (1H, d), 8.18 (1H, d), 2.83 (2H, q), 1.27 (3H, t) |
| 1-191 | (500 MHz/CDCl₃) 13.50 (1H, brs), 9.18 (1H, s), 8.82 (1H, s), 8.18 (1H, s), 5.60 (1H, s), 3.43 (6H, s), 2.84 (2H, q), 1.28 (3H, t) |
| 1-192 | (500 MHz/CDCl₃) 12.52 (1H, s), 9.40 (1H ,s), 8.84 (1H, s), 8.19 (1H, d), 2.86 (2H, q), 1.29 (3H, t) |
| 1-195 | (300 MHz/CDCl₃) 13.85 (1H, brs), 9.05 (1H, d), 8.85 (1H, dd), 8.20 (1H, dd), 2.87 (2H, q), 2.39 (3H, s), 1.31 (3H, t) |
| 1-196 | (300 MHz/CDCl₃) 14.00 (1H, brs), 8.86 (1H, dd), 8.80 (1H, s), 8.20 (1H, dd), 3.80 (3H, s), 2.86 (2H, q), 2.32 (3H, s), 1.31 (3H, t) |
| 1-197 | (300 MHz/CDCl₃) 13.49 (1H, brs), 9.22 (1H, s), 8.85 (1H, dd), 8.20 (1H, dd), 5.78 (1H, s), 4.26-4.40 (2H, m), 3.97-4.13 (2H, m), 2.87 (2H, q), 2.22-2.45 (1H, m), 1.45-1.60 (1H, m), 1.31 (3H, t) |
| 1-198 | (300 MHz/CDCl₃) 13.45 (1H, brs), 9.19 (1H, s), 8.85 (1H, d), 8.20 (1H, d), 5.80 (1H, s), 4.25-4.45 (2H, m), 3.95-4.14 (2H, m), 2.86 (2H, q), 2.20-2.45 (1H, m), 1.43-1.62 (1H, m), 1.31 (3H, t) |
| 1-199 | (500 MHz/CDCl₃) 8.81 (1H, s), 8.13 (1H, s), 6.02 (1H, s), 2.54-3.01 (2H, m), 2.33 (3H, brs), 2.23 (3H, brs), 1.25 (3H, t) |
| 1-200 | (500 MHz/CDCl₃) 8.81 (1H, s), 8.15 (1H, d), 6.40 (1H, s), 2.81 (2H, q), 1.27 (3H, t) |
| 1-201 | (500 MHz/CDCl₃) 8.82 (1H, s), 8.17 (1H, d), 6.54 (1H, s), 2.84 (2H, q), 2.43 (3H, s), 1.29 (3H, t) |
| 1-204 | (500 MHz/CDCl₃) 13.30 (1H, brs), 9.02 (1H, d), 8.75 (1H, s), 7.88 (1H, dd), 6.65 (1H, d), 2.86 (2H, q), 1.30 (3H, t) |
| 1-205 | (500 MHz/CDCl₃) 13.09 (1H, s), 9.10 (1H, d), 8.75 (1H, s), 7.88 (1H, d), 6.52 (1H, d), 2.86 (2H, q), 1.30 (3H, t) |
| 1-206 | (500 MHz/CDCl₃) 13.03 (1H, s), 9.15 (1H, d), 8.76 (1H, s), 7.88 (1H, d), 6.43 (1H, d), 2.86 (2H, q), 1.30 (3H, t) |
| 1-207 | (500 MHz/CDCl₃) 13.25 (1H, brs), 9.03 (1H, d), 8.86 (1H, s), 8.33 (1H, d), 6.65 (1H, d), 2.85 (2H, q), 1.29 (3H, t) |
| 1-208 | (500 MHz/CDCl₃) 12.99 (1H, s), 9.16 (1H, d), 8.86 (1H, s), 8.34 (1H, d), 6.42 (1H, d), 2.85 (2H, q), 1.29 (3H, t) |
| 1-209 | (500 MHz/CDCl₃) 13.05 (1H, brs), 9.11 (1H, d), 8.86 (1H, s), 8.34 (1H, d), 6.51 (1H, d), 2.85 (2H, q), 1.29 (3H, t) |
| 1-210 | (300 MHz/CDCl₃) 12.96 (1H, s), 9.18 (1H, d), 8.77 (1H, d), 8.01 (1H, d), 6.42 (1H, d), 2.84 (2H, q), 2.61 (2H, q), 1.28 (3H, t), 1.24 (3H, t) |
| 1-212 | (500 MHz/CDCl₃) 12.59 (1H, s), 9.07 (1H, dd), 8.86 (1H, d), 8.34 (1H, d), 6.07 (1H, dd), 2.84 (2H, q), 1.28 (3H, t) |
| 1-213 | (300 MHz/CDCl₃) 13.21 (1H, brs), 9.06 (1H, d), 8.72-8.82 (1H, m), 7.94-8.02 (1H, m), 6.65 (1H, d), 2.85 (2H, q), 2.30 (3H, s), 1.28 (3H, t) |
| 1-214 | (500 MHz/CDCl₃) 12.62 (1H, brs), 9.05 (1H, d), 8.75 (1H, s), 7.89 (1H, dd), 6.08 (1H, dd), 2.85 (2H, q), 1.29 (3H, t) |
| 1-215 | (500 MHz/CDCl₃) 12.95 (1H, s), 10.01 (1H, s), 8.77 (1H, s), 8.36 (1H, s), 7.91 (1H, dd), 2.88 (2H, q), 1.31 (3H, t) |
| 1-216 | (500 MHz/CDCl₃) 13.52 (1H, brs), 9.22 (1H, s), 8.76 (1H, s), 7.88 (1H, d), 7.71 (1H, s), 2.86 (2H, q), 1.30 (3H, t) |
| 1-217 | (500 MHz/CDCl₃) 13.63 (1H, brs), 9.13 (1H, d), 8.86 (1H, s), 8.34 (1H, d), 7.65 (1H, d), 2.84 (2H, q), 1.28 (3H, t) |
| 1-218 | (300 MHz/CDCl₃) 13.50 (1H, brs), 9.23 (1H, d), 8.87 (1H, d), 8.34 (1H, d), 7.72 (1H, d), 2.85 (2H, q), 1.29 (3H, t) |
| 1-219 | (300 MHz/CDCl₃) 13.30 (1H, brs), 9.25 (1H, d), 8.77 (1H, d), 8.01 (1H, d), 7.11 (1H, d), 2.84 (2H, q), 2.62 (2H, q), 1.28 (3H, t), 1.24 (3H, t) |
| 1-220 | (500 MHz/CDCl₃) 13.58 (1H, brs), 9.16 (1H, d), 8.76 (1H, s), 7.97 (1H, s), 7.65 (1H, d), 2.84 (2H, q), 2.30 (3H, s), 1.28 (3H, t) |
| 1-221 | (500 MHz/CDCl₃) 13.44 (1H, brs), 9.25 (1H, s), 8.76 (1H, s), 7.97 (1H, s), 7.71 (1H, s), 2.84 (2H, q), 2.30 (3H, s), 1.28 (3H, t) |
| 1-222 | (300 MHz/CDCl₃) 13.63 (1H, brs), 9.17 (1H, dd), 8.84 (1H, d),8.30 (1H, d), 7.67 (1H, dd), 6.53 (1H, dd), 5.94 (1H, d), 5.56 (1H, d), 2.87 (2H, q), 1.30 (3H, t) |
| 1-223 | (300 MHz/CDCl₃) 13.61 (1H, brs), 9.18 (1H, dd), 8.79 (1H, d), 8.04 (1H, d), 7.67 (1H, dd), 2.86 (2H, q), 2.64 (2H, q), 1.30 (3H, t), 1.26 (3H, t) |
| 1-224 | (300 MHz/CDCl₃) 12.48 (1H, s), 9.98 (1H, s), 8.90 (1H, d), 8.39 (1H, d), 2.91 (2H, q), 1.33 (3H, t) |
| 1-225 | (500 MHz/CDCl₃) 12.47 (1H, s), 9.94 (1H, s), 8.77 (1H, s), 7.91 (1H, d), 2.88 (2H, q), 1.31 (3H, t) |
| 1-226 | (500 MHz/CDCl₃) 12.23 (1H, s), 10.01 (1H, s), 8.87 (1H, s), 8.36 (1H, d), 2.87 (2H, q), 1.30 (3H, t) |
| 1-227 | (500 MHz/CDCl₃) 12.14 (1H, s), 10.02 (1H, s), 8.87 (1H, d), 8.37 (1H, d), 2.87 (2H, q), 1.30 (3H, t) |
| 1-231 | (500 MHz/CDCl₃) 12.17 (1H, s), 10.02 (1H, s), 8.77 (1H, s), 7.92 (1H, dd), 2.87 (2H, q), 1.30 (3H, t) |
| 1-232 | (500 MHz/CDCl₃) 12.26 (1H, s), 10.00 (1H, s), 8.77 (1H, s), 7.92 (1H, dd), 2.88 (2H, q), 1.31 (3H, t) |
| 1-233 | (500 MHz/CDCl₃) 13.21 (1H, brs), 9.95 (1H, s), 8.83 (1H, s), 8.20 (1H, d), 2.86 (2H, q), 2.70 (3H, s), 1.29 (3H, t) |
| 1-235 | (500 MHz/CDCl₃) 13.21 (1H, brs), 9.95 (1H, s), 8.87 (1H, s), 8.36 (1H, d), 2.86 (2H, q), 2.70 (3H, s), 1.29 (3H, t) |
| 1-236 | (500 MHz/CDCl₃) 13.24 (1H, brs), 9.94 (1H, s), 8.76 (1H, s), 7.91 (1H, dd), 2.87 (2H, q), 2.70 (3H, s), 1.30 (3H, t) |
| 1-237 | (500 MHz/CDCl₃) 12.49 (1H, s), 9.39 (1H, s), 8.88 (1H, s), 8.36 (1H, d), 2.87 (2H, q), 1.30 (3H, t) |
| 1-239 | (500 MHz/CDCl₃) 12.48 (1H, s), 9.36 (1H, s), 8.88 (1H, s), 8.36 (1H, d), 2.87 (2H, q), 1.30 (3H, t) |
| 1-240 | (500 MHz/CDCl₃) 12.52 (1H, s), 9.86 (1H, s), 8.76 (1H, s), 7.91 (1H, d), 4.19 (3H, s), 2.85 (2H, q), 1.30 (3H, t) |
| 1-241 | (300 MHz/CDCl₃) 12.53 (1H, s), 9.90 (1H, s), 8.82-8.92 (1H, m), 8.23 (1H, d), 4.22 (3H, s), 2.87 (2H, q), 1.32 (3H, t) |
| 1-242 | (500 MHz/CDCl₃) 12.43 (1H, s), 9.37 (1H, s), 8.76 (1H, s), 7.99 (1H, d), 2.86 (2H, q), 2.30 (3H, s), 1.28 (3H, t) |
| 1-243 | (500 MHz/CDCl₃) 12.63 (1H, s), 9.13 (1H, d), 8.75 (1H, s), 7.98 (1H, s), 2.84 (2H, q), 2.29 (3H, s), 1.27 (3H, t) |
| 1-244 | (300 MHz/CDCl₃) 12.39 (1H, s), 9.28-9.31 (1H, m), 8.75-8.79 (1H, m), 8.18-8.25 (1H, m), 6.41 (1H, q), 5.85 (1H, d), 5.48 (1H, d), 2.79 (2H, q), 1.22 (3H, t) |
| 1-245 | (300 MHz/CDCl₃) 12.36 (1H, s), 9.25-9.39 (1H, m), 8.69-8.72 (1H, m), 7.91-7.99 (1H, m), 2.78 (2H, q), 2.54 (2H, q), 1.21 (3H, t), 1.17 (3H, t) |
| 1-246 | (500 MHz/CDCl₃) 12.38 (1H, s), 9.33 (1H, s), 8.81 (1H, d), 8.00 (1H, d), 5.14 (1H, t), 4.94 (1H, s), 2.83 (2H, q), 2.00 (3H, s), 1.25 (3H, t) |
| 1-247 | (500 MHz/CDCl₃) 11.90 (1H, brs), 9.36 (1H, s), 8.75 (1H, d), 8.06 (1H, d), 2.87-2.80 (3H, m), 1.28-1.23 (9H, m) |
| 1-249 | (300 MHz/CDCl₃) 13.07 (1H, s), 8.99 (1H, d), 8.89 (1H, dd),8.36 (1H, dd), 2.87 (2H, q), 2.13 (3H, d), 1.31 (3H, t) |
| 1-250 | (500 MHz/CDCl₃) 12.98 (1H, brs), 8.98 (1H, s), 8.75 (1H, s), 7.97 (1H, d), 2.83 (2H, q), 2.29 (3H, s), 2.09 (3H, s), 1.27 (3H, t) |
| 1-251 | (500 MHz/CDCl₃) 13.74 (1H, brs), 9.13 (1H, s), 8.86 (1H, s), 8.33 (1H, s), 2.83 (2H, q), 2.37 (3H, s), 1.28 (3H, t) |
| 1-252 | (500 MHz/CDCl₃) 13.67 (1H, brs), 9.15 (1H, s), 8.75 (1H, s), 7.97 (1H, s), 2.83 (2H, q), 2.36 (3H, s), 2.30 (3H, s), 1.28 (3H, t) |
| 1-253 | (300 MHz/CDCl₃) 12.54 (1H, s), 9.44 (1H, d), 8.90 (1H, dd), 8.34-8.44 (1H, m), 2.89 (2H, q), 1.32 (3H, t) |
| 1-256 | (300 MHz/CDCl₃) 12.94 (1H, s), 9.32 (1H, m), 8.84 (1H, dd), 8.19 (1H, dd), 6.90 (1H, dd), 2.48 (3H, s) |
| 1-257 | (500 MHz/CDCl₃) 12.99 (1H, s), 9.32 (1H, d), 8.84 (1H, s), 8.20 (1H, d), 6.79 (1H, d), 2.82 (2H, br), 1.78 (2H, dt), 1.02 (3H, t) |
| 1-258 | (500 MHz/CDCl₃) 13.05 (1H, s), 9.31 (1H, d), 8.83 (1H, d), 8.19 (1H, d), 6.77 (1H, d), 3.44 (1H, m), 1.30 (6H, d) |
| 1-259 | (500 MHz/CDCl₃) 13.07 (1H, s), 9.33 (1H, d), 8.84 (1H, s), 8.20 (1H, d), 6.78 (1H, d), 3.18-3.28 (1H, m), 1.82-1.91 (1H, m), 1.55-1.66 (1H, m), 1.29 (3H, d), 0.95 (3H, t) |
| 1-260 | (500 MHz/CDCl₃) 13.38 (1H, s), 9.33 (1H, d), 8.82 (1H, s), 8.19 (1H, d), 6.77 (1H, d), 1.43 (9H, s) |
| 1-261 | (300 MHz/CDCl₃) 13.00 (1H, s), 9.34 (1H, m), 8.84 (1H, dd), 8.20 (1H, dd), 6.78 (1H, dd), 2.71 (2H, br), 2.14-2.28 (1H, m), 0.97 (6H, d) |
| 1-262 | (500 MHz/CDCl₃) 13.07 (1H, s), 9.31 (1H, d), 8.81 (1H, d), 8.17 (1H, s), 6.77 (1H, d), 2.39 (1H, m), 1.01 (4H, m) |
| 1-263 | (500 MHz/CDCl₃) 13.02 (1H, s), 9.31 (1H, d), 8.83 (1H, s), 8.18 (1H, d), 6.77 (1H, d), 3.47 (1H, quintet), 1.86-1.64 (8H, m) |
| 1-264 | (500 MHz/CDCl₃) 13.07 (1H, s), 9.31 (1H, d), 8.83 (1H, d), 8.18 (1H, d), 6.77 (1H, d), 3.10-3.07 (1H, m), 2.00 (2H, d), 1.85 (2H, d), 1.74 (1H, d), 1.53-1.36 (4H, m), 1.22-1.18 (1H, m) |
| 1-267 | (300 MHz/CDCl₃) 13.11 (1H, s), 9.30 (1H, d), 8.84 (1H, d), 8.20 (1H, d), 6.80 (1H, d), 4.62 (2H, brs), 3.50 (3H, s) |
| 1-270 | (300 MHz/CDCl₃) 13.23 (1H, brs), 9.28 (1H, m), 8.85 (1H, dd), 8.23 (1H, dd), 6.83 (1H, d), 6.72 (1H, t) |
| 1-272 | (300 MHz/CDCl₃) 13.24 (1H, s), 9.30 (1H, m), 8.85 (1H, dd), 8.22 (1H, dd), 6.82 (1H, dd), 4.57 (2H, s) |
| 1-273 | (500 MHz/CDCl₃) 13.06 (1H, s), 9.33 (1H, d), 8.88 (1H, s), 8.36 (1H, d), 6.78 (1H, d), 3.43 (1H, sep), 1.31 (6H, d) |
| 1-274 | (500 MHz/CDCl₃) 13.08 (1H, s), 9.32 (1H, d), 8.85 (1H, s), 8.33 (1H, d), 6.77 (1H, d), 2.39 (1H, tt), 0.91-1.12 (4H, m) |
| 1-275 | (500 MHz/CDCl₃) 13.08 (1H, s), 9.32 (1H, d), 8.74 (1H, s), 7.88 (1H, d), 6.78 (1H, d), 2.36-2.41 (1H, m), 1.02-1.05 (4H, m) |
| 1-276 | (500 MHz/CDCl₃) 13.09 (1H, s), 9.32 (1H, s), 8.77 (1H, s), 7.90 (1H, dd), 6.89 (1H, d), 3.42 (1H, sep), 1.32 (6H, d) |
| 1-277 | (500 MHz/CDCl₃) 12.92 (1H, s), 9.32 (1H, d), 8.88 (1H, s), 8.35 (1H, d), 6.78 (1H, d), 2.48 (3H, s) |
| 1-278 | (500 MHz/CDCl₃) 13.01 (1H, s), 9.36 (1H, d), 8.77 (1H, s), 8.00 (1H, s), 6.77 (1H, d), 3.42 (1H, sep), 2.29 (3H, s), 1.30 (6H, d) |
| 1-282 | (300 MHz/CDCl₃) 13.59 (1H, brs), 9.17 (1H, dd), 8.73-8.80 (1H, m), 7.95-8.02 (1H, m), 7.64 (1H, dd), 3.40 (1H, sep), 2.29 (3H, s), 1.29 (6H, d) |
| 1-283 | (500 MHz/CDCl₃) 13.03 (1H, s), 9.35 (1H, d), 8.75 (1H, s), 7.97 (1H, s), 6.78 (1H, d), 2.37-2.42 (1H, m), 2.27 (3H, s), 0.98-1.08 (4H, br) |
| 1-284 | (500 MHz/CDCl₃) 12.97 (1H, s), 9.32 (1H, s), 8.72 (1H, s), 7.90 (1H, d), 6.79 (1H, d), 2.50 (3H, s) |
| 1-285 | (500 MHz/CDCl₃) 13.72 (1H, s), 9.13 (1H, d), 8.82 (1H, s), 8.18 (1H, d), 7.64 (1H, d), 3.41-3.36 (1H, m), 1.94 (6H, d) |
| 1-286 | (500 MHz/CDCl₃) 12.55 (1H, s), 9.33 (1H, s), 8.22 (1H, d), 8.18 (1H, d), 3.39 (1H, sep), 1.28 (6H, d) |
| 1-287 | (500 MHz/CDCl₃) 12.22 (1H, s), 9.34 (1H, s), 8.83 (1H, d), 8.20 (1H, s), 3.39 (1H, sep), 1.29 (6H, d) |
| 1-289 | (500 MHz/CDCl₃) 12.45 (1H, s), 9.34 (1H, s), 8.83 (1H, s), 8.19 (1H, d), 2.47 (3H, s) |
| 1-291 | (500 MHz/CDCl₃) 12.13 (1H, brs), 10.02 (1H, s), 8.83 (1H, s), 8.21 (1H, s), 2.48 (3H, s) |
| 1-292 | (300 MHz/CDCl₃) 12.56 (1H, brs), 9.11-9.39 (1H, m), 8.81-8.91 (1H, m), 8.35-8.39 (1H, m), 3.33-3.49 (1H, m), 1.30 (6H, d) |
| 1-293 | (300 MHz/CDCl₃) 12.44 (1H, brs), 9.34-9.39 (1H, m), 8.82-8.91 (1H, m), 8.34-8.39 (1H, m), 2.48 (3H, s) |
| 1-294 | (300 MHz/CDCl₃) 12.48 (1H, s), 9.53-9.59 (1H, m), 8.86-8.93 (1H, m), 8.35-8.41 (1H, m), 2.90 (2H, q), 1.33 (3H, t) |
| 1-295 | (300 MHz/CDCl₃) 12.20 (1H, s), 10.34 (1H, s), 8.77 (1H, d), 8.04 (1H, d), 2.87 (2H, q), 2.61 (2H, q), 1.15-1.41 (6H, m) |
| 1-296 | (300 MHz/CDCl₃) 13.08 (1H, s), 9.07-9.13 (1H, m), 8.83-8.90 (1H, m), 8.18-8.25 (1H, m), 2.88 (2H, q), 2.23-2.30 (3H, m), 1.32 (3H, t) |
| 1-297 | (300 MHz/CDCl₃) 13.05 (1H, s), 9.05-9.12 (1H, m), 8.84-8.91 (1H, m), 8.32-8.39 (1H, m), 2.86 (2H, q), 2.21-2.28 (3H, m), 1.30 (3H, t) |
| 1-298 | (300 MHz/CDCl₃) 13.00 (1H, s), 9.07-9.13 (1H, m), 8.74-8.80 (1H, m), 7.95-8.02 (1H, m), 2.86 (2H, q), 2.30 (3H, s), 2.21-2.28 (3H, m), 1.29 (3H, t) |
| 1-299 | (300 MHz/CDCl₃) 13.50 (1H, brs), 9.51 (1H, s), 8.73-8.80 (1H, m), 7.95-8.02 (1H, m), 2.85 (2H, q), 2.49 (3H, s), 2.31 (3H,s ), 1.29 (3H, t) |
| 1-300 | (300 MHz/CDCl₃) 12.43 (1H, s), 9.49-9.55 (1H, m), 8.84 (1H, d), 8.19 (1H, d), 2.48 (3H, s) |
| 1-301 | (300 MHz/CDCl₃) 12.52 (1H, brs), 9.35-9.41 (1H, m), 8.74-8.80 (1H, m), 7.97-8.03 (1H, m), 3.41 (1H, sep), 2.29 (3H, s), 1.30 (6H, d) |
| 2-1 | (300 MHz/CDCl₃) 12.80 (1H, s), 9.43 (1H, dd), 7.60-7.39 (5H, m), 6.76 (1H, d), 2.87 (2H, q), 1.31 (3H, t) |
| 2-2 | (500 MHz/CDCl₃) 12.81 (1H, s), 9.42 (1H, s), 7.39-7.32 (3H, m), 7.28 (1H, d), 6.75 (1H, d), 2.85 (2H, q), 2.16 (3H, s), 1.29 (3H, t) |
| 2-3 | (500 MHz/CDCl₃) 12.87 (1H, s), 9.34 (1H, d), 7.85 (1H, d), 7.72 (1H, t), 7.63 (1H, t), 7.47 (1H, d), 6.75 (1H, d), 2.83 (2H, brs), 1.28 (3H, t) |
| 2-4 | (500 MHz/CDCl₃) 12.86 (1H, s), 9.39 (1H, s), 7.91 (1H, s), 7.84 (1H, d), 7.68 (1H, d), 7.62 (1H, t), 6.77 (1H, d), 2.87 (2H, q), 1.32 (3H, t) |
| 2-6 | (500 MHz/CDCl₃) 12.87 (1H, s), 9.38 (1H, d), 7.78-7.74 (4H, m), 6.78 (1H, d), 2.87 (2H, q), 1.31 (3H, t) |
| 2-7 | (500 MHz/CDCl₃) 12.76 (1H, s), 9.42 (1H, d), 7.42 (2H, t), 7.29 (2H, t), 6.75 (1H, d), 2.85 (2H, q), 2.41 (3H, s), 1.30 (3H, t) |
| 2-8 | (500 MHz/CDCl₃) 12.89 (1H, s), 9.36 (1H, d), 7.61 (1H, dd), 7.39-7.35 (2H, m), 6.76 (1H, d), 2.87-2.82 (2H, m), 1.30 (3H, t) |
| 2-9 | (500 MHz/CDCl₃) 12.88 (1H, s), 9.37 (1H, d), 7.77-7.75 (1H, m), 7.48-7.43 (2H, m), 6.76 (1H, d), 2.88-2.82 (2H, m), 2.23 (3H, brd), 1.28 (3H, t) |
| 2-10 | (500 MHz/CDCl₃) 12.92 (1H, s), 9.35 (1H, d), 7.85 (1H, d), 7.64 (1H, d), 7.55 (1H, t), 6.77 (1H, d), 2.90-2.81 (2H, m), 1.29 (3H, t) |
| 2-11 | (500 MHz/CDCl₃) 12.89 (1H, s), 9.33 (1H, d), 7.67 (1H, d), 7.60 (1H, t), 7.34 (1H, d), 6.76 (1H, d), 2.90-2.76 (2H, m), 1.26 (3H, t) |
| 2-12 | (500 MHz/CDCl₃) 12.92 (1H, s), 9.34 (1H, d), 7.84 (1H, d), 7.71 (1H, dd), 7.59 (1H, d), 6.77 (1H, d), 2.87 (2H, q), 1.28 (3H, t) |
| 2-13 | (500 MHz/CDCl₃) 12.90 (1H, s), 9.35 (1H, d), 7.50 (1H, d), 7.45 (1H, d), 7.40 (1H, dd), 6.77 (1H, d), 2.85 (2H, q), 1.29 (3H, t) |
| 2-14 | (500 MHz/CDCl₃) 12.93 (1H, s), 9.35 (1H, d), 8.02 (1H, s), 7.76 (1H, dd), 7.58 (1H, d), 6.78 (1H, d), 2.86 (2H, br), 1.30 (3H, t) |
| 2-15 | (500 MHz/CDCl₃) 12.88 (1H, s), 9.38 (1H, s), 7.64 (1H, s), 7.62 (1H, d), 7.43 (1H, d), 6.78 (1H, d), 2.86 (2H, q), 2.24 (3H, s), 1.29 (3H, t) |
| 2-18 | (500 MHz/CDCl₃) 12.88 (1H, s), 9.36 (1H, d), 7.42 (1H, dd), 7.32 (1H, dd), 7.15 (1H, td), 6.76 (1H, d), 2.86-2.82 (2H, m), 1.28 (3H, t) |
| 2-19 | (500 MHz/CDCl₃) 12.88 (1H, s), 9.35 (1H, d), 7.73 (1H, d), 7.57 (1H, dd), 7.30 (1H, d), 6.76 (1H, d), 2.84 (2H, q), 1.28 (3H, t) |
| 2-20 | (500 MHz/CDCl₃) 12.92 (1H, s), 9.35 (1H, d), 8.22 (1H, s), 7.78 (1H, d), 7.52 (1H, d), 6.77 (1H, d), 2.86 (2H, brs), 1.30 (3H, t) |
| 2-23 | (500 MHz/CDCl₃) 12.88 (1H, s), 9.36 (1H, d), 7.97 (1H, d), 7.49 (1H, dd), 7.32 (1H, d), 6.76 (1H, d), 2.86 (2H, brs), 1.30 (3H, t) |
| 2-24 | (500 MHz/CDCl₃) 12.87 (1H, s), 9.35 (1H, d), 7.92 (1H, d), 7.76 (1H, dd), 7.15 (1H, d), 6.76 (1H, d), 2.84 (2H, q), 1.27 (3H, t) |
| 2-25 | (500 MHz/CDCl₃) 12.87 (1H, s), 9.36 (1H, s), 7.39 (1H, t), 7.30-7.27 (2H, m), 6.76 (1H, d), 2.84 (2H, q), 1.28 (3H, t) |
| 2-26 | (500 MHz/CDCl₃) 12.94 (1H, s), 9.30 (1H, d), 8.10 (1H, s), 7.99 (1H, d), 7.65 (1H, d), 6.77 (1H, d), 2.84 (2H, q), 1.26 (3H, t) |
| 2-27 | (500 MHz/CDCl₃) 12.90 (1H, s), 9.31 (1H, d), 7.81 (1H, d), 7.68 (1H, t), 7.42 (1H, d), 6.76 (1H, d), 2.83 (2H, brd), 1.25 (3H, t) |
| 2-28 | (500 MHz/CDCl₃) 12.91 (1H, s), 9.35 (1H, d), 7.95 (1H, s), 7.68 (1H, d), 7.45 (1H, d), 6.76 (1H, d), 6.47 (1H, dd), 5.84 (1H, d), 5.40 (1H, d), 2.85 (2H, q), 1.28 (3H, t) |
| 2-29 | (500 MHz/CDCl₃) 12.89 (1H, s), 9.38 (1H, s), 7.67 (1H, s), 7.62 (1H, d), 7.41 (1H, d), 6.77 (1H, d), 2.86 (2H, q), 2.55 (2H, q), 1.29 (3H, t), 1.20 (3H, t) |
| 2-30 | (500 MHz/CDCl₃) 12.84 (1H, s), 9.39 (1H, d), 7.34 (1H, d), 7.08 (1H, d), 6.94 (1H, dd), 6.75 (1H, d), 3.84 (3H, s), 2.84 (2H, qd), 1.28 (3H, t) |
| 2-32 | (500 MHz/CDCl₃) 12.88 (1H, s), 9.38 (1H, s), 7.63-7.60 (2H, m), 7.43 (1H, d), 6.77 (1H, d), 2.86 (2H, q), 2.24 (3H, s), 1.29 (3H, t) |
| 2-35 | (500 MHz/CDCl₃) 12.85 (1H, s), 9.35 (1H, d), 7.38 (1H, d), 7.30 (1H, d), 7.19 (1H, dd), 6.74 (1H, d), 3.89 (3H, d), 2.83 (2H, brt), 1.26 (3H, t) |
| 2-36 | (500 MHz/CDCl₃) 12.91 (1H, s), 9.36 (1H, d), 7.50-7.45 (2H, m), 7.30 (1H, dd), 6.76 (1H, d), 2.86 (2H, q), 1.29 (3H, t) |
| 2-37 | (500 MHz/CDCl₃) 12.83 (1H, s), 9.38 (1H, d), 7.46 (1H, d), 7.38 (1H, d), 7.28 (1H, s), 6.76 (1H, d), 3.88 (3H, s), 2.84 (2H, q), 1.29 (3H, t) |
| 2-38 | (500 MHz/CDCl₃) 12.82 (1H, s), 9.35 (1H, d), 7.67-7.65 (2H, m), 7.48 (1H, d), 6.75 (1H, d), 5.04 (1H, t), 4.81 (1H, s), 2.83 (2H, q), 1.98 (3H, s), 1.26 (3H, t) |
| 2-39 | (500 MHz/CDCl₃) 12.86 (1H, s), 9.38 (1H, s), 7.59 (1H, d), 7.42-7.40 (2H, m), 6.77 (1H, d), 2.86 (2H, brd), 1.78-1.72 (1H, m), 1.29 (3H, t), 0.85 (2H, d), 0.70 (2H, brs) |
| 2-40 | (500 MHz/CDCl₃) 12.88 (1H, s), 9.38 (1H, d), 7.72 (1H, s), 7.60 (1H, dd), 7.38 (1H, d), 6.76 (1H, d), 2.89-2.75 (3H, m), 1.27 (3H, t), 1.24 (6H, dd) |
| 2-41 | (500 MHz/CDCl₃) 12.92 (1H, s), 9.35 (1H, d), 7.63-7.57 (2H, m), 7.54 (1H, d), 6.77 (1H, d), 2.86 (2H, q), 1.29 (3H, t) |
| 2-42 | (500 MHz/CDCl₃) 12.88 (1H, brs), 9.36 (1H, d), 7.93 (1H, d), 7.76 (1H, t), 7.58 (1H, d), 6.77 (1H, d), 3.17 (1H, s), 2.86 (2H, q), 1.29 (3H, t) |
| 2-43 | (300 MHz/CDCl₃) 12.83 (1H, s), 9.40-9.39 (1H, m), 7.28 (1H, d), 7.08 (2H, d), 6.77 (1H, d), 6.76-6.29 (1H, t), 2.85 (2H, q), 2.17 (3H, s), 1.29 (3H, t) |
| 2-44 | (300 MHz/CDCl₃) 12.92 (1H, s), 9.37 (1H, dd), 7.97-7.92 (2H, m), 7.53 (1H, d), 6.79 (1H, d), 3.09 (3H, s), 2.86 (2H, q), 2.29 (3H, s), 1.29 (3H, t) |
| 2-45 | (300 MHz/CDCl₃) 12.83 (1H, s), 9.41 (1H, dd), 7.29 (3H, s), 6.76 (1H, d), 7.42 (2H, q), 2.85 (2H, q), 2.18 (3H, s), 1.29 (3H, s) |
| 2-46 | (500 MHz/CDCl₃) 12.88 (1H, s), 9.35 (1H, d), 7.58 (1H, d), 7.41 (1H, d), 7.38 (1H, s), 6.76 (1H, d), 2.86 (2H, q), 1.28 (3H, t) |
| 2-47 | (500 MHz/CDCl₃) 12.78 (1H, s), 9.42 (1H, d), 7.24 (1H, d), 7.18 (1H, d), 7.08 (1H, s), 6.75 (1H, d), 2.85 (2H, q), 2.36 (3H, s), 2.10 (3H, s), 1.28 (3H, t) |
| 2-48 | (500 MHz/CDCl₃) 12.92 (1H, s), 9.34 (1H, d), 7.72 (3H, d), 6.77 (1H, d), 2.86 (2H, q), 1.29 (3H, t) |
| 2-49 | (500 MHz/CDCl₃) 12.90 (1H, d), 9.36 (1H, d), 7.60-7.58 (2H, m), 7.49 (1H, dd), 6.77 (1H, d), 2.85 (2H, brt), 1.29 (3H, t) |
| 2-50 | (500 MHz/CDCl₃) 12.89 (1H, s), 9.36 (1H, d), 7.24-7.14 (3H, m), 6.77 (1H, d), 2.85 (2H, q), 1.29 (3H, t) |
| 2-51 | (500 MHz/CDCl₃) 12.85 (1H, s), 9.39 (1H, d), 7.36 (1H, dd), 7.30-7.27 (2H, m), 6.76 (1H, d), 2.87 (2H, q), 2.13 (3H, s), 1.29 (3H, t) |
| 2-52 | (500 MHz/CDCl₃) 12.86 (1H, s), 9.39 (1H, t), 7.44 (1H, d), 7.25-7.22 (2H, m), 6.75 (1H, d), 2.85 (2H, qd), 2.39 (3H, s), 1.29 (3H, t) |
| 2-53 | (500 MHz/CDCl₃) 12.86 (1H, s), 9.39 (1H, d), 7.45 (1H, d), 6.99 (1H, d), 6.96 (1H, dd), 6.75 (1H, d), 3.82 (3H, s), 2.86-2.83 (2H, m), 1.29 (3H, t) |
| 2-54 | (500 MHz/CDCl₃) 12.90 (1H, s), 9.36 (1H, s), 7.70 (1H, dd), 7.20 (1H, dd), 7.11 (1H, td), 6.76 (1H, d), 2.85-2.84 (2H, m), 1.29 (3H, t) |
| 2-55 | (500 MHz/CDCl₃) 12.89 (1H, s), 9.36 (1H, d), 7.54 (1H, dd), 7.21-7.15 (2H, m), 6.76 (1H, d), 2.85 (2H, q), 1.29 (3H, t) |
| 2-56 | (500 MHz/CDCl₃) 12.91 (1H, s), 9.35 (1H, d), 7.60 (1H, d), 7.34-7.30 (2H, m), 6.77 (1H, d), 2.85 (2H, q), 1.29 (3H, t) |
| 2-57 | (500 MHz/CDCl₃) 12.89 (1H, s), 9.36 (1H, d), 7.60-7.55 (2H, m), 7.14 (1H, t), 6.76 (1H, d), 2.85 (2H, q), 1.29 (3H, t) |
| 2-58 | (500 MHz/CDCl₃) 12.89 (1H, s), 9.36 (1H, d), 7.45 (1H, dd), 7.43-7.40 (1H, m), 7.19 (1H, t), 6.76 (1H, d), 2.85 (2H, q), 1.29 (3H, t) |
| 2-59 | (500 MHz/CDCl₃) 12.90 (1H, s), 9.35 (1H, d), 7.59 (1H, d), 7.56 (1H, dd), 7.43 (1H, d), 6.76 (1H, d), 2.85 (2H ,q), 1.28 (3H, t) |
| 2-60 | (500 MHz/CDCl₃) 12.90 (1H, s), 9.36 (1H, s), 7.89 (1H, d), 7.41 (1H, d), 7.19 (1H, dd), 6.77 (1H, s), 2.86 (2H, brs), 1.30 (3H, t) |
| 2-61 | (500 MHz/CDCl₃) 12.90 (1H, s), 9.35 (1H, d), 7.67 (1H, d), 7.44 (1H, d), 7.35 (1H, dd), 6.77 (1H, d), 2.86-2.83 (2H, m), 1.29 (3H, t) |
| 2-62 | (500 MHz/CDCl₃) 12.88 (1H, s), 9.35 (1H, d), 7.76-7.73 (2H, m), 7.01 (1H, t), 6.76 (1H, d), 2.84 (2H, q), 1.29 (3H, t) |
| 2-63 | (500 MHz/CDCl₃) 12.89 (1H, s), 9.35 (1H, d), 7.74 (1H, d), 7.66 (1H, dd), 7.45 (1H, d), 6.76 (1H, d), 2.87-2.81 (2H, m), 1.29 (3H, t) |
| 2-64 | (500 MHz/CDCl₃) 12.89 (1H, s), 9.35 (1H, d), 7.75-7.72 (2H, m), 7.29 (1H, d), 6.76 (1H, d), 2.85 (2H, q), 1.29 (3H, t) |
| 2-65 | (500 MHz/CDCl₃) 12.93 (1H, s), 9.37 (1H, s), 7.48 (2H, d), 7.38 (1H, t), 6.76 (1H, d), 2.87 (2H, q), 1.29 (3H, t) |
| 2-66 | (300 MHz/CDCl₃) 12.87 (1H, s), 9.36-9.35 (1H, m), 7.69-7.48 (3H, m), 6.76-6.75 (1H, m), 2.95-2.74 (2H, m), 2.16 (3H, s), 1.26 (3H, t) |
| 2-67 | (500 MHz/CDCl₃) 12.92 (1H, s), 9.33 (1H, d), 7.78 (1H, d), 7.76 (1H, d), 7.57 (1H, t), 6.76 (1H, d), 2.92-2.78 (2H, m), 1.26 (3H, t) |
| 2-68 | (500 MHz/CDCl₃) 12.85 (1H, s), 9.37 (1H, d), 7.77 (1H, d), 7.56 (1H, d), 7.52 (1H, dd), 6.77 (1H, d), 2.87 (2H, q), 1.30 (3H, t) |
| 2-69 | (500 MHz/CDCl₃) 12.91 (1H, s), 9.36 (1H, s), 7.72 (1H, t), 7.65-7.62 (2H, m), 6.79 (1H, d), 2.88 (2H, q), 1.32 (3H, t) |
| 2-70 | (500 MHz/CDCl₃) 12.91 (1H, s), 9.36 (1H, d), 7.89 (1H, s), 7.81 (1H, d), 7.73 (1H, d), 6.79 (1H, d), 2.88 (2H, q), 1.32 (3H, t) |
| 2-71 | (500 MHz/CDCl₃) 12.88 (1H, s), 9.36 (1H, d), 8.01 (1H, d), 7.80 (1H, t), 7.62 (1H, d), 6.78 (1H, d), 2.87 (2H, q), 1.31 (3H, t) |
| 2-72 | (500 MHz/CDCl₃) 12.88 (1H, s), 9.36 (1H, d), 7.58 (2H, d), 7.40 (1H, t), 6.78 (1H, d), 2.86 (2H, q), 1.31 (3H, t) |
| 2-73 | (500 MHz/CDCl₃) 12.48 (1H, s), 9.37 (1H, s), 7.38 (2H, s), 2.86 (2H, q), 1.28 (3H, t) |
| 2-74 | (500 MHz/CDCl₃) 12.91 (1H, s), 9.31 (1H, d), 7.77 (1H, d), 7.60 (1H, d), 7.49 (1H, s), 6.76 (1H, d), 2.83 (2H, q), 1.26 (3H, t) |
| 2-75 | (500 MHz/CDCl₃) 12.87 (1H, s), 9.37 (1H, s), 7.15 (1H, q), 7.08 (1H, dd), 6.77 (1H, d), 2.85 (2H, q), 2.11 (3H, d), 1.28 (3H, t) |
| 2-76 | (500 MHz/CDCl₃) 12.93 (1H, s), 9.33 (1H, d), 7.61 (1H, d), 7.38 (1H, d), 6.77 (1H, d), 2.85 (2H, qd), 1.28 (3H, t) |
| 2-77 | (500 MHz/CDCl₃) 12.96 (1H, s), 9.34 (1H, d), 7.55 (1H, d), 7.45 (1H, d), 6.77 (1H, d), 2.86 (2H, q), 1.28 (3H, t) |
| 2-78 | (500 MHz/CDCl₃) 12.91 (1H, s), 9.32 (1H, s), 7.67 (1H, s), 7.54 (1H, s), 6.76 (1H, s), 2.85-2.82 (2H, m), 1.29-1.26 (3H, m) |
| 2-79 | (500 MHz/CDCl₃) 12.91 (1H, s), 9.33 (1H, d), 7.49 (1H, t), 7.27 (1H, t), 6.77 (1H, d), 2.84 (2H, q), 1.28 (3H, t) |
| 2-80 | (500 MHz/CDCl₃) 12.90 (1H, s), 9.34 (1H, d), 7.35-7.30 (1H, m), 7.15-7.10 (1H, m), 6.77 (1H, d), 2.84 (2H, q), 1.28 (3H, t) |
| 2-81 | (500 MHz/CDCl₃) 12.91 (1H, s), 9.33 (1H, s), 7.35 (1H, dd), 7.29 (1H, dd), 6.77 (1H, d), 2.84 (2H, q), 1.28 (3H, t) |
| 2-82 | (500 MHz/CDCl₃) 12.92 (1H, s), 9.33 (1H, s), 8.16 (1H, d), 7.22 (1H, d), 6.77 (1H, d), 2.86 (2H, brd), 1.30 (3H, t) |
| 2-83 | (500 MHz/CDCl₃) 12.92 (1H, s), 9.34 (1H, d), 7.77 (1H, t), 7.28 (1H, dd), 6.77 (1H, d), 2.86 (2H, brs), 1.30 (3H, t) |
| 2-84 | (500 MHz/CDCl₃) 12.96 (1H, s), 9.32 (1H, d), 8.23 (1H, s), 7.72 (1H, s), 6.78 (1H, d), 2.87 (2H, brd), 1.30 (3H, t) |
| 2-85 | (500 MHz/CDCl₃) 12.91 (1H, s), 9.33 (1H, s), 7.65 (1H, d), 7.53 (1H, d), 6.77 (1H, d), 2.84 (2H, brt), 1.29 (3H, t) |
| 2-86 | (500 MHz/CDCl₃) 12.91 (1H, s), 9.33 (1H, d), 7.58 (1H, d), 7.40 (1H, d), 6.77 (1H, d), 2.84 (2H, q), 1.29 (3H, t) |
| 2-87 | (500 MHz/CDCl₃) 12.92 (1H, s), 9.33 (1H, s), 7.55 (1H, d), 7.51 (1H, d), 6.77 (1H, d), 2.84 (2H, brt), 1.28 (3H, t) |
| 2-88 | (500 MHz/CDCl₃) 12.92 (1H, s), 9.33 (1H, d), 7.63 (1H, d), 7.29 (1H, d), 6.77 (1H, d), 2.84 (2H, q), 1.28 (3H, t) |
| 2-89 | (500 MHz/CDCl₃) 12.92 (1H, s), 9.33 (1H, d), 7.59 (1H, t), 7.33-7.30 (1H, m), 6.77 (1H, d), 2.85 (2H, brd), 1.28 (3H, t) |
| 2-90 | (500 MHz/CDCl₃) 12.91 (1H, s), 9.33 (1H, d), 7.57-7.54 (2H, m), 6.77 (1H, d), 2.84 (2H, q), 1.28 (3H, t) |
| 2-91 | (500 MHz/CDCl₃) 12.90 (1H, s), 9.33 (1H, d), 7.67 (1H, t), 7.09 (1H, t), 6.77 (1H, d), 2.84 (2H, q), 1.29 (3H, t) |
| 2-92 | (500 MHz/CDCl₃) 12.91 (1H, s), 9.33 (1H, s), 7.41 (1H, dd), 7.32 (1H, dd), 6.77 (1H, d), 2.84 (2H, q), 1.28 (3H, t) |
| 2-93 | (500 MHz/CDCl₃) 12.95 (1H, s), 9.32 (1H, d), 7.51 (1H, dd), 7.39 (1H, dd), 6.78 (1H, d), 2.85 (2H, q), 1.29 (3H, t) |
| 2-94 | (500 MHz/CDCl₃) 12.95 (1H, s), 9.31 (1H, d), 7.82 (1H, d), 7.36 (1H, d), 6.78 (1H, d), 2.85 (2H, q), 1.28 (3H, t) |
| 2-95 | (500 MHz/CDCl₃) 12.96 (1H, s), 9.32 (1H, d), 8.05 (1H, s), 7.60 (1H, s), 6.78 (1H, d), 2.87 (2H, q), 1.29 (3H, t) |
| 2-96 | (500 MHz/CDCl₃) 12.86 (1H, s), 9.37 (1H, d), 7.50 (1H, d), 7.13 (1H, d), 6.77 (1H, d), 2.84 (2H, q), 2.12 (3H, s), 1.28 (3H, t) |
| 2-97 | (500 MHz/CDCl₃) 12.98 (1H, s), 9.32 (1H, d), 7.75 (2H, s), 6.78 (1H, d), 2.87 (2H, q), 1.28(3H, t) |
| 2-98 | (500 MHz/CDCl₃) 12.94 (1H, s), 9.33 (1H, s), 7.50 (2H, s), 6.77(1H, d), 2.85 (2H, q), 1.28 (3H, t) |
| 2-99 | (500 MHz/CDCl₃) 13.00 (1H, s), 9.34 (1H, d), 7.97 (2H, s), 6.79 (1H, d), 2.88 (2H, q), 1.30 (3H, t) |
| 2-100 | (500 MHz/CDCl₃) 12.92 (1H, s), 9.40 (1H, d), 7.47 (2H, s), 6.78 (1H, d), 2.87 (2H, q), 2.16 (6H, s), 1.26 (3H, t) |
| 2-101 | (500 MHz/CDCl₃) 12.99 (1H, s), 9.32 (1H, d), 7.39 (2H, d), 6.79 (1H,d), 2.85 (2H, q), 1.29 (3H, t) |
| 2-102 | (500 MHz/CDCl₃) 12.98 (1H, s), 9.34 (1H, d), 7.39 (2H, s), 6.78 (1H, d), 2.87 (2H, q), 1.30 (3H, t) |
| 2-103 | (500 MHz/CDCl₃) 12.98 (1H, s), 9.34 (1H, d), 8.11 (1H, d), 7.81 (1H, d), 6.78 (1H, d), 2.94-2.81 (2H, m), 1.30 (3H, t) |
| 2-104 | (500 MHz/CDCl₃) 12.98 (1H, s), 9.31 (1H, d), 7.66 (1H, s), 7.48 (1H, d), 6.77 (1H, d), 2.88-2.83 (2H, m), 1.28 (3H, t) |
| 2-105 | (500 MHz/CDCl₃) 12.98 (1H, s), 9.33 (1H, d), 7.91 (1H, s), 7.79 (1H, d), 6.78 (1H, d), 2.87 (2H, qd), 1.29 (3H, t) |
| 2-106 | (500 MHz/CDCl₃) 12.98 (1H, s), 9.34 (1H, d), 8.15 (1H, s), 7.97 (1H, s), 6.78 (1H, d), 2.92-2.83 (2H, m), 1.30 (3H, t) |
| 2-107 | (500 MHz/CDCl₃) 12.90 (1H, s), 9.37 (1H, d), 7.41 (1H, d), 7.27 (1H, d), 6.76 (1H, d), 2.86 (2H, q), 2.15 (3H, s), 1.27 (3H, t) |
| 2-108 | (500 MHz/CDCl₃) 12.90 (1H, s), 9.38 (1H, d), 7.01 (2H, s), 6.75 (1H, d), 3.84 (3H, s), 2.86 (2H, q), 1.28 (3H, t) |
| 2-109 | (500 MHz/CDCl₃) 12.97 (1H, s), 9.35 (1H, d), 7.58 (2H, s), 6.78 (1H, d), 2.87 (2H, q), 1.30 (3H, t) |
| 2-110 | (500 MHz/CDCl₃) 12.91 (1H, s), 9.38 (1H, d), 7.33 (2H, s), 6.75 (1H, d), 2.97-2.90 (1H, m), 2.86 (2H, q), 1.30-1.26 (9H, m) |
| 2-111 | (500 MHz/CDCl₃) 12.94 (1H, s), 9.36 (1H, d), 7.67 (1H, s), 7.54 (1H, s), 6.77 (1H, d), 2.87 (2H, q), 2.24 (3H, s), 1.28 (3H, t) |
| 2-112 | (500 MHz/CDCl₃) 12.94 (1H, s), 9.36 (1H, d), 7.83 (1H, s), 7.58 (1H, s), 6.77 (1H, d), 2.92-2.81 (2H, q), 2.25 (3H, s), 1.30 (3H, t) |
| 2-113 | (500 MHz/CDCl₃) 12.91 (1H, s), 9.38 (1H, d), 7.47 (2H, s), 6.76 (1H, d), 2.88-2.83 (2H, m), 1.34 (9H, s), 1.29 (3H, t) |
| 2-114 | (500 MHz/CDCl₃) 12.94 (1H, s), 9.35 (1H, d), 7.84 (2H, s), 6.76 (1H, d), 6.35 (2H, dd), 5.83 (2H, d), 5.38 (2H, d), 2.86 (2H, q), 1.27 (3H, t) |
| 2-115 | (500 MHz/CDCl₃) 12.91 (1H,s), 9.39 (1H, d), 7.50 (2H, s), 6.77 (1H, d), 2.86 (2H, q), 2.37-2.50 (4H, m), 1.28 (3H, t), 1.18 (6H, t) |
| 2-116 | (500 MHz/CDCl₃) 12.94 (1H, s), 9.33 (1H, d), 7.56 (1H, t), 7.28 (1H, dd), 6.77 (1H, d), 2.91-2.79 (2H, m), 1.28 (3H, t) |
| 2-117 | (500 MHz/CDCl₃) 12.94 (1H, s), 9.35 (1H, d), 7.85 (2H, s), 6.77 (1H, d), 2.86 (2H, q), 1.29 (3H, t) |
| 2-118 | (500 MHz/CDCl₃) 12.89 (1H, s), 9.38 (1H, d), 7.31 (1H, dd), 7.04 (1H, dd), 6.76 (1H, d), 2.91-2.81 (2H, m), 2.17 (3H, s), 1.28 (3H, t) |
| 2-119 | (500 MHz/CDCl₃) 12.98 (1H, s), 9.31 (1H, d), 7.82 (1H, s), 7.51 (1H, dd), 6.78 (1H, d), 2.92-2.80 (2H, m), 1.29 (3H, t) |
| 2-120 | (500 MHz/CDCl₃) 12.92 (1H, s), 9.36 (1H, d), 7.29 (1H, s), 7.14 (1H, s), 6.77 (1H, d), 2.86 (2H, q), 2.19 (3H, s), 1.28 (3H, t) |
| 2-121 | (500 MHz/CDCl₃) 12.92 (1H, s), 9.37 (1H, d), 7.45 (1H, s), 7.18 (1H, s), 6.77 (1H, d), 2.90-2.81 (2H, m), 2.20 (3H, s), 1.29 (3H, t) |
| 2-122 | (500 MHz/CDCl₃) 12.96 (1H, s), 9.35 (1H, s), 7.28 (2H, s), 6.78 (1H, d), 2.86 (2H, q), 1.29 (3H, t) |
| 2-123 | (500 MHz/CDCl₃) 12.87 (1H, s), 9.40 (1H, dd), 7.04 (2H, s), 6.77 (1H, d), 2.86 (2H, q), 2.10 (6H, s), 1.28 (3H, t) |
| 2-126 | (500 MHz/CDCl₃) 12.94 (1H, s), 9.34 (1H, d), 6.87 (2H, dd), 6.77 (1H, d), 2.84 (2H, q), 1.29 (3H, t) |
| 2-127 | (300 MHz/CDCl₃) 12.93 (1H, brs), 9.36-9.35 (1H, m), 7.44 (1H, d), 7.38 (1H, d), 6.78 (1H, dd), 2.90-2.82 (2H, m), 2.26 (3H, s), 1.29 (3H, t) |
| 2-128 | (500 MHz/CDCl₃) 12.85 (1H, s), 9.41 (1H, d), 6.90 (2H, d), 6.77 (1H, d), 2.86 (2H, q), 2.09 (6H, s), 1.28 (3H, t) |
| 2-129 | (300 MHz/CDCl₃) 12.92 (1H, s), 9.38(1H, dd), 7.65 (1H, d), 7.28 (1H, d), 6.78 (1H, d), 2.88 (2H, q), 1.76-1.67 (1H, m), 1.29 (3H, t), 0.94-0.68 (4H, m) |
| 2-130 | (300 MHz/CDCl₃) 12.96 (1H, s), 9.35 (1H, dd), 7.84 (1H, s), 7.74 (1H, d), 6.77 (1H, d), 6.43 (1H, dd), 5.86 (1H, d), 5.43 (1H, d), 2.88 (2H, q), 1.28 (3H, t) |
| 2-131 | (300 MHz/CDCl₃) 12.94 (1H, s), 9.36 (1H, dd), 7.68 (1H, d), 7.57 (1H, d), 6.77 (1H, d), 2.87 (2H, q), 2.54 (2H, qd), 1.28 (3H, t), 1.21 (3H, t) |
| 2-133 | (500 MHz/CDCl₃) 9.36 (1H, s), 7.56 (1H, d), 7.43 (1H, d), 6.76 (1H, d), 2.85 (2H, q), 2.14 (3H, s), 1.27 (3H, t) |
| 2-136 | (500 MHz/CDCl₃) 12.95 (1H, s), 9.36 (1H, d), 7.65 (1H, s), 7.50 (1H, s), 6.78 (1H, s), 2.87 (2H, q), 2.25 (3H, s), 1.28 (3H, t) |
| 2-137 | (500 MHz/CDCl₃) 13.02 (1H, s), 9.29 (1H, s), 7.62 (1H, d), 6.79 (1H, d), 2.86 (2H, qd), 1.28 (3H, t) |
| 2-138 | (500 MHz/CDCl₃) 13.05 (1H, brs), 9.27 (1H, d), 6.79 (1H, d), 2.86 (2H, q), 1.27 (3H, t) |
| 2-139 | (500 MHz/CDCl₃) 13.06 (1H, s), 9.27 (1H, s), 6.80 (1H, d), 2.86 (2H, q), 1.28 (3H, t) |
| 2-140 | (500 MHz/CDCl₃) 12.51 (1H, s), 9.13 (1H, t), 7.44 (2H, s), 6.06 (1H, q), 2.83 (2H, q), 2.15 (6H, s), 1.26 (3H, t) |
| 2-143 | (500 MHz/CDCl₃) 12.91 (1H, brs), 9.22 (1H, d), 7.44 (2H, s), 6.42 (1H, s), 2.84 (2H, q), 2.15 (6H, s), 1.27 (3H, t) |
| 2-144 | (500 MHz/CDCl₃) 12.97 (1H, s), 9.18 (1H, d), 7.45 (2H, s), 6.51 (1H, d), 2.85 (2H, q), 2.15 (6H, s), 1.27 (3H, t) |
| 2-147 | (300 MHz/CDCl₃) 14.32 (1H, brs), 9.11-9.19 (1H, m), 7.39-7.49 (2H, m), 6.32-6.41 (1H, m), 2.83 (2H, q), 2.41 (3H, s), 2.16 (6H, s), 1.27 (3H, t) |
| 2-149 | (300 MHz/CDCl₃) 14.33 (1H, brs), 9.06 (1H, d), 7.37 (2H, s), 6.19 (1H, d), 3.41-3.26 (1H, m), 2.33 (3H, s), 2.07 (6H, s), 1.20 (6H, d) |
| 2-151 | (300 MHz/CDCl₃) 13.37 (1H, brs), 9.06 (1H, d), 7.45 (2H, s), 5.91 (1H, s), 4.01 (3H, s), 2.81 (2H, q), 2.15 (6H, s), 1.27 (3H, t) |
| 2-153 | (300 MHz/CDCl₃) 12.84 (1H, brs), 9.44 (1H, d), 7.48 (2H, s), 6.98 (1H, d), 3.01 (3H, s), 2.87 (2H, q), 2.15-2.22 (6H, m), 1.30 (3H, t) |
| 2-154 | (300 MHz/CDCl₃) 12.59 (1H, brs), 9.45 (1H, d), 7.49 (2H, s), 7.04 (1H, d), 3.26 (3H, s), 2.89 (2H, q), 2.19 (6H, s), 1.31 (3H, t) |
| 2-167 | (500 MHz/CDCl₃) 13.27 (1H, brs), 9.03 (1H, d), 7.75 (2H, s), 6.65 (1H, d), 2.85 (2H, q), 1.28 (3H, t) |
| 2-168 | (500 MHz/CDCl₃) 13.28 (1H, brs), 9.03 (1H, d), 7.75 (2H, s), 6.65 (1H, d), 2.85 (2H, q), 1.28 (3H, t) |
| 2-169 | (300 MHz/CDCl₃) 12.61 (1H, brs), 9.07 (1H, t), 7.20-7.78 (2H, m), 6.08 (1H, q), 2.83 (2H, q), 1.27 (3H, t) |
| 2-171 | (300 MHz/CDCl₃) 14.39 (1H, brs), 9.12 (1H, d), 7.28 (1H, dd), 7.13 (1H, dd), 6.27 (1H, d), 2.83 (2H, q), 2.40 (3H, s), 2.19 (3H, s), 1.28 (3H, t) |
| 2-172 | (300 MHz/CDCl₃) 14.40 (1H, brs), 9.10 (1H, dd), 7.66 (1H, dd), 7.52 (1H, dd), 6.27 (1H, t), 2.85 (2H, q), 2.40 (3H, s), 2.23 (3H, s), 1.28 (3H, t) |
| 2-173 | (300 MHz/CDCl₃) 12.96 (1H, brs), 9.20 (1H, d), 7.67 (1H, dd), 7.53 (1H, dd), 6.42 (1H, d), 2.84 (2H, q), 2.23 (3H, s), 1.27 (3H, t) |
| 2-175 | (300 MHz/CDCl₃) 12.95 (1H, brs), 9.21 (1H, d), 7.29 (1H, dd), 7.14-7.13 (1H, m), 6.42 (1H, d), 2.85 (2H, q), 2.19 (3H, s), 1.28 (3H, t) |
| 2-177 | (300 MHz/CDCl₃) 13.02 (1H, brs), 9.15 (1H, d), 7.67 (1H, dd), 7.53 (1H, dd), 6.52 (1H, d), 2.86 (2H, q), 2.23 (3H, s), 1.28 (3H, t) |
| 2-178 | (300 MHz/CDCl₃) 13.01 (1H, brs), 9.16 (1H, d), 7.29 (1H, dd), 7.14 (1H, dd), 6.51 (1H, d), 2.84 (2H, q), 2.19 (3H, s), 1.28 (3H, t) |
| 2-179 | (300 MHz/CDCl₃) 12.54 (1H, s), 9.11 (1H, t), 7.25-7.35 (1H, m), 7.10-7.18 (1H, m), 6.07 (1H, q), 2.83 (2H, q), 2.19 (3H, s), 1.27 (3H, t) |
| 2-180 | (500 MHz/CDCl₃) 14.25 (1H, brs), 9.22 (1H, d), 7.77 (1H, d), 7.74 (2H, s), 6.51 (1H, t), 2.85 (2H, q), 1.28 (3H, t) |
| 2-181 | (500 MHz/CDCl₃) 14.14 (1H, brs), 9.27 (1H, d), 7.77 (1H, d), 7.61 (2H, t), 7.43 (1H, d), 6.50 (1H, t), 2.84 (2H, q), 2.24 (3H, s), 1.28 (3H, t) |
| 2-182 | (500 MHz/CDCl₃) 14.19 (1H, brs), 9.25 (1H, d), 7.77 (1H, d), 7.66 (1H, s), 7.53 (1H, d), 6.50 (1H, t), 2.85 (2H, q), 2.24 (3H, s), 1.27 (3H, t) |
| 2-183 | (500 MHz/CDCl₃) 14.18 (1H, brs), 9.26 (1H, d), 7.77 (1H, d), 7.28 (1H, d), 7.13 (1H, s), 6.50 (1H, t), 2.84 (2H, q), 2.19 (3H, s), 1.27 (3H, t) |
| 2-184 | (500 MHz/CDCl₃) 14.13 (1H, brs), 9.29 (1H, d), 7.78 (1H, d), 7.45 (2H, s), 6.50 (1H, t), 2.85 (2H, q), 2.16 (6H, s), 1.27 (3H, t) |
| 2-185 | (300 MHz/CDCl₃) 14.10 (1H, brs), 9.30 (1H, dd), 7.78 (1H, dd), 7.04 (2H, s), 6.50 (1H, dd), 2.84 (2H, q), 2.11 (6H, s), 1.28 (3H, t) |
| 2-186 | (500 MHz/CDCl₃) 9.23 (1H, d), 7.03 (2H, s), 6.42 (1H, d), 2.84 (2H, q), 2.01 (6H, s), 1.26 (3H, t) |
| 2-189 | (300 MHz/CDCl₃) 12.91 (1H, brs), 9.23 (1H, d), 7.48 (2H, s), 6.43 (1H, d), 2.84 (2H, q), 2.12 (6H, s), 1.27 (3H, t) |
| 2-190 | (300 MHz/CDCl₃) 12.96 (1H, brs), 9.22 (1H, d), 7.60 (2H, d), 6.44 (1H, d), 2.85 (2H, q), 2.20 (6H, s), 1.28 (3H, t) |
| 2-191 | (300 MHz/CDCl₃) 12.99 (1H, brs), 9.19 (1H, d), 7.86 (2H, s), 6.45 (1H, d), 2.85 (2H, q), 2.23 (6H, s), 1.28 (3H, t) |
| 2-192 | (300 MHz/CDCl₃) 13.19 (1H, s), 9.10 (1H, d), 7.45 (2H, s), 6.65 (1H, d), 2.84 (2H, q), 2.15 (6H, s), 1.28 (3H, t) |
| 2-195 | (500 MHz/CDCl₃) 12.96 (1H, s), 9.19 (1H, d), 7.03 (2H, s), 6.50 (1H, d), 2.84 (2H, q), 2.09 (6H, s), 1.27 (3H, t) |
| 2-197 | (500 MHz/CDCl₃) 12.86 (1H, s), 9.41 (1H, d), 7.19 (2H, s), 6.77 (1H, d), 2.85 (2H, q), 2.07 (6H, s), 1.28 (3H, t) |
| 2-198 | (500 MHz/CDCl₃) 12.88 (1H, s), 9.24 (1H, d), 7.18 (2H, s), 6.42 (1H, d), 2.83 (2H, q), 2.07 (6H, s), 1.27 (3H, t) |
| 2-206 | (300 MHz/CDCl₃) 12.96 (1H, brs), 9.22 (1H, d), 7.79 (2H, s), 6.45 (1H, d), 3.08 (3H, s), 2.85 (2H, q), 2.20 (6H, s), 1.29 (3H, t) |
| 2-207 | (300 MHz/CDCl₃) 12.95 (1H, brs), 9.22 (1H, d), 7.75 (2H, s), 6.44 (1H, d), 3.15 (2H, q), 2.85 (2H, q), 2.20 (6H, s), 1.36 (3H, t), 1.28 (3H, t) |
| 2-208 | (300 MHz/CDCl₃) 12.89 (1H, brs), 9.29 (1H, d), 7.15 (2H, s), 6.44 (1H, d), 3.37 (2H, q), 2.87 (2H, q), 2.12 (6H, s), 1.31 (3H, t) |
| 2-209 | (300 MHz/CDCl₃) 12.89 (1H, brs), 9.26 (1H, d), 6.96 (2H, s), 6.53 (1H, t), 6.43 (1H, d), 2.85 (2H, q), 2.11 (6H, s), 1.28 (3H, t) |
| 2-211 | (300 MHz/CDCl₃) 12.82 (1H, brs), 9.17 (1H, d), 7.26 (2H, s), 6.55 (1H, t), 6.35 (1H, d), 2.76 (2H, q), 2.06 (6H, s), 1.20 (3H, t) |
| 2-215 | (300 MHz/CDCl₃) 12.83 (1H, brs), 9.22 (1H, d), 7.30 (2H, s), 6.40 (1H, d), 3.05 (1H, s), 2.81 (2H, q), 2.05 (6H, s), 1.25 (3H, t) |
| 2-218 | (300 MHz/CDCl₃) 12.90 (1H, s), 9.26 (1H, d), 7.36 (2H, s), 6.44 (1H, d), 2.85 (2H, q), 2.08 (6H, s), 1.28 (3H, t) |
| 2-222 | (300 MHz/CDCl₃) 12.91 (1H, s), 9.22 (1H, d), 7.57-7.66 (2H, m), 7.38-7.46 (1H, m), 6.43 (1H, d), 2.84 (2H, q), 2.24 (3H, s), 1.28 (3H, t) |
| 2-224 | (300 MHz/CDCl₃) 12.87 (1H, brs), 9.14 (1H, d), 7.70 (1H, t), 7.44 (1H, s), 6.34-6.23 (2H, m), 5.72 (1H, dd), 5.28(1H, d), 2.76 (2H, q), 2.08 (3H, s), 1.19 (3H, t) |
| 2-234 | (500 MHz/CDCl₃) 13.55 (1H, s), 9.21 (1H, d), 7.66 (1H, d), 7.46 (2H, s), 2.84 (2H, q), 2.16 (6H, s), 1.27 (3H, t) |
| 2-235 | (500 MHz/CDCl₃) 13.42 (1H, s), 9.30 (1H, s), 7.72 (1H, s), 7.46 (2H, s), 2.84 (2H, q), 2.15 (6H, s), 1.27 (3H, t) |
| 2-236 | (300 MHz/CDCl₃) 13.39 (1H, brs), 9.30 (1H, d), 7.72 (1H, d), 7.46 (2H, s), 2.44 (3H, s), 2.16 (6H, s) |
| 2-237 | (500 MHz/CDCl₃) 13.50 (1H, brs), 9.30 (1H, s), 7.71 (1H, s), 7.45 (2H, s), 3.41 (1H, sep), 2.13 (6H, s), 1.27 (6H, d) |
| 2-238 | (300 MHz/CDCl₃) 13.40 (1H, s), 9.34 (1H, s), 7.74 (1H, s), 7.45 (2H, s), 2.84 (2H, q), 2.15 (6H, s), 1.27 (3H, t) |
| 2-239 | (300 MHz/CDCl₃) 13.37 (1H, brs), 9.34 (1H, s), 7.75 (1H, s), 7.45 (2H, s), 2.44 (3H, s), 2.16 (6H, s) |
| 2-240 | (300 MHz/CDCl₃) 13.49 (1H, brs), 9.35 (1H, s), 7.74 (1H, s), 7.45 (2H, s), 3.47-3.37 (1H, m), 2.14 (6H, s), 1.27 (6H, d) |
| 2-241 | (300 MHz/CDCl₃) 14.16 (1H, brs), 9.00-9.75 (1H, m), 7.57 (1H, s), 7.45 (2H, s), 2.83 (2H, q), 2.14-2.21 (9H, m), 1.27 (3H, t) |
| 2-243 | (300 MHz/CDCl₃) 14.21 (1H, brs), 9.06 (1H, s), 7.59 (1H, s), 7.47 (2H, s), 3.51-3.37 (1H, m), 2.18 (3H, s), 2.17 (6H, s), 1.29 (6H, d) |
| 2-262 | (500 MHz/CDCl₃) 13.46 (1H, brs), 9.25 (1H, s), 8.01 (1H, s), 7.74 (1H, d), 7.71 (1H, s), 7.55 (1H, d), 2.83 (2H, br), 1.28 (3H, t) |
| 2-263 | (500 MHz/CDCl₃) 13.64 (1H, s), 9.13 (1H, d), 7.74 (2H, s), 7.65 (1H, d), 2.84 (2H, q), 1.26 (3H, t) |
| 2-266 | (500 MHz/CDCl₃) 13.65 (1H, s), 9.15 (1H, d), 7.95 (1H, d), 7.66 (1H, d), 2.85 (2H, q), 1.29 (3H, t) |
| 2-270 | (500 MHz/CDCl₃) 13.60 (1H, s), 9.16 (1H, s), 8.01 (1H, s), 7.75 (1H, d), 7.65 (1H, d), 7.55 (1H, d), 2.83 (2H, br), 1.28 (3H, t) |
| 2-271 | (500 MHz/CDCl₃) 13.41 (1H, s), 9.29 (1H, s), 7.77 (1H, s), 7.63 (1H, s), 7.61 (1H, d), 7.41 (1H, d), 2.83 (2H, q), 2.24 (3H, s), 1.28 (3H, t) |
| 2-274 | (500 MHz/CDCl₃) 13.55 (1H, s), 9.20 (1H, d), 7.65 (1H, d), 7.63 (1H, s), 7.61 (1H, d), 7.42 (1H, d), 2.83 (2H, q), 2.24 (3H, s), 1.28 (3H, t) |
| 2-277 | (300 MHz/CDCl₃) 13.50 (1H, brs), 9.11 (1H, d), 7.70 (1H, s), 7.57 (1H, d), 7.45 (1H, s), 6.28 (1H, dd), 5.73 (1H, d), 5.28 (1H, d), 2.75 (2H, q), 2.08 (3H, s), 1.18 (3H, t) |
| 2-278 | (300 MHz/CDCl₃) 13.47 (1H, brs), 9.13 (1H, d), 7.58 (1H, d), 7.41 (1H, s), 7.39 (1H, s), 2.76 (2H, q), 2.47-2.28 (2H, m), 2.07 (3H, s), 1.19 (3H, t), 1.10 (3H, t) |
| 2-279 | (300 MHz/CDCl₃) 13.58 (1H, s), 9.19 (1H, dd), 7.65 (1H, dd), 7.29 (1H, dd), 7.14 (1H, dd), 2.84 (2H, q), 2.19 (3H, s), 1.27 (3H, t) |
| 2-281 | (500 MHz/CDCl₃) 13.57 (1H, s), 9.19 (1H, dd), 7.65 (1H, dd), 7.45 (1H, d), 7.18 (1H, dd), 2.84 (2H, qd), 2.20 (3H, s), 1.28 (3H, t) |
| 2-283 | (500 MHz/CDCl₃) 13.46 (1H, s), 9.26 (1H, s), 7.70 (1H, s), 7.42 (1H, s), 7.35 (1H, d), 2.86-2.78 (2H, m), 2.24 (3H, s), 1.26 (3H, t) |
| 2-284 | (300 MHz/CDCl₃) 13.59 (1H, s), 9.18 (1H, dd), 7.67-7.64 (2H, m), 7.54 (1H, d), 2.85 (2H, q), 2.24 (3H, t), 1.27 (3H, t) |
| 2-285 | (300 MHz/CDCl₃) 13.64 (1H, s), 9.14 (1H, dd), 7.82 (1H, s), 7.65 (1H, dd), 7.52 (1H, dd), 2.91-2.76 (2H, m), 1.28 (3H, t) |
| 2-286 | (300 MHz/CDCl₃) 13.51 (1H, brs), 9.23 (1H, d), 7.82 (1H, s), 7.71 (1H, d), 7.52 (1H, dd), 2.93-2.74 (2H, m), 1.28 (3H, t) |
| 2-287 | (300 MHz/CDCl₃) 13.45 (1H, brs), 9.27 (1H, d), 7.83 (1H, dd), 7.71 (1H, d), 7.57 (1H, dd), 2.93-2.78 (2H, m), 2.25 (3H, s), 1.28 (3H, t) |
| 2-288 | (300 MHz/CDCl₃) 13.52 (1H, brs), 9.19 (1H, dd), 7.65-7.59 (3H, m), 7.42 (1H, d), 2.84 (2H, q), 2.23 (3H, s), 1.27 (3H, t) |
| 2-291 | (300 MHz/CDCl₃) 13.58 (1H, brs), 9.18 (1H, dd), 7.83 (1H, dd), 7.65 (1H, dd), 7.57 (1H, dd), 2.88-2.80 (2H, m), 2.25 (3H, s), 1.28 (3H, t) |
| 2-292 | (300 MHz/CDCl₃) 13.46 (1H, brs), 9.27 (1H, d), 7.71 (1H, t), 7.67 (1H, t), 7.54 (1H, dd), 2.85 (2H, q), 2.24 (3H, s), 1.27 (3H, s) |
| 2-293 | (500 MHz/CDCl₃) 13.61 (1H, s), 9.16 (1H, d), 7.64 (1H, d), 7.26 (2H, d), 2.82 (2H, q), 1.28 (3H, t) |
| 2-294 | (500 MHz/CDCl₃) 13.61 (1H, s), 9.50 (1H, d), 7.64 (1H, d), 6.86 (2H, dd), 2.81 (2H, q), 1.27 (3H, t) |
| 2-295 | (300 MHz/CDCl₃) 13.45 (1H, brs), 9.17 (1H, d), 7.69 (2H, d), 7.65 (1H, d), 2.78 (2H, q), 1.21 (3H, t) |
| 2-296 | (300 MHz/CDCl₃) 13.59 (1H, brs), 9.17 (1H, dd), 7.65 (1H, dd), 7.43 (1H, d), 7.35 (1H, d), 2.87-2.79 (2H, m), 2.25 (3H, s), 1.27 (3H, t) |
| 2-297 | (300 MHz/CDCl₃) 13.54 (1H, brs), 9.31 (1H, d), 7.78 (2H, d), 7.77 (1H, d), 2.87 (2H, q), 1.30 (3H, t) |
| 2-298 | (300 MHz/CDCl₃) 13.57 (1H, brs), 9.34 (1H, d), 7.81 (1H, d), 7.78 (2H, d), 2.87 (2H, q), 1.30 (3H, t) |
| 2-299 | (500 MHz/CDCl₃) 13.52 (1H, s), 9.25 (1H, s), 7.96 (2H, s), 7.72 (1H, s), 2.85 (2H, q), 1.29 (3H, t) |
| 2-300 | (300 MHz/CDCl₃) 13.45 (1H, brs), 9.29 (1H, d), 7.71 (1H, d), 7.29 (1H, t), 7.14 (1H, dd), 2.84 (2H, q), 2.19 (3H, s), 1.27 (3H, t) |
| 2-301 | (300 MHz/CDCl₃) 13.42 (1H, brs), 9.26 (1H, d), 7.71 (1H, d), 7.43 (1H, s), 7.36 (1H, d), 2.88-2.79 (2H, m), 2.25 (3H, s), 1.27 (3H, t) |
| 2-302 | (300 MHz/CDCl₃) 14.22 (1H, brs), 9.00 (1H, t), 7.66 (1H, dd), 7.57 (1H, s), 7.53 (1H, dd), 2.84 (2H, q), 2.23 (3H, s), 2.16 (3H, s), 1.28 (3H, t) |
| 2-303 | (300 MHz/CDCl₃) 14.20 (1H, brs), 9.02 (1H, t), 7.56 (1H, s), 7.28 (1H, dd), 7.13 (1H, dd), 2.84 (2H, q), 2.19 (3H, s), 2.16 (3H, s), 1.27 (3H, t) |
| 2-304 | (500 MHz/CDCl₃) 13.50 (1H, s), 9.23 (1H, d), 7.64 (1H, d), 6.89 (2H, d), 2.83 (2H, q), 2.08 (6H, s), 1.27 (3H, t) |
| 2-309 | (300 MHz/CDCl₃) 13.45 (1H, brs), 9.31 (1H, d), 7.74 (1H, d), 7.67 (1H, dd), 7.54 (1H, dd), 2.84 (2H, q), 2.23 (3H, s), 1.27 (3H, t) |
| 2-310 | (300 MHz/CDCl₃) 13.43 (1H, brs), 9.33 (1H, d), 7.74 (1H, d), 7.29 (1H, dd), 7.14 (1H, dd), 2.85 (2H, q), 2.19 (3H, s), 1.27 (3H, t) |
| 2-311 | (300 MHz/CDCl₃) 13.64 (1H, brs), 9.15 (1H, dd), 7.91 (1H, dd), 7.79 (1H, dd), 7.66 (1H, dd), 2.88-2.80 (2H, m), 1.28 (3H, t) |
| 2-313 | (500 MHz/CDCl₃) 13.33 (1H, s), 9.65 (1H, s), 7.98 (1H, s), 7.67 (1H, s), 7.54 (1H, s), 2.87 (2H, q), 2.24 (3H, s), 1.28 (3H, t) |
| 2-314 | (500 MHz/CDCl₃) 13.30 (1H, brs), 9.67 (1H, s), 7.97 (1H, s), 7.29 (1H, d), 7.14 (1H, s), 2.86 (2H, q), 2.18 (3H, s), 1.27 (3H, t) |
| 2-317 | (300 MHz/CDCl₃) 13.61 (1H, s), 9.16 (1H, dd), 7.84 (1H, s), 7.73 (1H, d), 7.65 (1H, dd), 6.41 (1H, dd), 5.86 (1H, d), 5.45 (1H, d), 2.84 (2H, q), 1.26 (3H, t) |
| 2-318 | (300 MHz/CDCl₃) 13.58 (1H, brs), 9.17 (1H, dd), 7.68-7.64 (2H, m), 7.56 (1H, d), 2.85 (2H, q), 2.60-2.41 (2H, m), 1.26 (3H, t), 1.20 (3H, t) |
| 2-319 | (500 MHz/CDCl₃) 9.21 (1H, d), 7.64 (1H, d), 7.04 (2H, s), 2.83 (2H, q), 2.10 (6H, s), 1.26 (3H, t) |
| 2-323 | (500 MHz/CDCl₃) 13.66 (1H, s), 9.13 (1H, d), 7.65 (1H, d), 7.38 (2H, d), 2.82 (2H, q), 1.27 (3H, t) |
| 2-324 | (500 MHz/CDCl₃) 13.42 (1H, brs), 9.37 (1H, s), 7.78 (1H, s), 7.45 (2H, s), 2.84 (2H, q), 2.14 (6H, s), 1.26 (3H, t) |
| 2-325 | (500 MHz/CDCl₃) 13.38 (1H, s), 9.36 (1H, s), 7.73 (1H, s), 7.04 (2H, s), 2.84 (2H, q), 2.10 (6H, s), 1.27 (3H, t) |
| 2-326 | (300 MHz/CDCl₃) 13.34 (1H, brs), 9.32 (1H, d), 7.71 (1H, d), 7.05 (2H, s), 2.82 (2H, q), 2.11 (6H, s), 1.28 (3H, t) |
| 2-328 | (500 MHz/CDCl₃) 13.51 (1H, s), 9.22 (1H, d), 7.64 (1H, d), 7.05 (2H, s), 2.82 (2H, q), 2.07 (6H, s), 1.26 (3H, t) |
| 2-329 | (500 MHz/CDCl₃) 13.51 (1H, s), 9.21 (1H, d), 7.64 (1H, d), 7.34 (2H, s), 2.82 (2H, q), 2.06 (6H, s), 1.25 (3H, t) |
| 2-335 | (500 MHz/CDCl₃) 12.51 (1H, s), 9.40 (1H, s), 7.77 (2H, s), 2.86 (2H, q), 1.29 (3H, t) |
| 2-336 | (500 MHz/CDCl₃) 13.24 (1H, s), 9.96 (1H, s), 7.77 (2H, s), 2.86 (2H, q), 2.71 (3H, q), 1.29 (3H, t) |
| 2-338 | (500 MHz/CDCl₃) 12.51 (1H, s), 9.37 (1H, s), 7.77 (2H, s), 2.86 (2H, q), 1.29 (3H, t) |
| 2-340 | (500 MHz/CDCl₃) 12.18 (1H, brs), 10.05 (1H, s), 7.78 (2H, s), 2.88 (2H, q), 1.29 (3H, t) |
| 2-342 | (500 MHz/CDCl₃) 12.39 (1H, s), 9.40 (1H, s), 7.63 (1H, s), 7.61 (1H, d), 7.41 (1H, d), 2.84 (2H, q), 2.23 (3H, s), 1.28 (3H, t) |
| 2-345 | (500 MHz/CDCl₃) 12.43 (1H, s), 9.36 (1H, s), 8.00 (1H, s), 7.74 (1H, d), 7.54 (1H, d), 2.83 (2H, br), 1.27 (3H, t) |
| 2-347 | (500 MHz/CDCl₃) 12.43 (1H, s), 9.36 (1H, s), 7.56 (1H, d), 7.50 (1H, d), 2.84 (2H, brd), 1.28 (3H, t) |
| 2-348 | (300 MHz/CDCl₃) 12.43 (1H, s), 9.37 (1H, d), 7.58 (1H, d), 7.56 (1H, d), 2.85 (2H, q), 1.29 (3H, t) |
| 2-349 | (500 MHz/CDCl₃) 12.41 (1H, s), 9.38 (1H, s), 7.50 (1H, d), 7.44-7.41 (2H, m), 2.84 (2H, q), 1.28 (3H, t) |
| 2-352 | (500 MHz/CDCl₃) 12.91 (1H, s), 9.36 (1H, d), 7.89 (2H, d), 6.78 (1H, d), 2.88 (2H, q), 1.32 (3H, t) |
| 2-353 | (500 MHz/CDCl₃) 12.45 (1H, s), 9.36 (1H, s), 7.56 (1H, s), 7.28-7.26 (1H, m), 2.90-2.78 (2H, m), 1.27 (3H, t) |
| 2-356 | (500 MHz/CDCl₃) 12.38 (1H, s), 9.39 (1H, s), 7.48 (1H, d), 7.13 (1H, d), 2.83 (2H, q), 2.11 (3H, s), 1.27 (3H, t) |
| 2-357 | (500 MHz/CDCl₃) 12.38 (1H, s), 9.38 (1H, s), 7.54 (1H, s), 7.46 (1H, s), 2.83 (2H, q), 2.09 (3H, t), 1.27 (3H, t) |
| 2-358 | (500 MHz/CDCl₃) 12.46 (1H, s), 9.38 (1H, s), 7.85 (2H, s), 2.86 (2H, q), 1.28 (3H, t) |
| 2-359 | (500 MHz/CDCl₃) 12.46 (1H, brs), 9.40 (1H, s), 7.46 (2H, t), 2.86 (2H, q), 1.29 (3H, t) |
| 2-360 | (500 MHz/CDCl₃) 12.39 (1H, s), 9.40 (1H, s), 7.15 (1H, q), 7.07-7.04 (1H, m), 2.84 (2H, q), 2.09 (3H, d), 1.27 (3H, t) |
| 2-361 | (500 MHz/CDCl₃) 12.38 (1H, s), 9.38 (1H, s), 7.62 (1H, s), 7.39 (1H, s), 2.84 (2H, q), 2.11 (3H, s), 1.27 (3H, t) |
| 2-362 | (500 MHz/CDCl₃) 12.40 (1H, s), 9.39 (1H, s), 7.46 (1H, dd), 7.03 (1H, dd), 2.84 (2H, q), 2.14 (3H, s), 1.27 (3H, t) |
| 2-363 | (500 MHz/CDCl₃) 12.38 (1H, s), 9.38 (1H, s), 7.56 (1H, d), 7.09 (1H, s), 2.83 (2H, q), 2.10 (3H, t), 1.27 (3H, t) |
| 2-364 | (500 MHz/CDCl₃) 12.44 (1H, s), 9.35 (1H, s), 7.30-7.27 (2H, m), 2.83 (2H, q), 1.26 (3H, t) |
| 2-365 | (500 MHz/CDCl₃) 12.38 (1H, s), 9.39 (1H, s), 7.17-7.11 (2H, m), 2.83 (2H, q), 2.10 (3H, s), 1.27 (3H, t) |
| 2-366 | (500 MHz/CDCl₃) 12.40 (1H, s), 9.38 (1H, s), 7.75 (1H, d), 7.47 (1H, dd), 7.35 (1H, d), 2.84-2.82 (2H, brt), 1.27 (3H, t) |
| 2-367 | (500 MHz/CDCl₃) 12.42 (1H, s), 9.40 (1H, s), 7.72 (1H, d), 7.47 (1H, s), 2.91-2.79 (2H, m), 2.14 (3H, s), 1.27 (3H, t) |
| 2-368 | (500 MHz/CDCl₃) 12.46 (1H, brs), 9.39 (1H, s), 7.71 (2H, s), 2.86 (2H, q), 1.28 (3H, t) |
| 2-369 | (500 MHz/CDCl₃) 12.42 (1H, s), 9.42 (1H, s), 7.31 (1H, dd), 7.04 (1H, dd), 2.91-2.79 (2H, m), 2.16 (3H, s), 1.28 (3H, t) |
| 2-370 | (500 MHz/CDCl₃) 12.41 (1H, s), 9.40 (1H, s), 7.56 (1H, d), 7.43 (1H, d), 2.84 (2H, q), 2.13 (3H, s), 1.26 (3H, t) |
| 2-371 | (500 MHz/CDCl₃) 12.39 (1H, s), 9.38 (1H, s), 7.53 (1H, t), 7.01 (1H, d), 2.83 (2H, q), 2.10 (3H, d), 1.27 (3H, t) |
| 2-372 | (500 MHz/CDCl₃) 12.40 (1H, s), 9.38 (1H, s), 7.64 (1H, d), 7.09 (1H, d), 2.84 (2H, qd), 2.22 (3H, s), 1.27 (3H, t) |
| 2-373 | (500 MHz/CDCl₃) 12.42 (1H, s), 9.43 (1H, s), 7.47 (2H, s), 2.86 (2H, q), 2.15 (6H, s), 1.28 (3H, t) |
| 2-377 | (500 MHz/CDCl₃) 12.46 (1H, s), 9.39 (1H, s), 7.67 (1H, s), 7.54 (1H, s), 2.86 (2H, q), 2.23 (3H, s), 1.27 (3H, t) |
| 2-378 | (500 MHz/CDCl₃) 12.46 (1H, s), 9.39 (1H, s), 7.85 (1H, s), 7.58 (1H, s), 2.91-2.80 (2H, m), 2.24 (3H, s), 1.28 (3H, t) |
| 2-379 | (500 MHz/CDCl₃) 12.34 (1H, brs), 9.38 (1H, s), 7.67 (2H, d), 7.48 (1H, d), 5.04 (1H, t), 4.80 (1H, s), 2.83 (2H, q), 1.99 (3H, s), 1.26 (3H, t) |
| 2-380 | (500 MHz/CDCl₃) 12.37 (1H, s), 9.42 (1H, s), 7.58 (1H, d), 7.41 (2H, d), 2.86 (2H, q), 1.76-1.71 (1H, m), 1.27 (3H, t), 0.88-0.80 (2H, m), 0.68 (2H, brs) |
| 2-381 | (500 MHz/CDCl₃) 12.39 (1H, s), 9.40 (1H, d), 7.71 (1H, s), 7.60 (1H, dd), 7.36 (1H, d), 2.87-2.71 (3H, m), 1.29-1.19 (9H, m) |
| 2-382 | (500 MHz/CDCl₃) 12.33 (1H, s), 9.41 (1H, s), 7.45 (1H, d), 7.38 (1H, d), 7.28 (1H, s), 3.88 (3H, s), 2.83 (2H, q), 1.28 (3H, t) |
| 2-383 | (500 MHz/CDCl₃) 12.39 (1H, brs), 9.39 (1H, s), 7.93 (1H, d), 7.77 (1H, t), 7.55 (1H, d), 3.18 (1H, s), 2.85 (2H, q), 1.28 (3H, t) |
| 2-384 | (500 MHz/CDCl₃) 12.47 (1H, s), 9.39 (1H, s), 7.28 (2H, s), 2.85 (2H, q), 1.29 (3H, t) |
| 2-385 | (500 MHz/CDCl₃) 12.44 (1H, s), 9.41 (1H, s), 7.45 (1H, d), 7.19 (1H, s), 2.90-2.81 (2H, m), 2.19 (3H, s), 1.28 (3H, t) |
| 2-386 | (500 MHz/CDCl₃) 12.41 (1H, s), 9.38 (1H, s), 7.48-7.45 (2H, m), 7.30 (1H, dd), 2.84 (2H, q), 1.28 (3H, t) |
| 2-387 | (500 MHz/CDCl₃) 12.39 (1H, s), 9.40 (1H, s), 7.63-7.60 (2H, m), 7.41 (1H, d), 2.85 (2H, q), 2.22 (3H, s), 1.27 (3H, t) |
| 2-388 | (300 MHz/CDCl₃) 12.44 (1H, brs), 9.41 (1H, d), 7.46 (1H, dd), 7.19 (1H, dd), 2.86 (2H, qd), 2.20 (3H, s), 1.29 (3H, t) |
| 2-389 | (500 MHz/CDCl₃) 12.44 (1H, s), 9.37 (1H, s), 6.87 (2H, dd), 2.83 (2H, q), 1.28 (3H, t) |
| 2-390 | (300 MHz/CDCl₃) 9.07 (1H, d), 7.68 (2H, d), 2.76 (2H, q), 2.30 (3H, s), 1.21 (3H, t) |
| 2-393 | (300 MHz/CDCl₃) 13.09 (1H, brs), 9.00 (1H, d), 7.77 (2H, d), 2.86 (2H, q), 2.12 (3H, d), 1.30 (3H, t) |
| 2-394 | (300 MHz/CDCl₃) 12.49 (1H, brs), 9.20 (1H, s), 7.69 (2H, d), 2.78 (2H, q), 1.21 (3H, t) |
| 2-395 | (300 MHz/CDCl₃) 12.37 (1H, s), 9.07 (1H, s), 7.58 (2H, d), 2.67 (2H, q), 1.10 (3H, t) |
| 2-396 | (300 MHz/CDCl₃) 12.51 (1H, s), 9.19 (1H, s), 7.69 (2H, d), 2.78 (2H, q), 1.20 (3H, t) |
| 2-398 | (300 MHz/CDCl₃) 12.45 (1H, brs), 9.41 (1H, d), 7.30 (1H, dd), 7.15 (1H, dd), 2.87 (2H, q), 2.19 (3H, s), 1.28 (3H, t) |
| 2-399 | (300 MHz/CDCl₃) 12.35 (1H, s), 9.62 (1H, d), 7.68 (1H, s), 7.54 (1H, s), 2.87 (2H, q), 2.23 (3H, s), 1.28 (3H, t) |
| 2-401 | (300 MHz/CDCl₃) 12.33 (1H, s), 9.64-9.70 (1H, m), 7.49 (2H, s), 2.88 (2H, q), 2.17 (6H, s), 1.30 (3H, t) |
| 2-403 | (300 MHz/CDCl₃) 12.34 (1H, s), 9.63 (1H, d), 7.30 (1H, dd), 7.15 (1H, dd), 2.86 (2H, q), 2.18 (3H, s), 1.28 (3H, t) |
| 2-404 | (300 MHz/CDCl₃) 12.50 (1H, s), 9.37 (1H, d), 7.92 (1H, dd), 7.80 (1H, dd), 2.891-2.83 (2H, m), 1.29 (3H, t) |
| 2-406 | (300 MHz/CDCl₃) 12.44 (1H, s), 9.41 (1H, d), 7.65 (1H, d), 7.28 (1H, d), 2.88 (2H, q), 1.74-1.65 (1H, m), 1.28 (3H, t), 0.94-0.70 (4H, m) |
| 2-407 | (300 MHz/CDCl₃) 12.47 (1H, brs), 9.38 (1H, dd), 7.85 (1H, d), 7.74 (1H, d), 6.40 (1H, dd), 5.86 (1H ,d), 5.45 (1H, d), 2.87 (2H, q), 1.28 (3H, t) |
| 2-408 | (300 MHz/CDCl₃) 12.45 (1H, brs), 9.39 (1H, d), 7.68 (1H, d), 7.57 (1H, d), 2.85 (2H, q), 2.51 (2H, qd), 1.28 (3H, t), 1.20 (3H, t) |
| 2-409 | (500 MHz/CDCl₃) 13.64 (1H, s), 9.20 (1H, s), 7.44 (2H, s), 2.83 (2H, q), 2.36 (3H, s), 2.14 (6H, s), 1.26 (3H, t) |
| 2-412 | (500 MHz/CDCl₃) 12.45 (1H, s), 9.32 (1H, s), 7.45 (2H, s), 2.84 (2H, q), 2.13 (6H, s), 1.26 (3H, t) |
| 2-413 | (300 MHz/CDCl₃) 12.44 (1H, s), 9.34 (1H, s), 7.04 (2H, s), 2.85 (2H, q), 2.09 (6H, s), 1.27 (3H, t) |
| 2-414 | (300 MHz/CDCl₃) 13.64 (1H, s), 9.22 (1H, d), 7.04 (2H, s), 2.84 (2H, q), 2.37 (3H, s), 2.10 (6H, s), 1.28 (3H, t) |
| 2-415 | (300 MHz/CDCl₃) 12.39 (1H, s), 9.44 (1H, d), 7.05 (2H, s), 2.85 (2H, q), 2.11 (6H, s), 1.28 (3H, t) |
| 2-422 | (500 MHz/CDCl₃) 8.43 (1H, s), 7.44 (2H, s), 2.82 (2H, q), 2.18 (6H, s), 1.27 (3H, t) |
| 2-423 | (500 MHz/CDCl₃) 13.10 (1H, s), 9.33 (1H, d), 7.75 (2H, s), 6.79 (1H, d), 2.44-2.39 (1H, m), 1.06-0.98 (4H, m) |
| 2-424 | (500 MHz/CDCl₃) 13.06 (1H, s), 9.33 (1H, d), 7.76 (2H, s), 6.78 (1H, d), 3.43 (1H, sep), 1.30 (6H, d) |
| 2-426 | (500 MHz/CDCl₃) 12.95 (1H, s), 9.32 (1H, s), 7.76 (2H, s), 6.78 (1H, d), 2.48 (3H, s) |
| 2-428 | (300 MHz/CDCl₃) 13.00 (1H, s), 9.38 (1H, dd), 7.29 (1H, dd), 7.14 (1H, dd), 6.77 (1H, d), 3.49-3.36 (1H, m), 2.18 (3H, s), 1.30 (6H, dd) |
| 2-429 | (300 MHz/CDCl₃) 12.90 (1H, brs), 9.37 (1H, dd), 7.29 (1H, s), 7.15 (1H, s), 6.78 (1H, d), 2.47 (3H, s), 2.20 (3H, s) |
| 2-430 | (300 MHz/CDCl₃) 12.96 (1H, s), 9.33-9.31 (1H, m), 7.82 (1H, s), 7.52 (1H, dd), 6.78 (1H, d), 2.47 (3H, s) |
| 2-431 | (300 MHz/CDCl₃) 13.02 (1H, s), 9.37 (1H, dd), 7.84 (1H, dd), 7.58 (1H, dd), 6.78 (1H, d), 3.51-3.37 (1H, m), 2.25 (3H, s), 1.30 (6H, dd) |
| 2-432 | (500 MHz/CDCl₃) 13.02 (1H, s), 9.37 (1H, d), 7.67 (1H, s), 7.54 (1H, s), 6.77 (1H, d), 3.47-3.40 (1H, m), 2.23 (3H, s), 1.29 (6H, dd) |
| 2-433 | (300 MHz/CDCl₃) 12.92 (1H, brs), 9.37-3.35 (1H, m), 7.68-7.67 (1H, m), 7.55-7.54 (1H, m), 6.78 (1H, dd), 2.47 (3H, s), 2.25 (3H, s) |
| 2-434 | (300 MHz/CDCl₃) 12.92 (1H, s), 9.37-3.35 (1H, m), 7.84-7.83 (1H, m), 7.59-7.58 (1H, m), 6.78 (1H, d), 2.48 (3H, s), 2.26 (3H, s) |
| 2-435 | (300 MHz/CDCl₃) 12.89 (1H, s), 9.36 (1H, dd), 7.03-6.99 (2H, m), 6.78 (1H, d), 2.46 (3H, s), 2.21 (3H, s) |
| 2-436 | (300 MHz/CDCl₃) 12.91 (1H, s), 9.41 (1H, d), 7.48 (2H, s), 6.81 (1H, d), 2.49 (3H, s), 2.18 (6H, s) |
| 2-437 | (500 MHz/CDCl₃) 13.52 (1H, s), 9.20 (1H, d), 7.66 (1H, s), 7.45 (2H, s), 2.44 (3H, s), 2.16 (6H, s) |
| 2-438 | (300 MHz/CDCl₃) 12.91 (1H, s), 9.32 (1H, d), 7.39 (2H, s), 6.71 (1H, d), 3.41-3.32 (1H, m), 2.07 (6H, s), 1.22 (6H, d) |
| 2-439 | (500 MHz/CDCl₃) 13.63 (1H, s), 9.21 (1H, d), 7.65 (1H, d), 7.45 (2H, s), 3.41 (1H, sep), 2.14 (6H, s), 1.27 (6H, d) |
| 2-440 | (300 MHz/CDCl₃) 12.94 (1H, s), 9.31 (1H, d), 7.37 (2H, s), 6.71 (1H, d), 2.37-2.29 (1H, m), 2.04 (6H, s), 0.99-0.85 (4H, m) |
| 2-441 | (300 MHz/CDCl₃) 13.59 (1H, brs), 9.20 (1H, d), 7.66 (1H, d), 7.43 (2H, s), 2.41-2.35 (1H, m), 2.11 (6H, s), 1.02-0.91 (4H, m) |
| 2-444 | (300 MHz/CDCl₃) 13.01 (1H, brs), 9.22 (1H, d), 7.48 (2H, s), 6.47 (1H, d), 4.61 (2H, s), 3.49 (3H, s), 2.18 (6H, s) |
| 2-446 | (300 MHz/CDCl₃) 13.37 (1H, brs), 9.17 (1H, d), 7.48 (2H, s), 6.49 (1H, d), 2.16 (6H, s) |
| 2-447 | (300 MHz/CDCl₃) 12.81 (1H, brs), 9.15 (1H, d), 7.37 (2H, s), 6.35 (1H, d), 2.36 (3H, s), 2.07 (6H, s) |
| 2-448 | (300 MHz/CDCl₃) 12.92 (1H, brs), 9.16 (1H, d), 7.37 (2H, d), 6.35 (1H, d), 3.42-3.27 (1H, m), 2.07 (6H, s), 1.20 (6H, d) |
| 2-449 | (300 MHz/CDCl₃) 12.85 (1H, s), 9.15 (1H, d), 7.37 (2H, s), 6.35 (1H, d), 2.72 (2H, t), 2.08 (6H, s), 1.74-1.62 (2H, m), 0.92 (3H, t) |
| 2-450 | (300 MHz/CDCl₃) 12.96 (1H, brs), 9.26 (1H, d), 7.47 (2H, s), 6.45 (1H, d), 2.84 (2H, t), 2.18 (6H, s), 1.79-1.67 (2H, m), 1.49-1.37 (2H, m), 0.97 (3H, t) |
| 2-451 | (300 MHz/CDCl₃) 12.94 (1H, brs), 9.24 (1H, d), 7.46 (2H, s), 6.43 (1H, d), 2.81 (2H, t), 2.16 (6H, s), 1.77-1.70 (2H, m), 1.44-1.30 (4H, m), 0.90 (3H, t) |
| 2-452 | (300 MHz/CDCl₃) 12.95 (1H, brs), 9.15 (1H, d), 7.35 (2H, d), 6.35 (1H, d), 2.37-2.27 (1H, m), 2.03 (6H, s), 0.97-0.91 (4H, m) |
| 2-453 | (500 MHz/CDCl₃) 13.67 (1H, brs), 9.18 (1H, d), 7.66-7.63 (2H, m), 7.52 (1H, s), 3.44-3.36 (1H, m), 2.22 (3H, s), 1.27 (6H, dd) |
| 2-454 | (300 MHz/CDCl₃) 13.66 (1H, s), 9.19 (1H, dd), 7.64 (1H, dd), 7.28 (1H, dd), 7.13 (1H, dd), 3.47-3.33 (1H, m), 2.17 (3H, s), 1.28 (6H, d) |
| 2-455 | (300 MHz/CDCl₃) 12.54 (1H, s), 9.40 (1H, d), 7.67 (1H, t), 7.54 (1H, t), 3.49-3.35 (1H, m), 2.22 (3H, s), 1.29 (6H, dd) |
| 2-456 | (300 MHz/CDCl₃) 13.54 (1H, s), 9.28 (1H, d), 7.71 (1H, d), 7.67 (1H, s), 7.53 (1H, s), 3.48-3.34 (1H, m), 2.22 (3H, s), 1.28 (6H, dd) |
| 2-457 | (500 MHz/CDCl₃) 13.74 (1H, s), 9.16 (1H, d), 7.95 (2H, s), 7.65 (1H, d), 3.42 (1H, sep), 1.29 (6H, s) |
| 2-458 | (300 MHz/CDCl₃) 13.61 (1H, s), 9.13 (1H, d), 7.82 (1H, s), 7.66 (1H, dd), 7.51 (1H, dd), 2.44 (3H, s) |
| 2-459 | (300 MHz/CDCl₃) 13.67 (1H, brs), 9.18 (1H, dd), 7.83 (1H, dd), 7.64 (1H, dd), 7.57 (1H, dd), 3.48-3.34 (1H, m), 2.24 (3H, s), 1.28 (6H, dd) |
| 2-460 | (300 MHz/CDCl₃) 13.55 (1H, brs), 9.17 (1H, d), 7.68-7.65 (2H, m), 7.54-7.53(1H, m), 2.44 (3H, s), 2.24 (3H, s) |
| 2-461 | (300 MHz/CDCl₃) 13.52 (1H, brs), 9.17 (1H, dd), 7.83 (1H, dd), 7.66 (1H, dd), 7.57 (1H, dd), 2.44 (3H, s), 2.25 (3H, s) |
| 2-462 | (300 MHz/CDCl₃) 12.47 (1H, brs), 9.36-9.35 (1H, m), 7.83 (1H, brs), 7.53 (1H, dd), 2.46 (3H, s) |
| 2-463 | (500 MHz/CDCl₃) 13.62 (1H, s), 9.15 (1H, d), 7.95 (2H, s), 7.66 (1H, d), 2.46 (3H, s) |
| 2-464 | (300 MHz/CDCl₃) 13.53 (1H, brs), 9.16 (1H, dd), 7.65 (1H, dd), 7.43 (1H, d), 7.34 (1H, dd), 2.43(3H, s), 2.25 (3H, s) |
| 2-465 | (300 MHz/CDCl₃) 12.44 (1H, brs), 9.39 (1H, d), 7.68 (1H, t), 7.55 (1H, d), 2.47 (3H, s), 2.24 (3H, s) |
| 2-466 | (300 MHz/CDCl₃) 12.44 (1H, brs), 9.39 (1H, d), 7.84 (1H, d), 7.58 (1H, d), 2.47 (3H, s), 2.25 (3H, s) |
| 2-467 | (300 MHz/CDCl₃) 12.54 (1H, brs), 9.40 (1H, d), 7.84 (1H, dd), 7.58 (1H, dd), 3.62-3.27 (1H, m), 2.24 (3H, s), 1.29 (6H, dd) |
| 2-468 | (300 MHz/CDCl₃) 13.54 (1H, brs), 9.28 (1H, d), 7.83 (1H, dd), 7.71 (1H, d), 7.57 (1H, dd), 3.53-3.31 (1H, m), 2.24 (3H, s), 1.28 (6H, dd) |
| 2-469 | (300 MHz/CDCl₃) 13.42 (1H, brs), 9.26 (1H, d), 7.72 (1H, d), 7.67 (1H, dd), 7.53 (1H, dd), 2.44 (3H, s), 2.24 (3H, s) |
| 2-470 | (300 MHz/CDCl₃) 13.47 (1H, brs), 9.24 (1H, d), 7.71 (1H, d), 7.45-7.43 (1H, m), 7.15 (1H, ddd), 2.44 (3H, s) |
| 2-471 | (300 MHz/CDCl₃) 13.48 (1H, brs), 9.23 (1H, d), 7.82 (1H, s), 7.71 (1H, d), 7.51 (1H, dd), 2.44 (3H, s) |
| 2-472 | (300 MHz/CDCl₃) 12.95 (1H, s), 9.33 (1H, dd), 7.44 (1H, s), 7.15 (1H, ddd), 6.78 (1H, d), 2.47 (3H, s) |
| 2-473 | (300 MHz/CDCl₃) 12.46 (1H, s), 9.36 (1H, d), 7.45-7.44 (1H, m), 7.16 (1H, ddd), 2.46 (3H, s) |
| 2-474 | (300 MHz/CDCl₃) 13.59 (1H, brs), 9.14 (1H, dd), 7.66 (1H, dd), 7.44-7.43 (1H, m), 7.15 (1H, ddd), 2.43 (3H, s) |
| 2-475 | (300 MHz/CDCl₃) 13.44 (1H, brs), 9.27 (1H, d), 7.83 (1H, dd), 7.72 (1H, d), 7.58 (1H, dd), 2.45 (3H, s), 2.25 (3H, s) |
| 2-476 | (300 MHz/CDCl₃) 13.42 (1H, brs), 9.28 (1H, d), 7.71 (1H, d), 7.29 (1H, dd), 7.14 (1H, dd), 2.44 (3H, s), 2.19 (3H, s) |
| 2-477 | (300 MHz/CDCl₃) 13.53 (1H, brs), 9.29 (1H, d), 7.70 (1H, d), 7.29 (1H, t), 7.14 (1H, dd), 3.48-3.33 (1H, m), 2.18 (3H, s), 1.28 (6H, t) |
| 2-478 | (300 MHz/CDCl₃) 12.91 (1H, s), 9.36-9.34 (1H, m), 7.44 (1H, d), 7.38 (1H, d), 6.79-6.78 (1H, m), 2.46 (3H, s), 2.26 (3H, s) |
| 2-479 | (300 MHz/CDCl₃) 12.43 (1H, brs), 9.38 (1H, d), 7.44 (1H, d), 7.38(1H, d), 2.46 (3H, s), 2.25 (3H, s) |
| 2-480 | (300 MHz/CDCl₃) 12.42 (1H, brs), 9.40 (1H, d), 7.30 (1H, dd), 7.15 (1H, dd), 2.46 (3H, s), 2.19 (3H, s) |
| 2-481 | (300 MHz/CDCl₃) 12.52 (1H, brs), 9.41 (1H, t), 7.29 (1H, dd), 7.15 (1H, dd), 3.49-3.34 (1H, m), 2.17 (3H, s), 1.29 (6H, dd) |
| 2-482 | (500 MHz/CDCl₃) 13.40 (1H, brs), 9.26 (1H, s), 7.70 (1H, s), 7.02 (1H, s), 6.99 (1H, d), 2.42 (3H, s), 2.20 (3H, s) |
| 2-483 | (500 MHz/CDCl₃) 13.41 (1H, brs), 9.25 (1H, s), 7.71 (1H, s), 7.42 (1H, s), 7.35 (1H, d), 2.43 (3H, s), 2.25 (3H, s) |
| 2-484 | (300 MHz/CDCl₃) 12.40 (1H, brs), 9.39 (1H, t), 7.04-6.98 (2H, m), 2.45 (3H, s), 2.20 (3H, s) |
| 2-485 | (300 MHz/CDCl₃) 13.53 (1H, brs), 9.17 (1H, dd), 7.65 (1H, dd), 7.02-6.98 (2H, m), 2.42 (3H, s), 2.20 (3H, s) |
| 2-487 | (300 MHz/CDCl₃) 12.91 (1H, brs), 9.21 (1H, d), 7.78 (2H, s), 6.44 (1H, d), 3.07 (3H, s), 2.45 (3H, s), 2.20 (6H, s) |
| 2-488 | (300 MHz/CDCl₃) 12.86 (1H, brs), 9.21 (1H, d), 7.74 (2H, s), 6.45 (1H, d), 3.14 (2H, q), 2.46 (3H, s), 2.20 (6H, s), 1.35 (3H, t) |
| 2-489 | (300 MHz/CDCl₃) 12.85 (1H, brs), 9.28 (1H, d), 7.15 (2H, s), 6.44 (1H, d), 3.36 (2H, q), 2.47 (3H, s), 2.13 (6H, s) |
| 2-490 | (300 MHz/CDCl₃) 12.87 (1H, brs), 9.27 (1H, d), 6.96 (2H, s), 6.53 (1H, t), 6.45 (1H, d), 2.47 (3H, s), 2.12 (6H, s) |
| 2-493 | (300 MHz/CDCl₃) 13.35 (1H, brs), 9.27 (1H, d), 7.47 (2H, s), 6.45 (1H, d), 2.17 (6H, s), 1.45 (9H, s) |
| 2-494 | (300 MHz/CDCl₃) 12.20 (1H, s), 10.12 (1H, s), 7.50 (2H, s), 2.89 (2H, q), 2.18 (6H, s), 1.31 (3H, t) |
| 2-495 | (300 MHz/CDCl₃) 12.10 (1H, s), 10.14 (1H, s), 7.50 (2H, d), 2.89 (2H, q), 2.17 (6H, s), 1.31 (3H, t) |
| 2-496 | (300 MHz/CDCl₃) 12.40 (1H, s), 9.41-9.48 (1H, m), 7.58-7.67 (2H, m), 7.37-7.46 (1H, m), 2.85 (2H, q), 2.23 (3H, s), 1.28 (3H, t) |
| 2-497 | (300 MHz/CDCl₃) 12.40 (1H, s), 9.41-9.48 (1H, m), 7.58-7.67 (2H, m), 7.41 (1H, d), 2.85 (2H, q), 2.23 (3H, s), 1.28 (3H,t) |
| 2-498 | (300 MHz/CDCl₃) 13.04 (1H, brs), 9.15 (1H, d), 8.05 (2H, s), 6.44 (1H, d), 3.13 (3H, s), 2.85 (2H, q), 1.28 (3H, t) |
| 2-499 | (300 MHz/CDCl₃) 13.04 (1H, brs), 9.16 (1H, d), 8.25 (2H, s), 6.44 (1H, d), 3.13 (3H, s), 2.85 (2H, q), 1.29 (3H, t) |
| 2-500 | (300 MHz/CDCl₃) 12.92 (1H, brs), 9.21 (1H, d), 7.78 (1H, s), 7.52 (1H, s), 6.43 (1H, d), 6.36 (1H, dd), 5.81 (1H, d), 5.36 (1H, d), 2.45 (3H, s), 2.16 (3H, s) |
| 2-501 | (300 MHz/CDCl₃) 13.03 (1H, brs), 9.21 (1H, d), 7.77 (1H, s), 7.52 (1H, s), 6.42 (1H, d), 6.34 (1H, dd), 5.78 (1H, d), 5.34 (1H, d), 3.48-3.34 (1H, m), 2.15 (3H, s), 1.27 (6H, d) |
| 2-502 | (300 MHz/CDCl₃) 13.54 (1H, brs), 9.18 (1H, d), 7.78 (1H, brs), 7.66 (1H, d), 7.52 (1H, brs), 6.36 (1H, dd), 5.81 (1H, d), 5.36 (1H, d), 2.44 (3H, s), 2.17 (3H, s) |
| 2-503 | (300 MHz/CDCl₃) 13.67 (1H, brs), 9.19 (1H, d), 7.77 (1H, brs), 7.65 (1H, d), 7.52 (1H, brs), 6.34 (1H, dd), 5.78 (1H, d), 5.34 (1H, d), 3.48-3.34 (1H, m), 2.15 (3H, s), 1.26 (6H, d) |
| 2-504 | (300 MHz/CDCl₃) 12.90 (1H, brs), 9.23 (1H, d), 7.47 (1H, brs), 7.46 (1H, brs), 6.43 (1H, d), 2.52-2.38 (5H, m), 2.15 (3H, s), 1.18 (3H, t) |
| 2-505 | (300 MHz/CDCl₃) 13.01 (1H, brs), 9.23 (1H, d), 7.47 (1H, brs), 7.46 (1H, brs), 6.42 (1H, d), 3.48-3.35 (1H, m), 2.55-2.34 (2H, m), 2.12 (3H, s), 1.27 (6H, d), 1.16 (3H, t) |
| 2-506 | (300 MHz/CDCl₃) 13.52 (1H, brs), 9.20 (1H, d), 7.66 (1H, d), 7.48 (1H, brs), 7.46 (1H, brs), 2.50-2.39 (5H, m), 2.15 (3H, s), 1.18 (3H, t) |
| 2-507 | (300 MHz/CDCl₃) 13.65 (1H, brs), 9.21 (1H, d), 7.64 (1H,d), 7.48 (1H, brs), 7.47 (1H, brs), 3.50-3.35 (1H, m), 2.55-2.34 (2H, m), 2.13 (3H, s), 1.27 (6H, d), 1.16 (3H, t) |
| 2-508 | (300 MHz/CDCl₃) 9.24 (1H, d), 7.47 (2H, s), 6.40 (1H, d), 2.85 (2H,q), 2.64 (3H, s), 2.18 (6H, s), 1.30 (3H, t) |
| 2-509 | (300 MHz/CDCl₃) 13.91 (1H, brs), 9.26 (1H, d), 7.81 (1H, d), 7.47-7.54 (2H, m), 2.19 (6H, s) |
| 2-510 | (300 MHz/CDCl₃) 12.60 (1H, brs), 9.31 (1H, s), 8.09 (2H, d), 7.93 (2H, d), 3.48-3.37 (1H, m), 3.12 (3H, s), 1.34 (6H, d) |
| 2-511 | (300 MHz/CDCl₃) 9.17 (1H, dd), 7.75 (1H, dd), 7.50 (2H, s), 2.19 (6H, s) |
| 2-512 | (300 MHz/CDCl₃) 12.69 (1H, brs), 9.14 (1H, d), 7.47 (2H, s), 6.04 (1H, d), 4.64 (2H, q), 2.84 (2H, q), 2.18 (6H, s), 1.29 (3H ,t) |
| 2-513 | (300 MHz/CDCl₃) 12.31 (1H, brs), 9.10 (1H, d), 7.76 (2H, s), 6.29 (1H, d), 3.47-3.38 (1H, m), 2.43 (3H, s), 1.30 (6H, d) |
| 2-514 | (300 MHz/CDCl₃) 14.44 (1H, brs), 9.00 (1H, d), 7.65 (2H, s), 6.20 (1H, d), 2.37-2.28 (4H, m), 0.97-0.88 (4H, m) |
| 2-515 | (300 MHz/CDCl₃) 13.55 (1H, brs), 9.15 (1H, d), 7.50 (2H, s), 6.60 (1H, d), 2.18 (6H, s) |
| 2-516 | (300 MHz/CDCl₃) 8.93 (1H, d), 7.29-7.35 (2H, m), 6.18 (1H, d), 2.28 (3H, s), 2.01 (6H, s) |
| 2-517 | (300 MHz/CDCl₃) 13.40 (1H, brs), 9.32-9.40 (1H, m), 7.52 (2H, s), 6.83-6.90 (1H, m), 2.19 (6H, s) |
| 2-519 | (300 MHz/CDCl₃) 14.28 (1H, brs), 8.91 (1H, s), 7.66 (2H, s), 7.50 (1H, s), 2.38-2.28 (1H, m), 2.09 (3H, s), 0.96-0.88 (4H, m) |
| 2-520 | (300 MHz/CDCl₃) 14.28 (1H, brs), 9.06 (1H, s), 7.60 (1H, s), 7.45 (2H, s), 2.47-2.37 (1H, m), 2.18 (3H, s), 2.13 (6H, s), 1.05-0.94 (4H, m) |
| 2-521 | (300 MHz/CDCl₃) 14.52 (1H, brs), 9.07 (1H, s), 7.58 (1H, s), 7.46 (2H, s), 2.18 (3H, s), 2.16 (6H, s), 1.44 (9H, s) |
| 2-522 | (300 MHz/CDCl₃) 13.46 (1H, brs), 9.19 (1H, s), 7.47 (2H, s), 2.83 (2H, q), 2.17 (6H, s), 2.01-2.16 (1H, m), 1.28 (3H, t), 1.06-1.19 (2H, m), 0.96-1.08 (2H, m) |
| 2-523 | (300 MHz/CDCl₃) 12.85 (1H, s), 9.24 (1H, d), 7.45-7.51 (2H, m), 6.44 (1H, d), 3.79-3.97 (1H, m), 2.21-2.43 (4H, m), 2.19 (6H, s), 1.98-2.17 (1H, m), 1.81-1.97 (1H, m) |
| 2-524 | (300 MHz/CDCl₃) 12.94 (1H, brs), 9.08 (1H, d), 7.30 (2H, d), 6.36 (1H, d), 2.75 (2H, q), 1.21 (3H, t) |
| 2-525 | (300 MHz/CDCl₃) 14.77 (1H, brs), 9.18 (1H, d), 7.46 (2H, s), 6.29 (1H, d), 2.44 (3H, s), 2.16 (6H, s), 1.45 (9H, s) |
| 2-526 | (300 MHz/CDCl₃) 14.49 (1H, brs), 9.15 (1H, d), 7.44 (2H, s), 6.28 (1H, d), 2.46-2.37 (4H, m), 2.13 (6H, s), 1.05-0.93 (4H, m) |
| 2-527 | (300 MHz/CDCl₃) 13.02 (1H, brs), 9.18 (1H, d), 7.79 (2H, s), 6.45 (1H, d), 2.86 (2H, q), 1.30 (3H, t) |
| 2-529 | (300 MHz/CDCl₃) 12.79 (1H, brs), 9.26-9.32 (1H, m), 7.42 (2H, s), 2.09 (6H, s) |
| 2-530 | (300 MHz/CDCl₃) 9.13-9.19 (1H, m), 7.47-7.53 (2H, m), 2.41 (3H, s), 2.18 (6H, s) |
| 2-531 | (300 MHz/CDCl₃) 8.96-9.03 (1H, m), 7.50 (2H, s), 2.18 (6H, s), 2.12-2.17 (3H, m) |
| 2-532 | (300 MHz/CDCl₃) 9.24 (1H, s), 7.50 (2H, s), 2.70-2.84 (4H, m), 2.19 (6H, s), 1.34 (3H, t), 1.27 (3H, t) |
| 2-533 | (300 MHz/CDCl₃) 12.99 (1H, s), 9.03-9.09 (1H, m), 7.47 (2H, s), 2.85 (2H, q), 2.17 (6H, s), 2.09-2.16 (3H, m), 1.29 (3H, t) |
| 2-534 | (300 MHz/CDCl₃) 13.01 (1H, brs), 9.14-9.21 (1H, m), 7.48 (2H, s), 2.87 (2H, q), 2.23-2.29 (3H, m), 2.18 (6H, s), 1.30 (3H, t) |
| 2-535 | (300 MHz/CDCl₃) 13.33 (1H, s), 9.41 (1H, d), 7.46 (2H, s), 6.79 (1H, d), 2.15 (6H, s), 1.44 (9H, s) |
| 2-536 | (300 MHz/CDCl₃) 14.01 (1H, brs), 9.22 (1H, d), 7.64 (1H, d), 7.45 (2H, s), 2.14 (6H, s), 1.41 (9H, s) |
| 2-537 | (300 MHz/CDCl₃) 13.54 (1H, brs), 9.30 (1H, s), 7.72 (1H, s), 7.43 (2H, s), 2.43-2.36 (1H, m), 2.11 (6H, s), 1.03-0.96 (4H, m) |
| 2-538 | (300 MHz/CDCl₃) 13.87 (1H, brs), 9.32 (1H, s), 7.71 (1H, s), 7.45 (2H, s), 2.13 (6H , s), 1.42 (9H, s) |
| 2-539 | (300 MHz/CDCl₃) 12.95 (1H, s), 9.18 (1H, d), 7.76-7.82 (2H, m), 6.45 (1H, d), 3.79-3.97 (1H, m), 2.23-2.44 (4H, m), 1.98-2.20 (1H, m), 1.81-1.99 (1H, m) |
| 2-540 | (300 MHz/CDCl₃) 13.53 (1H, s), 9.35 (1H, s), 7.75 (1H, s), 7.44 (2H, s), 2.43-2.37 (1H, m), 2.11 (6H, s), 1.03-0.96 (4H, m) |
| 2-541 | (300 MHz/CDCl₃) 13.86 (1H, s), 9.36 (1H, s), 7.73 (1H, s), 7.45 (2H, s), 2.14 (6H, s), 1.42 (9H, s) |
| 2-543 | (300 MHz/CDCl₃) 13.18 (1H, s), 9.38 (1H, d), 7.49 (2H, s), 6.63-6.87 (2H, m), 2.88 (2H, q), 2.18 (6H, s), 1.30 (3H, t) |
| 2-544 | (300 MHz/CDCl₃) 13.13 (1H, s), 9.14-9.20 (1H, m), 7.46 (2H, s), 2.35-2.49 (1H, m), 2.23-2.40 (3H, m), 2.13 (6H, s), 0.87-1.10 (4H, m) |
| 2-545 | (300 MHz/CDCl₃) 13.26 (1H, brs), 9.69 (1H, s), 8.00 (1H, s), 7.47 (2H, s), 2.47 (3H, s), 2.16 (6H, s) |
| 2-546 | (300 MHz/CDCl₃) 13.29 (1H, s), 9.69 (1H, s), 7.99 (1H, s), 7.47 (2H, s), 2.86 (2H, q), 2.16 (6H, s), 1.28 (3H, t) |
| 2-547 | (300 MHz/CDCl₃) 13.37 (1H, s), 9.70 (1H, s), 7.99 (1H, s), 7.46 (2H, s), 3.48-3.39 (1H, m), 2.15 (6H, s), 1.28 (6H, d) |
| 2-548 | (300 MHz/CDCl₃) 13.40 (1H, s), 9.70 (1H, s), 8.00 (1H, s), 7.45 (2H, s), 2.46-2.36 (1H, m), 2.11 (6H, s), 1.06-0.92 (4H, m) |
| 2-549 | (300 MHz/CDCl₃) 13.74 (1H, s), 9.71 (1H, s), 7.98 (1H, s), 7.46 (2H, s), 2.14 (6H, s), 1.43 (9H, s) |
| 2-550 | (300 MHz/CDCl₃) 12.46 (1H, s), 9.33-9.38 (1H, m), 7.33-7.42 (2H, m), 2.24-2.38 (1H, m), 2.30 (6H, s), 0.84-1.01 (4H, m) |
| 2-552 | (300 MHz/CDCl₃) 13.42 (1H, s), 9.19 (1H, s), 7.48 (2H, s), 2.24-2.28 (3H, m), 2.16 (6H, s), 1.45 (9H, s) |
| 2-554 | (300 MHz/CDsOD) 7.72 (1H, d), 7.56 (1H, d), 7.48 (2H, s), 2.73 (2H, q), 2.23 (6H, s), 1.21 (3H, t) |
| 2-555 | (300 MHz/CD₃OD) 7.59 (1H, dd), 7.53 (1H, dd), 7.48 (2H, s), 3.20-3.32 (8H, m), 2.71 (2H, q), 2.21 (6H, s), 1.60-1.77 (8H, m), 1.35-1.53 (8H, m), 1.20 (3H, t), 0.99-1.10 (12H, m) |
| 4-1 | (300 MHz/CDCl₃) 11.50 (1H, brs), 7.98 (2H, s), 7.43 (2H, s), 2.86 (2H, q), 2.19 (6H, s), 1.28 (3H, t) |
| 4-3 | (300 MHz/CDCl₃) 12.04 (1H, brs), 7.66 (1H, s), 7.35 (2H, s), 2.77 (2H, q), 2.41 (3H, s), 2.10 (6H, s), 1.20 (3H, t) |
| 4-5 | (300 MHz/CDCl₃) 7.94 (1H, s), 7.45 (2H, s), 3.47-3.37 (1H, m), 2.18 (6H, s), 1.29 (6H, d) |
| 5-1 | (500 MHz/CDCl₃) 12.79 (1H, brs), 9.80 (1H, s), 8.84 (1H, s), 8.24 (1H, s), 8.20 (1H, d), 2.88 (2H, q), 1.30 (3H, t) |
| 5-2 | (300 MHz/CDCl₃) 12.00 (1H, s), 9.72 (1H, s), 8.86 (1H, d), 8.23 (1H, d), 2.90 (2H, q), 1.32 (3H, t) |
| 5-4 | (300 MHz/CDCl₃) 12.00 (1H, s), 9.73 (1H, s), 8.90 (1H, d), 8.39 (1H, d), 2.90 (2H, q), 1.33 (3H, t) |
| 5-21 | (300 MHz/CDCl₃) 11.75 (1H, d), 9.57 (1H, d), 8.87 (1H, dd), 8.23 (1H, dd), 2.89 (2H, q), 1.32 (3H, t) |
| 5-22 | (300 MHz/CDCl₃) 11.74 (1H, d), 9.57 (1H, d), 8.90 (1H, dd), 8.39 (1H, d), 2.89 (2H, q), 1.32 (3H, t) |
| 5-23 | (300 MHz/CDCl₃) 12.05 (1H, s), 9.70 (1H, s), 8.86 (1H, dd), 8.39 (1H, dd), 2.90 (2H, q), 1.32 (3H, t) |
| 5-24 | (300 MHz/CDCl₃) 12.05 (1H, s), 9.70 (1H, s), 8.90 (1H, dd), 8.20-8.27 (1H, m), 2.90 (2H, q), 1.33 (3H, t) |
| 5-25 | (300 MHz/CDCl₃) 13.03 (1H, brs), 9.67 (1H, s), 8.84-8.89 (1H, m), 8.20-8.24 (1H, m), 2.89 (2H, q), 2.58 (3H, s), 1.32 (3H, t) |
| 5-26 | (300 MHz/CDCl₃) 13.03 (1H, brs), 9.68 (1H, s), 8.86-8.94 (1H, m), 8.36-8.40 (1H, m), 2.88 (2H, q), 2.58 (3H, s), 1.32 (3H, t) |
| 5-27 | (300 MHz/CDCl₃) 11.85 (1H, s), 9.91-9.97 (1H, m), 8.84-8.91 (1H, m), 8.21-8.27 (1H,m), 2.92 (2H, q), 1.34 (3H, t) |
| 5-28 | (300 MHz/CDCl₃) 11.84 (1H, s), 9.91-9.97 (1H, m), 8.87-8.94 (1H, m), 8.37-8.46 (1H,m), 2.92 (2H, q), 1.34 (3H, t) |
| 5-50 | (300 MHz/CDCl₃) 11.93 (1H, brs), 9.72 (1H, s), 8.77 (1H, d), 8.03 (1H, d), 2.87 (2H, q), 2.62 (2H, q), 1.21-1.32 (6H, m) |
| 5-51 | (300 MHz/CDCl₃) 11.78 (1H, s), 9.91-9.98 (1H, m), 8.75-8.81 (1H, m), 7.97-8.05 (1H,m), 2.89 (2H, q), 2.32 (3H, s), 1.31 (3H, t) |
| 6-1 | (500 MHz/CDCl₃) 11.84 (1H, s), 9.92 (1H, s), 7.78 (2H, s), 2.89 (2H, q), 1.30 (3H, t) |
| 6-2 | (500 MHz/CDCl₃) 11.85 (1H, s), 9.93 (1H, s), 7.98 (2H, s), 2.90 (2H, q), 1.31 (3H, t) |
| 6-5 | (500 MHz/CDCl₃) 11.77 (1H, s), 9.99 (1H, s), 7.47 (2H, s), 2.89 (2H, q), 2.16 (6H, s), 1.29 (3H, t) |
| 6-7 | (300 MHz/CDCl₃) 11.75 (1H, brs), 9.99 (1H, d), 7.06 (2H, s), 2.88 (2H, q), 2.11 (6H, s), 1.29 (3H, t) |
| 6-10 | (500 MHz/CDCl₃) 11.98 (1H, s), 9.70 (1H, s), 7.77 (2H, s), 2.87 (2H, q), 1.29 (3H, t) |
| 6-11 | (300 MHz/CDCl₃) 12.09 (1H, brs), 9.72 (1H, s), 7.79 (2H, s), 3.49-3.39 (1H, m), 1.31 (6H, d) |
| 6-12 | (500 MHz/CDCl₃) 12.04 (1H, s), 9.67 (1H, s), 7.77 (2H, s), 2.87 (2H, q), 1.29 (3H, t) |
| 6-13 | (500 MHz/CDCl₃) 11.99 (1H, s), 9.71 (1H, s), 7.97 (2H, s), 2.88 (2H, q), 1.30 (3H, t) |
| 6-14 | (500 MHz/CDCl₃) 12.04 (1H, s), 9.68 (1H, s), 7.97 (2H, s), 2.88 (2H, q), 1.30 (3H, t) |
| 6-18 | (500 MHz/CDCl₃) 11.91 (1H, s), 9.76 (1H, s), 7.47 (2H, s), 2.87 (2H, q), 2.16 (6H, s), 1.28 (3H, t) |
| 6-19 | (300 MHz/CDCl₃) 9.75 (1H, s), 7.64 (1H, s), 7.62 (1H, d), 7.42 (1H, d), 2.86 (2H, q), 2.24 (3H, s), 1.29 (3H, t) |
| 6-21 | (300 MHz/CDCl₃) 11.88 (1H, brs), 9.77 (1H, s), 7.05 (2H, s), 2.86 (2H, q), 2.11 (6H, s), 1.28 (3H, t) |
| 6-22 | (500 MHz/CDCl₃) 11.87 (1H, s), 9.77 (1H, s), 7.19 (2H, s), 2.85 (2H, q), 2.07 (6H, s), 1.27 (3H, t) |
| 6-23 | (300 MHz/CDCl₃) 11.88 (1H, s), 9.79 (1H, s), 7.37 (2H, s), 2.87 (2H, q), 2.09 (6H, s), 1.29 (3H, t) |
| 6-30 | (300 MHz/CDCl₃) 11.86 (1H, brs), 9.80 (1H, s), 7.15 (2H, s), 3.37 (2H, q), 2.88 (2H, q), 2.11 (6H, s), 1.32-1.27 (3H, m) |
| 6-31 | (300 MHz/CDCl₃) 11.86 (1H, brs), 9.76 (1H, s), 6.94 (2H, s), 6.51 (1H, t), 2.85 (2H, q), 2.08 (6H, s), 1.25 (3H, t) |
| 6-33 | (300 MHz/CDCl₃) 11.91 (1H, s), 9.80 (1H, s), 7.37 (2H, s), 6.65 (1H, t), 2.88 (2H, q), 2.16 (6H, s), 1.30 (3H, t) |
| 6-37 | (300 MHz/CDCl₃) 11.84 (1H, brs), 9.76 (1H, s), 7.31 (2H, s), 3.07 (1H, s), 2.84 (2H, q), 2.05 (6H, s), 1.25 (3H, t) |
| 6-38 | (300 MHz/CDCl₃) 11.75 (1H, brs), 9.73 (1H, s), 7.15 (2H, s), 6.62 (1H, dd), 5.68 (1H, d), 5.21 (1H, d), 2.78 (2H, q), 2.01 (6H, s), 1.21 (3H, t) |
| 6-40 | (300 MHz/CDCl₃) 11.96 (1H, brs), 9.77 (1H, s), 7.81 (1H, brs), 7.56 (1H, brs), 6.36 (1H, dd), 5.83 (1H, d), 5.39 (1H, d), 2.89 (2H, q), 2.19 (3H, s), 1.29 (3H, t) |
| 6-41 | (300 MHz/CDCl₃) 11.84 (1H, brs), 9.68 (1H, s), 7.42 (1H, brs), 7.40 (1H, brs), 2.79 (2H, q), 2.45-2.30 (2H, m), 2.07 (3H, s), 1.20 (3H, t), 1.10 (3H, t) |
| 6-45 | (300 MHz/CDCl₃) 12.72 (1H, brs), 9.87 (1H, s), 8.25 (1H, s), 7.46 (2H, s), 2.88 (2H, q), 2.16 (6H, s), 1.28 (3H, t) |
| 6-47 | (500 MHz/CDCl₃) 12.81 (1H, s), 9.81 (1H, s), 8.25 (1H, s), 7.77 (2H, s), 2.88 (2H, q), 1.29 (3H, t) |
| 6-48 | (500 MHz/CDCl₃) 12.81 (1H, s), 9.81 (1H, s), 8.25 (1H, s), 7.97 (2H, s), 2.88 (2H, q), 1.30 (3H, t) |
| 6-49 | (500 MHz/CDCl₃) 11.87 (1H, s), 9.77 (1H, s), 7.19 (2H, s), 2.85 (2H, q), 2.07 (6H, s), 1.27 (3H, t) |
| 6-51 | (500 MHz/CDCl₃) 11.94 (1H, s), 9.73 (1H, s), 7.45 (2H, s), 2.86 (2H, q), 2.14 (6H, s), 1.27 (3H, t) |
| 6-52 | (300 MHz/CDCl₃) 12.95 (1H, brs), 9.74 (1H, s), 7.48 (2H, s), 2.88 (2H, q), 2.58 (3H, s), 2.18 (6H, s), 1.30 (3H, t) |
| 6-64 | (500 MHz/CDCl₃) 11.93 (1H, s), 9.74 (1H, s), 7.04 (2H, s), 2.86 (2H, q), 2.09 (6H, s), 1.27 (3H, t) |
| 6-65 | (500 MHz/CDCl₃) 11.42 (1H, brs), 9.98 (1H, s), 7.49 (2H, s), 2.91 (2H, q), 2. 17 (6H, s), 1.30 (3H, t) |
| 6-66 | (300 MHz/CDCl₃) 12.70 (1H, brs), 9.88 (1H, s), 8.24 (1H, s), 7.05 (2H, s), 2.86 (2H, q), 2.11 (6H, s), 1.28 (3H, t) |
| 6-67 | (500 MHz/CDCl₃) 11.93 (1H, s), 9.75 (1H, s), 7.20 (2H, s), 2.86 (2H, q), 2.07 (6H, s), 1.28 (3H, t) |
| 6-71 | (500 MHz/CDCl₃) 7.45 (2H, s), 2.82 (2H, q), 2.18 (6H, s), 1.28 (3H, t) |
| 6-73 | (300 MHz/CDCl₃) 11.75 (1H, brs), 9.99 (1H, s), 7.48 (2H, s), 2.18 (3H, s), 2.17 (6H, s) |
| 6-74 | (300 MHz/CDCl₃) 11.61-11.67 (1H, m), 9.63 (1H, d), 7.49 (2H, s), 2.49 (3H, s), 2.18 (6H, s) |
| 6-75 | (300 MHz/CDCl₃) 11.88 (1H, s), 9.77 (1H, s), 7.48 (2H, s), 2.49 (3H, s), 2.17 (6H, s) |
| 6-76 | (300 MHz/CDCl₃) 11.96 (1H, s), 9.76 (1H, s), 7.49 (2H, s), 2.50 (3H, s), 2.18 (6H, s) |
| 6-78 | (300 MHz/CDCl₃) 11.86 (1H, brs), 9.99 (1H, s), 7.48 (2H, s), 3.50-3.41 (1H, m), 2.15 (6H, s), 1.30 (6H, d) |
| 6-79 | (300 MHz/CDCl₃) 11.73 (1H, brs), 9.61 (1H, d), 7.44-7.50 (2H, m), 3.42 (1H, sep), 2.15 (6H, s), 1.28 (6H, d) |
| 6-80 | (300 MHz/CDCl₃) 12.00 (1H, s), 9.79 (1H, s), 7.48 (2H, s), 3.53-3.38 (1H, m), 2.17 (6H, s), 1.31 (6H, d) |
| 6-81 | (300 MHz/CDCl₃) 11.86 (1H, brs), 9.99 (1H, s), 7.46 (2H, s), 2.44-2.38 (1H, m), 2.12 (6H, s), 1.09-0.96 (4H, m) |
| 6-82 | (300 MHz/CDCl₃) 11.74 (1H, brs), 9.60 (1H, d), 7.45 (2H, s), 2.29-2.47 (1H, m), 2.11 (6H, s), 0.90-1.11 (4H, m) |
| 6-83 | (300 MHz/CDCl₃) 12.01 (1H, brs), 9.78 (1H, s), 7.47 (2H, s), 2.47-2.38 (1H, m), 2.13 (6H, s), 1.10-0.95 (4H, m) |
| 6-84 | (300 MHz/CDCl₃) 11.93 (1H, s), 9.78 (1H, s), 7.49 (2H, s), 2.84 (2H, t), 2.18 (6H, s), 1.85-1.72 (2H, m), 1.02 (3H, t) |
| 6-85 | (300 MHz/CDCl₃) 11.95 (1H, s), 9.78 (1H, s), 7.49 (2H, s), 2.86 (2H, t), 2.17 (6H, s), 1.77-1.66 (2H, m), 1.51-1.36 (2H, m), 0.97 (3H, t) |
| 6-86 | (300 MHz/CDCl₃) 11.94 (1H, brs), 9.79 (1H, s), 7.49 (2H, s), 2.85 (2H, t), 2.18 (6H, s), 1.81-1.69 (2H, m), 1.45-1.35 (4H, m), 0.92 (3H, t) |
| 6-89 | (300 MHz/CDCl₃) 12.03 (1H, brs), 9.75 (1H, s), 7.49 (2H, s), 4.62 (2H, s), 3.49 (3H, s), 2.18 (6H, s) |
| 6-91 | (300 MHz/CD₃OD) 8.65 (1H, s), 7.54 (2H, s), 2.22 (6H, s) |
| 6-94 | (300 MHz/CDCl₃) 11.84 (1H, brs), 9.80 (1H, s), 7.15 (2H, s), 3.37 (2H, q), 2.49 (3H, s), 2.11 (6H, s) |
| 6-95 | (300 MHz/CDCl₃) 11.85 (1H, brs), 9.79 (1H, s), 6.97 (2H, s), 6.53 (1H, t), 2.48 (3H, s), 2.11 (6H, s) |
| 6-98 | (300 MHz/CDCl₃) 11.90 (1H, s), 9.76 (1H, s), 7.50 (2H, s), 2.86 (2H, q), 2.12 (6H, s), 1.28 (3H, t) |
| 6-102 | (300 MHz/CDCl₃) 12.35 (1H, brs), 9.81 (1H, s), 7.48 (2H, s), 2.17 (6H, s), 1.45 (9H, s) |
| 6-103 | (300 MHz/CDCl₃) 11.64-11.70 (1H, m), 9.64 (1H, d), 7.08 (2H, s), 2.88 (2H, q), 2.13 (6H, s), 1.30 (3H, t) |
| 6-104 | (300 MHz/CDCl₃) 11.84 (1H, s), 9.77 (1H, s), 7.50 (2H, s), 3.80-3.97 (1H, m), 2.24-2.46 (4H, m), 2.19 (6H, s), 2.00-2.18 (1H, m), 1.81-2.00 (1H, m) |
| 6-105 | (300 MHz/CDCl₃) 12.08 (1H, brs), 9.94 (1H, s), 7.45-7.51 (2H, m), 6.86 (1H,t), 2.88 (2H, q), 2.16 (6H, s), 1.29 (3H, t) |
| 6-106 | (300 MHz/CDCl₃) 11.83 (1H, brs), 9.73 (1H, s), 7.79 (1H, brs), 7.54 (1H, brs), 6.34 (1H, dd), 5.82 (1H, d), 5.38 (1H, d), 2.47 (3H, s), 2.17 (3H, s) |
| 6-107 | (300 MHz/CDCl₃) 12.01 (1H, brs), 9.74 (1H, s), 7.78 (1H, brs), 7.53 (1H, brs), 6.32 (1H, dd), 5.79 (1H, d), 5.36 (1H, d), 3.47-3.38 (1H, m), 2.15 (3H, s), 1.29 (6H, d) |
| 6-108 | (300 MHz/CDCl₃) 11.80 (1H, brs), 9.74 (1H, s), 7.50 (1H, s), 7.47 (1H, s), 2.50-2.40 (5H, m), 2.14 (3H, s), 1.18 (3H, t) |
| 6-109 | (300 MHz/CDCl₃) 11.99 (1H, brs), 9.76 (1H, s), 7.49 (1H, brs), 7.47 (1H, brs), 3.48-3.38 (1H, m), 2.52-2.33 (2H, m), 2.12 (3H, s), 1.28 (6H, d), 1.18 (3H, t) |
| 6-110 | (500 MHz/DMSO-d₆) 8.44 (1H, s), 7.48 (2H, s), 3.81 (3H, s), 3.24 (1H, sep), 2.09 (6H, s), 1.03 (6H, d) |
| 6-111 | (300 MHz/CDCl₃) 11.86 (1H, brs), 9.75 (1H, s), 7.64 (1H, s), 7.62 (1H, d), 7.41 (1H, d), 2.47 (3H, s), 2.24 (3H, s) |
| 6-112 | (300 MHz/CDCl₃) 12.17 (1H, brs), 9.94 (1H, s), 7.48 (2H, s), 6.86 (1H, t), 3.45 (1H, sep), 2.15 (6H, s), 1.30 (6H, d) |
| 6-113 | (300 MHz/CDCl₃) 12.53 (1H, brs), 9.96 (1H, s), 7.47 (2H, s), 6.86 (1H, t), 2.15 (6H, s), 1.44 (9H, s) |
| 6-114 | (300 MHz/CDCl₃) 12.18 (1H, brs), 9.94 (1H, s), 7.46 (2H, s), 6.86 (1H, t), 2.34-2.49 (1H, m), 2.12 (6H, s), 1.01-1.12 (2H, m), 0.91-1.01 (2H, m) |
| 6-115 | (300 MHz/CDCl₃) 12.08 (1H, brs), 9.63 (1H, d), 7.46 (2H, s), 2.14 (6H, s), 1.42 (9H, s) |
| 6-116 | (300 MHz/CDCl₃) 11.98 (1H, brs), 9.76 (1H, s), 7.58-7.67 (2H, m), 7.38-7.47 (1H, m), 3.41 (1H, sep), 2.23 (3H, s), 1.29 (6H, d) |
| 6-117 | (500 MHz/CDCl₃) 11.94 (1H, brs), 9.72 (1H, s), 8.22 (1H, s), 7.75-7.81 (1H, m), 7.49-7.52 (1H, m), 2.81-2.92 (2H, m), 1.30 (3H, t) |
| 6-118 | (300 MHz/CDCl₃) 12.25 (1H, s), 9.59 (1H, s), 7.48 (2H, s), 4.86 (2H, q), 3.43 (1H, sep), 2.17 (6H, s), 1.30 (6H, d) |
| 6-119 | (300 MHz/CDCl₃) 12.06 (1H, s), 9.77 (1H, s), 7.49 (2H, s), 3.45 (1H, sep),2.17 (6H, s), 1.31 (6H, d) |
| 6-120 | (300 MHz/CDCl₃) 13.04 (1H, brs), 9.74 (1H, s), 7.48 (2H, s), 3.44 (1H, sep), 2.58 (3H, s), 2.17 (6H, s), 1.31 (6H, d) |
| 6-121 | (300 MHz/CDCl₃) 12.10 (1H, s), 9.72 (1H, s), 7.77 (2H, s), 2.46-2.38 (1H, m), 1.10-1.01 (4H, m) |
| 6-122 | (300 MHz/CDCl₃) 12.21 (1H, brs), 9.69 (1H, s), 7.57 (2H, s), 2.89 (2H, q), 2.59 (3H, s), 1.26 (3H, t) |
| 6-123 | (300 MHz/CDCl₃) 12.05 (1H, brs), 9.51 (1H, s), 7.59 (2H, s), 2.41 (3H, s), 2.29-2.20 (1H, m), 0.92-0.82 (4H, m) |
| 6-124 | (300 MHz/CDCl₃) 12.01 (1H, s), 9.71 (1H, s), 7.42 (2H, d), 2.88 (2H, q), 1.31 (3H, t) |
| 6-125 | (300 MHz/CDCl₃) 12.09 (1H, s), 9.74 (1H, s), 7.99 (2H, s), 3.50-3.40 (1H, m), 1.32 (6H, d) |
| 6-126 | (300 MHz/CDCl₃) 12.01 (1H, brs), 9.64 (1H, s), 7.87 (2H, s), 2.39-2.29 (1H, m), 1.01-0.98 (4H, m) |
| 6-128 | (300 MHz/CDCl₃) 11.96 (1H, brs), 9.66 (1H, s), 7.36 (2H, s), 2.37-2.28 (1H, m), 2.03 (6H, s), 0.99-0.85 (4H, m) |
| 6-129 | (300 MHz/CDCl₃) 12.42 (1H, s), 9.78 (1H, s), 7.48 (2H, s), 2.16 (6H, s), 1.45 (9H, s) |
| 6-131 | (300 MHz/CDCl₃) 13.03 (1H, brs), 9.73 (1H, s), 7.46 (2H, s), 2.58 (3H, s), 2.43-2.38 (1H, m), 2.13 (6H, s), 1.07-0.97 (4H, m) |
| 6-132 | (300 MHz/CDCl₃) 13.41 (1H, brs), 9.77 (1H, s), 7.47 (2H, s), 2.59 (3H, s), 2.17 (6H, s), 1.45 (9H, s) |
| 6-133 | (300 MHz/CDCl₃) 12.21 (1H, s), 10.01 (1H, s), 7.47 (2H, s), 2.15 (6H, s), 1.44 (9H, s) |
| 6-134 | (500 MHz/CDCl₃) 13.10 (1H, brs), 9.71 (1H, s), 7.45 (2H, s), 3.41 (1H, sep), 2.90 (2H, q), 2.13 (6H, s), 1.39 (3H, t), 1.27 (6H, d) |
| 6-135 | (500 MHz/CDCl₃) 13.16 (1H, s), 9.72 (1H, s), 7.45 (2H, s), 3.41 (1H, sep), 3.21 (1H, sep), 2.14 (6H, s), 1.41 (6H, d), 1.28 (6H, d) |
| 6-136 | (300 MHz/CDCl₃) 11.94 (1H, brs), 9.73 (1H, s), 7.51 (2H, s), 2.86 (2H, q), 2.13 (6H, s), 1.27 (3H, t) |
| 6-139 | (300 MHz/CDCl₃) 11.95 (1H, brs), 9.75 (1H, s), 7.62 (2H, d), 2.87 (2H, q), 2.20 (6H, s), 1.27 (3H, t) |

**Table 9 (Continued)**

| No. | ¹H-NMR (300 MHz or 500 MHz/solvent) δ ppm |
|---|---|
| 8-1 | (300 MHz/CDCl₃) 12.55 (1H, brs), 9.41 (1H, d), 7.91 (1H, d), 7.47 (2H, d), 2.90 (2H, q), 2.17 (6H, s), 1.30 (3H, t) |
| 8-2 | (300 MHz/CDCl₃) 12.10 (1H, brs), 9.43 (1H, s), 7.49 (2H, s), 2.92 (2H, q), 2.18 (6H, s), 1.32 (3H, t) |
| 8-4 | (300 MHz/CDCl₃) 12.64 (1H, brs), 9.13 (1H, s), 7.49 (2H, s), 2.91 (2H, q), 2.48 (3H, s), 2.18 (6H, s), 1.31 (3H, t) |
| 8-6 | (300 MHz/CDCl₃) 10.59 (1H, brs), 7.45 (2H, s), 2.86 (2H, q), 2.19 (6H, s), 1.29 (3H, t) |
| 8-7 | (300 MHz/CDCl₃) 10.57 (1H, brs), 7.45 (2H, s), 3.49-3.34 (1H, m), 2.18 (6H, s), 1.30 (6H, d) |
| 8-8 | (300 MHz/CDCl₃) 10.61 (1H, brs), 7.45 (2H, s), 2.81 (2H, t), 2.19 (6H, t), 1.83-1.70 (2H, m), 1.01 (3H, t) |
| 8-10 | (300 MHz/CDCl₃) 10.63 (1H, brs), 7.43 (2H, s), 2.42-2.32 (1H, m), 2.14 (6H, s), 1.07-0.95 (4H, m) |
| 8-11 | (300 MHz/CDCl₃) 10.69 (1H, brs), 7.44 (2H, s), 2.17 (6H, s), 1.43 (9H, s) |
| 8-12 | (300 MHz/CDCl₃) 12.17 (1H, brs), 9.44 (1H, s), 7.49 (2H, s), 3.55-3.45 (1H, m), 2.17 (6H, s), 1.32 (6H, d) |

### Test Example 1

### Test on effect against brown planthopper (Nilaparvata lugens)

Rice seedlings were dipped in a chemical solution of a test compound at a concentration of 200 ppm or 50 ppm. After the chemical solution dried in air, the root was wrapped with wet adsorbent cotton, and the seedlings were put into a test tube. About 10 second to third instar nymphs of brown planthopper were released, the test tube was covered with a gauze, and the test tube was left in a constant temperature chamber with lighting at 25°C. On the 5th day after release, the number of dead nymphs was counted, and the mortality (%) was calculated by the following formula.$Mortality \left(%\right) = \left[\left(number of dead nymphs\right)/\left(number of released nymphs\right)\right] \times 100$

As a result, the following compounds showed a mortality of 90% or higher at a concentration of 200 ppm.

Compound Nos. 1-1, 1-2, 1-3, 1-4, 1-5, 1-6, 1-7, 1-8, 1-10, 1-11, 1-12, 1-13, 1-25, 1-42, 1-43, 1-45, 1-46, 1-47, 1-48, 1-49, 1-51, 1-52, 1-57, 1-58, 1-60, 1-61, 1-63, 1-64, 1-65, 1-66, 1-68, 1-69, 1-70, 1-71, 1-73, 1-74, 1-76, 1-77, 1-78, 1-80, 1-87, 1-100, 1-101, 1-103, 1-104, 1-105, 1-106, 1-107, 1-109, 1-110, 1-111, 1-113, 1-115, 1-116, 1-118, 1-128, 1-129, 1-130, 1-131, 1-132, 1-133, 1-135, 1-136, 1-138, 1-139, 1-140, 1-144, 1-146, 1-147, 1-148, 1-149, 1-150, 1-151, 1-152, 1-155, 1-156, 1-157, 1-158, 1-160, 1-161, 1-162, 1-163, 1-164, 1-165, 1-166, 1-167, 1-168, 1-170, 1-171, 1-175, 1-176, 1-177, 1-178, 1-179, 1-180, 1-181, 1-182, 1-183, 1-184, 1-186, 1-187, 1-188, 1-189, 1-190, 1-191, 1-192, 1-195, 1-196, 1-197, 1-198, 1-199, 1-201, 1-204, 1-205, 1-206, 1-207, 1-208, 1-209, 1-210, 1-212, 1-213, 1-214, 1-215, 1-216, 1-217, 1-218, 1-219, 1-220, 1-221, 1-222, 1-223, 1-224, 1-225, 1-233, 1-235, 1-236, 1-237, 1-239, 1-240, 1-241, 1-242, 1-244, 1-245, 1-246, 1-247, 1-249, 1-250, 1-251, 1-252, 1-253, 1-256, 1-257, 1-258, 1-259, 1-260, 1-261, 1-262, 1-263, 1-264, 1-267, 1-270, 1-273, 1-274, 1-275, 1-276, 1-277, 1-278, 1-282, 1-283, 1-284, 1-285, 1-286, 1-287, 1-289, 1-291, 1-292, 1-293, 1-294, 1-295, 1-296, 1-297, 1-298, 1-299, 1-300, 1-301, 2-1, 2-3, 2-6, 2-8, 2-9, 2-10, 2-12, 2-13, 2-14, 2-15, 2-18, 2-19, 2-20, 2-23, 2-24, 2-25, 2-26, 2-27, 2-28, 2-29, 2-32, 2-36, 2-37, 2-38, 2-40, 2-41, 2-42, 2-43, 2-44, 2-45, 2-46, 2-47, 2-48, 2-49, 2-52, 2-54, 2-55, 2-56, 2-58, 2-59, 2-60, 2-62, 2-63, 2-64, 2-66, 2-67, 2-69, 2-70, 2-71, 2-74, 2-76, 2-77, 2-78, 2-79, 2-80, 2-81, 2-83, 2-85, 2-86, 2-87, 2-88, 2-89, 2-91, 2-92, 2-93, 2-94, 2-96, 2-97, 2-98, 2-99, 2-100, 2-101, 2-102, 2-103, 2-104, 2-105, 2-106, 2-107, 2-109, 2-111, 2-112, 2-114, 2-115, 2-116, 2-117, 2-118, 2-119, 2-120, 2-121, 2-122, 2-123, 2-126, 2-127, 2-128, 2-129, 2-130, 2-131, 2-133, 2-136, 2-137, 2-139, 2-140, 2-143, 2-144, 2-147, 2-149, 2-151, 2-153, 2-154, 2-167, 2-168, 2-169, 2-171, 2-172, 2-173, 2-175, 2-177, 2-178, 2-179, 2-180, 2-181, 2-182, 2-183, 2-184, 2-185, 2-186, 2-190, 2-191, 2-192, 2-195, 2-197, 2-198, 2-206, 2-207, 2-209, 2-215, 2-218, 2-222, 2-224, 2-234, 2-235, 2-236, 2-237, 2-238, 2-239, 2-240, 2-241, 2-243, 2-262, 2-263, 2-266, 2-270, 2-271, 2-274, 2-277, 2-278, 2-279, 2-283, 2-284, 2-285, 2-286, 2-287, 2-288, 2-291, 2-292, 2-293, 2-294, 2-295, 2-296, 2-297, 2-298, 2-299, 2-300, 2-301, 2-302, 2-303, 2-304, 2-309, 2-310, 2-311, 2-313, 2-314, 2-317, 2-318, 2-319, 2-323, 2-324, 2-325, 2-326, 2-328, 2-329, 2-335, 2-336, 2-338, 2-340, 2-342, 2-345, 2-347, 2-348, 2-349, 2-352, 2-353, 2-356, 2-357, 2-359, 2-360, 2-362, 2-364, 2-365, 2-366, 2-369, 2-373, 2-377, 2-378, 2-379, 2-380, 2-381, 2-382, 2-383, 2-384, 2-385, 2-386, 2-387, 2-388, 2-389, 2-390, 2-393, 2-394, 2-395, 2-396, 2-398, 2-399, 2-401, 2-403, 2-404, 2-406, 2-407, 2-408, 2-409, 2-413, 2-414, 2-415, 2-422, 2-423, 2-424, 2-426, 2-428, 2-429, 2-430, 2-431, 2-432, 2-433, 2-434, 2-435, 2-436, 2-437, 2-438, 2-439, 2-441, 2-444, 2-446, 2-447, 2-448, 2-449, 2-450, 2-451, 2-452, 2-453, 2-454, 2-455, 2-456, 2-457, 2-458, 2-459, 2-460, 2-461, 2-462, 2-463, 2-464, 2-465, 2-466, 2-467, 2-468, 2-469, 2-470, 2-471, 2-472, 2-473, 2-474, 2-475, 2-476, 2-477, 2-478, 2-479, 2-480, 2-481, 2-482, 2-483, 2-484, 2-485, 2-487, 2-488, 2-489, 2-490, 2-493, 2-494, 2-495, 2-496, 2-497, 2-498, 2-500, 2-501, 2-502, 2-503, 2-504, 2-505, 2-506, 2-507, 2-508, 2-509, 2-510, 2-511, 2-512, 2-513, 2-514, 2-515, 2-516, 2-517, 2-519, 2-520, 2-521, 2-522, 2-523, 2-524, 2-525, 2-526, 2-527, 2-529, 2-530, 2-531, 2-532, 2-533, 2-534, 2-536, 2-537, 2-539, 2-540, 2-541, 2-543, 2-544, 2-545, 2-546, 2-547, 2-548, 2-549, 2-550, 2-552, 2-554, 2-555, 4-1, 4-3, 4-5, 5-1, 5-2, 5-4, 5-21, 5-22, 5-23, 5-24, 5-25, 5-26, 5-27, 5-28, 5-50, 5-51, 6-1, 6-2, 6-5, 6-7, 6-10, 6-11, 6-12, 6-13, 6-14, 6-18, 6-19, 6-21, 6-22, 6-23, 6-31, 6-37, 6-38, 6-41, 6-45, 6-47, 6-48, 6-49, 6-51, 6-52, 6-64, 6-65, 6-66, 6-67, 6-71, 6-73, 6-74, 6-75, 6-76, 6-78, 6-79, 6-80, 6-81, 6-82, 6-83, 6-84, 6-85, 6-86, 6-89, 6-91, 6-95, 6-102, 6-103, 6-104, 6-105, 6-106, 6-107, 6-108, 6-109, 6-110, 6-111, 6-112, 6-113, 6-114, 6-115, 6-116, 6-117, 6-119, 6-120, 6-121, 6-123, 6-124, 6-125, 6-128, 6-129, 6-131, 6-132, 6-133, 6-134, 6-135, 6-136, 6-139, 8-1, 8-2, 8-4, 8-6, 8-8, 8-10, 8-11 and 8-12.

Further, Compound Nos. 1-226, 1-227, 1-231, 1-232, 2-39, 2-189, 2-281, 2-412, 2-440, 2-499, 2-535, 2-538, 6-40, 6-98, 6-122, 6-126 and 8-7 showed a mortality of 90% or higher at a concentration of 50 ppm.

### Test Example 2

### Test on effect against green peach aphid (Myzus persicae)

A Japanese radish leaf was inserted in a test tube in which water was put, and 5 adults of green peach aphid were released on the leaf. On the next day, the adults were removed, and the number of nymphs parasitic on the leaf was counted as the number of tested nymphs, and then the leaf was dipped in a chemical solution of a test compound at a concentration of 200 ppm or 50 ppm. After the chemical solution dried in air, the leaf was left in a constant temperature chamber with lighting at 25°C. On the 5th day after the application, the number of dead nymphs was counted, and the mortality (%) was calculated by the following formula. Nymphs that dropped from the leaf or nymphs that were moribund were counted as dead nymphs. $Mortality \left(%\right) = \left[\left(Number of dead nymphs\right)/\left(number of tested nymphs\right)\right] \times 100$

As a result, the following compounds showed a mortality of 90% or higher at a concentration of 200 ppm.

Compound Nos. 1-1, 1-2, 1-3, 1-4, 1-5, 1-7, 1-8, 1-10, 1-11, 1-13, 1-45, 1-47, 1-49, 1-51, 1-52, 1-53, 1-57, 1-58, 1-60, 1-61, 1-63, 1-64, 1-65, 1-66, 1-67, 1-68, 1-69, 1-70, 1-71, 1-73, 1-74, 1-76, 1-77, 1-100, 1-101, 1-103, 1-105, 1-106, 1-107, 1-109, 1-110, 1-111, 1-113, 1-116, 1-128, 1-129, 1-132, 1-133, 1-135, 1-136, 1-139, 1-140, 1-144, 1-146, 1-148, 1-149, 1-151, 1-152, 1-156, 1-157, 1-158, 1-160, 1-161, 1-162, 1-163, 1-164, 1-165, 1-166, 1-167, 1-168, 1-170, 1-175, 1-176, 1-178, 1-180, 1-183, 1-184, 1-191, 1-192, 1-195, 1-196, 1-197, 1-198, 1-201, 1-205, 1-206, 1-207, 1-208, 1-209, 1-210, 1-212, 1-213, 1-214, 1-215, 1-217, 1-218, 1-219, 1-220, 1-221, 1-222, 1-223, 1-225, 1-226, 1-231, 1-233, 1-236, 1-237, 1-239, 1-240, 1-241, 1-242, 1-243, 1-245, 1-250, 1-251, 1-252, 1-253, 1-256, 1-257, 1-258, 1-260, 1-261, 1-262, 1-267, 1-273, 1-274, 1-275, 1-276, 1-277, 1-278, 1-282, 1-283, 1-284, 1-285, 1-286, 1-289, 1-292, 1-293, 1-294, 1-296, 1-297, 1-298, 1-299, 1-300, 1-301, 2-1, 2-2, 2-6, 2-12, 2-13, 2-14, 2-15, 2-18, 2-19, 2-20, 2-23, 2-24, 2-25, 2-26, 2-28, 2-29, 2-32, 2-36, 2-37, 2-41, 2-42, 2-43, 2-44, 2-45, 2-46, 2-50, 2-54, 2-57, 2-58, 2-65, 2-66, 2-67, 2-75, 2-81, 2-82, 2-83, 2-85, 2-87, 2-88, 2-89, 2-91, 2-92, 2-93, 2-94, 2-96, 2-97, 2-98, 2-99, 2-100, 2-101, 2-102, 2-103, 2-104, 2-105, 2-106, 2-107, 2-111, 2-112, 2-115, 2-116, 2-119, 2-120, 2-121, 2-122, 2-123, 2-126, 2-127, 2-128, 2-129, 2-130, 2-131, 2-133, 2-140, 2-143, 2-144, 2-147, 2-149, 2-151, 2-153, 2-167, 2-168, 2-169, 2-171, 2-172, 2-173, 2-175, 2-177, 2-178, 2-179, 2-180, 2-181, 2-182, 2-183, 2-184, 2-185, 2-186, 2-190, 2-191, 2-192, 2-195, 2-197, 2-198, 2-206, 2-208, 2-209, 2-211, 2-215, 2-218, 2-222, 2-224, 2-234, 2-235, 2-236, 2-237, 2-238, 2-239, 2-240, 2-241, 2-243, 2-262, 2-263, 2-266, 2-270, 2-271, 2-274, 2-277, 2-278, 2-279, 2-283, 2-284, 2-285, 2-286, 2-287, 2-288, 2-291, 2-292, 2-293, 2-295, 2-296, 2-297, 2-299, 2-300, 2-301, 2-302, 2-303, 2-304, 2-309, 2-310, 2-311, 2-313, 2-314, 2-317, 2-318, 2-319, 2-323, 2-324, 2-325, 2-326, 2-328, 2-329, 2-336, 2-338, 2-342, 2-345, 2-347, 2-348, 2-353, 2-356, 2-359, 2-360, 2-363, 2-364, 2-365, 2-366, 2-367, 2-368, 2-369, 2-370, 2-373, 2-382, 2-383, 2-384, 2-385, 2-387, 2-388, 2-390, 2-393, 2-394, 2-395, 2-396, 2-398, 2-399, 2-401, 2-403, 2-407, 2-409, 2-413, 2-414, 2-415, 2-422, 2-423, 2-424, 2-426, 2-428, 2-429, 2-430, 2-431, 2-432, 2-433, 2-434, 2-435, 2-436, 2-437, 2-438, 2-439, 2-441, 2-444, 2-446, 2-447, 2-448, 2-449, 2-450, 2-452, 2-453, 2-454, 2-456, 2-457, 2-458, 2-459, 2-460, 2-461, 2-462, 2-463, 2-464, 2-465, 2-466, 2-467, 2-468, 2-469, 2-470, 2-471, 2-472, 2-474, 2-475, 2-476, 2-477, 2-478, 2-479, 2-480, 2-482, 2-483, 2-484, 2-485, 2-490, 2-493, 2-495, 2-496, 2-497, 2-498, 2-500, 2-501, 2-502, 2-503, 2-504, 2-505, 2-506, 2-507, 2-508, 2-509, 2-510, 2-511, 2-512, 2-513, 2-515, 2-516, 2-517, 2-519, 2-520, 2-521, 2-522, 2-523, 2-524, 2-525, 2-526, 2-527, 2-530, 2-531, 2-532, 2-533, 2-534, 2-536, 2-537, 2-539, 2-540, 2-541, 2-543, 2-544, 2-545, 2-546, 2-547, 2-548, 2-549, 2-550, 2-552, 2-554, 2-555, 4-1, 4-3, 4-5, 5-1, 5-2, 5-4, 5-21, 5-22, 5-23, 5-24, 5-25, 5-26, 5-27, 5-28, 5-50, 5-51, 6-1, 6-2, 6-5, 6-7, 6-11, 6-12, 6-13, 6-14, 6-18, 6-19, 6-21, 6-22, 6-23, 6-30, 6-31, 6-33, 6-38, 6-41, 6-45, 6-47, 6-48, 6-49, 6-51, 6-52, 6-64, 6-65, 6-66, 6-67, 6-73, 6-74, 6-75, 6-76, 6-78, 6-79, 6-80, 6-81, 6-82, 6-83, 6-84, 6-85, 6-89, 6-94, 6-95, 6-102, 6-103, 6-104, 6-105, 6-106, 6-107, 6-108, 6-109, 6-111, 6-112, 6-113, 6-114, 6-115, 6-116, 6-117, 6-118, 6-119, 6-120, 6-121, 6-123, 6-124, 6-125, 6-128, 6-129, 6-131, 6-132, 6-133, 6-134, 6-135, 6-136, 6-139, 8-1, 8-2, 8-4, 8-6, 8-8, 8-10, 8-11 and 8-12.

Further, Compound Nos. 2-39, 2-189, 2-281, 2-412, 2-440, 2-499, 2-535, 2-538, 6-10, 6-40, 6-98, 6-122 and 6-126 showed a mortality of 90% or higher at a concentration of 50 ppm.

### Test Example 3

### Test on effects against two-spotted spider mite (Tetranychus urticae)

In a chemical solution of a test compound at a concentration of 800 ppm, 200 ppm or 50 ppm, a leaf of kidney bean on which about 20 adults of two-spotted spider mite had been released, was dipped. After the chemical solution dried in air, the leaf was left in a constant temperature chamber with lighting at 25°C. On the 3rd day after the application, the number of dead adults was counted, and the mortality (%) was calculated by the following formula. Adults that dropped from the leaf or adults that were moribund were counted as dead adults. $Mortality \left(%\right) = \left[\left(number of dead adults\right)/\left(number of released adults\right)\right] \times 100$

As a result, the following compounds showed a mortality of 80% or higher at a concentration of 800 ppm.

Compound Nos. No. 1-1, 1-2, 1-3, 1-4, 1-5, 1-6, 1-7, 1-8, 1-12, 1-27, 1-52, 1-53, 1-54, 1-60, 1-63, 1-65, 1-69, 1-77, 1-96, 1-100, 1-101, 1-104, 1-105, 1-106, 1-107, 1-109, 1-110, 1-111, 1-113, 1-115, 1-128, 1-129, 1-131, 1-132, 1-133, 1-136, 1-138, 1-140, 1-147, 1-148, 1-149, 1-151, 1-155, 1-156, 1-164, 1-166, 1-167, 1-170, 1-171, 1-175, 1-178, 1-186, 1-189, 1-190, 1-191, 1-192, 1-198, 1-205, 1-206, 1-207, 1-208, 1-209, 1-212, 1-213, 1-214, 1-215, 1-216, 1-217, 1-220, 1-221, 1-222, 1-223, 1-224, 1-225, 1-226, 1-232, 1-236, 1-237, 1-239, 1-240, 1-241, 1-242, 1-251, 1-252, 1-253, 1-257, 1-258, 1-259, 1-260, 1-262, 1-264, 1-270, 1-273, 1-274, 1-275, 1-276, 1-277, 1-278, 1-283, 1-284, 1-285, 1-286, 1-289, 2-3, 2-4, 2-8, 2-9, 2-11, 2-12, 2-13, 2-14, 2-15, 2-18, 2-19, 2-23, 2-24, 2-25, 2-26, 2-27, 2-29, 2-32, 2-36, 2-37, 2-38, 2-40, 2-41, 2-46, 2-48, 2-49, 2-50, 2-51, 2-52, 2-54, 2-55, 2-56, 2-57, 2-59, 2-60, 2-63, 2-67, 2-68, 2-69, 2-70, 2-72, 2-74, 2-75, 2-76, 2-78, 2-79, 2-80, 2-81, 2-82, 2-83, 2-84, 2-85, 2-86, 2-87, 2-88, 2-89, 2-90, 2-91, 2-92, 2-93, 2-94, 2-95, 2-96, 2-97, 2-98, 2-99, 2-100, 2-101, 2-102, 2-103, 2-104, 2-105, 2-107, 2-109, 2-110, 2-111, 2-112, 2-113, 2-114, 2-115, 2-116, 2-117, 2-119, 2-120, 2-121, 2-122, 2-123, 2-126, 2-127, 2-137, 2-139, 2-167, 2-168, 2-234, 2-263, 2-266, 2-270, 2-274, 2-279, 2-283, 2-284, 2-292, 2-293, 2-294, 2-295, 2-296, 2-335, 2-336, 2-338, 2-340, 2-342, 2-345, 2-347, 2-348, 2-349, 2-352, 2-353, 2-356, 2-357, 2-358, 2-359, 2-360, 2-361, 2-362, 2-363, 2-364, 2-365, 2-366, 2-367, 2-368, 2-370, 2-371, 2-372, 2-377, 2-378, 2-379, 2-380, 2-383, 2-384, 2-386, 2-389, 2-424, 2-426, 2-428, 2-429, 2-432, 2-453, 2-455, 2-456, 2-457, 2-462, 2-463, 2-464 and 2-469.

The following Compounds showed a mortality of 80% or higher at a concentration of 200 ppm.

Compound Nos. 1-219, 1-292, 1-293, 1-297, 2-6, 2-20, 2-42, 2-71, 2-73, 2-106, 2-128, 2-130, 2-131, 2-133, 2-136, 2-140, 2-143, 2-144, 2-149, 2-151, 2-169, 2-171, 2-172, 2-173, 2-175, 2-177, 2-178, 2-179, 2-180, 2-181, 2-182, 2-183, 2-184, 2-185, 2-186, 2-192, 2-195, 2-197, 2-198, 2-218, 2-222, 2-224, 2-235, 2-236, 2-237, 2-238, 2-239, 2-241, 2-243, 2-262, 2-271, 2-277, 2-278, 2-285, 2-286, 2-287, 2-288, 2-291, 2-297, 2-298, 2-299, 2-300, 2-301, 2-302, 2-303, 2-304, 2-309, 2-310, 2-311, 2-313, 2-314, 2-317, 2-318, 2-319, 2-323, 2-324, 2-325, 2-326, 2-328, 2-329, 2-369, 2-373, 2-387, 2-388, 2-390, 2-393, 2-394, 2-395, 2-396, 2-398, 2-399, 2-403, 2-404, 2-409, 2-413, 2-414, 2-415, 2-430, 2-431, 2-433, 2-434, 2-435, 2-436, 2-437, 2-438, 2-439, 2-441, 2-446, 2-448, 2-449, 2-450, 2-451, 2-452, 2-454, 2-458, 2-459, 2-460, 2-461, 2-465, 2-466, 2-467, 2-468, 2-470, 2-472, 2-473, 2-474, 2-475, 2-476, 2-477, 2-478, 2-480, 2-481, 2-482, 2-483, 2-484, 2-485, 2-493, 2-496, 2-497, 2-498, 2-502, 2-503, 2-504, 2-505, 2-506, 2-507, 2-509, 2-512, 2-513, 2-516, 2-521, 2-525, 2-526, 2-533, 2-534, 2-536, 2-537, 2-540, 2-541, 2-543, 2-544, 2-545, 2-546, 2-547, 2-548, 2-549, 2-554, 5-24, 6-5, 6-7, 6-10, 6-12, 6-13, 6-14, 6-18, 6-21, 6-22, 6-23, 6-41, 6-45, 6-47, 6-49, 6-51, 6-64, 6-67, 6-74, 6-75, 6-80, 6-83, 6-84, 6-89, 6-102, 6-104, 6-105, 6-106, 6-107, 6-108, 6-109, 6-111, 6-113, 6-116, 6-119, 6-120, 6-125, 6-129 and 6-134.

Further, Compound Nos. No.2-281 and 2-447 showed a mortality of 80% or higher at a concentration of 50 ppm.

Now, comparison between effects of the compound of the present invention and effects of comparative compounds not having the predetermined diarylpyridazinone structure of the present invention, are shown in the following Comparative Tests.

### Comparative Test Example 1

Rice seedlings were dipped in a chemical solution of a test compound at a concentration of 200 ppm or 50 ppm. After the chemical solution dried in air, the root was wrapped with wet adsorbent cotton, and the seedlings were put into a test tube. About 10 second to third instar nymphs of brown planthopper (Nilaparvata lugens)were released, the test tube was covered with a gauze, and the test tube was left in a constant temperature chamber with lighting at 25°C. On the 5th day after release, the number of dead nymphs was counted, and the mortality (%) was calculated by the following formula. $Mortality \left(%\right) = \left[\left(number of dead nymphs\right)/\left(number of released nymphs\right)\right] \times 100$

The test compounds are Compound No. 2-12 as the compound of the present invention and the following Comparative Compounds (A) and (B) as the comparative compounds.

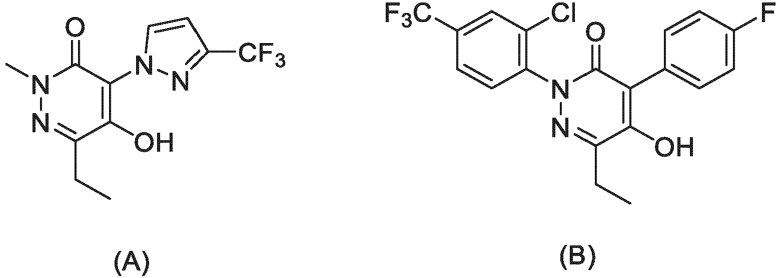

Test results are shown in the following Table 10.

**Table 10**

| Test compounds | Chemical structure | Mortality (%) | |
|---|---|---|---|
| | | Active ingredient concentration | |
| | | 200 ppm | 50 ppm |
| Compound No. 2-12 | 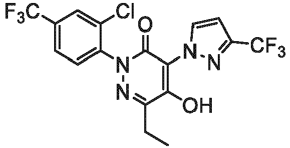 | 100 | 100 |
| Comparative compound (A) | 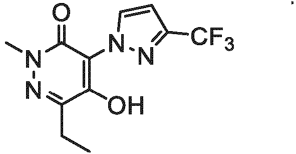 | 0 | 0 |
| Comparative compound (B) | 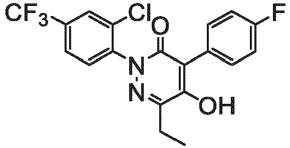 | 0 | 0 |

### Comparative Test Example 2

A Japanese radish leaf was inserted in a test tube in which water was put, and 5 adults of green peach aphid (Myzus persicae) were released on the leaf. On the next day, the adults were removed, and the number of nymphs parasitic on the leaf was counted as the number of tested nymphs, and then the leaf was dipped in a chemical solution of a test compound at a concentration of 200 ppm or 50 ppm. After the chemical solution dried in air, the leaf was left in a constant temperature chamber with lighting at 25°C. On the 5th day after the application, the number of dead nymphs was counted, and the mortality (%) was calculated by the following formula. Nymphs that dropped from the leaf or nymphs that were moribund were counted as dead nymphs.$Mortality \left(%\right) = \left[\left(Number of dead larvae\right)/\left(number of tested nymphs\right)\right] \times 100$

The test compounds were the same as in Comparative Test Example 1.

The results are shown in the following Table 11.

**Table 11**

| Test compounds | Chemical structure | Mortality (%) | |
|---|---|---|---|
| | | Active ingredient concentration | |
| | | 200 ppm | 50 ppm |
| Compound No. 2-12 | 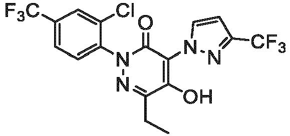 | 100 | 100 |
| Comparative compound (A) | 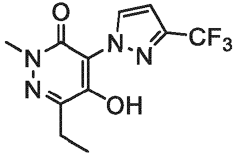 | 20 | 0 |
| Comparative compound (B) | 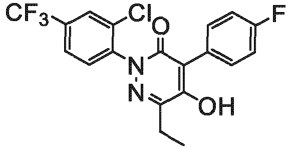 | 30 | 0 |

Formulation Examples will be described.

### Formulation Example 1

| | | |
|---|---|---|
| (1) | Compound of the present invention | 20 parts by weight |
| (2) | Clay | 70 parts by weight |
| (3) | White carbon | 5 parts by weight |
| (4) | Sodium polycarboxylate | 3 parts by weight |
| (5) | Sodium alkylnaphthalenesulfonate | 2 parts by weight |

The above ingredients are uniformly mixed to obtain a wettable powder.

### Formulation Example 2

| | | |
|---|---|---|
| (1) | Compound of the present invention | 5 parts by weight |
| (2) | Talc | 60 parts by weight |
| (3) | Calcium carbonate | 34.5 parts by weight |
| (4) | Liquid paraffin | 0.5 part by weight |

The above ingredients are uniformly mixed to obtain a dust.

### Formulation Example 3

| | | |
|---|---|---|
| (1) | Compound of the present invention | 20 parts by weight |
| (2) | N,N-dimethylacetamide | 20 parts by weight |
| (3) | Polyoxyethylene tristyrylphenyl ether | 10 parts by weight |
| (4) | Calcium dodecylbenzenesulfonate | 2 parts by weight |
| (5) | Xylene | 48 parts by weight |

The above ingredients are uniformly mixed and dissolved to obtain an emulsifiable concentrate.

### Formulation Example 4

| | | |
|---|---|---|
| (1) | Clay | 68 parts by weight |
| (2) | Sodium ligninsulfonate | 2 parts by weight |
| (3) | Polyoxyethylene alkylaryl sulfate | 5 parts by weight |
| (4) | White carbon | 25 parts by weight |

A mixture of the above ingredients is mixed with the compound of the present invention in a weight ratio of 4:1 to obtain a wettable powder.

### Formulation Example 5

| | | |
|---|---|---|
| (1) | Compound of the present invention | 50 parts by weight |
| (2) | Sodium alkylnaphthalenesulfonate condensed with formaldehyde | 2 parts by weight |
| (3) | Silicone oil | 0.2 part by weight |
| (4) | Water | 47.8 parts by weight |

The above ingredients are uniformly mixed and pulverized to obtain a liquid concentrate. Furthermore,

| | | |
|---|---|---|
| (5) | Sodium polycarboxylate | 5 parts by weight and |
| (6) | Anhydrous sodium sulfate | 42.8 parts by weight |

are added thereto and uniformly mixed. The mixture is granulated and dried to obtain a water dispersible granule.

### Formulation Example 6

| | | |
|---|---|---|
| (1) | Compound of the present invention | 5 parts by weight |
| (2) | Polyoxyethylene octylphenyl ether | 1 part by weight |
| (3) | Polyoxyethylene alkyl ether phosphate | 0.1 part by weight |
| (4) | Particulate calcium carbonate | 93.9 parts by weight |

(1) to (3) are uniformly mixed beforehand, and this mixture is diluted with an adequate amount of acetone. Thereafter, the diluted mixture is sprayed on (4) and the acetone is removed to obtain a granule.

### Formulation Example 7

| | | |
|---|---|---|
| (1) | Compound of the present invention | 2.5 parts by weight |
| (2) | N,N-dimethylacetamide | 2.5 parts by weight |
| (3) | Soybean oil | 95.0 parts by weight |

The above ingredients are uniformly mixed and dissolved to obtain an ultra low volume formulation.

### Formulation Example 8

| | | |
|---|---|---|
| (1) | Compound of the present invention | 10 parts by weight |
| (2) | Diethylene glycol monoethyl ether | 80 parts by weight |
| (3) | Polyoxyethylene alkyl ether | 10 parts by weight |

The above ingredients are uniformly mixed to obtain a liquid formulation.

The entire disclosure of Japanese Patent Application No. 2022-056258 filed on March 30, 2022 including specification, claims, drawings and abstract is incorporated herein by reference in its entirety.

## Claims

1. A pyridazinone compound represented by the formula (I) or a salt thereof: formula (I): 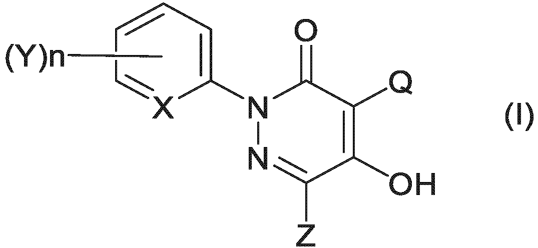 wherein Q is Q¹, Q², Q³ or Q⁴: 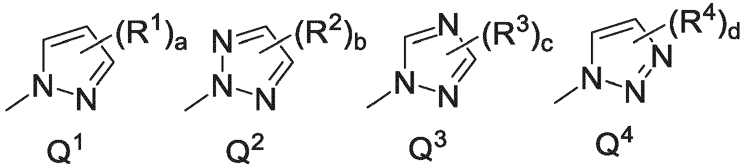
X is CH, C(R^{A}) or N;
Y and R^{A} are each a halogen, an alkyl, an alkenyl, an alkynyl, a cycloalkyl, a haloalkyl, an alkoxy, a haloalkoxy, an alkylthio, an alkylsulfinyl, an alkylsulfonyl, a haloalkylthio, a haloalkylsulfinyl, a haloalkylsulfonyl, hydroxy, cyano, nitro, -SFs, - NR^{B}R^{C}, -C(=O)R^{D} or -C(=O)NR^{E}R^{F};
Z is an alkyl, an alkenyl, an alkynyl, a cycloalkyl, an alkoxyalkyl, a cycloalkylalkyl or a haloalkyl;
R¹, R², R³ and R⁴ are each a halogen, an alkyl, an alkenyl, an alkynyl, a cycloalkyl, a haloalkyl, an alkoxy, a cycloalkoxy, a haloalkoxy, an alkylthio, an alkylsulfinyl, an alkylsulfonyl, a haloalkylthio, a haloalkylsulfinyl, a haloalkylsulfonyl, hydroxy, cyano, nitro, -NR^{G}R^{H}, -C(=O)R^{I}, -C(=O)NR^{J}R^{K}, -C(=NOR^{L})R^{M}, dimethoxymethyl, 1,3-dioxan-2-yl or 1,3-dioxolan-2-yl;
R^{B}, R^{C}, R^{E}, R^{F}, R^{G}, R^{H}, R^{J} and R^{K} are each a hydrogen atom, an alkyl or a haloalkyl;
R^{D} and R^{I} are each a hydrogen atom, hydroxy, an alkyl, a haloalkyl or an alkoxy; R^{L} and R^{M} are each a hydrogen atom or an alkyl;
n is an integer of from 0 to 4;
a is an integer of from 0 to 3; and
b, c and d are each 0, 1 or 2.

2. The pyridazinone compound or a salt thereof according to Claim 1, wherein
Y and R^{A} are each a halogen, a (C₁-C₄)alkyl, a (C₂-C₄)alkenyl, a (C₂-C₄)alkynyl, a (C₃-C₆)cycloalkyl, a (C₁-C₄)haloalkyl, a (C₁-C₄)alkoxy, a (C₁-C₄)haloalkoxy, a (C₁-C₄)alkylthio, a (C₁-C₄)alkylsulfinyl, a (C₁-C₄)alkylsulfonyl, a (C₁-C₄)haloalkylthio, a (C₁-C₄)haloalkylsulfinyl, a (C₁-C₄)haloalkylsulfonyl, hydroxy, cyano, nitro, -SFs, -NR^{B}R^{C}, - C(=O)R^{D} or a -C(=O)NR^{E}R^{F};
Z is a (C₁-C₅)alkyl, a (C₂-C₄)alkenyl, a (C₂-C₄)alkynyl, a (C₃-C₆)cycloalkyl, a (C₁-C₄)alkoxy(C₁-C₄)alkyl, a (C₃-C₆)cycloalkyl(C₁-C₄)alkyl or a (C₁-C₄)haloalkyl;
R¹, R², R³ and R⁴ are each a halogen, a (C₁-C₄)alkyl, a (C₂-C₄)alkenyl, a (C₂-C₄)alkynyl, a (C₃-C₆)cycloalkyl, a (C₁-C₄)haloalkyl, a (C₁-C₄)alkoxy, a (C₃-C₆)cycloalkoxy, a (C₁-C₄)haloalkoxy, a (C₁-C₄)alkylthio, a (C₁-C₄)alkylsulfinyl, a (C₁-C₄)alkylsulfonyl, a (C₁-C₄)haloalkylthio, a (C₁-C₄)haloalkylsulfinyl, a (C₁-C₄)haloalkylsulfonyl, hydroxy, cyano, nitro, -NR^{G}R^{H}, -C(=O)R^{I}, -C(=O)NR^{J}R^{k}, - C(=NOR^{L})R^{M}, dimethoxymethyl, 1,3-dioxan-2-yl or 1,3-dioxolan-2-yl;
R^{B}, R^{C}, R^{E}, R^{F}, R^{G}, R^{H}, R^{J} and R^{K} are each a hydrogen atom, a (C₁-C₄)alkyl or a (C₁-C₄)haloalkyl;
R^{D} and R^{I} are each a hydrogen atom, hydroxy, a (C₁-C₄)alkyl, a (C₁-C₄)haloalkyl or a (C₁-C₄)alkoxy; and
R^{L} and R^{M} are each a hydrogen atom or a (C₁-C₄)alkyl.

3. The pyridazinone compound or a salt thereof according to Claim 1, wherein
Y and R^{A} are each a halogen, a (C₁-C₄)alkyl, a (C₂-C₄)alkenyl, a (C₂-C₄)alkynyl, a (C₃-C₆)cycloalkyl, a (C₁-C₄)haloalkyl, a (C₁-C₄)alkoxy, a (C₁-C₄)haloalkoxy, a (C₁-C₄)alkylsulfonyl, a (C₁-C₄)haloalkylthio, a (C₁-C₄)haloalkylsulfinyl, a (C₁-C₄)haloalkylsulfonyl, cyano, nitro or -C(=O)R^{D};
Z is a (C₁-C₅)alkyl, a (C₃-C₆)cycloalkyl, a (C₁-C₄)alkoxy(C₁-C₄)alkyl or a (C₁-C₄)haloalkyl;
R¹, R², R³ and R⁴ are each a halogen, a (C₁-C₄)alkyl, a (C₂-C₄)alkynyl, a (C₃-C₆)cycloalkyl, a (C₁-C₄)haloalkyl, a (C₁-C₄)alkoxy, a (C₁-C₄)haloalkoxy, a (C₁-C₄)alkylthio, a (C₁-C₄)alkylsulfinyl, a (C₁-C₄)alkylsulfonyl, cyano, nitro, -NR^{G}R^{H}, - C(=O)R^{I}, -C(=NOR^{L})R^{M}, dimethoxymethyl, 1,3-dioxan-2-yl or 1,3-dioxolan-2-yl;
R^{G} and R^{H} are each a hydrogen atom or a (C₁-C₄)alkyl;
R^{D} and R^{I} are each a hydrogen atom, a (C₁-C₄)alkyl or a (C₁-C₄)alkoxy, and
R^{L} and R^{M} are each a hydrogen atom or a (C₁-C₄)alkyl.

4. The pyridazinone compound or a salt thereof according to Claim 1, wherein Q is Q¹ or Q³.

5. The pyridazinone compound or a salt thereof according to Claim 1, wherein X is CH or C(R^{A}).

6. The pyridazinone compound or a salt thereof according to Claim 1, wherein X is N.

7. The pyridazinone compound or a salt thereof according to Claim 1, wherein when X is CH, n is an integer of from 1 to 3, and when X is C(R^{A}) or N, n is 1 or 2.

8. The pyridazinone compound or a salt thereof according to Claim 1, wherein a, b, c and d are each 1 or 2.

9. A pest control agent comprising as an active ingredient the compound or a salt thereof as defined in any one of Claims 1 to 8.

10. An agricultural and horticultural insecticide, miticide, nematicide or soil pesticide, comprising as an active ingredient the compound or a salt thereof as defined in any one of Claims 1 to 8.

11. A method for controlling pests, which comprises applying an effective amount of the compound or a salt thereof as defined in any one of Claims 1 to 8.
